# EUROPEAN PATENT SPECIFICATION

(11) **EP 3 119 418 B1**
(45) Date of publication and mention of the grant of the patent: **23.02.2022**
(21) Application number: 15765863.4
(22) Date of filing: 20.03.2015
(51) Int. Cl.: A61K 38/17, A61K 39/395, A61P 7/06

(54) **COMPOSITION FOR USE IN A METHOD OF TREATING OR PREVENTING ANEMIA BY INHIBITING ACTIVIN B AND GDF11**
ZUSAMMENSETZUNG ZUR VERWENDUNG IN EINEM VERFAHREN ZUR BEHANDLUNG ODER VORBEUGUNG VON ANÄMIE DURCH DIE HEMMUNG VON ACTIVIN B UND GDF11
COMPOSITION POUR SON UTILISATION DANS UNE METHODE DE TRAITEMENT OU DE PREVENTION DE L'ANEMIE PAR L'INHIBITION DE L'ACTIVINE B ET DU GDF11

(30) Priority: 21.03.2014 US 201461969073 P; 08.07.2014 US 201462021923 P
(43) Date of publication of application: 25.01.2017
(73) Proprietor: Acceleron Pharma Inc., Cambridge, MA 02139 (US)
(72) Inventor: KUMAR, Ravindra, Acton, MA 01720 (US); SURAGANI, Naga Venkata, Sai Rajasekhar, Norwood, MA 02062 (US); KNOPF, John, Carlisle, MA 01741 (US)
(74) Representative: Varma, Anita
(86) International application number: PCT/US2015/021880
(87) International publication number: WO 2015/143403

(56) References cited:
- WO-A1-2010/019261
- WO-A1-2011/020045
- WO-A1-2013/059347
- WO-A2-2008/076437
- WO-A2-2012/027065
- WO-A2-2014/066487
- US-A1- 2011 038 831
- CARRANCIO, S. ET AL.: 'An activin receptor IIA ligand trap promotes erythropoiesis resulting in a rapid induction of red blood cells and haemoglobin' BRITISH JOURNAL OF HAEMATOLOGY vol. 165, 2014, pages 870 - 882, XP055227789

## Description

### BACKGROUND OF THE INVENTION

The mature red blood cell, or erythrocyte, is responsible for oxygen transport in the circulatory systems of vertebrates. Red blood cells contain high concentrations of hemoglobin, a protein that binds to oxygen in the lungs at relatively high partial pressure of oxygen (_{P}O₂) and delivers oxygen to areas of the body with a relatively low pO₂.

Mature red blood cells are produced from pluripotent hematopoietic stem cells in a process termed erythropoiesis. Postnatal erythropoiesis occurs primarily in the bone marrow and in the red pulp of the spleen. The coordinated action of various signaling pathways controls the balance of cell proliferation, differentiation, survival, and death. Under normal conditions, red blood cells are produced at a rate that maintains a constant red cell mass in the body, and production may increase or decrease in response to various stimuli, including increased or decreased oxygen tension or tissue demand. The process of erythropoiesis begins with the formation of lineage committed precursor cells and proceeds through a series of distinct precursor cell types. The final stages of erythropoiesis occur as reticulocytes are released into the bloodstream and lose their mitochondria and ribosomes while assuming the morphology of mature red blood cell. An elevated level of reticulocytes, or an elevated reticulocyte:erythrocyte ratio, in the blood is indicative of increased red blood cell production rates.

Erythropoietin (EPO) is widely recognized as the most significant positive regulator of postnatal erythropoiesis in vertebrates. EPO regulates the compensatory erythropoietic response to reduced tissue oxygen tension (hypoxia) and low red blood cell levels or low hemoglobin levels. In humans, elevated EPO levels promote red blood cell formation by stimulating the generation of erythroid progenitors in the bone marrow and spleen. In the mouse, EPO enhances erythropoiesis primarily in the spleen.

Effects of EPO are mediated by a cell-surface receptor belonging to the cytokine receptor superfamily. The human EPO receptor gene encodes a 483 amino acid transmembrane protein; however, the active EPO receptor is thought to exist as a multimeric complex even in the absence of ligand [see, *e.g.,* U.S. Pat. No. 6,319,499]. The cloned full-length EPO receptor expressed in mammalian cells binds EPO with an affinity similar to that of the native receptor on erythroid progenitor cells. Binding of EPO to its receptor causes a conformational change resulting in receptor activation and biological effects including increased proliferation of immature erythroblasts, increased differentiation of immature erythroblasts, and decreased apoptosis in erythroid progenitor cells [see, *e.g.,* Liboi et al. (1993) Proc Natl Acad Sci USA 90:11351-11355 and Koury et al. (1990) Science 248:378-381].

Various forms of recombinant EPO are used by physicians to increase red blood cell levels in a variety of clinical settings, particularly in the treatment of anemia. Anemia is a broadly-defined condition characterized by lower than normal levels of hemoglobin or red blood cells in the blood. In some instances, anemia is caused by a primary disorder in the production or survival of red blood cells *(e.g.,* a thalassemia disorder or sickle cell anemia). More commonly, anemia is secondary to diseases of other systems [see, *e.g.*, Weatherall & Provan (2000) Lancet 355, 1169-1175]. Anemia may result from a reduced rate of production or increased rate of destruction of red blood cells or by loss of red blood cells due to bleeding. Anemia may result from a variety of disorders that include, for example, acute or chronic renal failure or end stage renal disease, chemotherapy treatment, a myelodysplastic syndrome, rheumatoid arthritis, and bone marrow transplantation.

Treatment with EPO typically causes a rise in hemoglobin by about 1-3 g/dL in healthy humans over a period of weeks. When administered to anemic individuals, this treatment regimen often provides substantial increases in hemoglobin and red blood cell levels and leads to improvements in quality of life and prolonged survival. However, EPO is not uniformly effective, and many individuals are refractory to even high doses [see, *e.g.,* Horl et al. (2000) Nephrol Dial Transplant 15, 43-50]. For example, over 50% of patients with cancer have an inadequate response to EPO, approximately 10% with end-stage renal disease are hyporesponsive to EPO [see, *e.g.,* Glaspy et al. (1997) J Clin Oncol 15, 1218-1234 and Demetri et al. (1998) J Clin Oncol 16, 3412-3425], and less than 10% with myelodysplastic syndrome respond favorably to EPO [see Estey (2003) Curr Opin Hematol 10, 60-670]. Several factors, including inflammation, iron and vitamin deficiency, inadequate dialysis, aluminum toxicity, and hyperparathyroidism may predict a poor therapeutic response. The molecular mechanisms of resistance to EPO are as yet unclear. Recent evidence suggests that higher doses of EPO may be associated with an increased risk of cardiovascular morbidity, tumor growth, and mortality in some patient populations [see, *e.g.,* Krapf et al. (2009) Clin J Am Soc Nephrol 4:470-480 and Glaspy (2009) Annu Rev Med 60:181-192] . It has been therefore recommended that EPO-based therapeutic compounds (e.g., erythropoietin-stimulating agents, ESAs) be administered at the lowest dose required to avoid red blood cell transfusions [see, *e.g.,* Jelkmann et al. (2008) Crit Rev Oncol. Hematol 67:39-61].

Ineffective erythropoiesis is a term used to describe a group of erythroid disorders in which erythrocyte production is decreased despite increased numbers of earlier-stage erythroid cells [see, *e.g.,* Tanno (2010) Adv Hematol 2010:358283]. Ineffective erythropoiesis often gives rise to anemia, elevated erythropoietin levels, formation of excessive numbers of red blood cell precursors, and iron overload. If they persist, these conditions can lead to splenomegaly, liver and heart disorders, and bone damage as well as other complications. As endogenous erythropoietin levels are commonly very high in patients with ineffective erythropoiesis, EPO-based therapeutics often will not treat the anemia in these patients and/or may cause an aggravation of other aspects of the disease, such as splenomegaly and iron overload. WO2011/020045 describes compositions for increasing red blood cell levels or treatment of anemia comprising erythropoietin receptor activators and a variant Activin RIIB (ActRIIB) polypeptide having a leucine residue replaced by an acidic amino acid residue, where the variant ActRIIB polypeptide has a reduced affinity for activin B and may be called a GDF trap.

Thus, it is an object of the present disclosure to provide alternative methods for increasing red blood cell levels and/or addressing other disorders in the context of ineffective erythropoiesis.

### SUMMARY OF THE INVENTION

The invention is defined in the claims. In certain aspects, the disclosure provides methods for increasing red blood cell levels, treating or preventing an anemia, and/or treating or preventing ineffective erythropoiesis in a subject comprising administering to a subject in need thereof an effective amount of an agent, or combination of agents, that antagonizes (inhibits) at least activin B and/or GDF11. In some embodiments, the agent, or combination of agents, that inhibits activin B further inhibits one or more additional ligands that bind to ActRIIB and signal through Smad 2/3. In some embodiments, the agent, or combination of agents, that inhibits GDF11 further inhibits one or more additional ligands that bind to ActRIIB and signal through Smad 2/3. For example, the agent, or combination of agents, that inhibits activin B and/or GDF11 may further inhibit GDF8. Optionally, the agent, or combination of agents, that inhibits activin B and/or GDF11 does not inhibit activin A. In certain embodiments, the agent, or combination of agents, that inhibits activin B and/or GDF11 further inhibits one or more of GDF8, activin A, activin E, activin C, and BMP6. In certain embodiments, the agent, or combination of agents, may further inhibit one or more of GDF15, Nodal, GDF3, BMP3, and BMP3B. In certain embodiments, the agent, or combination of agents, that inhibits activin B and/or GDF11 further inhibits BMP9 from interacting with a type II receptor of the TGFβ superfamily (e.g., ActRIIA and/or ActRIIB) and/or BMP10 from interacting with a type II receptor of the TGFβ superfamily (e.g., ActRIIA and/or ActRIIB). Preferably, the agent, or combination of agents, to be used in accordance with the methods of the present disclosure do not inhibit, or substantially inhibit, interaction (e.g., binding, activation of Smad 2/3 signaling, *etc.*) between BMP9 and ALK1 and/or BMP10 and ALK1. Inhibition may be assessed by a variety of biochemical assays known in the art as well as those provided herein *(e.g.,* protein-based assays, cell-based assays, *etc*.).

In some embodiments, the agent, or combination of agents, inhibits at least activin B and/or GDF11 signaling (Smad 2/3 signaling) in a cell-based assay. In some embodiments, the agent, or combination of agents, binds to activin B and/or GDF11. In some embodiments, the agent, or combination of agents, does not substantially inhibit activin A signaling in a cell-based assay. In some embodiments, the agent, or combination of agents, does not substantially bind to activin A. In some embodiments, the agent, or combination of agents, that inhibits GDF11 and/or activin B signaling in a cell-based assay further inhibits one or more of GDF8, BMP6, activin C, activin A, activin E, GDF15, Nodal, GDF3, BMP3, BMP3B, BMP9, and BMP10 signaling in a cell-based assay. In some embodiments, the agent, or combination of agents, that binds to GDF11 and/or activin B further binds to one or more of GDF8, BMP6, activin C, activin A, activin E, GDF15, Nodal, GDF3, BMP3, BMP3B, BMP9, and BMP10.

In some embodiments, the agent is a multispecific antibody, or combination of multispecific antibodies, that binds to and/or inhibits at least GDF11 and activin B. In some embodiments, the multispecific antibody, or combination of multispecific antibodies, does not substantially bind to and/or inhibit activin A. In some embodiments, the multispecific antibody, or combination of multispecific antibodies, further binds to and/or inhibits GDF8. In some embodiments, the multispecific antibody, or combination of multispecific antibodies, that binds to and/or inhibits GDF11 and/or activin B further binds to and/or inhibits one or more of activin C, activin E, activin A, GDF8, ActRIIA, ActRIIB, BMP6, GDF15, Nodal, GDF3, BMP3, BMP3B, BMP9, and BMP10.

In some embodiments, the multispecific antibody is a bispecific antibody, or combination of bispecific antibodies. In some embodiments, the bispecific antibody, or combination of bispecific antibodies, binds to and/or inhibits at least GDF11 and activin B. In some embodiments, the bispecific antibody, or combination of bispecific antibodies, does not substantially bind to and/or inhibit activin A.

In some embodiments, bispecific antibody comprises two different monospecific antibodies that are associated with one another. In some embodiments, the antibody is a chimeric antibody. In some embodiments, the antibody is a humanized antibody. In some embodiments, the antibody is a human antibody.

In some embodiments, the antibody is a single-chain antibody. In some embodiments, the antibody is an F(ab')₂ fragment. In some embodiments, the antibody is a single-chain diabody, a tandem single-chain Fv fragment, a tandem single-chain diabody, a or a fusion protein comprising a single-chain diabody and at least a portion of an immunoglobulin heavy chain constant region.

In some embodiments, the antibody is a dual variable-domain immunoglobulin.

In some embodiments, the antibody comprises a heterologous moiety. In some embodiments, the heterologous moiety is a sugar, a detectable label, or a stabilization moiety.

In some embodiments, the agent is a GDF11/activin B trap comprising an amino acid sequence that is at least 80% *(e.g.* at least 80%, 85%, 90%, 95%, 96%, 97%, 98%, 99%, 100%) identical to amino acids 29-109 of SEQ ID NO:1. In some embodiments, the GDF11/activin B trap does not substantially bind to/and or inhibit activin A. In some embodiments, the GDF11/activin A trap does not comprise an acidic amino acid at the position corresponding to position 79 of SEQ ID NO:1. In some embodiments, the agent is a GDF11/activin B trap comprising an amino acid sequence that is at least 80% *(e.g.* at least 80%, 85%, 90%, 95%, 96%, 97%, 98%, 99%, 100%) identical to amino acids 29-109 of SEQ ID NO:1, and wherein the GDF11/activin B trap binds to activin B with a K_{D} of less than 100 pM, less than 10pM, or less than 1pM. In some embodiments, the GDF11/activin B trap binds to activin B with a K_{D} of 1 nM-750 pM, 750 pM-500 pM, 500 pM-250 pM, 250pM-100 pM, 100 pM - 50 pM, 50-25 pM, 25-10 pM, or 10-1 pM. In some embodiments, the agent is a GDF11/activin B trap comprising an amino acid sequence that is at least 80% identical (*e.g*. at least 80%, 85%, 90%, 95%, 96%, 97%, 98%, 99%, or 100%) to amino acids 29-109 of SEQ ID NO:1, and wherein the GDF11/ activin B trap has a binding affinity for GDF11 that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand binding domain of a ActRIIB receptor. In some embodiments, the agent is a GDF11/activin B trap comprising an amino acid sequence that is at least 80% *(e.g.* at least 80%, 85%, 90%, 95%, 96%, 97%, 98%, 99%, or 100%) identical to amino acids 29-109 of SEQ ID NO:1, and wherein the GDF11/activin B trap has a binding affinity for activin B that is 3-fold less, 4-fold higher, 5-fold higher, 6-fold higher, 7-fold higher, 8-fold higher, 9-fold higher, 10-fold higher, 15-fold higher, or 20-fold higher than the binding affinity of a wild-type ligand binding domain of a ActRIIB receptor.

In some embodiments, the GDF11/activin B trap comprises an amino acid sequence that is at least 85%, 90%, 95%, 96%, 97%, 98%, 99%, or 100% identical to amino acids 29-109 of SEQ ID NO:1. In some embodiments, the GDF11/activin B trap comprises an amino acid sequence that is at least 80%, 85%, 90%, 95%, 96%, 97%, 98%, 99%, or 100% identical to amino acids 25-131 of SEQ ID NO:1. In some embodiments, the GDF11/activin B trap comprises an amino acid sequence that is at least 80%, 85%, 90%, 95%, 96%, 97%, 98%, 99%, or 100% identical to the amino acid sequence of SEQ ID NO: 3 or 4. In some embodiments, the GDF11/activin B trap comprises a polypeptide comprising an amino acid sequence that is at least 80%, 85%, 90%, 95%, 96%, 97%, 98%, 99%, 100% identical to the amino acid sequence of SEQ ID NO: 5 or 6.

In some embodiments, the GDF11/activin B trap comprises an amino acid sequence that is at least 80%, 85%, 90%, 95%, 96%, 97%, 98%, 99%, or 100% identical to the amino acid sequence of SEQ ID NO:23.

In some embodiments, the GDF11/activin B trap comprises an amino acid sequence that is at least 80%, 85%, 90%, 95%, 96%, 97%, 98%, 99%, or 100% identical to the amino acid sequence of SEQ ID NO:48.

In some embodiments, the GDF11/activin B trap comprises an amino acid sequence that is at least 80%, 85%, 90%, 95%, 96%, 97%, 98%, 99%, or 100% identical to the amino acid sequence of SEQ ID NO:49.

In some embodiments, the agent is a GDF11/activin B trap comprising an amino acid sequence that is at least 80% identical to amino acids 30-110 of SEQ ID NO:9. In some embodiments, the agent is a GDF11/activin B trap comprising an amino acid sequence that is at least 80% identical to amino acids 30-110 of SEQ ID NO:9, and wherein the GDF11/activin trap has a binding affinity for GDF11 that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand-binding domain of a ActRIIA receptor. In some embodiments, the agent is a GDF11/activin B trap comprising an amino acid sequence that is at least 80% identical to amino acids 30-110 of SEQ ID NO:9, and wherein the GDF11/activin B trap has a binding affinity for activin B that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand-binding domain of a ActRIIA receptor. In some embodiments, the GDF11/activin B trap comprises an amino acid sequence that is at least 85%, 90%, 95%, 96%, 97%, 98%, 99%, or 100% identical to amino acids 30-110 of SEQ ID NO:9.

In some embodiments, the GDF11/activin B trap comprises an amino acid sequence that is at least 80%, 85%, 90%, 95%, 96%, 97%, 98%, 99%, or 100% identical to the amino acid sequence of SEQ ID NO:10.

In some embodiments, the GDF11/activin B trap comprises a polypeptide comprising an amino acid sequence that is at least 80%, 85%, 90%, 95%, 96%, 97%, 98%, 99%, or 100% identical to the amino acid sequence of SEQ ID NO:11.

In some embodiments, the GDF11/activin B trap comprises an amino acid sequence that is at least 80%, 85%, 90%, 95%, 96%, 97%, 98%, 99%, or 100% identical to the amino acid sequence of SEQ ID NO:20.

In some embodiments, the agent may be any one of the GDF11, activin B and GDF11/activin B traps disclosed herein, or a combination thereof.

In some embodiments, the GDF11/activin B trap is a fusion protein comprising, in addition to a GDF11/activin B trap polypeptide domain, one or more heterologous polypeptide domains that enhance one or more of: *in vivo* half-life, *in vitro* half-life, uptake/administration, tissue localization or distribution, formation of protein complexes, multimerization of the fusion protein and/or purification. In some embodiments, the GDF11/activin B trap fusion protein comprises a heterologous polypeptide domain selected from: an immunoglobulin Fc domain and a serum albumin. In some embodiments, the immunoglobulin Fc domain is an IgG1 Fc domain. In some embodiments, the immunoglobulin Fc domain comprises an amino acid sequence selected from SEQ ID NO: 16 or 17. In some embodiments, fusion protein further comprises a linker domain positioned between the Trap polypeptide domain and the immunoglobulin Fc domain. In some embodiments, the linker domain is a TGGG linker (SEQ ID NO: 45). In some embodiments, the linker domain may be any of the linker domains disclosed herein. In some embodiments, a fusion protein may include a purification subsequence, such as an epitope tag, a FLAG tag, a polyhistidine sequence, and a GST fusion. In certain embodiments, a GDF11/activin B trap fusion comprises a leader sequence. The leader sequence may be a native leader sequence or a heterologous leader sequence. In certain embodiments, the leader sequence is a tissue plasminogen activator (TPA) leader sequence. In an embodiment, a GDF11/activin B trap fusion protein comprises an amino acid sequence as set forth in the formula A-B-C. The B portion is a GDF11/activin B trap of the disclosure. The A and C portions may be independently zero, one or more than one amino acids, and both A and C portions are heterologous to B. The A and/or C portions may be attached to the B portion via a linker sequence. In some embodiments, the GDF11/activin B trap fusion protein may comprise any of the fusion proteins disclosed herein.

In some embodiments, the GDF11/activin B trap comprises one or more amino acid modifications selected from: a glycosylated amino acid, a PEGylated amino acid, a farnesylated amino acid, an acetylated amino acid, a biotinylated amino acid, an amino acid conjugated to a lipid moiety, an amino acid conjugated to an organic derivatizing agent. In some embodiments, the GDF11/activin B trap is glycosylated and has a mammalian glycosylation pattern. In some embodiments, the GDF11/activin B trap has a glycosylation pattern obtainable from a Chinese hamster ovary cell line. In general, it is preferable that a GDF trap be expressed in a mammalian cell line that mediates suitably natural glycosylation of the GDF trap so as to diminish the likelihood of an unfavorable immune response in a patient. Human and CHO cell lines have been used successfully, and it is expected that other common mammalian expression vectors will be useful. In some embodiments, the GDF11/activin B trap may comprise any of the amino acid modifications disclosed herein, or a combination thereof.

In certain aspects, the disclosure provides nucleic acids encoding a GDF11/activin B trap polypeptide. An isolated polynucleotide may comprise a coding sequence for a soluble GDF trap polypeptide, such as described above. Nucleic acids disclosed herein may be operably linked to a promoter for expression, and the disclosure provides cells transformed with such recombinant polynucleotides. Preferably the cell is a mammalian cell such as a CHO cell. In some embodiments, the host cell may be selected from any of the cells disclosed herein for such purpose.

In certain aspects, the disclosure provides methods for making a GDF11/activin B trap polypeptide. Such a method may include expressing any of the nucleic acids disclosed herein in a suitable cell, such as a Chinese hamster ovary (CHO) cell. Such a method may comprise: a) culturing a cell under conditions suitable for expression of the GDF11/activin B trap polypeptide, wherein said cell is transformed with a GDF11/activin B trap expression construct; and b) recovering the GDF11/activin B trap polypeptide so expressed. GDF11/activin B trap polypeptides may be recovered as crude, partially purified or highly purified fractions using any of the well-known techniques for obtaining protein from cell cultures.

In certain aspects, the disclosure provides a method for increasing red blood cell levels or treating or preventing an anemia in a subject comprising administering to a subject in need thereof an effective amount of a combination of agents that inhibit signaling of activin B and GDF11. In some embodiments, the combination of agents inhibits activin B and GDF11 signaling in a cell based assay. In some embodiments, the combination of agents does not substantially inhibit activin A signaling. In some embodiments, the combination of agents does not substantially inhibit activin A signaling in a cell based assay. In some embodiments, the combination of agents does not substantially bind to activin A. In some embodiments, one or more of the agents that inhibit GDF11 and/or activin B further inhibits the signaling of one or more of GDF8, BMP6, activin A, activin C, activin E, GDF15, Nodal, GDF3, BMP3B, BMP9, and BMP10.

In some embodiments, the combination of agents comprises at least one agent that is an antibody or antigen-binding fragment thereof that binds to GDF11. In some embodiments, the combination of agents comprises at least one agent that is an antibody or antigen-binding fragment thereof that binds to activin B. In some embodiments, the combination of agents comprises a combination of two or more antibodies or antigen-binding fragments thereof directed against two or more targets disclosed herein (e.g., activin A, activin B, activin C, activin E, GDF11, GDF8, BMP6, GDF15, Nodal, GDF3, BMP3, BMP3B, BMP9, and BMP10).

In some embodiments, the antibody or antigen-binding fragment thereof is a chimeric antibody or fragment thereof. In some embodiments, the antibody or antigen-binding fragment thereof is a humanized antibody or fragment thereof. In some embodiments, the antibody or antigen-binding fragment thereof is a human antibody or fragment thereof. In some embodiments, the antibody or antigen-binding fragment thereof is a single-chain antibody. In some embodiments, the antigen-binding fragment is selected from the group consisting of: Fab, Fab', F(ab')₂, F(ab')₃, Fd, Fv, domain antibody. In some embodiments, the antibody or antigen-binding fragment thereof comprises a heterologous moiety. In some embodiments, the heterologous moiety is a sugar, a detectable label, or a stabilization moiety.

In some embodiments, the combination of agents comprises at least one agent that is a GDF11 trap, and wherein the GDF11 trap comprises a polypeptide comprising an amino acid sequence that is at least 80% *(e.g.* at least 80%, 85%, 90%, 95%, 96%, 97%, 98%, 99%, or 100%) identical to amino acids 29-109 of SEQ ID NO:1. In some embodiments, the combination of agents comprises at least one agent that is an activin B trap, and wherein the activin B trap comprises a polypeptide comprising an amino acid sequence that is at least 80% *(e.g.* at least 80%, 85%, 90%, 95%, 96%, 97%, 98%, 99%, or 100%) identical to amino acids 29-109 of SEQ ID NO:1.

In some embodiments, the combination of agents comprises at least one GDF11 trap or activin B trap comprising an amino acid sequence that is at least 80% *(e.g.* at least 80%, 85%, 90%, 95%, 96%, 97%, 98%, 99%, or 100%) identical to amino acids 29-109 of SEQ ID NO:1, and wherein the GDF11 trap or activin B trap does not comprise an acidic amino acid at the position corresponding to position 79 of SEQ ID NO:1. In some embodiments, the agent is a GDF11 trap comprising an amino acid sequence that is at least 80% *(e.g.* at least 80%, 85%, 90%, 95%, 96%, 97%, 98%, 99%, or 100%) identical to amino acids 29-109 of SEQ ID NO:1, and wherein the GDF11 trap binds to activin B with a K_{D} of less than 100 pM, less than 10pM, or less than 1pM. In some embodiments, the GDF11 trap binds to activin B with a K_{D} of 1 nM-750 pM, 750 pM-500 pM, 500 pM-250 pM, 250pM-100 pM, 100 pM - 50 pM, 50-25 pM, 25-10 pM, or 10-1 pM. In some embodiments, the agent is a GDF11 trap comprising an amino acid sequence that is at least 80% identical (*e.g*. at least 80%, 85%, 90%, 95%, 96%, 97%, 98%, 99%, or 100%) to amino acids 29-109 of SEQ ID NO:1, and wherein the GDF11 trap has a binding affinity for GDF11 that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand binding domain of a ActRIIB receptor. In some embodiments, the agent is a activin B trap comprising an amino acid sequence that is at least 80% *(e.g.* at least 80%, 85%, 90%, 95%, 96%, 97%, 98%, 99%, or 100%) identical to amino acids 29-109 of SEQ ID NO:1, and wherein the GDF11 trap has a binding affinity for activin B that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand binding domain of a ActRIIB receptor.

In some embodiments, the GDF11 trap or activin B trap comprises an amino acid sequence that is at least 85%, 90%, 95%, 96%, 97%, 98%, 99%, or 100% identical to amino acids 29-109 of SEQ ID NO:1. In some embodiments, the GDF11 trap or activin B trap comprises an amino acid sequence that is at least 80%, 85%, 90%, 95%, 96%, 97%, 98%, 99%, or 100% identical to amino acids 25-131 of SEQ ID NO:1. In some embodiments, the GDF11 trap or activin B trap comprises an amino acid sequence that is at least 80%, 85%, 90%, 95%, 96%, 97%, 98%, 99%, or 100% identical to the amino acid sequence of SEQ ID NO: 3 or 4. In some embodiments, the GDF11 trap or activin B trap comprises a polypeptide comprising an amino acid sequence that is at least 80%, 85%, 90%, 95%, 96%, 97%, 98%, 99%, or 100% identical to the amino acid sequence of SEQ ID NO: 5 or 6.

In some embodiments, the GDF11 trap or activin B trap comprises an amino acid sequence that is at least 80%, 85%, 90%, 95%, 96%, 97%, 98%, 99%, or 100% identical to the amino acid sequence of SEQ ID NO:23.

In some embodiments, the combination of agents comprises at least one agent that is a GDF11 trap, and wherein the GOF11 trap comprises a polypeptide comprising an amino acid sequence that is at least 80% *(e.g.* at least 80%, 85%, 90%, 95%, 96%, 97%, 98%, 99%, or 100%) identical to amino acids 30-110 of SEQ ID NO:9. In some embodiments, the combination of agents comprises at least one agent that is an activin B trap, and wherein the activin B trap comprises a polypeptide comprising an amino acid sequence that is at least 80% *(e.g.* at least 80%, 85%, 90%, 95%, 96%, 97%, 98%, 99%, or 100%) identical to amino acids 30-110 of SEQ ID NO:9. In some embodiments, the agent is a GDF trap comprising an amino acid sequence that is at least 80% identical to amino acids 30-110 of SEQ ID NO:9, and wherein the GDF11 trap has a binding affinity for GOF11 that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand-binding domain of a ActRIIA receptor. In some embodiments, the agent is an activin B trap comprising an amino acid sequence that is at least 80% identical to amino acids 30-110 of SEQ ID NO:9, and wherein the activin B trap has a binding affinity for activin B that is 3-fold higher, 4-fold higher, 5-fold higher, 6-fold higher, 7-fold higher, 8-fold higher, 9-fold higher, 10-fold higher, 15-fold higher, or 20-fold higher than the binding affinity of a wild-type ligand-binding domain of a ActRIIA receptor. In some embodiments, the GDF11 trap or activin B trap comprises an amino acid sequence that is at least 85%, 90%, 95%, 96%, 97%, 98%, 99%, or 100% identical to amino acids 30-110 of SEQ ID NO:9.

In some embodiments, the GDF11 trap or activin B trap comprises an amino acid sequence that is at least 80%, 85%, 90%, 95%, 96%, 97%, 98%, 99%, or 100% identical to the amino acid sequence of SEQ ID NO:10.

In some embodiments, the GDF11 trap or activin B trap comprises a polypeptide comprising an amino acid sequence that is at least 80%, 85%, 90%, 95%, 96%, 97%, 98%, 99%, or 100% identical to the amino acid sequence of SEQ ID NO:11.

In some embodiments, the GDF11 trap or activin B trap comprises an amino acid sequence that is at least 80%, 85%, 90%, 95%, 96%, 97%, 98%, 99%, or 100% identical to the amino acid sequence of SEQ ID NO:20.

In some embodiments, the GDF11 trap or activin B trap is a fusion protein comprising, in addition to a GDF trap or activin B trap polypeptide domain, one or more heterologous polypeptide domains that enhance one or more of: *in vivo* half-life, *in vitro* half-life, uptake/administration, tissue localization or distribution, formation of protein complexes, multimerization of the fusion protein and/or purification. In some embodiments, the GDF11 trap or activin B trap fusion protein comprises a heterologous polypeptide domain selected from: an immunoglobulin Fc domain and a serum albumin. In some embodiments, the immunoglobulin Fc domain is an IgG1 Fc domain. In some embodiments, the immunoglobulin Fc domain comprises an amino acid sequence selected from SEQ ID NO: 16 or 17. In some embodiments, fusion protein further comprises a linker domain positioned between the trap polypeptide domain and the immunoglobulin Fc domain. In some embodiments, the linker domain is a TGGG linker. In some embodiments, the linker domain may be any of the linker domains disclosed herein. In some embodiments, a fusion protein may include a purification subsequence, such as an epitope tag, a FLAG tag, a polyhistidine sequence, and a GST fusion. In certain embodiments, a GDF11 trap or activin B trap fusion comprises a leader sequence. The leader sequence may be a native leader sequence or a heterologous leader sequence. In certain embodiments, the leader sequence is a tissue plasminogen activator (TPA) leader sequence. In an embodiment, a GDF11 trap or activin B trap fusion protein comprises an amino acid sequence as set forth in the formula A-B-C. The B portion is a GDF11 trap or activin B trap of the disclosure. The A and C portions may be independently zero, one or more than one amino acids, and both A and C portions are heterologous to B. The A and/or C portions may be attached to the B portion via a linker sequence. In some embodiments, the GDF11 trap or activin B trap fusion protein may comprise any of the fusion proteins disclosed herein.

In some embodiments, the GDF11 trap or activin B trap comprises one or more amino acid modifications selected from: a glycosylated amino acid, a PEGylated amino acid, a farnesylated amino acid, an acetylated amino acid, a biotinylated amino acid, an amino acid conjugated to a lipid moiety, an amino acid conjugated to an organic derivatizing agent. In some embodiments, the GDF11 trap or activin B trap is glycosylated and has a mammalian glycosylation pattern. In some embodiments, the GDF11 trap or activin B trap has a glycosylation pattern obtainable from a Chinese hamster ovary cell line. In general, it is preferable that a GDF11 trap be expressed in a mammalian cell line that mediates suitably natural glycosylation of the GDF11 trap so as to diminish the likelihood of an unfavorable immune response in a patient. Human and CHO cell lines have been used successfully, and it is expected that other common mammalian expression vectors will be useful. In some embodiments, the GDF11 trap or activin B trap may comprise any of the amino acid modifications disclosed herein, or a combination thereof.

In certain aspects, the disclosure provides nucleic acids encoding a GDF11 trap or activin B trap polypeptide. An isolated polynucleotide may comprise a coding sequence for a soluble GDF11 trap polypeptide or activin B trap polypeptide, such as described above. Nucleic acids disclosed herein may be operably linked to a promoter for expression, and the disclosure provides cells transformed with such recombinant polynucleotides. Preferably the cell is a mammalian cell such as a CHO cell. In some embodiments, the host cell may be selected from any of the cells disclosed herein for such purpose.

In certain aspects, the disclosure provides methods for making a GDF11 trap or activin B trap polypeptide. Such a method may include expressing any of the nucleic acids disclosed herein in a suitable cell, such as a Chinese hamster ovary (CHO) cell. Such a method may comprise: a) culturing a cell under conditions suitable for expression of the GDF11 trap or activin B trap polypeptide, wherein said cell is transformed with a GDF11 trap or activin B trap expression construct; and b) recovering the GDF11/activin B trap polypeptide so expressed. GDF11/activin B trap polypeptides may be recovered as crude, partially purified or highly purified fractions using any of the well-known techniques for obtaining protein from cell cultures.

In some embodiments, two or more of any of the GDF11, activin B and GDF11/activin B traps disclosed herein may be combined.

In some embodiments, the GDF11 and/or activin B antagonist is a small-molecule antagonist, or combination of small-molecule antagonists. In some embodiments, the combination of agents comprises at least one agent that is a small-molecule antagonist of activin B. In some embodiments, the combination of agents comprises at least one agent that is a small-molecule antagonist of GDF11. In some embodiments, the GDF11 and/or activin B small-molecule antagonist, or combination of small-molecule antagonists, does not bind to and/or inhibit activin A. In some embodiments, the GDF11 and/or activin B small-molecule antagonist, or combination of small-molecule antagonists, further bind to and/on inhibit GDF8. In some embodiments, the GDF11 and/or activin B small-molecule antagonist, or combination of small-molecule antagonists, further bind to and/or inhibit one or more of activin A, activin C, activin E, GDF8, BMP6, GDF15, Nodal, GDF3, BMP3 and BMP10.

In another aspect, an antagonist agent, or combination of agents, of the present disclosure is a polynucleotide antagonist that inhibits at least GDF11 and/or activin B. In some embodiments, an antagonist polynucleotide of the disclosure inhibits the expression (e.g., transcription, translation, and/or cellular secretion) of at least GDF11 and/or activin B. Optionally, a polynucleotide antagonist, or combinations of polynucleotide antagonists, of the disclosure does not inhibit activin A (*e.g*. inhibits expression and/or activity of activin A). Optionally, a polynucleotide antagonist, or combinations of polynucleotide antagonists, of the disclosure further inhibit GDF8 (*e.g*. inhibits expression and/or activity of GDF8). In some embodiments, a polynucleotide antagonist, or combinations of polynucleotide antagonists, of the disclosure that inhibits GDF11 and/or activin B (*e.g*. expression and/or activity of GDF11 and/or activin B) further inhibits (e.g., inhibits expression and/or activity) one or more of activin E, activin C, activin A, GDF8, BMP6, GDF15, Nodal, GDF3, BMP3, and BMP3B.

In some embodiments, the polynucleotide molecule is an antisense oligonucleotide that hybridizes to a transcript of a gene selected from: activin B, activin C, activin E, GDF11, and GDF8, activin A, GDF15, GDF3, Nodal, BMP3, and BMP3B to inhibit expression of the gene. In some embodiments, the combination of agents comprises an antisense oligonucleotide that hybridizes to a transcript of activin B and inhibits activin B expression. In some embodiments, the combination of agents comprises an antisense oligonucleotide that hybridizes to a transcript of GDF11 and inhibits GDF11 expression. In some embodiments, the combination of agents comprises a combination of two or more antisense oligonucleotides that inhibit the expression of two or more targets of the disclosure (e.g., activin A, activin B, activin C, activin E, GDF11, GDF8, BMP6, GDF15, Nodal, GDF3, BMP3, and BMP3B).

In some embodiments, the polynucleotide molecule comprises an RNAi molecule that targets the transcript of a gene selected from: activin A, activin B, activin C, activin E, BMP6, GDF11, GDF8, GDF15, Nodal, GDF3, BMP3, and BMP3B. In some embodiments, the polynucleotide molecule comprises an RNAi molecule that targets the transcript of GDF11. In some embodiments, the polynucleotide molecule comprises an RNAi molecule that targets the transcript of activin B. In some embodiments, the combination of agents comprises a combination of two or more RNAi molecules that inhibit the expression of two or more targets of the disclosure (e.g., activin A, activin B, activin C, activin E, GDF11, GDF8, BMP6, GDF15, Nodal, GDF3, BMP3, and BMP3B).

In some embodiments, the RNAi molecule comprises an siRNA. In some embodiments, the siRNA is from about 19 to about 45 nucleotides in length. In some embodiments, the siRNA is from about 25 to about 30 nucleotides in length. In some embodiments, the siRNA is from about 10 to about 20 nucleotides in length. In some embodiments, the RNAi molecule comprises an shRNA. In some embodiments, the shRNA molecule has a stem length of 19-29 nucleotides. In some embodiments, the shRNA molecule has a stem length of 19-23 nucleotides. In some embodiments, the loop region of the shRNA has a length of 5-9 nucleotides.

In some embodiments, any of the disclosed GOF11 antagonists herein *(e.g.,* GDF11 trap polypeptide, anti-GDF11 antibody, small-molecule antagonist, polypeptide or polynucleotide antagonist) can be combined with an activin B antagonist of the disclosure (e.g., activin B trap polypeptide, anti-activin B antibody, small-molecule antagonist, polypeptide or polynucleotide antagonist) to inhibit both a GDF11 and an activin B activity *(e.g.,* the ability to bind to and/or activate an ActRIIA and/or ActRIIB receptor).

In some embodiments, methods of the disclosure are for increasing red blood cells in a subject in need thereof. In some embodiments, the method is for treating or preventing an anemia in a subject in need thereof. In some embodiments, the anemia is associated with one or more of: multiple myeloma, chronic or acute renal disease or failure, chemotherapeutic treatment of the subject, a myelodysplastic syndrome, and a thalassemia. In some embodiments, the thalassemia is beta-thalassemia. In some embodiments, the renal failure is end-stage renal failure. In some embodiments, the subject has sickle cell anemia.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
Figure 1 shows an alignment of the extracellular domains of human ActRIIA (SEQ ID NO: 36) and human ActRIIB (SEQ ID NO: 46) with the residues that are deduced herein, based on composite analysis of multiple ActRIIB and ActRIIA crystal structures to directly contact ligand (the ligand binding pocket) indicated with boxes.

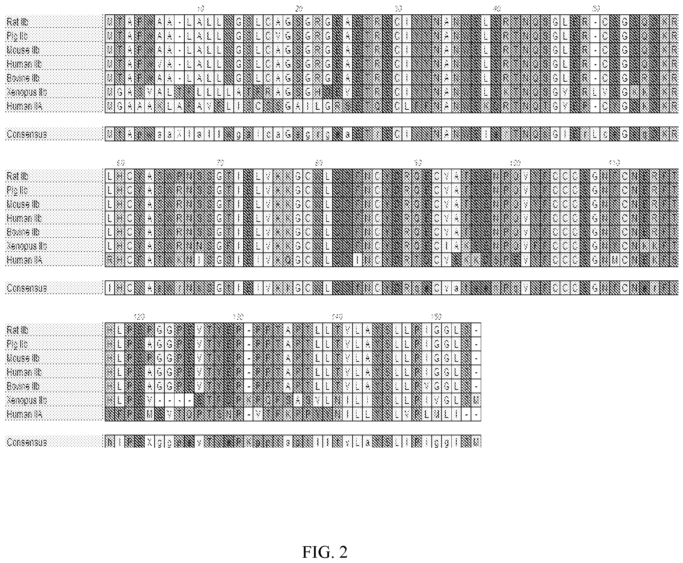

Figure 2 shows a multiple sequence alignment of various vertebrate ActRIIB proteins and human ActRIIA (SEQ ID NOs: 37-44).

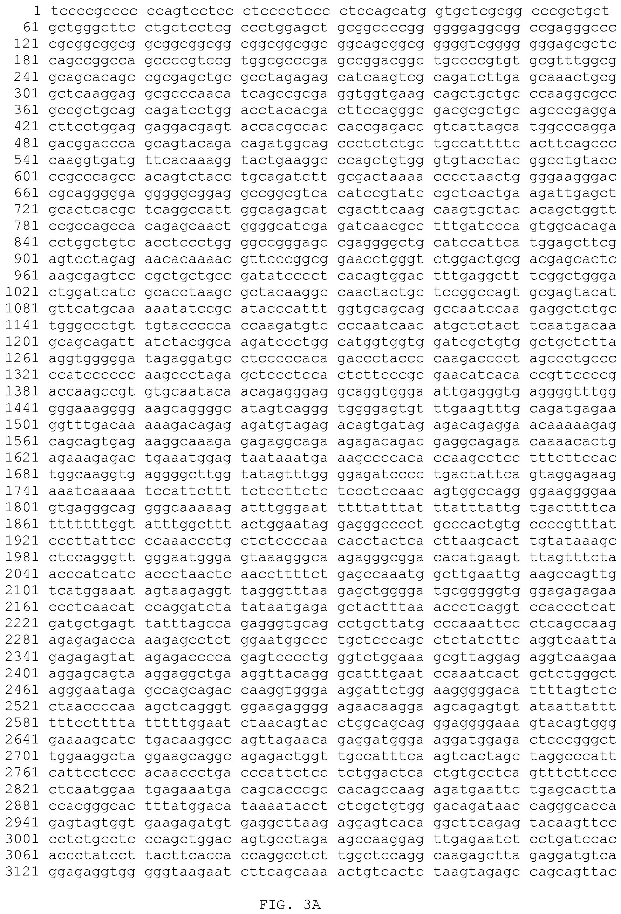

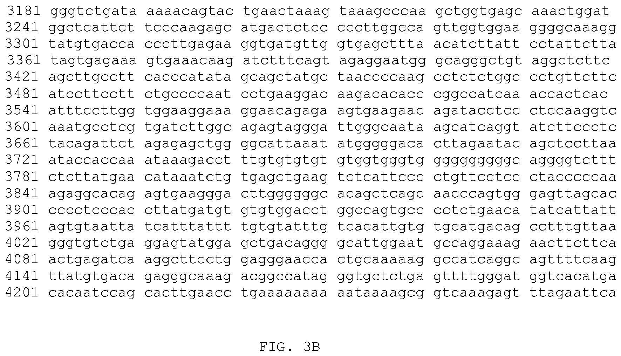

Figures 3A and 3B depict the human GDF11 cDNA sequence (NCBI Reference Sequence No. NM_005811.3) (SEQ ID NO: 24).

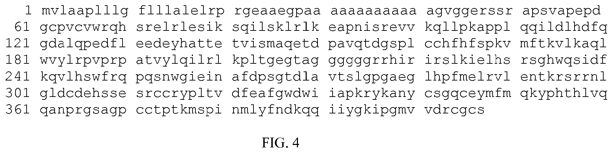

Figure 4 depicts the amino acid sequence of the human GDF11 precursor protein sequence (NCBI Reference Sequence No. NP_005802.1) (SEQ ID NO: 25).

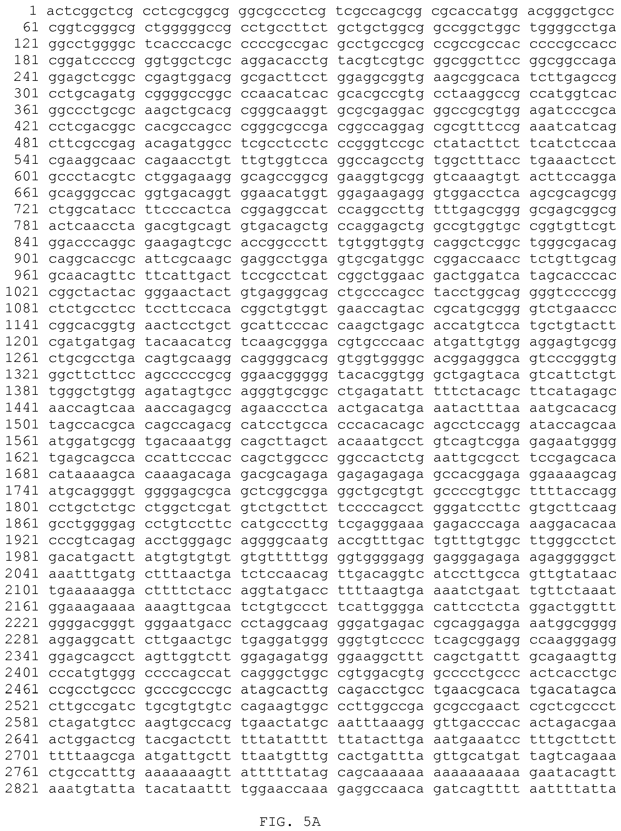

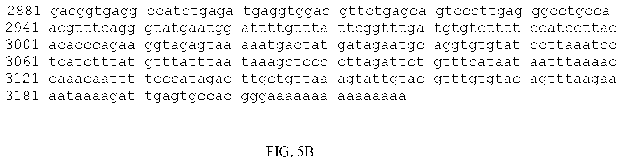

Figures 5A and 5B depict the human activin B cDNA sequence (NCBI Reference Sequence No. NM_002193.2) (SEQ ID NO: 26).

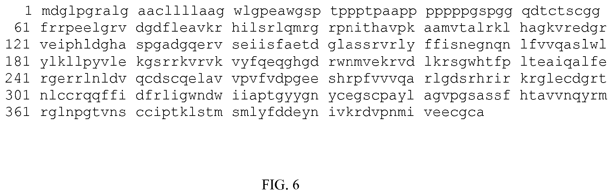

Figure 6 depicts the amino acid sequence of the human activin B precursor protein (NCBI Reference Sequence No. NP_002184.2) (SEQ ID NO: 27).

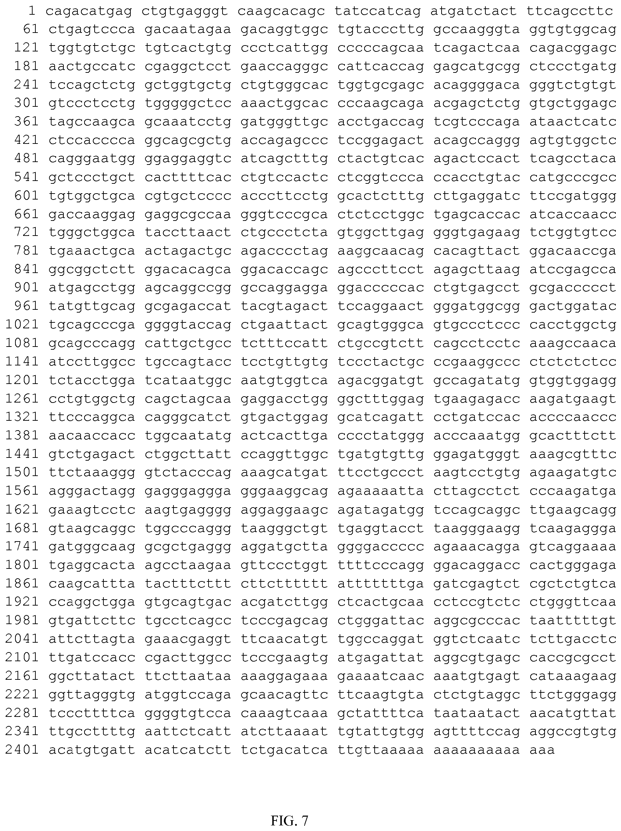

Figure 7 depicts the human activin E cDNA sequence (GenBank Accession No. NM_031479.3) (SEQ ID NO: 28).

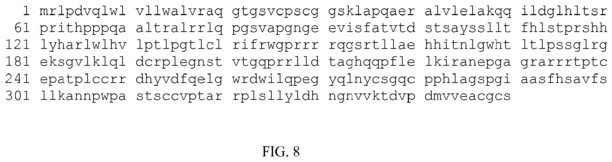

Figure 8 depicts the amino acid sequence of the human activin E precursor protein (GenBank Accession No. NP_113667.1) (SEQ ID NO: 29).

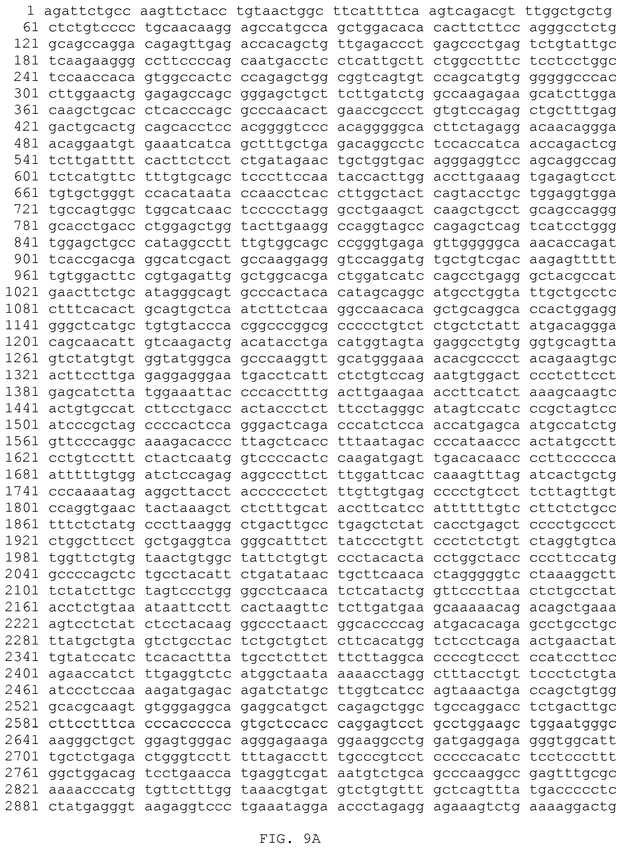

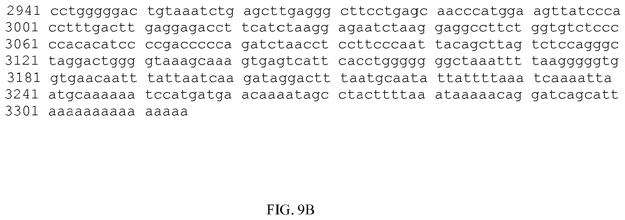

Figures 9A and 9B depict the human activin C cDNA sequence (NCBI Reference Sequence No. NM_005538.3) (SEQ ID NO: 30).

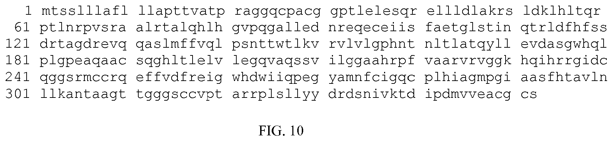

Figure 10 depicts the amino acid sequence of the human activin C precursor protein (NCBI Reference Sequence No. NP_005529.1) (SEQ ID NO: 31).

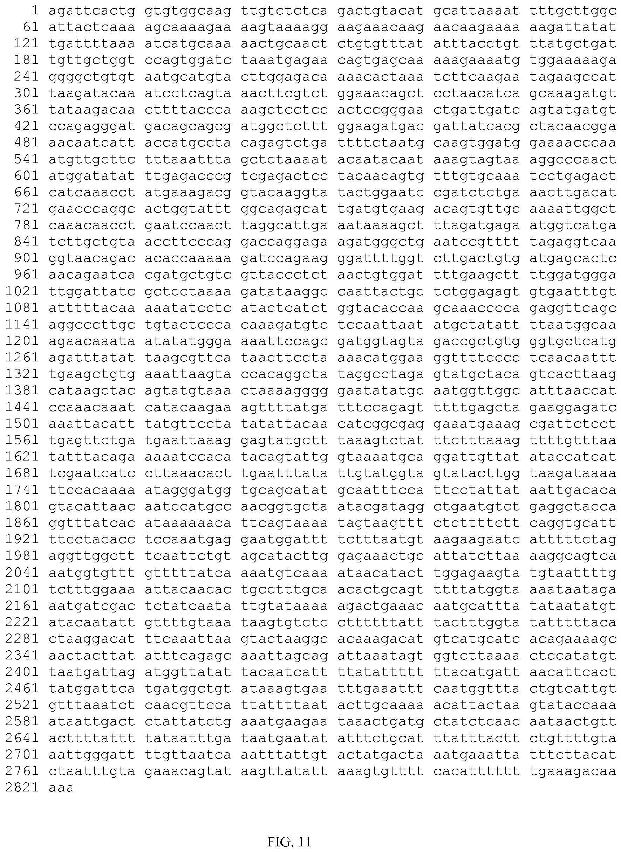

Figure 11 depicts the human GDF8 cDNA sequence (NCBI Reference Sequence No. NM_005259.2) (SEQ ID NO: 32).

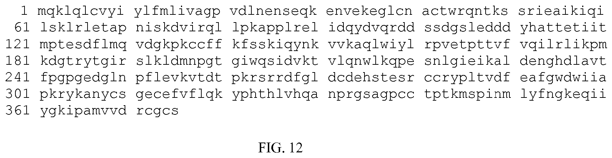

Figure 12 depicts the amino acid sequence of the human GDF8 precursor protein (NCBI Reference Sequence No. NP_005250.1) (SEQ ID NO: 33).
Figure 13 depicts the ligand binding profile for ActRIIB(L79D 25-131)-Fc (see, *e.g.,* U.S. Patent No. 8,058,229) characterized with respect to various ActRII ligands (GDF11, GDF8, activin A, activin B, BMP10, BMP6, and BMP9) as determined by surface plasmon resonance.

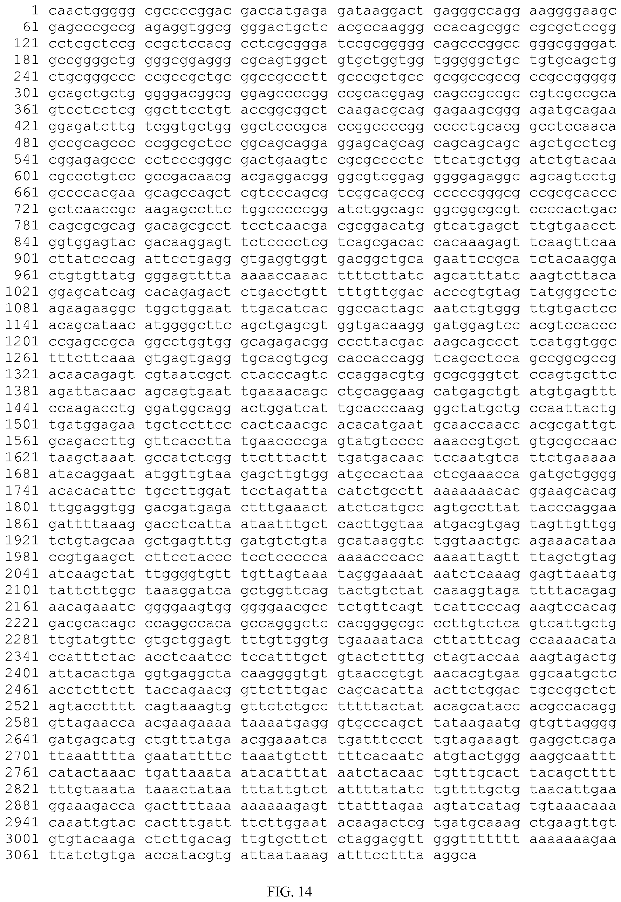

Figure 14 depicts the human BMP6 cDNA sequence (NCBI Reference Sequence No. NM_001718.4) (SEQ ID NO:34).

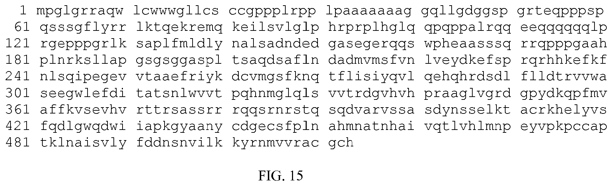

Figure 15 depicts the amino acid sequence of the human BMP6 precursor protein (NCBI Reference Sequence No. NP_001709.1) (SEQ ID NO: 35).
Figure 16 depicts the amino acid sequence of human GDF15 precursor protein (NCBI Reference Sequence No. NP_004855.2) (SEQ ID NO: 50).

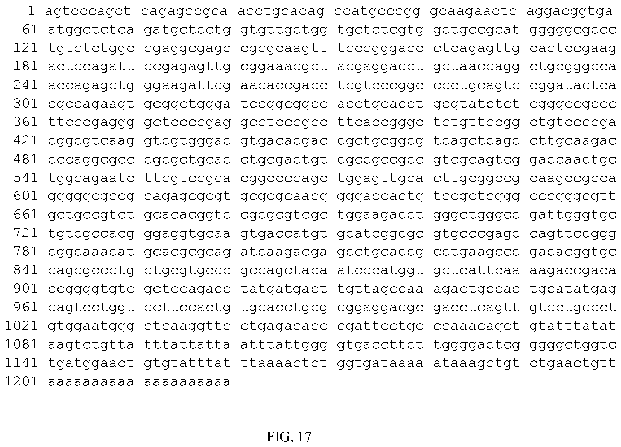

Figure 17 depicts the human GDF15 cDNA sequence (NCBI Reference Sequence No. NM_004864.2) (SEQ ID NO: 51).

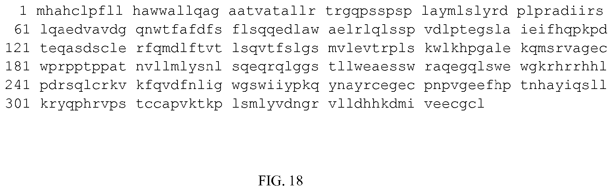

Figure 18 depicts the amino acid sequence of human Nodal precursor protein (NCBI Reference Sequence No. NP_060525.3) (SEQ ID NO: 52).

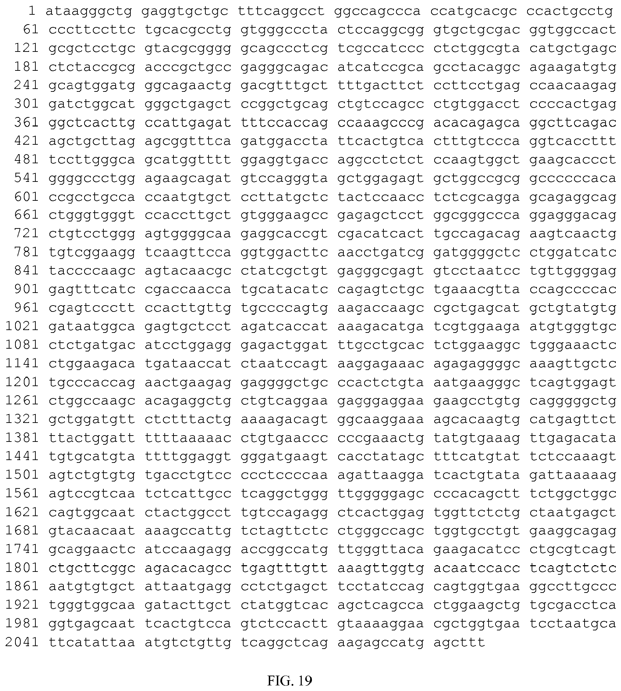

Figure 19 depicts the human Nodal cDNA sequence (NCBI Reference Sequence No. NM_018055.4) (SEQ ID NO: 53).

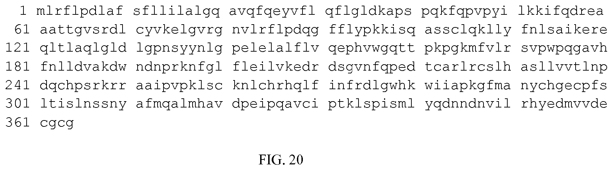

Figure 20 depicts the amino acid sequence of human GDF3 precursor protein (NCBI Reference Sequence No. NP_065685.1) (SEQ ID NO: 54).

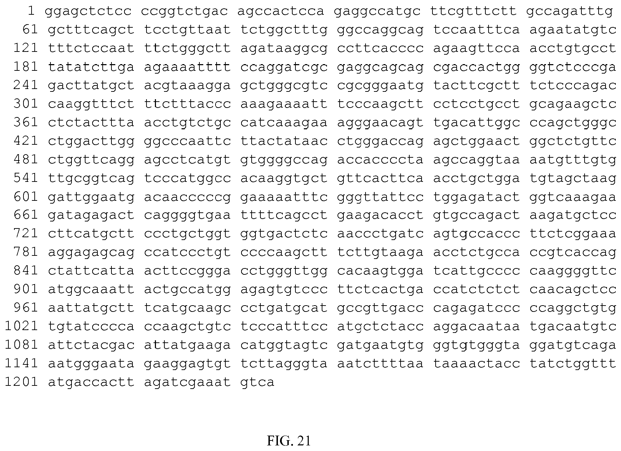

Figure 21 depicts the human GDF3 cDNA sequence (NCBI Reference Sequence No. NM_020634.1) (SEQ ID NO: 55).

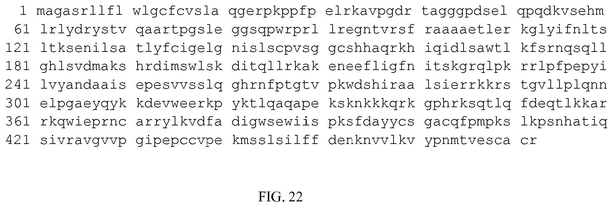

Figure 22 depicts the amino acid sequence of human BMP3 precursor protein (NCBI Reference Sequence No. NP_001192.2) (SEQ ID NO: 56).

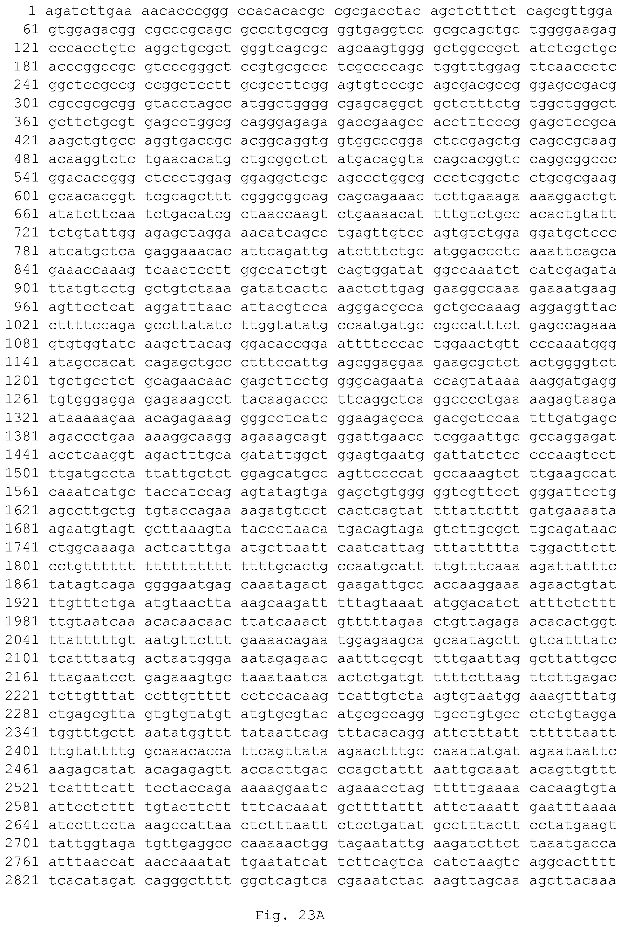

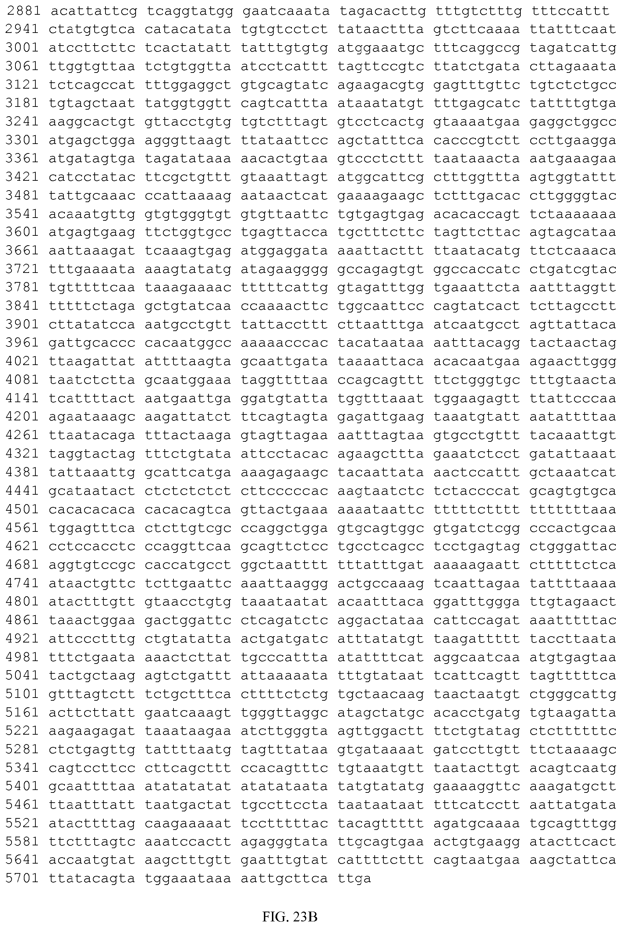

Figures 23A and 23B depict the human BMP3 cDNA sequence (NCBI Reference Sequence No. NM_001201.2) (SEQ ID NO: 57).

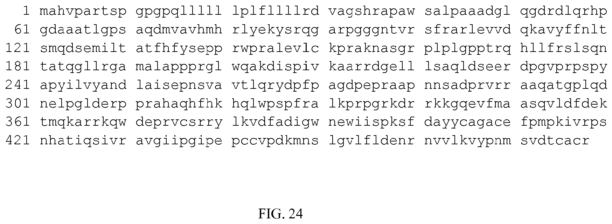

Figure 24 depicts the amino acid sequence of human BMP3B precursor protein (NCBI Reference Sequence No. NP_004953.1) (SEQ ID NO: 58).

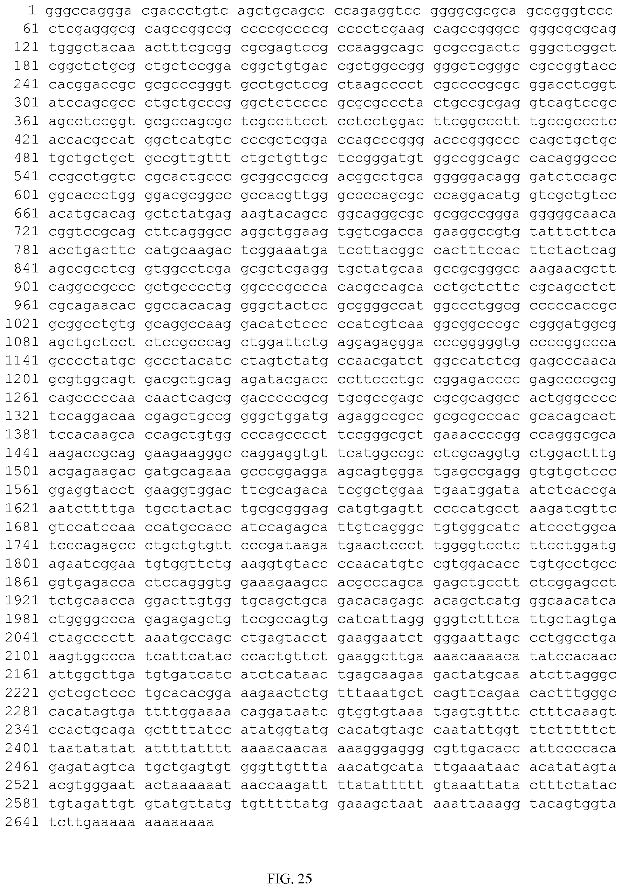

Figure 25 depicts the human BMP3B cDNA sequence (NCBI Reference Sequence No. NM_004962.3) (SEQ ID NO: 59).

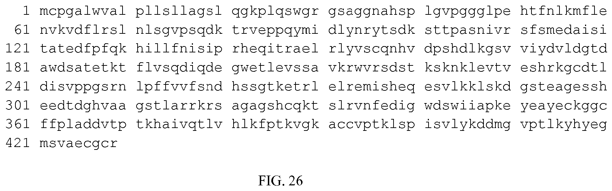

Figure 26 depicts the amino acid sequence of human BMP9 precursor protein (NCBI Reference Sequence No. NP_057288.1) (SEQ ID NO: 60).

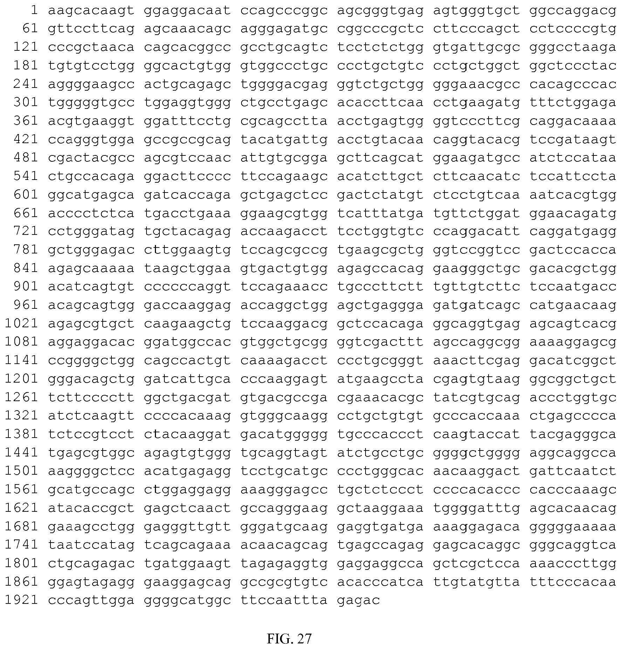

Figure 27 depicts the human BMP9 cDNA sequence (NCBI Reference Sequence No. NM_016204.2) (SEQ ID NO: 61).

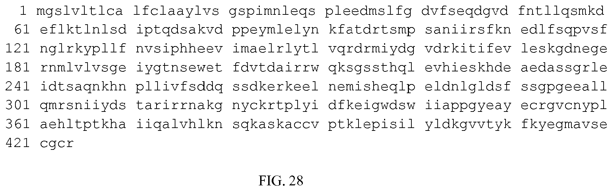

Figure 28 depicts the amino acid sequence of human BMP10 precursor protein (NCBI Reference Sequence No. NP_055297.1) (SEQ ID NO: 62).

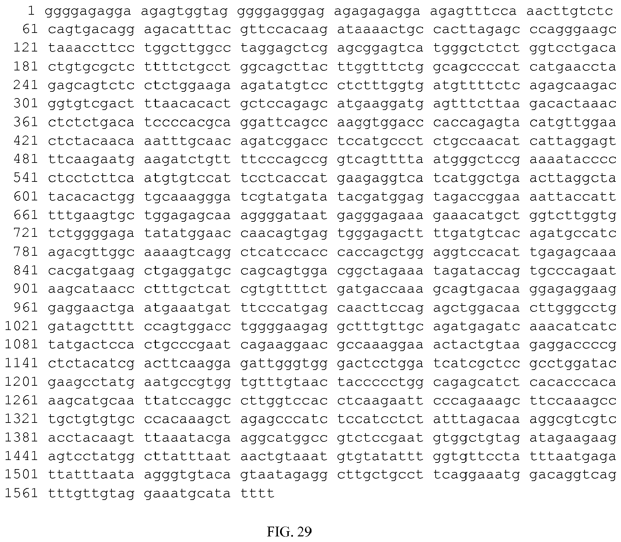

Figure 29 depicts the human BMP10 cDNA sequence (NCBI Reference Sequence No. NM_014482.1) (SEQ ID NO: 63).

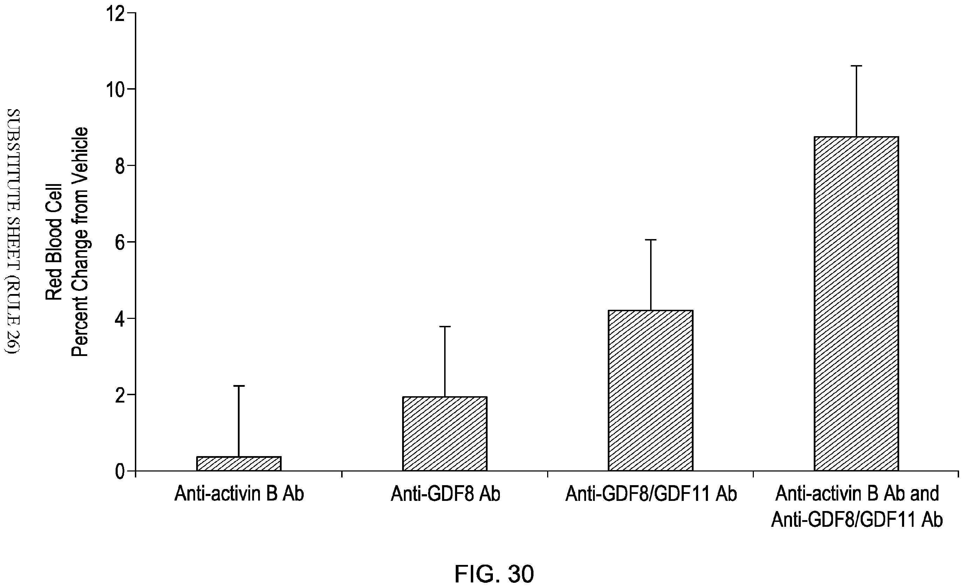

Figure 30 shows the effect of treatment with an anti-activin B antibody (Ab), an anti-GDF8 Ab, a bispecific anti-GDF8/GDF1 1 Ab, or a combination of an anti-activin B Ab and a bispecific anti-GDF8/GDF11 Ab on red blood cell levels in C57BL6 mice (n = 5 mice per group). Data is shown as the percent increase in red blood cell levels over that observed in vehicle (PBS) treated subjects.

### DETAILED DESCRIPTION OF THE INVENTION

### 1. Overview

The transforming growth factor-beta (TGF-β) superfamily contains a variety of growth factors that share common sequence elements and structural motifs. These proteins are known to exert biological effects on a large variety of cell types in both vertebrates and invertebrates. Members of the superfamily perform important functions during embryonic development in pattern formation and tissue specification and can influence a variety of differentiation processes, including adipogenesis, myogenesis, chondrogenesis, cardiogenesis, hematopoiesis, neurogenesis, and epithelial cell differentiation. By manipulating the activity of a member of the TGF-β family, it is often possible to cause significant physiological changes in an organism. For example, the Piedmontese and Belgian Blue cattle breeds carry a loss-of-function mutation in the GDF8 (also called myostatin) gene that causes a marked increase in muscle mass [see, *e.g.,* Grobet et al. (1997) Nat Genet. 17(1):71-4]. Furthermore, in humans, inactive alleles of GDF8 are associated with increased muscle mass and, reportedly, exceptional strength [see, *e.g.,* Schuelke et al. (2004) N Engl J Med, 350:2682-8.

TGF-β signals are mediated by heteromeric complexes of type I and type II serine/threonine kinase receptors, which phosphorylate and activate downstream Smad proteins *(e.g.,* Smad proteins 1, 2, 3, 5, and 8) upon ligand stimulation [see, *e.g.,* Massagué (2000) Nat. Rev. Mol. Cell Biol. 1: 169-178]. These type I and type II receptors are transmembrane proteins, composed of a ligand-binding extracellular domain with cysteinerich region, a transmembrane domain, and a cytoplasmic domain with predicted serine/threonine specificity. Type I receptors are essential for signaling. Type II receptors are required for binding ligands and for expression of type I receptors. Type I and II activin receptors form a stable complex after ligand binding, resulting in phosphorylation of type I receptors by type II receptors.

Activins are dimeric polypeptide growth factors belonging to the TGF-β superfamily. There are three principal activin forms (A, B, and AB) that are homo/heterodimers of two closely related β subunits (β_{A}β_{A}, β_{B}β_{B}, and β_{A}β_{B}, respectively). The human genome also encodes an activin C and an activin E, which are primarily expressed in the liver, and heterodimeric forms containing β_{C} or β_{E} are also known.

Two related type II receptors for activins have been identified, ActRIIA (encoded by the *ACVR2A* gene) and ActRIIB (encoded by the *ACVR2B* gene) [see, *e.g.,* Mathews and Vale (1991) Cell 65:973-982; and Attisano et al. (1992) Cell 68: 97-108]. Besides activins, ActRIIA and ActRIIB can interact biochemically with several other TGF-β family proteins including, for example, BMP7, Nodal, GDF8, and GDF11 [see, *e.g.,* Yamashita et al. (1995) J. Cell Biol. 130:217-226; Lee and McPherron (2001) Proc. Natl. Acad. Sci. 98:9306-9311; Yeo and Whitman (2001) Mol. Cell 7: 949-957; and Oh et al. (2002) Genes Dev. 16:2749-54]. Activin-like kinase-4 (ALK4) is the primary type I receptor for activins, particularly for activin A, and ALK7 may serve as a receptor for other activins as well, particularly for activin B. In certain embodiments, the present disclosure relates to antagonizing a ligand of an ActRIIA or ActRIIB receptor (also referred to as an ActRIIA ligand or an ActRIIB ligand) with one or more agents disclosed herein, particularly agents that can antagonize GDF11 and/or activin B.

As described herein, agents that bind to "activin B" are agents that specifically bind to the β_{B} subunit, whether in the context of an isolated β_{B} subunit or as a dimeric complex (e.g., a β_{B}β_{B} homodimer or a β_{A}β_{B} heterodimer). In the case of a heterodimer complex (e.g., a β_{A}β_{B} heterodimer), agents that bind to "activin B" are specific for epitopes present within the β_{B} subunit, but do not bind to epitopes present within the non-β_{B} subunit of the complex (e.g., the β_{A} subunit of the complex). Similarly, agents disclosed herein that antagonize (inhibit) "activin B" are agents that inhibit one or more activities as mediated by a β_{B} subunit, whether in the context of an isolated β_{B} subunit or as a dimeric complex (e.g., a β_{B}β_{B} homodimer or a β_{A}β_{B} heterodimer). In the case of β_{A}β_{B} heterodimers, agents that inhibit "activin B" are agents that specifically inhibit one or more activities of the β_{B} subunit, but do not inhibit the activity of the non-β_{B} subunit of the complex (e.g., the β_{A} subunit of the complex). This principle applies also to agents that *bind to* and/or *inhibit* "activin A", "activin C", and "activin E".

In the TGF-β superfamily, activins are unique and multifunctional factors that can stimulate hormone production in ovarian and placental cells, support neuronal cell survival, influence cell-cycle progress positively or negatively depending on cell type, and induce mesodermal differentiation at least in amphibian embryos [DePaolo et al. (1991) Proc Soc Ep Biol Med. 198:500-512; Dyson et al. (1997) Curr Biol. 7:81-84; and Woodruff (1998) Biochem Pharmacol. 55:953-963]. Moreover, an erythroid differentiation factor (EDF) isolated from the stimulated human monocytic leukemic cells was found to be identical to activin A [Murata et al. (1988) PNAS, 85:2434]. It has been suggested that activin A promotes erythropoiesis in the bone marrow. In several tissues, activin signaling is antagonized by its related heterodimer, inhibin. For example, during the release of follicle-stimulating hormone (FSH) from the pituitary, activin promotes FSH secretion and synthesis, whereas inhibin prevents FSH secretion and synthesis. Other proteins that may regulate activin bioactivity and/or bind to activin include follistatin (FS), follistatin-related protein (FSRP), and α₂-macroglobulin.

Growth and differentiation factor-8 (GDF8) is also known as myostatin. GDF8 is a negative regulator of skeletal muscle mass. GDF8 is highly expressed in developing and adult skeletal muscle. GDF8 gene deletion in mice is characterized by a marked hypertrophy and hyperplasia of the skeletal muscle [McPherron et al., Nature (1997) 387:83-90]. Similar increases in skeletal muscle mass are evident in naturally occurring mutations of GDF8 in cattle [see, *e.g.,* Ashmore et al. (1974) Growth, 38:501-507; Swatland and Kieffer (1994) J. Anim. Sci. 38:752-757; McPherron and Lee (1997) Proc. Natl. Acad. Sci. USA 94:12457-12461; and Kambadur et al. (1997) Genome Res. 7:910-915] and, strikingly, in humans [see, *e.g.,* Schuelke et al. (2004) N Engl J Med 350:2682-8]. Studies have also shown that muscle wasting associated with HIV-infection in humans is accompanied by increases in GDF8 protein expression [see, *e.g.,* Gonzalez-Cadavid et al. (1998) PNAS 95:14938-43]. In addition, GDF8 can modulate the production of muscle-specific enzymes (e.g., creatine kinase) and modulate myoblast cell proliferation [see, e.g. international patent application publication no. WO 00/43781]. The GDF8 propeptide can noncovalently bind to the mature GDF8 domain dimer, inactivating its biological activity [see, *e.g.,* Miyazono et al. (1988) J. Biol. Chem., 263: 6407-6415; Wakefield et al. (1988) J. Biol. Chem., 263: 7646-7654; and Brown et al. (1990) Growth Factors, 3: 35-43]. Other proteins which bind to GDF8 or structurally related proteins and inhibit their biological activity include follistatin, and potentially, follistatin-related proteins [see, *e.g.,* Gamer et al. (1999) Dev. Biol., 208: 222-232].

Growth and differentiation factor-11 (GDF11), also known as bone morphogenetic protein-11 (BMP11), is a secreted protein first identified as a regulator of vertebrate development [McPherron et al. (1999) Nat. Genet. 22: 260-264]. GDF11 is expressed in the tail bud, limb bud, maxillary and mandibular arches, and dorsal root ganglia during mouse embryonic development [see, *e.g.,* Nakashima et al. (1999) Mech. Dev. 80: 185-189]. GDF11 plays a unique role in patterning both mesodermal and neural tissues [see, *e.g.,* Gamer et al. (1999) Dev Biol., 208:222-32] and was shown to be a negative regulator of chondrogenesis and myogenesis in the developing chick limb [see, *e.g.,* Gamer et al. (2001) Dev Biol. 229:407-20]. GDF11 is also implicated as a regulator of tissue homeostasis postnatally. For example, expression of GDF11 in muscle also suggests a role for this ligand in regulating muscle growth in a manner similar to that of GDF8. In addition, expression of GDF11 in brain suggests that GDF11 may also regulate nervous system function, and GDF11 has been found to inhibit neurogenesis in the olfactory epithelium [see, *e.g.,* Wu et al. (2003) Neuron. 37:197-207].

Bone morphogenetic protein (BMP7), also called osteogenic protein-1 (OP-1), is well known to induce cartilage and bone formation. In addition, BMP7 regulates a wide array of physiological processes. For example, BMP7 may be the osteoinductive factor responsible for the phenomenon of epithelial osteogenesis. BMP7 also plays a role in calcium regulation and bone homeostasis. Like activin, BMP7 binds to ActRIIA and ActRIIB. However, BMP7 and activin recruit distinct type I receptors into heteromeric receptor complexes. Whereas BMP7 signals preferentially through ALK2, activin signals through ALK4. This difference allows BMP7 and activin to activate different Smad pathways and elicit distinct biological responses [see, *e.g.,* Macias-Silva et al. (1998) J Biol Chem. 273:25628-36].

It has been previously reported that special biological properties are exhibited *in vitro* and *in vivo* by variant ActRIIB-Fc fusion proteins, one variant comprising amino acids 20-134 of instant SEQ ID NO:1 with an acidic amino acid at position 79 with respect to SEQ ID NO:1 [referenced hereafter as the "ActRIIB(L79D 20-134)-Fc" fusion protein] and a second, truncated variant comprising amino acids 25-131 of instant SEQ ID NO: 1 and also incorporating an acidic amino acid at position 79 [referenced hereafter as "ActRIIB(L79D 25-131)-Fc"]. See, *e.g.,* U.S. Patent No. 8,058,229. In comparison to the unmodified fusion proteins ActRIIB(20-134)-Fc and ActRIIB(25-131)-Fc, the corresponding L79D variants [ActRIIB(L79D 20-134)-Fc and ActRIIB(L79D 25-131)-Fc, respectively] are characterized, in part, by substantial loss of binding affinity for activin A, and therefore significantly diminished capacity to antagonize activin A activity, but retain near wild-type levels of binding and inhibition of GDF11. The ActRIIB(L79D 20-134)-Fc and ActRIIB(L79D 25-131)-Fc variants were found to be significantly more potent in the capacity to increase red blood cell levels in vivo in comparison to the unmodified ActRIIB(20-134)-Fc and ActRIIB(25-131)-Fc fusion proteins, respectively. These data therefore indicate that the observed biological activity is not dependent on activin A inhibition.

The instant application is directed, in part, to the insight that the ActRIIB(L79D 20-134)-Fc and ActRIIB(L9D 25-131)-Fc variants disclosed in U.S. Patent No. 8,058,229, while having significantly reduced binding affinity for activin A, are capable of inhibiting activin B as well as GDF11. Therefore, Applicants conclude herein that erythropoiesis can be increased by an agent, or combination of agents, that antagonizes both GDF11 and activin B activity. Although inhibition of activin A is not necessary to promote red blood cell formation, it is known that an ActRII-Fc fusion protein that inhibits activin A also promotes red blood cell formation. Therefore, agents that inhibit activin A are included in the scope of the present disclosure.

As demonstrated herein, multiple ActRII ligands (e.g., activin B, GDF11, and GDF8) appear to be regulators erythropoiesis as there is a trend toward increased levels of various blood parameters (e.g., hematocrit, hemoglobin, and red blood cell levels) as more of these ligands are inhibited. For example, the data presented herein demonstrate that administration of an agent that inhibits GDF8 and GDF11 activity has a more substantial effect on increasing red blood cell levels *in vivo* compared to an agent that only antagonizes GDF8. In addition, it is shown that combination therapy using agents that inhibit activin B, GDF8, and GDF11 have even a greater effect on increasing red blood cells compared to treatment with a GDF8/GDF11 antagonist. Accordingly, the data presented herein indicates that an effective strategy for promoting erythropoiesis in a subject is to target multiple (*i.e.,* two or more) ActRII ligands.

Accordingly, the present disclosure provides, in part, methods for increasing red blood cell levels, treating or preventing anemia, and/or treating or preventing ineffective erythropoiesis in a subject in need thereof with an agent, or combination of agents, that antagonizes (inhibits) GDF11 *(e.g.,* GDF11-mediated activation of Smad2/3 signaling through ActRIIA and/or ActRIIB) and/or activin B (e.g., activin B-mediated activation of Smad2/3 signaling through ActRIIA and/or ActRIIB). Optionally, an agent, or combination of agents, of the disclosure that antagonizes GDF11 and/or activin B does not substantially antagonizes activin A (e.g., activin A-mediated activation of Smad2/3 signaling through ActRIIA and/or ActRIIB). Optionally an agent, or combination of agents, of the disclosure that antagonizes GDF11 and/or activin B may further inhibit GDF8 activity. In certain embodiments, an agent, or combination of agents, of the disclosure that antagonizes GDF11 and/or activin B may further inhibit one or more of GDF8, BMP6, activin C, activin E, activin A, GDF15, Nodal, GDF3, BMP3, BMP3B, BMP9, and BMP10.

In some embodiments, an agent, or combination of agents, that inhibits GDF11 and/or activin B activity are agents that directly bind to GDF11 and/or activin B, including for example: a multispecific antibody (e.g., bispecific antibody) that binds to at least GDF11 and activin B (optionally such an agent, or combination of agents, does not substantially bind to activin A); a combination of antibodies comprising an anti-GDF11 antibody and an anti-activin B antibody; a variant ActRII polypeptide *(e.g.,* a variant ActRIIA or ActRIIB polypeptide) that binds to GDF11 and activin B (optionally may not bind to activin A); a combination of variant ActRII polypeptides (e.g., variant ActRIIA or ActRIIB polypeptides) comprising at least one variant ActRII polypeptide that binds to GDF11 (but does not substantially activin B) and at least one soluble, variant ActRII polypeptide that binds to activin B (but does not substantially bind to GDF11) (optionally one or both of the GDF- and activin B-binding ActRII polypeptides may not substantially bind to activin A); a small molecule that directly binds to GDF11 and/or activin B (optionally may not substantially bind to activin A); and a combination of small molecules comprising at least one small molecule that binds to GOF11 (but does not substantially bind to activin B) and one small molecule that binds to activin B (but does not substantially bind to GDF11) (one or both of the GDF- and activin B-binding small molecules may not substantially bind to activin A).

In alternative embodiments, an agent, or combination agents, that inhibit GDF11 and/or activin B activity are indirect antagonist agents that do not directly bind to GDF11 and/or activin B. For example, an indirect antagonist agent may be an antibody that binds to a native ActRII receptor *(e.g.,* an ActRIIA or ActRIIB receptor) and prevents GDF11 and/or activin B from binding to and/or activating the ActRII receptor. Optionally, such an agent, or combination of agents, does not substantially inhibit activin A from binding to and/or activating the ActRII receptor. Other indirect antagonist agents of the present disclosure include an agent, or combination of agents, that inhibit the expression (e.g., transcription, translation, and/or cellular secretion) of GDF11 and/or activin B. Optionally such an agent, or combination of agents, do not substantially affect the expression of activin A. Such indirect agents include, for example, small-molecule inhibitors of GDF11 and/or activin B expression as well as the use of various polynucleotide antagonists [e.g. antisense DNA, RNA or chemical analogues, and interfering RNA molecules including small interfering RNA (siRNA), small hairpin RNA (shRNA) or microRNA (miRNA) molecules, targeted to GDF11 and/or activin B mRNA] to inhibit GDF11 and/or activin B expression.

In some embodiments, an agent, or combination agents, of the disclosure that inhibit GDF11 and/or activin B activity optionally bind to and/or inhibit the activity of one or more of: GDF8, activin A, activin C, activin E, activin A, BMP6, GDF15, Nodal, GDF3, BMP3, BMP3B, BMP9, and BMP10.

Additionally, methods of the present disclosure are directed to the use of one or more antagonist agents described herein *(e.g.,* an agent or combination of agents that inhibit GDF11 and activin B) in combination with an EPO receptor activator to increase red blood cell formation as well as to treat or prevent various anemias and ineffective erythropoiesis disorders and associated conditions. It should be noted that hematopoiesis is a complex process, regulated by a variety of factors, including erythropoietin, G-CSF and iron homeostasis. The terms "increase red blood cell levels" and "promote red blood cell formation" refer to clinically observable metrics, such as hematocrit, red blood cell counts, and hemoglobin measurements, and are intended to be neutral as to the mechanism by which such changes occur.

EPO is a glycoprotein hormone involved in the growth and maturation of erythroid progenitor cells into erythrocytes. EPO is produced by the liver during fetal life and by the kidney in adults. Decreased production of EPO, which commonly occurs in adults as a consequence of renal failure, leads to anemia. EPO has been produced by genetic engineering techniques based on expression and secretion of the protein from a host cell transfected with the EPO gene. Administration of such recombinant EPO has been effective in the treatment of anemia. For example, Eschbach et al. (1987, N Engl J Med 316:73) describe the use of EPO to correct anemia caused by chronic renal failure.

Effects of EPO are mediated through its binding to, and activation of, a cell surface receptor belonging to the cytokine receptor superfamily and designated the EPO receptor. The human and murine EPO receptors have been cloned and expressed [see, *e.g.,* D'Andrea et al. (1989) Cell 57:277; Jones et al. (1990) Blood 76:31; Winkelman et al. (1990) Blood 76:24; and U.S. Pat. No. 5,278,065]. The human EPO receptor gene encodes a 483 amino acid transmembrane protein comprising an extracellular domain of approximately 224 amino acids and exhibits approximately 82% amino acid sequence identity with the murine EPO receptor [see, *e.g.,* U.S. Pat. No. 6,319,499. The cloned, full-length EPO receptor expressed in mammalian cells (66-72 kDa) binds EPO with an affinity (K_{D} = 100-300 nM) similar to that of the native receptor on erythroid progenitor cells. Thus, this form is thought to contain the main EPO binding determinant and is referred to as the EPO receptor. By analogy with other closely related cytokine receptors, the EPO receptor is thought to dimerize upon agonist binding. Nevertheless, the detailed structure of the EPO receptor, which may be a multimeric complex, and its specific mechanism of activation are not completely understood [see, *e.g.,* U.S. Pat. No. 6,319,499].

Activation of the EPO receptor results in several biological effects. These include increased proliferation of immature erythroblasts, increased differentiation of immature erythroblasts, and decreased apoptosis in erythroid progenitor cells [see, *e.g.,* Liboi et al. (1993) Proc Natl Acad Sci USA 90:11351-11355; Koury et al. (1990) Science 248:378-381]. The EPO receptor signal transduction pathways mediating proliferation and differentiation appear to be distinct [see, *e.g.,* Noguchi et al. (1988) Mol Cell Biol 8:2604; Patel et al. (1992) J Biol Chem, 267:21300; and Liboi et al. (1993) Proc Natl Acad Sci USA 90:11351-11355)]. Some results suggest that an accessory protein may be required for mediation of the differentiation signal [see, *e.g.,* Chiba et al. (1993) Nature 362:646; and Chiba et al. (1993) Proc Natl Acad Sci USA 90:11593]. However, there is controversy regarding the role of accessory proteins in differentiation since a constitutively activated form of the receptor can stimulate both proliferation and differentiation [see, *e.g.,* Pharr et al. (1993) Proc Natl Acad Sci USA 90:938].

EPO receptor activators include small-molecule erythropoiesis-stimulating agents (ESAs) as well as EPO-based compounds. An example of the former is a dimeric peptide-based agonist covalently linked to polyethylene glycol (proprietary name Hematide), which has shown erythropoiesis-stimulating properties in healthy volunteers and in patients with both chronic kidney disease and endogenous anti-EPO antibodies [see, *e.g.,* Stead et al. (2006) Blood 108:1830-1834; and Macdougall et al. (2009) N Engl J Med 361:1848-1855]. Other examples include nonpeptide-based ESAs [see, *e.g.,* Qureshi et al. (1999) Proc Natl Acad Sci USA 96:12156-12161].

EPO receptor activators also include compounds that stimulate erythropoiesis indirectly, without contacting EPO receptor itself, by enhancing production of endogenous EPO. For example, hypoxia-inducible transcription factors (HIFs) are endogenous stimulators of EPO gene expression that are suppressed (destabilized) under normoxic conditions by cellular regulatory mechanisms. Therefore, inhibitors of HIF prolyl hydroxylase enzymes are being investigated for EPO-inducing activity *in vivo.* Other indirect activators of EPO receptor include inhibitors of GATA-2 transcription factor [see, *e.g.,* Nakano et al. (2004) Blood 104:4300-4307], which tonically inhibits EPO gene expression, and inhibitors of hemopoietic cell phosphatase (HCP or SHP-1), which functions as a negative regulator of EPO receptor signal transduction [see, *e.g.,* Klingmuller et al. (1995) Cell 80:729-738].

The terms used in this specification generally have their ordinary meanings in the art, within the context of this disclosure and in the specific context where each term is used. Certain terms are discussed below or elsewhere in the specification, to provide additional guidance to the practitioner in describing the compositions and methods of the disclosure and how to make and use them. The scope or meaning of any use of a term will be apparent from the specific context in w

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions.

The term "sequence similarity," in all its grammatical forms, refers to the degree of identity or correspondence between nucleic acid or amino acid sequences that may or may not share a common evolutionary origin.

However, in common usage and in the instant application, the term "homologous," when modified with an adverb such as "highly," may refer to sequence similarity and may or may not relate to a common evolutionary origin.

"Percent (%)sequence identity" with respect to a reference polypeptide (or nucleotide) sequence is defined as the percentage of amino acid residues (or nucleic acids) in a candidate sequence that are identical with the amino acid residues (or nucleic acids) in the reference polypeptide (nucleotide) sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid (nucleic acid) sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, Calif., or may be compiled from the source code. The ALIGN-2 program should be compiled for use on a UNIX operating system, including digital UNIX V4.0D. All sequence comparison parameters are set by the ALIGN-2 program and do not vary.

As used herein "does not substantially bind to X" is intended to mean that an agent has a K_{D} that is greater than about 10⁻⁷, 10⁻⁶, 10⁻⁵, 10⁻⁴ or greater *(e.g.,* no detectable binding by the assay used to determine the K_{D}) for "X".

### 2. Antagonist Agents

### A. Antibody Antagonists

In certain aspects, antagonist agents, or combinations of agents, of the present disclosure are antibodies that bind to and/or inhibit the activity of at least activin B and/or GDF11 (e.g., activation of ActRIIA- or ActRIIB-based Smad 2/3 signaling). Optionally, an antibody, or combination of antibodies, of the disclosure does not bind to and/or inhibit the activity of activin A (e.g., activin A-mediated activation of ActRIIA- or ActRIIB-based Smad 2/3 signaling). Optionally, an antibody, or combination of antibodies, of the present disclosure further binds to and/or inhibits the activity of GDF8 (e.g., GDF8-mediated activation of ActRIIA or ActRIIB Smad 2/3 signaling). In some embodiments, an antibody, or combination of antibodies, of the disclosure that binds to and/or inhibits the activity of at least activin B and/or GDF11 further binds to and/or inhibits the activity of one of more of GDF8, activin C, activin E, BMP6, activin A, GDF15, Nodal, GDf3, BMP3, BMP3B, BMP9, or BMP10 (e.g., activation of ActRIIA- or ActRIIB-based Smad 2/3 and/or Smad 1/5/8 signaling).

In another aspect, an antibody, or combination of antibodies, of the present disclosure is an anti-ActRII receptor antibody (*e.g*. an ActRIIA or ActRIIB receptor antibody) that binds to an ActRII receptor and prevents binding and/or activation of the ActRII receptor by at least activin B and/or GDF11. Optionally, an anti-ActRII receptor antibody, or combination of antibodies, of the disclosure does not substantially inhibit activin A from binding to and/or activating an ActRII receptor. Optionally, an anti-ActRII receptor antibody, or combination of antibodies, of the disclosure further prevents binding and/or activation of the ActRII receptor by GDF8. In some embodiments, an anti-ActRII receptor antibody, or combination of antibodies, of the disclosure that binds to an ActRII receptor and prevents binding and/or activation of the ActRII receptor by activin B and/or GDF11 further prevents binding and/or activation of the ActRII receptor by one or more of activin A, activin C, activin E, GDF8, BMP6, GDF15, Nodal, GDF3, BMP3, BMP3B, BMP9 and BMP10.

The term antibody is used herein in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. An antibody fragment refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multispecific antibodies formed from antibody fragments [see, *e.g.,* Hudson et al. (2003) Nat. Med. 9:129-134; Plückthun, in The Pharmacology of Monoclonal Antibodies, vol. 113, Rosenburg and Moore eds., (Springer-Verlag, New York), pp. 269-315 (1994); WO 93/16185; and U.S. Pat. Nos. 5,571,894; 5,587,458; and 5,869,046]. Antibodies disclosed herein may be polyclonal antibodies or monoclonal antibodies. In certain embodiments, the antibodies of the present disclosure comprise a label attached thereto and able to be detected *(e.g.,* the label can be a radioisotope, fluorescent compound, enzyme, or enzyme co-factor). In preferred embodiments, the antibodies of the present disclosure are isolated antibodies.

Diabodies are antibody fragments with two antigen-binding sites that may be bivalent or bispecific [see, *e.g.,* EP 404,097; WO 1993/01161; Hudson et al. (2003) Nat. Med. 9:129-134 (2003); and Hollinger et al. (1993) Proc. Natl. Acad. Sci. USA 90: 6444-6448]. Triabodies and tetrabodies are also described in Hudson et al. (2003) Nat. Med. 9:129-134.

Single-domain antibodies are antibody fragments comprising all or a portion of the heavy-chain variable domain or all or a portion of the light-chain variable domain of an antibody. In certain embodiments, a single-domain antibody is a human single-domain antibody [see, *e.g.,* U.S. Pat. No. 6,248,516].

Antibody fragments can be made by various techniques, including but not limited to proteolytic digestion of an intact antibody as well as production by recombinant host cells (e.g., E. coli or phage), as described herein.

The antibodies herein may be of any class. The class of an antibody refers to the type of constant domain or constant region possessed by its heavy chain. There are five major classes of antibodies: IgA, IgD, IgE, IgG, and IgM, and several of these may be further divided into subclasses (isotypes), for example, IgG₁, IgG₂, IgG₃, IgG₄, IgA₁, and IgA₂. The heavy chain constant domains that correspond to the different classes of immunoglobulins are called alpha, delta, epsilon, gamma, and mu.

In general, an antibody for use in the methods disclosed herein specifically binds to its target antigen, preferably with high binding affinity. Affinity may be expressed as a K_{D} value and reflects the intrinsic binding affinity (e.g., with minimized avidity effects). Typically, binding affinity is measured *in vitro,* whether in a cell-free or cell-associated setting. Any of a number of assays known in the art, including those disclosed herein, can be used to obtain binding affinity measurements including, for example, Biacore, radiolabeled antigen-binding assay (RIA), and ELISA. In some embodiments, antibodies of the present disclosure bind to their target antigens (*e.g*. GDF11, activin B, GDF8, activin E, activin C, BMP6, GDF15, Nodal, GDF3, BMP3, BMP3B, BMP9, or BMP10) with at least a K_{D} of 1× 10⁻⁷ or stronger, 1×10⁻⁸ or stronger, 1×10⁻⁹ or stronger, 1×10⁻¹⁰ or stronger, 1×10⁻¹¹ or stronger, 1×10⁻¹² or stronger, 1×10⁻¹³ or stronger, or 1×10⁻¹⁴ or stronger.

In certain embodiments, K_{D} is measured by RIA performed with the Fab version of an antibody of interest and its target antigen as described by the following assay. Solution binding affinity of Fabs for the antigen is measured by equilibrating Fab with a minimal concentration of radiolabeled antigen (e.g., ¹²⁵I-labeled) in the presence of a titration series of unlabeled antigen, then capturing bound antigen with an anti-Fab antibody-coated plate [see, *e.g.,* Chen et al. (1999) J. Mol. Biol. 293:865-881]. To establish conditions for the assay, multi-well plates (*e.g.,* MICROTITER^{®} from Thermo Scientific) are coated *(e.g.,* overnight) with a capturing anti-Fab antibody (e.g., from Cappel Labs) and subsequently blocked with bovine serum albumin, preferably at room temperature (approximately 23°C). In a non-adsorbent plate, radiolabeled antigen are mixed with serial dilutions of a Fab of interest [e.g., consistent with assessment of the anti-VEGF antibody, Fab-12, in Presta et al., (1997) Cancer Res. 57:4593-4599], The Fab of interest is then incubated, preferably overnight but the incubation may continue for a longer period (e.g., about 65 hours) to ensure that equilibrium is reached. Thereafter, the mixtures are transferred to the capture plate for incubation, preferably at room temperature for about one hour. The solution is then removed and the plate is washed times several times, preferably with polysorbate 20 and PBS mixture. When the plates have dried, scintillant (e.g., MICROSCINT^{®} from Packard) is added, and the plates are counted on a gamma counter (e.g., TOPCOUNT^{®} from Packard).

According to another embodiment, K_{D} is measured using surface plasmon resonance assays using, for example a BIACORE^{®} 2000 or a BIACORE^{®} 3000 (BIAcore, Inc., Piscataway, N.J.) with immobilized antigen CM5 chips at about 10 response units (RU). Briefly, carboxymethylated dextran biosensor chips (CM5, BIACORE, Inc.) are activated with N-ethyl-N'-(3-dimethylaminopropyl)-carbodiimide hydrochloride (EDC) and N-hydroxysuccinimide (NHS) according to the supplier's instructions. For example, an antigen can be diluted with 10 mM sodium acetate, pH 4.8, to 5 µg/ml (about 0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{®}) surfactant (PBST) at at a flow rate of approximately 25 µl/min. Association rates (kₒₙ) and dissociation rates (k_{off}) are calculated using, for example, a simple one-to-one Langmuir binding model (BIACORE^{®} Evaluation Software version 3.2) by simultaneously fitting the association and dissociation sensorgrams. The equilibrium dissociation constant (K_{D}) is calculated as the ratio k_{off} / kₒₙ [see, *e.g.,* Chen et al., (1999) J. Mol. Biol. 293:865-881]. If the on-rate exceeds, for example, 10⁶ M⁻¹ s⁻¹ by the surface plasmon resonance assay above, then the on-rate can be determined by using a fluorescent quenching technique that measures the increase or decrease in fluorescence emission intensity (e.g., excitation=295 nm; emission=340 nm, 16 nm bandpass) of a 20 nM anti-antigen antibody (Fab form) in PBS in the presence of increasing concentrations of antigen as measured in a spectrometer, such as a stop-flow equipped spectrophometer (Aviv Instruments) or a 8000-series SLM-AMINCO^{®} spectrophotometer (ThermoSpectronic) with a stirred cuvette.

In general, an anti-GDF11 antibody refers to an antibody that is capable of binding to GDF11 with sufficient affinity such that the antibody is useful as a diagnostic and/or therapeutic agent in targeting GDF11. In certain embodiments, the extent of binding of an anti-GDF11 antibody to an unrelated, non-GDF11 protein is less than about 10%, 9%, 8%, 7%, 6%, 5%, 4%, 3%, 2%, or less than 1% of the binding of the antibody to GDF11 as measured, for example, by a radioimmunoassay (RIA). In certain embodiments, an anti-GDF11 antibody binds to an epitope of GDF11 that is conserved among orthologous GDF11 proteins from different species. In preferred embodiments, an anti-GDF11 antibody of the present disclosure is an antagonist antibody that can substantially inhibit GDF11 activity. For example, an anti-GDF11 antibody of the disclosure may substantially inhibit GDF11 from binding to a cognate receptor (e.g., ActRIIA or ActRIIB receptor) and/or substantially inhibit GDF11-mediated signal transduction (activation) of a cognate receptor, such as Smad2/3 signaling by ActRIIA and/or ActRIIB receptors. In some embodiments, anti-GDF11 antibodies of the present disclosure do not substantially bind to and/or inhibit activity of activin A. It should be noted that GOF11 has high sequence identity with GDF8 on the amino acid level and therefore antibodies that bind and/or to GDF11, in many cases, may also bind to and/or inhibit GDF8.

In general, an anti-activin B antibody refers to an antibody that is capable of binding to activin B with sufficient affinity such that the antibody is useful as a diagnostic and/or therapeutic agent in targeting activin B. In certain embodiments, the extent of binding of an anti-activin B antibody to an unrelated, non-activin B protein is less than about 10%, 9%, 8%, 7%, 6%, 5%, 4%, 3%, 2%, or less than 1% of the binding of the antibody to activin B as measured, for example, by a radioimmunoassay (RIA). In certain embodiments, an anti-activin B antibody binds to an epitope of activin B that is conserved among orthologous activin B proteins from different species. In preferred embodiments, an anti-activin B antibody of the present disclosure is an antagonist antibody that can substantially inhibit activin B activity. For example, an anti-activin B antibody of the disclosure may substantially inhibit activin B from binding to a cognate receptor (e.g., ActRIIA or ActRIIB receptor) and/or substantially inhibit activin B-mediated signal transduction (activation) of a cognate receptor, such as Smad2/3 signaling by ActRIIA and/or ActRIIB receptors. In some embodiments, anti-activin B antibodies of the present disclosure do not substantially bind to and/or inhibit activity of activin A.

In general, an anti-activin C antibody refers to an antibody that is capable of binding to activin C with sufficient affinity such that the antibody is useful as a diagnostic and/or therapeutic agent in targeting activin C. In certain embodiments, the extent of binding of an anti-activin C antibody to an unrelated, non-activin C protein is less than about 10%, 9%, 8%, 7%, 6%, 5%, 4%, 3%, 2%, or less than 1% of the binding of the antibody to activin C as measured, for example, by a radioimmunoassay (RIA). In certain embodiments, an anti-activin C antibody binds to an epitope of activin C that is conserved among orthologous activin C proteins from different species. In preferred embodiments, an anti-activin C antibody of the present disclosure is an antagonist antibody that can substantially inhibit activin C activity. For example, an anti-activin C antibody of the disclosure may substantially inhibit activin C from binding to a cognate receptor (e.g., ActRIIA or ActRIIB receptor) and/or substantially inhibit activin C-mediated signal transduction (activation) of a cognate receptor, such as Smad2/3 signaling by ActRIIA and/or ActRIIB receptors. In some embodiments, anti-activin C antibodies of the present disclosure do not substantially bind to and/or inhibit activity of activin A.

In general, an anti-activin A antibody refers to an antibody that is capable of binding to activin A with sufficient affinity such that the antibody is useful as a diagnostic and/or therapeutic agent in targeting activin A. In certain embodiments, the extent of binding of an anti-activin A antibody to an unrelated, non-activin A protein is less than about 10%, 9%, 8%, 7%, 6%, 5%, 4%, 3%, 2%, or less than 1% of the binding of the antibody to activin A as measured, for example, by a radioimmunoassay (RIA). In certain embodiments, an anti-activin A antibody binds to an epitope of activin A that is conserved among orthologous activin A proteins from different species. In preferred embodiments, an anti-activin A antibody of the present disclosure is an antagonist antibody that can substantially inhibit activin A activity. For example, an anti-activin A antibody of the disclosure may substantially inhibit activin A from binding to a cognate receptor (e.g., ActRIIA or ActRIIB receptor) and/or substantially inhibit activin A-mediated signal transduction (activation) of a cognate receptor, such as Smad2/3 signaling by ActRIIA and/or ActRIIB receptors.

In general, an anti-activin E antibody refers to an antibody that is capable of binding to activin E with sufficient affinity such that the antibody is useful as a diagnostic and/or therapeutic agent in targeting activin E. In certain embodiments, the extent of binding of an anti-activin E antibody to an unrelated, non-activin E protein is less than about 10%, 9%, 8%, 7%, 6%, 5%, 4%, 3%, 2%, or less than 1% of the binding of the antibody to activin E as measured, for example, by a radioimmunoassay (RIA). In certain embodiments, an anti-activin E antibody binds to an epitope of activin E that is conserved among orthologous activin E proteins from different species. In preferred embodiments, an anti-activin E antibody of the present disclosure is an antagonist antibody that can substantially inhibit activin E activity. For example, an anti-activin E antibody of the disclosure may substantially inhibit activin E from binding to a cognate receptor (e.g., ActRIIA or ActRIIB receptor) and/or substantially inhibit activin E-mediated signal transduction (activation) of a cognate receptor, such as Smad2/3 signaling by ActRIIA and/or ActRIIB receptors. In some embodiments, anti-activin E antibodies of the present disclosure do not substantially bind to and/or inhibit activity of activin A.

In general, an anti-GDF8 antibody refers to an antibody that is capable of binding to GDF8 with sufficient affinity such that the antibody is useful as a diagnostic and/or therapeutic agent in targeting GDF8. In certain embodiments, the extent of binding of an anti-GDF8 antibody to an unrelated, non-GDF8 protein is less than about 10%, 9%, 8%, 7%, 6%, 5%, 4%, 3%, 2%, or less than 1% of the binding of the antibody to GDF8 as measured, for example, by a radioimmunoassay (RIA). In certain embodiments, an anti-GDF8 antibody binds to an epitope of GDF8 that is conserved among orthologous GDF8 proteins from different species. In preferred embodiments, an anti-GDF8 antibody of the present disclosure is an antagonist antibody that can substantially inhibit GDF8 activity. For example, an anti-GDF8 antibody of the disclosure may substantially inhibit GDF8 from binding to a cognate receptor (e.g., ActRIIA or ActRIIB receptor) and/or substantially inhibit GDF8-mediated signal transduction (activation) of a cognate receptor, such as Smad2/3 signaling by ActRIIA and/or ActRIIB receptors. In some embodiments, anti-GDF8 antibodies of the present disclosure do not substantially bind to and/or inhibit activity of activin A. It should be noted that GDF8 has high sequence homology to GDF11 and therefore antibodies that bind and/or to GDF8, in many cases, may also bind to and/or inhibit GDF11.

In general, an anti-BMP6 antibody refers to an antibody that is capable of binding to BMP6 with sufficient affinity such that the antibody is useful as a diagnostic and/or therapeutic agent in targeting BMP6. In certain embodiments, the extent of binding of an anti-BMP6 antibody to an unrelated, non-BMP6 protein is less than about 10%, 9%, 8%, 7%, 6%, 5%, 4%, 3%, 2%, or less than 1% of the binding of the antibody to BMP6 as measured, for example, by a radioimmunoassay (RIA). In certain embodiments, an anti-BMP6 antibody binds to an epitope of BMP6 that is conserved among orthologous BMP6 proteins from different species. In preferred embodiments, an anti-BMP6 antibody of the present disclosure is an antagonist antibody that can substantially inhibit BMP6 activity. For example, an anti-BMP6 antibody of the disclosure may substantially inhibit BMP6 from binding to a cognate receptor (e.g., ActRIIA or ActRIIB receptor) and/or substantially inhibit BMP6-mediated signal transduction (activation) of a cognate receptor, such as Smad2/3 signaling by ActRIIA and/or ActRIIB receptors. In some embodiments, anti- BMP6 antibodies of the present disclosure do not substantially bind to and/or inhibit activity of activin A.

In general, an anti-GDF15 antibody refers to an antibody that is capable of binding to GDF15 with sufficient affinity such that the antibody is useful as a diagnostic and/or therapeutic agent in targeting GDF15. In certain embodiments, the extent of binding of an anti-GDF15 antibody to an unrelated, non-GDF15 protein is less than about 10%, 9%, 8%, 7%, 6%, 5%, 4%, 3%, 2%, or less than 1% of the binding of the antibody to GDF15 as measured, for example, by a radioimmunoassay (RIA). In certain embodiments, an anti-GDF15 antibody binds to an epitope of GDF15 that is conserved among orthologous GDF15 proteins from different species. In preferred embodiments, an anti-GDF15 antibody of the present disclosure is an antagonist antibody that can substantially inhibit GDF15 activity. For example, an anti-GDF15 antibody of the disclosure may substantially inhibit GDF15 from binding to a cognate receptor (e.g., ActRIIA or ActRIIB receptor) and/or substantially inhibit GDF15-mediated signal transduction (activation) of a cognate receptor, such as Smad 2/3 signaling by ActRIIA and/or ActRIIB receptors. In some embodiments, anti-GDF15 antibodies of the present disclosure do not substantially bind to and/or inhibit activity of activin A.

In general, an anti-Nodal antibody refers to an antibody that is capable of binding to Nodal with sufficient affinity such that the antibody is useful as a diagnostic and/or therapeutic agent in targeting Nodal. In certain embodiments, the extent of binding of an anti-Nodal antibody to an unrelated, non-Nodal protein is less than about 10%, 9%, 8%, 7%, 6%, 5%, 4%, 3%, 2%, or less than 1% of the binding of the antibody to Nodal as measured, for example, by a radioimmunoassay (RIA). In certain embodiments, an anti-Nodal antibody binds to an epitope of Nodal that is conserved among orthologous Nodal proteins from different species. In preferred embodiments, an anti-Nodal antibody of the present disclosure is an antagonist antibody that can substantially inhibit Nodal activity. For example, an anti-Nodal antibody of the disclosure may substantially inhibit Nodal from binding to a cognate receptor (e.g., ActRIIA or ActRIIB receptor) and/or substantially inhibit Nodal-mediated signal transduction (activation) of a cognate receptor, such as Smad 2/3 signaling by ActRIIA and/or ActRIIB receptors. In some embodiments, anti-Nodal antibodies of the present disclosure do not substantially bind to and/or inhibit activity of activin A.

In general, an anti-GDF3 antibody refers to an antibody that is capable of binding to GDF3 with sufficient affinity such that the antibody is useful as a diagnostic and/or therapeutic agent in targeting GDF3. In certain embodiments, the extent of binding of an anti-GDF3 antibody to an unrelated, non-GDF3 protein is less than about 10%, 9%, 8%, 7%, 6%, 5%, 4%, 3%, 2%, or less than 1% of the binding of the antibody to GDF3 as measured, for example, by a radioimmunoassay (RIA). In certain embodiments, an anti-GDF3 antibody binds to an epitope of GDF3 that is conserved among orthologous GDF3 proteins from different species. In preferred embodiments, an anti-GDF3 antibody of the present disclosure is an antagonist antibody that can substantially inhibit GDF3 activity. For example, an anti-GDF3 antibody of the disclosure may substantially inhibit GDF3 from binding to a cognate receptor (e.g., ActRIIA or ActRIIB receptor) and/or substantially inhibit GDF3-mediated signal transduction (activation) of a cognate receptor, such as Smad 2/3 signaling by ActRIIA and/or ActRIIB receptors. In some embodiments, anti-GDF3 antibodies of the present disclosure do not substantially bind to and/or inhibit activity of activin A.

In general, an anti-BMP3 antibody refers to an antibody that is capable of binding to BMP3 with sufficient affinity such that the antibody is useful as a diagnostic and/or therapeutic agent in targeting BMP3. In certain embodiments, the extent of binding of an anti-BMP3 antibody to an unrelated, non-BMP3 protein is less than about 10%, 9%, 8%, 7%, 6%, 5%, 4%, 3%, 2%, or less than 1% of the binding of the antibody to BMP3 as measured, for example, by a radioimmunoassay (RIA). In certain embodiments, an anti-BMP3 antibody binds to an epitope of BMP3 that is conserved among orthologous BMP3 proteins from different species. In preferred embodiments, an anti-BMP3 antibody of the present disclosure is an antagonist antibody that can substantially inhibit BMP3 activity. For example, an anti-BMP3 antibody of the disclosure may substantially inhibit BMP3 from binding to a cognate receptor (e.g., ActRIIA or ActRIIB receptor) and/or substantially inhibit BMP3-mediated signal transduction (activation) of a cognate receptor, such as Smad 2/3 signaling by ActRIIA and/or ActRIIB receptors. In some embodiments, anti-BMP3 antibodies of the present disclosure do not substantially bind to and/or inhibit activity of activin A.

In general, an anti-BMP3B antibody refers to an antibody that is capable of binding to BMP3B with sufficient affinity such that the antibody is useful as a diagnostic and/or therapeutic agent in targeting BMP3B. In certain embodiments, the extent of binding of an anti-BMP3B antibody to an unrelated, non-BMP3B protein is less than about 10%, 9%, 8%, 7%, 6%, 5%, 4%, 3%, 2%, or less than 1% of the binding of the antibody to BMP3B as measured, for example, by a radioimmunoassay (RIA). In certain embodiments, an anti-BMP3B antibody binds to an epitope of BMP3B that is conserved among orthologous BMP3B proteins from different species. In preferred embodiments, an anti-BMP3B antibody of the present disclosure is an antagonist antibody that can substantially inhibit BMP3B activity. For example, an anti-BMP3B antibody of the disclosure may substantially inhibit BMP3B from binding to a cognate receptor (e.g., ActRIIA or ActRIIB receptor) and/or substantially inhibit BMP3B-mediated signal transduction (activation) of a cognate receptor, such as Smad 2/3 signaling by ActRIIA and/or ActRIIB receptors. In some embodiments, anti-BMP3B antibodies of the present disclosure do not substantially bind to and/or inhibit activity of activin A.

In general, an anti-BMP9 antibody refers to an antibody that is capable of binding to BMP9 with sufficient affinity such that the antibody is useful as a diagnostic and/or therapeutic agent in targeting BMP9. In certain embodiments, the extent of binding of an anti-BMP9 antibody to an unrelated, non-BMP9 protein is less than about 10%, 9%, 8%, 7%, 6%, 5%, 4%, 3%, 2%, or less than 1% of the binding of the antibody to BMP9 as measured, for example, by a radioimmunoassay (RIA). In certain embodiments, an anti-BMP9 antibody binds to an epitope of BMP9 that is conserved among orthologous BMP9 proteins from different species. In preferred embodiments, an anti-BMP9 antibody of the present disclosure is an antagonist antibody that can substantially inhibit BMP9 activity. For example, an anti-BMP9 antibody of the disclosure may substantially inhibit BMP9 from binding to a cognate receptor (e.g., ActRIIA or ActRIIB receptor) and/or substantially inhibit BMP9-mediated signal transduction (activation) of a cognate receptor, such as Smad 2/3 signaling by ActRIIA and/or ActRIIB receptors. In some embodiments, anti-BMP9 antibodies of the present disclosure do not substantially bind to and/or inhibit activity of activin A. In certain embodiments, anti-BMP9 antibodies inhibit the interaction between BMP9 and a type II receptor of the TGFβ superfamily (e.g., ActRIIA and/or ActRIIB). Preferably, anti-BMP9 antibodies do not inhibit, or substantially inhibit, interaction between BMP9 and ALK1.

In general, an anti-BMP 10 antibody refers to an antibody that is capable of binding to BMP10 with sufficient affinity such that the antibody is useful as a diagnostic and/or therapeutic agent in targeting BMP10. In certain embodiments, the extent of binding of an anti-BMP 10 antibody to an unrelated, non-BMP 10 protein is less than about 10%, 9%, 8%, 7%, 6%, 5%, 4%, 3%, 2%, or less than 1% of the binding of the antibody to BMP10 as measured, for example, by a radioimmunoassay (RIA). In certain embodiments, an anti-BMP10 antibody binds to an epitope of BMP10 that is conserved among orthologous BMP10 proteins from different species. In preferred embodiments, an anti-BMP10 antibody of the present disclosure is an antagonist antibody that can substantially inhibit BMP10 activity. For example, an anti-BMP10 antibody of the disclosure may substantially inhibit BMP10 from binding to a cognate receptor (e.g., ActRIIA or ActRIIB receptor) and/or substantially inhibit BMP10-mediated signal transduction (activation) of a cognate receptor, such as Smad 2/3 signaling by ActRIIA and/or ActRIIB receptors. In some embodiments, anti-BMP10 antibodies of the present disclosure do not substantially bind to and/or inhibit activity of activin A. In certain embodiments, anti-BMP10 antibodies inhibit the interaction between BMP10 and a type II receptor of the TGFβ superfamily (e.g., ActRIIA and/or ActRIIB). Preferably, anti-BMP 10 antibodies do not inhibit, or substantially inhibit, interaction between BMP10 and ALK1.

An anti-ActRIIA antibody refers to an antibody that is capable of binding to ActRIIA with sufficient affinity such that the antibody is useful as a diagnostic and/or therapeutic agent in targeting ActRIIA. In certain embodiments, the extent of binding of an anti-ActRIIA antibody to an unrelated, non-ActRIIA protein is less than about 10%, 9%, 8%, 7%, 6%, 5%, 4%, 3%, 2%, or less than 1% of the binding of the antibody to ActRIIA as measured, for example, by a radioimmunoassay (RIA). In certain embodiments, an anti-ActRIIA antibody binds to an epitope of ActRIIA that is conserved among orthologous ActRIIA proteins from different species. In preferred embodiments, an anti-ActRIIA antibody of the present disclosure is an antagonist antibody that can inhibit an ActRIIA activity. For example, an anti-ActRIIA antibody of the present disclosure may inhibit one or more ActRIIA ligands selected from activin B, activin C, activin E, GDF11, GDF8, activin A, BMP6, GDF15, Nodal, GDF3, BMP3, BMP3B, BMP9, or BMP10 from binding to the ActRIIA receptor and/or inhibit one of these ligands from activating ActRIIA signaling (e.g., through Smad2/3 and/or Smad 1/5/8 pathways). In preferred embodiments, anti-ActRIIA antibodies of the present disclosure inhibit GDF11 and/or activin B from binding to the ActRIIA receptor and/or inhibit GDF11 and/or activin B from activating ActRIIA signaling. Optionally, anti-ActRIIA antibodies of the disclosure further inhibit GDF8 from binding to the ActRIIA receptor and/or inhibit GDF8 from activating ActRIIA signaling. Optionally, anti-ActRIIA antibodies of the present disclosure do not substantially inhibit activin A from binding to the ActRIIA receptor and/or do not substantially inhibit activin A-mediated activation of ActRIIA signaling. In some embodiments, an anti-ActRIIA antibody of the disclosure that inhibits GDF11 and/or activin B from binding to and/or activating an ActRIIA receptor further inhibits one or more of activin C, activin E, activin A, GDF8, BMP6, GDF15, Nodal, GDF3, BMP3, BMP3B, BMP9, and BMP10 from binding to and/or activating the ActRIIA receptor.

An anti-ActRIIB antibody refers to an antibody that is capable of binding to ActRIIB with sufficient affinity such that the antibody is useful as a diagnostic and/or therapeutic agent in targeting ActRIIB. In certain embodiments, the extent of binding of an anti-ActRIIB antibody to an unrelated, non-ActRIIB protein is less than about 10%, 9%, 8%, 7%, 6%, 5%, 4%, 3%, 2%, or less than 1% of the binding of the antibody to ActRIIB as measured, for example, by a radioimmunoassay (RIA). In certain embodiments, an anti-ActRIIB antibody binds to an epitope of ActRIIB that is conserved among orthologous ActRIIB proteins from different species. In preferred embodiments, an anti-ActRIIB antibody of the present disclosure is an antagonist antibody that can inhibit an ActRIIB activity. For example, an anti-ActRIIB antibody of the present disclosure may inhibit one or more ActRIIB ligands selected from activin B, activin C, activin E, GDF11, GDF8, activin A, BMP6, GDF15, Nodal, GDF3, BMP3, BMP3B, BMP9, and BMP10 from binding to the ActRIIB receptor and/or inhibit one of these ligands from activating ActRIIB signaling (e.g., through Smad2/3 and/or Smad 1/5/8 pathways). In preferred embodiments, anti-ActRIIB antibodies of the present disclosure inhibit GDF11 and/or activin B from binding to the ActRIIB receptor and/or inhibit GDF11 and/or activin B from activating ActRIIB signaling. Optionally, anti-ActRIIB antibodies of the disclosure further inhibit GDF8 from binding to the ActRIIB receptor and/or inhibit GDF8 from activating ActRIIB signaling. Optionally, anti-ActRIIB antibodies of the present disclosure do not substantially inhibit activin A from binding to the ActRIIB receptor and/or do not substantially inhibit activin A-mediated activation of ActRIIB signaling. In some embodiments, an anti-ActRIIB antibody of the disclosure that inhibits GDF11 and/or activin B from binding to and/or activating an ActRIIB receptor further inhibits one or more of activin C, activin E, activin A, GDF8, BMP6, GDF15, Nodal, GDF3, BMP3, BMP3B, BMP9, and BMP10 from binding to and/or activating the ActRIIB receptor.

The nucleic acid and amino acid sequences of human GDF11, activin A, activin B, activin C, activin E, GDF8, BMP6, ActRIIB, ActRIIA, GDF15, Nodal, GDF3, BMP3, BMP3B, BMP9, and BMP9 are well known in the art. In addition, numerous methods for generating antibodies are well known in the art, some of which are described herein. Therefore antibody antagonists for use in accordance with this disclosure may be routinely made by the skilled person in the art based on the knowledge in the art and teachings provided herein.

In certain embodiments, an antibody provided herein *(e.g.,* an anti-GDF11 antibody, an anti-activin B antibody, an anti-ActRIIA antibody, or an anti-ActRIIB antibody) is a chimeric antibody. A chimeric antibody refers to an antibody in which a portion of the heavy and/or light chain is derived from a particular source or species, while the remainder of the heavy and/or light chain is derived from a different source or species. Certain chimeric antibodies are described, for example, in U.S. Pat. No. 4,816,567; and Morrison et al., (1984) Proc. Natl. Acad. Sci. USA, 81:6851-6855. In some embodiments, a chimeric antibody comprises a non-human variable region *(e.g.,* a variable region derived from a mouse, rat, hamster, rabbit, or non-human primate, such as a monkey) and a human constant region. In some embodiments, a chimeric antibody is a "class switched" antibody in which the class or subclass has been changed from that of the parent antibody. In general, chimeric antibodies include antigen-binding fragments thereof.

In certain embodiments, a chimeric antibody provided herein *(e.g.,* an anti-GDF11 antibody, an anti-activin B antibody, an anti-ActRIIA antibody, or an anti-ActRIIb antibody) is a humanized antibody. A humanized antibody refers to a chimeric antibody comprising amino acid residues from non-human hypervariable regions (HVRs) and amino acid residues from human framework regions (FRs). In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g*., a non-human antibody, refers to an antibody that has undergone humanization.

Humanized antibodies and methods of making them are reviewed, for example, in Almagro and Fransson (2008) Front. Biosci. 13:1619-1633 and are further described, for example, in Riechmann et al., (1988) Nature 332:323-329; Queen et al. (1989) Proc. Nat'l Acad. Sci. USA 86:10029-10033; U.S. Pat. Nos. 5,821,337; 7,527,791; 6,982,321; and 7,087,409; Kashmiri et al., (2005) Methods 36:25-34 [describing SDR (a-CDR) grafting]; Padlan, Mol. Immunol. (1991) 28:489-498 (describing "resurfacing"); Dall'Acqua et al. (2005) Methods 36:43-60 (describing "FR shuffling"); Osbourn et al. (2005) Methods 36:61-68; and Klimka et al. Br. J. Cancer (2000) 83:252-260 (describing the "guided selection" approach to FR shuffling).

Human framework regions that may be used for humanization include but are not limited to: framework regions selected using the "best-fit" method [see, *e.g.,* Sims et al. (1993) J. Immunol. 151:2296]; framework regions derived from the consensus sequence of human antibodies of a particular subgroup of light or heavy chain variable regions [see, *e.g.,* Carter et al. (1992) Proc. Natl. Acad. Sci. USA, 89:4285; and Presta et al. (1993) J. Immunol., 151:2623]; human mature (somatically mutated) framework regions or human germline framework regions [see, *e.g*., Almagro and Fransson (2008) Front. Biosci. 13:1619-1633]; and framework regions derived from screening FR libraries [see, *e.g.,* Baca et al., (1997) J. Biol. Chem. 272:10678-10684; and Rosok et al., (1996) J. Biol. Chem. 271:22611-22618].

In certain embodiments, an antibody provided herein *(e.g.,* an anti-GDF11 antibody, an anti-activin B antibody, an anti-ActRIIA antibody, or an anti-ActRIIB antibody) is a human antibody. Human antibodies can be produced using various techniques known in the art. Human antibodies are described generally in van Dijk and van de Winkel (2008) Curr. Opin. Pharmacol. 5: 368-74 (2001) and Lonberg, Curr. Opin. Immunol. 20:450-459.

Human antibodies may be prepared by administering an immunogen (e.g., a GDF11 polypeptide, an activin B polypeptide, an ActRIIA polypeptide, or an ActRIIB polypeptide) to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. For a review of methods for obtaining human antibodies from transgenic animals see, for example, Lonberg (2005) Nat. Biotech. 23:1117-1125; U.S. Pat. Nos. 6,075,181 and 6,150,584 (describing XFNOMOUSF^{™} technology); U.S. Pat. No. 5,770,429 (describing HuMab^{®} technology); U.S. Pat. No. 7,041,870 (describing K-M MOUSE^{®} technology); and U.S. Patent Application Publication No. 2007/0061900 (describing VelociMouse^{®} technology). Human variable regions from intact antibodies generated by such animals may be further modified, for example, by combining with a different human constant region.

Human antibodies provided herein can also be made by hybridoma-based methods. Human myeloma and mouse-human heteromyeloma cell lines for the production of human monoclonal antibodies have been described [see, *e.g.,* Kozbor J. Immunol., (1984) 133: 3001; Brodeur et al. (1987) Monoclonal Antibody Production Techniques and Applications, pp. 51-63, Marcel Dekker, Inc., New York; and Boerner et al. (1991) J. Immunol., 147: 86]. Human antibodies generated via human B-cell hybridoma technology are also described in Li et al., (2006) Proc. Natl. Acad. Sci. USA, 103:3557-3562. Additional methods include those described, for example, in U.S. Pat. No. 7,189,826 (describing production of monoclonal human IgM antibodies from hybridoma cell lines) and Ni, Xiandai Mianyixue (2006) 26(4):265-268 (2006) (describing human-human hybridomas). Human hybridoma technology (Trioma technology) is also described in Vollmers and Brandlein (2005) Histol. Histopathol., 20(3):927-937 (2005) and Vollmers and Brandlein (2005) Methods Find Exp. Clin. Pharmacol., 27(3):185-91.

Human antibodies provided herein *(e.g.,* an anti-GDF11 antibody, an anti-activin B antibody, an anti-ActRIIA antibody, or an anti-ActRIIB antibody) may also be generated by isolating Fv clone variable-domain sequences selected from human-derived phage display libraries. Such variable-domain sequences may then be combined with a desired human constant domain. Techniques for selecting human antibodies from antibody libraries are described herein.

For example, antibodies of the present disclosure may be isolated by screening combinatorial libraries for antibodies with the desired activity or activities. A variety of methods are known in the art for generating phage display libraries and screening such libraries for antibodies possessing the desired binding characteristics. Such methods are reviewed, for example, in Hoogenboom et al. (2001) in Methods in Molecular Biology 178:1-37, O'Brien et al., ed., Human Press, Totowa, N.J. and further described, for example, in the McCafferty et al. (1991) Nature 348:552-554; Clackson et al., (1991) Nature 352: 624-628; Marks et al. (1992) J. Mol. Biol. 222:581-597; Marks and Bradbury (2003) in Methods in Molecular Biology 248:161-175, Lo, ed., Human Press, Totowa, N.J.; Sidhu et al. (2004) J. Mol. Biol. 338(2):299-310; Lee et al. (2004) J. Mol. Biol. 340(5):1073-1093; Fellouse (2004) Proc. Natl. Acad. Sci. USA 101(34):12467-12472; and Lee et al. (2004) J. Immunol. Methods 284(1-2): 119-132.

In certain phage display methods, repertoires of VH and VL genes are separately cloned by polymerase chain reaction (PCR) and recombined randomly in phage libraries, which can then be screened for antigen-binding phage as described in Winter et al. (1994) Ann. Rev. Immunol., 12: 433-455. Phage typically display antibody fragments, either as single-chain Fv (scFv) fragments or as Fab fragments. Libraries from immunized sources provide high-affinity antibodies to the immunogen (e.g., GDF11, activin B, ActRIIA, or ActRIIB) without the requirement of constructing hybridomas. Alternatively, the naive repertoire can be cloned *(e.g.,* from human) to provide a single source of antibodies to a wide range of non-self and also self-antigens without any immunization as described by Griffiths et al. (1993) EMBO J, 12: 725-734. Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter (1992) J. Mol. Biol., 227: 381-388. Patent publications describing human antibody phage libraries include, for example: U.S. Pat. No. 5,750,373, and U.S. Patent Publication Nos. 2005/0079574, 2005/0119455, 2005/0266000, 2007/0117126, 2007/0160598, 2007/0237764, 2007/0292936, and 2009/0002360.

In certain embodiments, an antibody provided herein is a multispecific antibody, for example, a bispecific antibody. Multispecific antibodies (typically monoclonal antibodies) that have binding specificities for at least two different epitopes (e.g., two, three, four, five, or six or more) on one or more (e.g., two, three, four, five, six or more) antigens.

In certain embodiments, multispecific antibodies of the present disclosure comprise two or more binding specificities, with at least one of the binding specificities being for a GDF11 epitope, and optionally one or more additional binding specificities being for an epitope on a different ActRII ligand (e.g., GDF8, activin B, activin C, activin E, activin A, GDF15, Nodal, GDF3, GDF3B, BMP9, BMP10, and/or BMP6) and/or an ActRII receptor *(e.g.,* an ActRIIA and/or ActRIIB receptor). In certain embodiments, multispecific antibodies may bind to two or more different epitopes of GDF11. Preferably, a multispecific antibody of the disclosure that has binding affinity, in part, for an GDF11 epitope can be used to inhibit an GDF11 activity *(e.g.,* the ability to bind to and/or activate an ActRIIA and/or ActRIIB receptor), and optionally inhibit the activity of one or more different ActRII ligands *(e.g.,* GDF8, activin B, activin C, activin E, activin A, GDF15, Nodal, GDF3, GDF3B, BMP9, BMP10, and/or BMP6) and/or an ActRII receptor *(e.g.,* an ActRIIA or ActRIIB receptor). In preferred embodiments, multispecific antibodies of the present disclosure that bind to and/or inhibit GDF11 further bind to and/or inhibit at least activin B. Optionally, multispecific antibodies of the disclosure that bind to and/or inhibit GDF11 do not bind to and/or inhibit activin A. In some embodiments, multispecific antibodies of the disclosure that bind to and/or inhibit GDF11 further bind to and/or inhibit at least GDF8.

In certain embodiments, multispecific antibodies of the present disclosure comprise two or more binding specificities, with at least one of the binding specificities being for an activin B epitope, and optionally one or more additional binding specificities being for an epitope on a different ActRII ligand (e.g., GDF8, GDF11, activin C, activin E, activin A, GDF15, Nodal, GDF3, GDF3B, BMP9, BMP10, and/or BMP6) and/or an ActRII receptor *(e.g.,* an ActRIIA and/or ActRIIB receptor). In certain embodiments, multispecific antibodies may bind to two or more different epitopes of activin B. Preferably, a multispecific antibody of the disclosure that has binding affinity, in part, for an activin B epitope can be used to inhibit an activin B activity *(e.g.,* the ability to bind to and/or activate an ActRIIA and/or ActRIIB receptor), and optionally inhibit the activity of one or more different ActRII ligands *(e.g.,* GDF8, GDF11, activin C, activin E, activin A, GDF15, Nodal, GDF3, GDF3B, BMP9, BMP10, and/or BMP6) and/or an ActRII receptor *(e.g.,* an ActRIIA or ActRIIB receptor). In preferred embodiments, multispecific antibodies of the present disclosure that bind to and/or inhibit activin B further bind to and/or inhibit at least GDF11. Optionally, multispecific antibodies of the disclosure that bind to and/or inhibit activin B do not bind to and/or inhibit activin A. In some embodiments, multispecific antibodies of the disclosure that bind to and/or inhibit activin B further bind to and/or inhibit at least GDF8.

In certain embodiments, multispecific antibodies of the present disclosure comprise two or more binding specificities, with at least one of the binding specificities being for a GDF8 epitope, and optionally one or more additional binding specificities being for an epitope on a different ActRII ligand *(e.g.,* GDF11, activin B, activin C, activin E, activin A, GDF15, Nodal, GDF3, GDF3B, BMP9, BMP10, and/or BMP6) and/or an ActRII receptor *(e.g.,* an ActRIIA and/or ActRIIB receptor). In certain embodiments, multispecific antibodies may bind to two or more different epitopes of GDF8. Preferably, a multispecific antibody of the disclosure that has binding affinity, in part, for an GDF8 epitope can be used to inhibit an GDF8 activity *(e.g.,* the ability to bind to and/or activate an ActRIIA and/or ActRIIB receptor), and optionally inhibit the activity of one or more different ActRII ligands (e.g., GDF11, activin B, activin C, activin E, activin A, GDF15, Nodal, GDF3, GDF3B, BMP9, BMP10, and/or BMP6) and/or an ActRII receptor *(e.g.,* an ActRIIA or ActRIIB receptor). In preferred embodiments, multispecific antibodies of the present disclosure that bind to and/or inhibit GDF8 further bind to and/or inhibit at least GDF11 and/or activin B. Optionally, multispecific antibodies of the disclosure that bind to and/or inhibit GDF8 do not bind to and/or inhibit activin A.

In certain embodiments, multispecific antibodies of the present disclosure comprise two or more binding specificities, with at least one of the binding specificities being for an activin C epitope, and optionally one or more additional binding specificities being for an epitope on a different ActRII ligand (e.g., GDF8, GDF11, activin B, activin E, activin A, GDF15, Nodal, GDF3, GDF3B, BMP9, BMP10, and/or BMP6) and/or an ActRII receptor *(e.g.,* an ActRIIA and/or ActRIIB receptor). In certain embodiments, multispecific antibodies may bind to two or more different epitopes of activin C. Preferably, a multispecific antibody of the disclosure that has binding affinity, in part, for an activin C epitope can be used to inhibit an activin C activity *(e.g.,* the ability to bind to and/or activate an ActRIIA and/or ActRIIB receptor), and optionally inhibit the activity of one or more different ActRII ligands *(e.g.,* GDF8, GDF11, activin B, activin E, activin A, GDF15, Nodal, GDF3, GDF3B, BMP9, BMP10, and/or BMP6) and/or an ActRII receptor *(e.g.,* an ActRIIA or ActRIIB receptor). In preferred embodiments, multispecific antibodies of the present disclosure that bind to and/or inhibit activin C further bind to and/or inhibit at least GOF11 and/or activin B. Optionally, multispecific antibodies of the disclosure that bind to and/or inhibit activin C do not bind to and/or inhibit activin A. Optionally, multispecific antibodies of the disclosure that bind to and/or inhibit activin C further bind to and/or inhibit GDF8.

In certain embodiments, multispecific antibodies of the present disclosure comprise two or more binding specificities, with at least one of the binding specificities being for an activin E epitope, and optionally one or more additional binding specificities being for an epitope on a different ActRII ligand (e.g., GDF8, GDF11, activin C, activin B, activin A, GDF15, Nodal, GDF3, GDF3B, BMP9, BMP10, and/or BMP6) and/or an ActRII receptor *(e.g.,* an ActRIIA and/or ActRIIB receptor). In certain embodiments, multispecific antibodies may bind to two or more different epitopes of activin E. Preferably, a multispecific antibody of the disclosure that has binding affinity, in part, for an activin E epitope can be used to inhibit an activin E activity (e.g., the ability to bind to and/or activate an ActRIIA and/or ActRIIB receptor), and optionally inhibit the activity of one or more different ActRII ligands *(e.g.,* GDF8, GDF11, activin C, activin A, GDF15, Nodal, GDF3, GDF3B, BMP9, BMP10, and/or BMP6) and/or an ActRII receptor *(e.g.,* an ActRIIA or ActRIIB receptor). In preferred embodiments, multispecific antibodies of the present disclosure that bind to and/or inhibit activin E further bind to and/or inhibit at least GDF11 and/or activin B. Optionally, multispecific antibodies of the disclosure that bind to and/or inhibit activin E do not bind to and/or inhibit activin A. Optionally, multispecific antibodies of the disclosure that bind to and/or inhibit activin E further bind to and/or inhibit GDF8.

In certain embodiments, multispecific antibodies of the present disclosure comprise two or more binding specificities, with at least one of the binding specificities being for a BMP6 epitope, and optionally one or more additional binding specificities being for an epitope on a different ActRII ligand *(e.g.,* GDF11, activin B, activin C, activin E, GDF8, activin A, GDF15, Nodal, GDF3, GDF3B, BMP9, and BMP10) and/or an ActRII receptor *(e.g.,* an ActRIIA and/or ActRIIB receptor). In certain embodiments, multispecific antibodies may bind to two or more different epitopes of BMP6. Preferably, a multispecific antibody of the disclosure that has binding affinity, in part, for a BMP6 epitope can be used to inhibit a BMP6 activity (e.g., the ability to bind to and/or activate an ActRIIA and/or ActRIIB receptor), and optionally inhibit the activity of one or more different ActRII ligands (e.g., GDF11, activin B, activin C, activin E, GDF8, activin A, GDF15, Nodal, GDF3, GDF3B, BMP9, and BMP10) and/or an ActRII receptor *(e.g.,* an ActRIIA or ActRIIB receptor). In preferred embodiments, multispecific antibodies of the present disclosure that bind to and/or inhibit BMP6 further bind to and/or inhibit at least GDF11 and/or activin B. Optionally, multispecific antibodies of the disclosure that bind to and/or inhibit BMP6 do not bind to and/or inhibit activin A. Optionally, multispecific antibodies of the disclosure that bind to and/or inhibit BMP6 further bind to and/or inhibit GDF8.

In certain embodiments, multispecific antibodies of the present disclosure comprise two or more binding specificities, with at least one of the binding specificities being for a BMP9 epitope, and optionally one or more additional binding specificities being for an epitope on a different ActRII ligand *(e.g.,* GDF11, activin B, activin C, activin E, GDF8, activin A, GDF15, Nodal, GDF3, GDF3B, and BMP10) and/or an ActRII receptor *(e.g.,* an ActRIIA and/or ActRIIB receptor). In certain embodiments, multispecific antibodies may bind to two or more different epitopes of BMP9. Preferably, a multispecific antibody of the disclosure that has binding affinity, in part, for a BMP9 epitope can be used to inhibit a BMP9 activity (e.g., the ability to bind to and/or activate an ActRIIA and/or ActRIIB receptor), and optionally inhibit the activity of one or more different ActRII ligands (e.g., GDF11, activin B, activin C, activin E, GDF8, activin A, GDF15, Nodal, GDF3, GDF3B, and BMP10) and/or an ActRII receptor *(e.g.,* an ActRIIA or ActRIIB receptor). In preferred embodiments, multispecific antibodies of the present disclosure that bind to and/or inhibit BMP9 further bind to and/or inhibit at least GDF11 and/or activin B. Optionally, multispecific antibodies of the disclosure that bind to and/or inhibit BMP9 do not bind to and/or inhibit activin A. Optionally, multispecific antibodies of the disclosure that bind to and/or inhibit BMP9 further bind to and/or inhibit GDF8. In certain embodiments, multispecific antibodies that bind to BMP9 inhibit interaction between BMP9 and a type II receptor of the TGFβ superfamily (e.g., ActRIIA and/or ActRIIB). Preferably, multispecific antibodies that bind to BMP9 do not inhibit, or substantially inhibit, interaction between BMP9 and ALK1.

In certain embodiments, multispecific antibodies of the present disclosure comprise two or more binding specificities, with at least one of the binding specificities being for a BMP10 epitope, and optionally one or more additional binding specificities being for an epitope on a different ActRII ligand *(e.g.,* GDF11, activin B, activin C, activin E, GDF8, activin A, GDF15, Nodal, GDF3, GDF3B, and BMP9) and/or an ActRII receptor *(e.g.,* an ActRIIA and/or ActRIIB receptor). In certain embodiments, multispecific antibodies may bind to two or more different epitopes of BMP10. Preferably, a multispecific antibody of the disclosure that has binding affinity, in part, for a BMP10 epitope can be used to inhibit a BMP10 activity *(e.g.,* the ability to bind to and/or activate an ActRIIA and/or ActRIIB receptor), and optionally inhibit the activity of one or more different ActRII ligands (e.g., GDF11, activin B, activin C, activin E, GDF8, activin A, GDF15, Nodal, GDF3, GDF3B, and BMP9) and/or an ActRII receptor *(e.g.,* an ActRIIA or ActRIIB receptor). In preferred embodiments, multispecific antibodies of the present disclosure that bind to and/or inhibit BMP10 further bind to and/or inhibit at least GDF11 and/or activin B. Optionally, multispecific antibodies of the disclosure that bind to and/or inhibit BMP10 do not bind to and/or inhibit activin A. Optionally, multispecific antibodies of the disclosure that bind to and/or inhibit BMP10 further bind to and/or inhibit GDF8. In certain embodiments, multispecific antibodies that bind to BMP10 inhibit interaction between BMP10 and a type II receptor of the TGFβ superfamily (e.g., ActRIIA and/or ActRIIB). Preferably, multispecific antibodies that bind to BMP10 do not inhibit, or substantially inhibit, interaction between BMP10 and ALK1.

In certain embodiments, multispecific antibodies of the present disclosure comprise two or more binding specificities, with at least one of the binding specificities being for a GDF11 epitope, and one other binding specificity being for an activin B epitope. Preferably, a multispecific antibody of the disclosure that has binding affinity, in part, for a GDF11 epitope and an activin B epitope can be used to inhibit both a GDF11 and an activin B activity (e.g., the ability to bind to and/or activate an ActRIIA and/or ActRIIB receptor). In some embodiments, a multispecific antibody that binds to and/or inhibits GDF11 and activin B activity further binds to and/or inhibits one or more of GDF8, activin A, activin C, activin E, GDF15, Nodal, GDF3, GDF3B, BMP9, BMP10, and/or BMP6. Optionally, a multispecific antibody that binds to and/or inhibits GDF11 and activin B does not substantially bind to and/or inhibit activin A.

Techniques for making multispecific antibodies include, but are not limited to, recombinant co-expression of two immunoglobulin heavy-chain/light-chain pairs having different specificities [see, *e.g*., Milstein and Cuello (1983) Nature 305: 537; International patent publication no. WO 93/08829; and Traunecker et al. (1991) EMBO J. 10: 3655, and U.S. Pat. No. 5,731,168 ("knob-in-hole" engineering)]. Multispecific antibodies may also be made by engineering electrostatic steering effects for making antibody Fc-heterodimeric molecules (see, *e.g.,* WO 2009/089004A1); cross-linking two or more antibodies or fragments [see, *e.g.,* U.S. Pat. No. 4,676,980; and Brennan et al. (1985) Science, 229: 81]; using leucine zippers to produce bispecific antibodies [see, *e.g.,* Kostelny et al. (1992) J. Immunol., 148(5):1547-1553]; using "diabody" technology for making bispecific antibody fragments [see, *e.g.,* Hollinger et al. (1993) Proc. Natl. Acad. Sci. USA, 90:6444-6448]; using single-chain Fv (sFv) dimers [see, *e.g.,* Gruber et al. (1994) J. Immunol., 152:5368]; and preparing trispecific antibodies (see, *e.g.,* Tutt et al. (1991) J. Immunol. 147: 60. Multispecific antibodies can be prepared as full-length antibodies or antibody fragments.

Engineered antibodies with three or more functional antigen-binding sites, including "Octopus antibodies," are also included herein [see, *e.g.,* US 2006/0025576A1].

In certain embodiments, an antibody disclosed herein *(e.g.,* an anti-GDF11 antibody, an anti-activin B antibody, an anti-ActRIIA antibody, or an anti-ActRIIB antibody) is a monoclonal antibody. Monoclonal antibody refers to an antibody obtained from a population of substantially homogeneous antibodies, *i.e.,* the individual antibodies comprising the population are identical and/or bind the same epitope, except for possible variant antibodies, *e.g*., containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present methods may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

For example, by using immunogens derived from GDF11 or activin B, anti-protein/anti-peptide antisera or monoclonal antibodies can be made by standard protocols [see, *e.g.*, Antibodies: A Laboratory Manual ed. by Harlow and Lane (1988) Cold Spring Harbor Press: 1988]. A mammal, such as a mouse, hamster, or rabbit, can be immunized with an immunogenic form of the GOF11 or activin B polypeptide, an antigenic fragment which is capable of eliciting an antibody response, or a fusion protein. Techniques for conferring immunogenicity on a protein or peptide include conjugation to carriers or other techniques well known in the art. An immunogenic portion of a GDF11 or activin B polypeptide can be administered in the presence of adjuvant. The progress of immunization can be monitored by detection of antibody titers in plasma or serum. Standard ELISA or other immunoassays can be used with the immunogen as antigen to assess the levels of antibody production and/or level of binding affinity.

Following immunization of an animal with an antigenic preparation of GDF11 or activin B, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique [*see, e.g.,* Kohler and Milstein (1975) Nature, 256: 495-497], the human B cell hybridoma technique [*see, e.g.,* Kozbar et al. (1983) Immunology Today, 4:72], and the EBV-hybridoma technique to produce human monoclonal antibodies [Cole et al. (1985) Monoclonal Antibodies and Cancer Therapy, Alan R. Liss, Inc. pp. 77-96]. Hybridoma cells can be screened immunochemically for production of antibodies specifically reactive with a GDF11 or activin B polypeptide, and monoclonal antibodies isolated from a culture comprising such hybridoma cells.

In certain embodiments, one or more amino acid modifications may be introduced into the Fc region of an antibody provided herein *(e.g.,* an anti-GDF11 antibody, an anti-activin B antibody, an anti-ActRIIA antibody, or an anti-ActRIIB antibody), thereby generating an Fc region variant. The Fc region variant may comprise a human Fc region sequence (e.g., a human IgG1, IgG2, IgG3 or IgG4 Fc region) comprising an amino acid modification (e.g., a substitution, deletion, and/or addition) at one or more amino acid positions.

For example, the present disclosure contemplates an antibody variant that possesses some but not all effector functions, which make it a desirable candidate for applications in which the half-life of the antibody *in vivo* is important yet certain effector functions [e.g., complement-dependent cytotoxicity (CDC) and antibody-dependent cellular cytotoxicity (ADCC)] are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcyRIII only, whereas monocytes express FcyRI, FcyRII and FcyRIII. FcR expression on hematopoietic cells is summarized in, for example, Ravetch and Kinet (1991) Annu. Rev. Immunol. 9:457-492. Non-limiting examples of *in vitro* assays to assess ADCC activity of a molecule of interest are described in U.S. Pat. No. 5,500,362; Hellstrom, I. et al. (1986) Proc. Natl. Acad. Sci. USA 83:7059-7063]; Hellstrom, I et al. (1985) Proc. Natl. Acad. Sci. USA 82:1499-1502; U.S. Pat. No. 5,821,337; Bruggemann, M. et al. (1987) J. Exp. Med. 166:1351-1361. Alternatively, non-radioactive assays methods may be employed (e.g., ACTI^{™}, non-radioactive cytotoxicity assay for flow cytometry; CellTechnology, Inc. Mountain View, Calif; and CytoTox 96^{®} non-radioactive cytotoxicity assay, Promega, Madison, Wis.). Useful effector cells for such assays include peripheral blood mononuclear cells (PBMC) and natural killer (NK) cells. Alternatively, or additionally, ADCC activity of the molecule of interest may be assessed *in vivo,* for example, in an animal model such as that disclosed in Clynes et al. (1998) Proc. Natl. Acad. Sci. USA 95:652-656. C1q binding assays may also be carried out to confirm that the antibody is unable to bind C1q and hence lacks CDC activity [see, *e.g.,* C1q and C3c binding ELISA in WO 2006/029879 and WO 2005/100402]. To assess complement activation, a CDC assay may be performed [see, *e.g,* Gazzano-Santoro et al. (1996) J. Immunol. Methods 202:163; Cragg, M. S. et al. (2003) Blood 101:1045-1052; and Cragg, M. S, and M. J. Glennie (2004) Blood 103:2738-2743]. FcRn binding and *in vivo* clearance/half-life determinations can also be performed using methods known in the art [see, *e.g*., Petkova, S. B. et al. (2006) Intl. Immunol. 18(12):1759-1769].

Antibodies of the present disclosure (e.g., an anti-GDF11 antibody, an anti-activin B antibody, an anti-ActRIIA antibody, or an anti-ActRIIB antibody) with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

In certain embodiments, it may be desirable to create cysteine engineered antibodies, *e.g*., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, for example., in U.S. Pat. No. 7,521,541.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays *(e.g.,* the Biacore binding assay, Biacore AB, Uppsala, Sweden), sandwich assays *(e.g.,* the paramagnetic bead system of IGEN International, Inc., Gaithersburg, Maryland), western blots, immunoprecipitation assays, and immunohistochemistry.

In certain embodiments, amino acid sequence variants of the antibodies and/or the binding polypeptides provided herein are contemplated. For example, it may be desirable to improve the binding affinity and/or other biological properties of the antibody and/or binding polypeptide. Amino acid sequence variants of an antibody and/or binding polypeptides may be prepared by introducing appropriate modifications into the nucleotide sequence encoding the antibody and/or binding polypeptide, or by peptide synthesis. Such modifications include, for example, deletions from, and/or insertions into and/or substitutions of residues within the amino acid sequences of the antibody and/or binding polypeptide. Any combination of deletion, insertion, and substitution can be made to arrive at the final construct, provided that the final construct possesses the desired characteristics, *e.g.*, target-binding (GDF11 and/or activin B binding).

Alterations (e.g., substitutions) may be made in HVRs, for example, to improve antibody affinity. Such alterations may be made in HVR "hotspots," i.e., residues encoded by codons that undergo mutation at high frequency during the somatic maturation process [see, *e.g.*, Chowdhury (2008) Methods Mol. Biol. 207:179-196 (2008)], and/or SDRs (a-CDRs), with the resulting variant VH or VL being tested for binding affinity. Affinity maturation by constructing and reselecting from secondary libraries has been described in the art [see, *e.g.,* Hoogenboom et al., in Methods in Molecular Biology 178:1-37, O'Brien et al., ed., Human Press, Totowa, N.J., (2001). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, *e.g*., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind to the antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of the antibody and/or the binding polypeptide that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid *(e.g.,* alanine or polyalanine) to determine whether the interaction of the antibody-antigen is affected. Further substitutions may be introduced at the amino acid locations demonstrating functional sensitivity to the initial substitutions. Alternatively, or additionally, a crystal structure of an antigen-antibody complex is determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme (*e.g.*, for ADEPT) or a polypeptide which increases the serum half-life of the antibody.

In certain embodiments, an antibody and/or binding polypeptide provided herein may be further modified to contain additional nonproteinaceous moieties that are known in the art and readily available. The moieties suitable for derivatization of the antibody and/or binding polypeptide include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody and/or binding polypeptide may vary, and if more than one polymer are attached, they can be the same or different molecules. In general, the number and/or type of polymers used for derivatization can be determined based on considerations including, but not limited to, the particular properties or functions of the antibody and/or binding polypeptide to be improved, whether the antibody derivative and/or binding polypeptide derivative will be used in a therapy under defined conditions.

Any of the antibodies disclosed herein (*e.g.*, an anti-GDF11 antibody, an anti-activin A antibody, an anti-activin B antibody, an anti-activin C antibody, an anti-activin E antibody, an anti-GDF8 antibody, an anti-BMP6 antibody, an anti-ActRIIA antibody, an anti-GDF15 antibody, an anti-Nodal antibody, an anti-GDF3 antibody, an anti-BMP3 antibody, an anti-BMP3B antibody, an anti-BMP9 antibody, an anti-BMP 10 antibody, or an anti-ActRIIB antibody) can be combined with one or more additional antagonist agents of the disclosure to achieve the desired effect. An antibody disclosed herein (*e.g.*, an anti-GDF11 antibody, an anti-activin A antibody, an anti-activin B antibody, an anti-activin C antibody, an anti-activin E antibody, an anti-GDF11 antibody, an anti-GDF8 antibody, an anti-BMP6 antibody, an anti-ActRIIA antibody, an anti-GDF15 antibody, an anti-Nodal antibody, an anti-GDF3 antibody, an anti-BMP3 antibody, an anti-BMP3B antibody, an anti-BMP9 antibody, an anti-BMP10 antibody, or an anti-ActRIIB antibody) can be combined with another antibody disclosed herein, or a ligand trap polypeptide disclosed herein (*e.g.*, a GDF11 trap polypeptide, an activin B trap polypeptide, or a GDF11/activin B trap polypeptide), or a small-molecule antagonist directed to any of the targets of the disclosure (*e*.*g*., an activin A small-molecule antagonist, an activin B small-molecule antagonist, a GDF11 small-molecule antagonist, an activin C small-molecule antagonist, an activin E small-molecule antagonist, a GDF8 small-molecule antagonist, a BMP6 small-molecule antagonist, a GDF15 small-molecule antagonist, a Nodal small-molecule antagonist, a GDF3 small-molecule antagonist, a BMP3B small-molecule antagonist, a BMP3B small-molecule antagonist, a BMP9 small-molecule antagonist, or a BMP10 small-molecule antagonist), or a polynucleotide antagonist of the disclosure (*e*.*g*., a polynucleotide antagonist of activin A, activin B, activin C, activin E, GDF11, GDF8, GDF15, Nodal, GDF3, BMP3, BMP3B, or BMP6), or a non-antibody binding polypeptide disclosed herein (*e.g.*, a GDF11 binding polypeptide, an activin A binding polypeptide, an activin B binding polypeptide, an activin E binding polypeptide, an activin C binding polypeptide, a GDF8 binding polypeptide, a GDF15 binding polypeptide, a Nodal binding polypeptide, a GDF3binding polypeptide, a BMP3 binding polypeptide, a BMP3B polypeptide, a BMP9 binding polypeptide, a BMP10 binding polypeptide, or a BMP6 binding polypeptide). For example, an anti-GDF11 antibody can be combined with an activin B antagonist of the disclosure (*e*.*g*., an activin B trap polypeptide, an anti-activin B antibody, a small-molecule antagonist of activin B, a polynucleotide antagonist of activin B, or a non-antibody polypeptide antagonist of activin B) to inhibit both a GDF11 and an activin B activity (*e*.*g*., the ability to bind to and/or activate an ActRIIA and/or ActRIIB receptor). In an alternative embodiment, an anti-activin B antibody can be combined with a GDF11 antagonist of the disclosure (*e*.*g*., a GDF trap polypeptide, an anti-GDF11 antibody, a small-molecule antagonist of GDF11, a polynucleotide antagonist of GDF11, or a non-antibody polypeptide antagonist of GDF11) to inhibit both a GDF11 and an activin B activity.

### B. GDF11, Activin B, and GDF11/Activin B Trap Polypeptides

In one aspect, the antagonist (inhibitory) agents of the present disclosure are ActRII polypeptides and variants thereof. As used herein the term "ActRII" refers to the family of type II activin receptors. This family includes both the activin receptor type IIA (ActRIIA) and the activin receptor type IIB (ActRIIB). In some embodiments, the methods of the present disclosure relate to the use of one or more variant ActRII polypeptides (e.g., variant ActRIIA and ActRIIB polypeptides) that bind to and/or antagonize (inhibit) the activity of one or more of: GDF11, GDF8, activin B, activin C, activin E, activin A, BMP6, GDF15, Nodal, GDF3, BMP3, and BMP3B (e.g., activation of ActRIIA and/or ActRIIB Smad2/3 and/or Smad 1/5/8 signaling). In some embodiments, variant ActRII polypeptides are ligand "trap" polypeptides (e.g., GDF11 trap polypeptides, activin B trap polypeptides, GDF11/activin B trap polypeptides). In some embodiments, variant ActRII polypeptides, and fusion constructs thereof, have reduced binding affinity for BMP9 and/or BMP10.

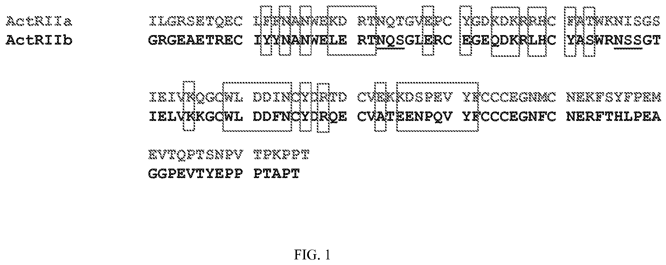

As used herein, the term "ActRIIB" refers to a family of activin receptor type IIB (ActRIIB) proteins from any species and variants derived from such ActRIIB proteins by mutagenesis or other modification. Reference to ActRIIB herein is understood to be a reference to any one of the currently identified forms. Members of the ActRIIB family are generally transmembrane proteins, composed of a ligand-binding extracellular domain with a cysteine-rich region, a transmembrane domain, and a cytoplasmic domain with predicted serine/threonine kinase activity. An alignment of the amino acid sequences of human ActRIIB soluble extracellular domain and the related ActRIIA soluble extracellular domain are illustrated in Figure 1.

The term "ActRIIB polypeptide" includes polypeptides comprising any naturally occurring polypeptide of an ActRIIB family member as well as any variants thereof (including mutants, fragments, fusions, and peptidomimetic forms) that retain a useful activity [see, *e*.*g*., international patent application publication no. WO 2006/012627]. Numbering of amino acids for all ActRIIB-related polypeptides described herein is based on the numbering for SEQ ID NO:1, unless specifically designated otherwise.

Applicants have ascertained that that an Fc fusion protein having the sequence disclosed by Hilden et al. [Blood (1994) 83(8): 2163-2170], which has an alanine at the position corresponding to amino acid 64 of SEQ ID NO:1 (A65), has a relatively low affinity for activin and GDF11. By contrast, the same Fc fusion protein with an arginine at position (R65) has an affinity for activin and GDF11 in the low nanomolar to high picomolar range. Therefore, a sequence with an R64 is used as the "wild-type" reference sequence for human ActRIIB in this disclosure.

The human ActRIIB precursor protein sequence is as follows:

The signal peptide is indicated with single underlined; the extracellular domain is indicated in **bold** font; and the potential, native N-linked glycosylation sites are indicated with double underlining.

A form with an alanine at position 64 is also reported in the literature, as follows:

The human ActRIIB soluble (extracellular), processed polypeptide sequence is as follows:

The alternative form with an A64 is as follows:

In some embodiments, the protein may be produced with an "SGR..." sequence at the N-terminus. The C-terminal "tail" of the extracellular domain is indicated by single underlining. The sequence with the "tail" deleted (a Δ15 sequence) is as follows:

GRGEAETRECIYYNANWELERTNQSGLERCEGEQDKRLHCYASWRNSSG TIELVKKGCWLDDFNCYDRQECVATEENPQVYFCCCEGNFCNERFTHLPEA (SEQ ID NO:5).

The alternative form with an A64 is as follows:

The nucleic acid sequence encoding a human ActRIIB precursor protein is as follows: (nucleotides 5-1543 of Genbank entry NM_001106; the sequence as shown provides an alanine at position 64, and may be modified to provide an arginine instead)

The nucleic acid sequence encoding a human ActRIIB soluble (extracellular) polypeptide is as follows (the sequence as shown provides an alanine at position 64, and may be modified to provide an arginine instead):

In certain embodiments, the present disclosure relates to ActRIIA polypeptides. As used herein, the term "ActRIIA" refers to a family of activin receptor type IIA (ActRIIA) proteins from any species and variants derived from such ActRIIA proteins by mutagenesis or other modification. Reference to ActRIIA herein is understood to be a reference to any one of the currently identified forms. Members of the ActRIIA family are generally transmembrane proteins, composed of a ligand-binding extracellular domain with a cysteine-rich region, a transmembrane domain, and a cytoplasmic domain with predicted serine/threonine kinase activity.

The term "ActRIIA polypeptide" includes polypeptides comprising any naturally occurring polypeptide of an ActRIIA family member as well as any variants thereof (including mutants, fragments, fusions, and peptidomimetic forms) that retain a useful activity (see, *e*.*g*., international patent application publication no. WO/2006/012627). Numbering of amino acids for all ActRIIA-related polypeptides described herein is based on the numbering for SEQ ID NO:9, unless specifically designated otherwise.

The human ActRIIA precursor protein sequence is as follows:

The signal peptide is indicated by single underlined; the extracellular domain is indicated in **bold** font; and the potential N-linked glycosylation sites are indicated by double underlined.

The human ActRIIA soluble (extracellular), processed polypeptide sequence is as follows:

The C-terminal "tail" of the extracellular domain is indicated by single underlining. The sequence with the "tail" deleted (a Δ15 sequence) is as follows:

The nucleic acid sequence encoding human ActRIIA precursor protein is as follows (nucleotides 164-1705 of Genbank entry NM_001616):

The nucleic acid sequence encoding a human ActRIIA soluble (extracellular) polypeptide is as follows:

In certain embodiments, the present disclosure relates to ActRII polypeptides (ActRIIA and ActRIIB polypeptides) which are soluble ActRII polypeptides. As described herein, the term "soluble ActRII polypeptide" generally refers to polypeptides comprising an extracellular domain of an ActRII protein. The term "soluble ActRII polypeptide," as used herein, includes any naturally occurring extracellular domain of an ActRII protein as well as any variants thereof (including mutants, fragments, and peptidomimetic forms) that retain a useful activity (e.g., a GDF11 trap, an activin B trap, and a GDF11/activin B trap as described herein). Other examples of soluble ActRII polypeptides comprise a signal sequence in addition to the extracellular domain of an ActRII protein. For example, the signal sequence can be a native signal sequence of an ActRIIA or ActRIIB protein, or a signal sequence from another protein including, for example, a tissue plasminogen activator (TPA) signal sequence or a honey bee melittin (HBM) signal sequence.

An example of a HBM signal sequence is as follows:

MKFLVNVALVFMVVYISYIYA (SEQ ID NO:14).

An example of a TPA signal sequence is as follows:

MDAMKRGLCCVLLLCGAVFVSP (SEQ ID NO:15).

In certain embodiments, the present disclosure contemplates making mutations in the extracellular domain (also referred to as ligand-binding domain) of an ActRII polypeptide such that the ActRII polypeptide has an altered ligand-binding activity (e.g., binding specificity). In certain aspects, such "ligand trap" polypeptides have altered (elevated or reduced) binding affinity for one or more specific ActRII ligands. In other aspects, ligand trap polypeptides have altered binding specificity for one or more ActRII ligands.

For example, the present disclosure provides methods of using ligand trap polypeptides that preferentially bind to at least GDF11 and/or activin B, particularly methods for increasing red blood cell levels and/or treating anemia in a subject in need thereof. Optionally, trap polypeptides of the disclosure do not bind to and/or inhibit activin A. Optionally, trap polypeptides of the disclosure further bind to GDF8. In some embodiments, trap polypeptides of the disclosure that bind to and/or inhibit GDF11 and/or activin B further bind to and/or inhibit one or more of activin C, activin E, activin A, BMP6, GDF15, Nodal, GDF3, BMP3, BMP3B, and GDF8. Optionally, trap polypeptides of the disclosure have reduced to no binding affinity for BMP9 and/or BMP10.

As disclosed herein, GDF11/activin B traps are variant ActRII polypeptides (e.g. variant ActRIIA or ActRIIB polypeptides) that bind to and/or antagonize (inhibit) the activity of both GDF11 and activin B (*e.g.*, GDF11- and activin B-mediated activation of the ActRIIA or ActRIIB Smad2/3 signaling pathway). Thus, the present disclosure provides GDF11/activin B traps that comprise an altered ligand-binding domain of an ActRII receptor [*e.g.*, comprising one or more amino acid mutations (*e.g.*, amino acid substitutions, additions, or deletions)] which are characterized, in part, by increased selectivity for GDF11 and activin B relative to an unmodified (wild-type) ligand-binding domain of an ActRII receptor. Optionally, GDF11/activin B traps do not substantially bind to and/or inhibit activity of activin A (*e*.*g*., activin A-mediated activation of the ActRIIA or ActRIIB Smad2/3 signaling pathway).

In some embodiments, GDF11/activin B traps of the disclosure have equivalent or increased binding affinity (*e.g.*, 2-, 3-, 10-, 100-, 1000-fold increased binding affinity) for GDF11 and/or activin B relative to an unmodified (wild-type) ligand-binding domain of an ActRII receptor. Optionally, the GDF11/activin B trap comprises an altered ActRII ligand-binding domain that has a ratio of K_{D} for activin A binding to K_{D} for GDF11 binding that is at least 2-, 5-, 10-, 100-, or even 1000-fold greater relative to the ratio for the unmodified (wild-type) ActRII ligand-binding domain. Optionally, the GDF11/activin B trap comprises an altered ActRII ligand-binding domain that has a ratio of K_{D} for activin A binding to K_{D} for activin B binding that is at least 2-, 5-, 10-, 100-, or even 1000-fold greater relative to the ratio for the unmodified (wild-type) ActRII ligand-binding domain. Optionally, the GDF11/activin B trap comprises an altered ActRII ligand-binding domain that has a ratio of IC₅₀ for inhibiting activin A to IC₅₀ for inhibiting GDF11 that is at least 2-, 5-, 10-, 100- or even 1000-fold greater relative to the unmodified (wild-type) ActRII ligand-binding domain. Optionally, the GDF11/activin B trap comprises an altered ActRII ligand-binding domain that has a ratio of IC₅₀ for inhibiting activin A to IC₅₀ for inhibiting activin B that is at least 2-, 5-, 10-, 100- or even 1000-fold greater relative to the unmodified (wild-type) ActRII ligand-binding domain. Optionally, the GDF11/activin B trap comprises an altered ligand-binding domain that inhibits GDF11 with an IC₅₀ at least 2-, 5-, 10-, or even 100-times less than the IC₅₀ for inhibiting activin A. Optionally, the GDF11/activin B trap comprises an altered ligand-binding domain that inhibits activin B with an IC₅₀ at least 2-, 5-, 10-, or even 100-times less than the IC₅₀ for inhibiting activin A.

In some embodiments, a GDF11/activin B trap of the present disclosure optionally further binds to and/or inhibits activity of one or more of BMP6, activin C, activin E, activin A, GDF15, Nodal, GDF3, BMP3, BMP3B, BMP9, and BMP10, and GDF8.

In some embodiments, a GDF11/activin B trap of the present disclosure comprises an amino acid sequence that is at least 80%, 85%, 90%, 95%, 96%, 97%, 98%, 99%, or 100% identical to amino acids 29-109 of SEQ ID NO:1 or 2. In preferred embodiments, GDF11/activin B traps of the present disclosure do not comprise or consist of amino acids 29-109 of SEQ ID NO:1 or 2. In other preferred embodiments, GDF11/activin B traps of the present disclosure do not comprise an acidic amino acid [e.g., aspartic (D) or glutamic (E) acid] at position 79 with respect to SEQ ID NO: 1 or 2.

In other embodiments, the GDF11/activin B trap comprises an amino acid sequence that is at least 80%, 85%, 90%, 95%, 96%, 97%, 98%, 99%, or 100%to any one of SEQ ID Nos: 3, 4, 5, 6, 21, 23, 48, and 49. In preferred embodiments, GDF11/activin B traps of the present disclosure do not comprise or consist of the amino acid sequence of any one of SEQ ID Nos: 3, 4, 5, 6, 21, 23, 48, and 49.

In some embodiments, a GDF11/activin B trap of the present disclosure comprises an amino acid sequence that is at least 80%, 85%, 90%, 95%, 96%, 97%, 98%, 99%, or 100% identical to amino acids 30-110 of SEQ ID NO:9. In preferred embodiments, a GDF11/activin B trap of the present disclosure does not comprise or consist of amino acids 30-110 of SEQ ID NO:9. In other embodiments, the GDF11/activin trap comprises an amino acid sequence that is at least 80%, 85%, 90%, 95%, 96%, 97%, 98%, or 99% to any one of SEQ ID Nos: 10, 11, 18, and 20. In preferred embodiments, GDF11/activin B traps of the present disclosure do not comprise or consist of the amino acid sequence of any one of SEQ ID Nos: 10, 11, 18, and 20.

As disclosed herein, GDF11 traps are variant ActRII polypeptides (e.g. variant ActRIIA or ActRIIB polypeptides) that bind to and/or antagonize (inhibit) the activity of GDF11 (*e.g.*, GDF11-mediated activation of the ActRIIA or ActRIIB Smad2/3 signaling pathway), but that do not substantially bind to and/or inhibit activity of activin B (e.g., activin B-mediated activation of the ActRIIA or ActRIIB Smad2/3 signaling pathway). Thus, the present disclosure provides GDF11 traps that comprise an altered ligand-binding domain of an ActRII receptor [*e*.*g*., comprising one or more amino acid mutations (*e.g.*, amino acid substitutions, additions, or deletions)] which are characterized, in part, by increased selectivity for GDF11 relative to an unmodified (wild-type) ligand-binding domain of an ActRII receptor. Optionally, GDF11 traps do not bind to and/or inhibit activin A activity (*e*.*g*., activin A-mediated activation of the ActRIIA or ActRIIB Smad2/3 signaling pathway).

In some embodiments, GDF11 traps of the disclosure have equivalent or enhanced increased binding affinity (*e.g.*, 2-, 3-, 10-, 100-, 1000-fold increased binding affinity) binding affinity for GDF11 relative to an unmodified (wild-type) ligand-binding domain of an ActRII receptor. Optionally, the GDF11 trap comprises an altered ActRII ligand-binding domain that has a ratio of K_{D} for activin A binding to K_{D} for GDF11 binding that is at least 2-, 5-, 10-, 100-, or even 1000-fold greater relative to the ratio for the unmodified (wild-type) ActRII ligand-binding domain. Optionally, the GDF11 trap comprises an altered ActRII ligand-binding domain that has a ratio of K_{D} for activin B binding to K_{D} for GDF11 binding that is at least 2-, 5-, 10-, 100-, or even 1000-fold greater relative to the ratio for the unmodified (wild-type) ActRII ligand-binding domain. Optionally, the GDF11 trap comprises an altered ActRII ligand-binding domain that has a ratio of IC₅₀ for inhibiting activin A to IC₅₀ for inhibiting GDF11 that is at least 2-, 5-, 10-, 100- or even 1000-fold greater relative to the unmodified (wild-type) ActRII ligand-binding domain. Optionally, the GDF11 trap comprises an altered ActRII ligand-binding domain that has a ratio of IC₅₀ for inhibiting activin B to IC₅₀ for inhibiting GDF11 that is at least 2-, 5-, 10-, 100- or even 1000-fold greater relative to the unmodified (wild-type) ActRII ligand-binding domain. Optionally, the GDF11 trap comprises an altered ligand-binding domain that inhibits GDF11 with an IC₅₀ at least 2-, 5-, 10-, or even 100-fold less than the IC₅₀ for inhibiting activin A. Optionally, the GDF11 trap comprises an altered ligand-binding domain that inhibits GDF11 with an IC₅₀ at least 2-, 5-, 10-, or even 100-fold less than the IC₅₀ for inhibiting activin B.

In some embodiments, a GDF11 trap of the present disclosure optionally further binds to and/or inhibits activity of one or more of BMP6, activin C, activin E, activin A, GDF15, Nodal, GDF3, BMP3, BMP3B, BMP9, BMP10, and GDF8.

In some embodiments, a GDF11 trap of the present disclosure comprises an amino acid sequence that is at least 80%, 85%, 90%, 95%, 96%, 97%, 98%, 99%, or 100% identical to amino acids 29-109 of SEQ ID NO:1 or 2. In preferred embodiments, GDF11 traps of the present disclosure do not comprise or consist of amino acids 29-109 of SEQ ID NO:1 or 2. In other preferred embodiments, GDF11 traps of the present disclosure do not comprise an acidic amino acid [e.g., aspartic (D) or glutamic (E) acid] at position 79 with respect to SEQ ID NO: 1 or 2.

In other embodiments, the GDF11 trap comprises an amino acid sequence that is at least 80%, 85%, 90%, 95%, 96%, 97%, 98%, 99%, or 100% to any one of SEQ ID Nos: 3, 4, 5, 6, 21, 23, 48 and 49. In preferred embodiments, GDF11 traps of the present disclosure do not comprise or consist of the amino acid sequence of any one of SEQ ID Nos: 3, 4, 5, 6, 21, 23, 48, and 49.

In some embodiments, a GDF11 trap of the present disclosure comprises an amino acid sequence that is at least 80%, 85%, 90%, 95%, 96%, 97%, 98%, 99%, or 100% identical to amino acids 30-110 of SEQ ID NO:9. In preferred embodiments, a GDF11 trap of the present disclosure does not comprise or consist of amino acids 30-110 of SEQ ID NO:9. In other embodiments, the GDF11 trap comprises an amino acid sequence that is at least 80%, 85%, 90%, 95%, 96%, 97%, 98%, 99%, or 100% to any one of SEQ ID Nos: 10, 11, 18, and 20. In preferred embodiments, GDF11 traps of the present disclosure do not comprise or consist of the amino acid sequence of any one of SEQ ID Nos: 10, 11, 18, and 20.

As disclosed herein, activin B traps are variant ActRII polypeptides (*e*.*g*. variant ActRIIA or ActRIIB polypeptides) that bind to and/or antagonize (inhibit) the activity of activin B (e.g., activin B-mediated activation of the ActRIIA or ActRIIB Smad2/3 signaling pathway), but that do not substantially bind to and/or inhibit activity of GDF11 (*e.g.*, GDF11-mediated activation of the ActRIIA or ActRIIB Smad2/3 signaling pathway). Thus, the present disclosure provides activin B traps that comprise an altered ligand-binding domain of an ActRII receptor [*e*.*g*., comprising one or more amino acid mutations (*e.g.*, amino acid substitutions, additions, or deletions)] which are characterized, in part, by increased selectivity for activin B relative to an unmodified (wild-type) ligand-binding domain of an ActRII receptor. Optionally, activin B traps do not substantially bind to and/or inhibits activin A activity (*e*.*g*., activin A-mediated activation of the ActRIIA or ActRIIB Smad2/3 signaling pathway)

In some embodiments, activin B traps of the disclosure have equivalent or increased binding affinity (*e.g.*, 2-, 3-, 10-, 100-, 1000-fold increased binding affinity) for activin B relative to an unmodified (wild-type) ligand-binding domain of an ActRII receptor. Optionally, the activin B Trap comprises an altered ActRII ligand-binding domain that has a ratio of K_{D} for activin A binding to K_{D} for activin B binding that is at least 2-, 5-, 10-, 100-, or even 1000-fold greater relative to the ratio for the unmodified (wild-type) ActRII ligand-binding domain. Optionally, the activin B trap comprises an altered ActRII ligand-binding domain that has a ratio of K_{D} for GDF11 binding to K_{D} for activin B binding that is at least 2-, 5-, 10-, 100-, or even 1000- fold greater relative to the ratio for the unmodified (wild-type) ActRII ligand-binding domain. Optionally, the activin B trap comprises an altered ActRII ligand-binding domain that has a ratio of IC₅₀ for inhibiting activin A to IC₅₀ for inhibiting activin B that is at least 2-, 5-, 10-, 100- or even 1000-fold greater relative to the unmodified (wild-type) ActRII ligand-binding domain. Optionally, the activin B trap comprises an altered ActRII ligand-binding domain that has a ratio of IC₅₀ for inhibiting GDF11 to IC₅₀ for inhibiting activin B that is at least 2-, 5-, 10-, 100- or even 1000-fold greater relative to the unmodified (wild-type) ActRII ligand-binding domain. Optionally, the activin B trap comprises an altered ligand-binding domain that inhibits activin B with an IC₅₀ at least 2-, 5-, 10-, or even 100-fold less than the IC₅₀ for inhibiting activin A. Optionally, the activin B trap comprises an altered ligand-binding domain that inhibits activin B with an IC₅₀ at least 2-, 5-, 10-, or even 100-fold less than the IC₅₀ for inhibiting GDF11.

In some embodiments, an activin B trap of the present disclosure optionally further binds to and/or inhibits activity of one or more of BMP6, activin C, activin E, activin A, GDF15, Nodal, BMP3, GDF3, BMP3B, BMP9, BMP10, and GDF8.

In some embodiments, an activin B trap of the present disclosure comprises an amino acid sequence that is at least 80%, 85%, 90%, 95%, 96%, 97%, 98%, 99%, or 100% identical to amino acids 29-109 of SEQ ID NO:1 or 2. In preferred embodiments, activin B traps of the present disclosure do not comprise or consist of amino acids 29-109 of SEQ ID NO:1 or 2. In other preferred embodiments, GDF11 traps of the present disclosure do not comprise an acidic amino acid [e.g., aspartic (D) or glutamic (E) acid] at position 79 with respect to SEQ ID NO: 1 or 2.

In other embodiments, the activin B trap comprises an amino acid sequence that is at least 80%, 85%, 90%, 95%, 96%, 97%, 98%, 99%, or 100% to any one of SEQ ID Nos: 3, 4, 5, 6, 21, 23, 48, and 49. In preferred embodiments, activin B traps of the present disclosure do not comprise or consist of the amino acid sequence of any one of SEQ ID Nos: 3, 4, 5, 6, 21, 23, 48, and 49.

In some embodiments, an activin B trap of the present disclosure comprises an amino acid sequence that is at least 80%, 85%, 90%, 95%, 96%, 97%, 98%, 99%, or 100% identical to amino acids 30-110 of SEQ ID NO:9. In preferred embodiments, an activin B trap of the present disclosure does not comprise or consist of amino acids 30-110 of SEQ ID NO:9. In other embodiments, the activin B trap comprises an amino acid sequence that is at least 80%, 85%, 90%, 95%, 96%, 97%, 98%, 99%, or 100% to any one of SEQ ID Nos: 10, 11, 18, and 20. In preferred embodiments, activin B traps of the present disclosure do not comprise or consist of the amino acid sequence of any one of SEQ ID Nos: 10, 11, 18, and 20.

As will be recognized by one of skill in the art, most of the described mutations, variants or modifications may be made at the nucleic acid level or, in some cases, by post translational modification or chemical synthesis. Such techniques are well known in the art and described herein.

ActRII proteins have been well characterized in the art in terms of structural and functional characteristics, particularly with respect to ligand-binding [see, *e.g.*, Attisano et al. (1992) Cell 68(1):97-108; Greenwald et al. (1999) Nature Structural Biology 6(1): 18-22; Allendorph et al. (2006) PNAS 103(20: 7643-7648; Thompson et al. (2003) The EMBO Journal 22(7): 1555-1566; and U.S. Patent Nos: 7,709,605; 7,612,041; and 7,842,663]. For example, Attisano *et al.* showed that a deletion of the proline knot at the C-terminus of the extracellular domain of ActRIIB reduced the affinity of the receptor for activin. An ActRIIB-Fc fusion protein containing amino acids 20-119 of SEQ ID NO:1, "ActRIIB(20-119)-Fc", has reduced binding to GDF-11 and activin relative to an ActRIIB(20-134)-Fc, which includes the proline knot region and the complete juxtamembrane domain (see, *e.g.*, U.S. Patent No. 7,842,663). However, an ActRIIB(20-129)-Fc protein retains similar but somewhat reduced activity relative to the wild type, even though the proline knot region is disrupted. Thus, ActRIIB extracellular domains that stop at amino acid 134, 133, 132, 131, 130 and 129 (with respect to SEQ ID NO:1 or 2) are all expected to be active, but constructs stopping at 134 or 133 may be most active. Similarly, mutations at any of residues 129-134 (with respect to SEQ ID NO:1 or 2) are not expected to alter ligand-binding affinity by large margins. In support of this, mutations of P129 and P130 (with respect to SEQ ID NO:1 or 2) do not substantially decrease ligand binding. Therefore, an ActRIIB trap polypeptide of the present disclosure may end as early as amino acid 109 (the final cysteine), however, forms ending at or between 109 and 119 are expected to have reduced ligand-binding. Amino acid 119 (with respect to SEQ ID NO:1 or 2) is poorly conserved and so is readily altered or truncated. ActRIIB-based ligand traps ending at 128 (with respect to SEQ ID NO:1 or 2) or later retain ligand-binding activity. ActRIIB-based ligand traps ending at or between 119 and 127 (with respect to SEQ ID NO:1 or 2) will have an intermediate binding ability. Any of these forms may be desirable to use, depending on the clinical or experimental setting.

At the N-terminus of ActRIIB, it is expected that a protein beginning at amino acid 29 or before (with respect to SEQ ID NO:1 or 2) will retain ligand-binding activity. Amino acid 29 represents the initial cysteine. An alanine to asparagine mutation at position 24 (with respect to SEQ ID NO:1 or 2) introduces an N-linked glycosylation sequence without substantially affecting ligand-binding (see, *e.g.*, U.S. Patent No. 7,842,663). This confirms that mutations in the region between the signal cleavage peptide and the cysteine cross-linked region, corresponding to amino acids 20-29 are well tolerated. In particular, ActRIIB-based ligand trap constructs beginning at position 20, 21, 22, 23, and 24 (with respect to SEQ ID NO:1 or 2) will retain general ligand-biding activity, and ActRIIB-based ligand trap constructs beginning at positions 25, 26, 27, 28, and 29 (with respect to SEQ ID NO:1 or 2) are also expected to retain ligand-biding activity. Data shown in, *e.g.*, U.S. Patent No. 7,842,663 demonstrates that, surprisingly, an ActRIIB construct beginning at 22, 23, 24, or 25 will have the most activity.

Taken together, an active portion (*e*.*g*., ligand binding activity) of ActRIIB comprises amino acids 29-109 of SEQ ID NO:1 or 2. Therefore ActRIIB-based ligand trap constructs of the present disclosure may, for example, comprise an amino acid sequence that is at least 80%, 85%, 90%, 95%, 96%, 97%, 98% 99%, or 100% identical to a portion of ActRIIB beginning at a residue corresponding to amino acids 20-29 (*e*.*g*., beginning at amino acid 20, 21, 22, 23, 24, 25, 26, 27, 28, or 29) of SEQ ID NO: 1 or 2 and ending at a position corresponding to amino acids 109-134 (*e.g.*, ending at amino acid 109, 110, 111, 112, 113, 114, 115, 116, 117, 118, 119, 120, 121, 122, 123, 124, 125, 126, 127, 128, 129, 130, 131, 132, 133, or 134) of SEQ ID NO: 1 or 2. In preferred embodiments, ActRIIB-based ligand trap polypeptides of the present disclosure do not comprise an acidic amino acid [e.g., aspartic (D) or glutamic (E) acid] at the position corresponding to position 79 of SEQ ID NO: 1 or 2. In other preferred embodiments, ActRIIB-based ligand trap polypeptides of the present disclosure do not comprise or consist of amino acids 29-109 of SEQ ID NO:1 or 2. Other examples include ActRIIB-based ligand trap constructs that begin at a position from 20-29 (*e.g.*, position 20, 21, 22, 23, 24, 25, 26, 27, 28, or 29) or 21-29 (*e.g.*, position 21, 22, 23, 24, 25, 26, 27, 28, or 29) and end at a position from 119-134 (*e*.*g*., 119, 120, 121, 122, 123, 124, 125, 126, 127, 128, 129, 130, 131, 132, 133, or 134), 119-133 (*e.g.*, 119, 120, 121, 122, 123, 124, 125, 126, 127, 128, 129, 130, 131, 132, or 133), 129-134 (*e.g.*, 129, 130, 131, 132, 133, or 134), or 129-133 (*e.g.*, 129, 130, 131, 132, or 133) of SEQ ID NO: 1 or 2. Other examples include constructs that begin at a position from 20-24 (*e.g.*, 20, 21, 22, 23, or 24), 21-24 (*e.g.*, 21, 22, 23, or 24), or 22-25 (*e.g.*, 22, 22, 23, or 25) and end at a position from 109-134 (*e.g.*, 109, 110, 111, 112, 113, 114, 115, 116, 117, 118, 119, 120, 121, 122, 123, 124, 125, 126, 127, 128, 129, 130, 131, 132, 133, or 134), 119-134 (*e.g.*, 119, 120, 121, 122, 123, 124, 125, 126, 127, 128, 129, 130, 131, 132, 133, or 134) or 129-134 (*e.g.*, 129, 130, 131, 132, 133, or 134) of SEQ ID NO: 1 or 2. Variants within these ranges are also contemplated, particularly those having at least 80%, 85%, 90%, 95%, 96%, 97%, 98%, 99%, or 100%identity to the corresponding portion of SEQ ID NO: 1 or 2, optionally such ActRIIB-based ligand trap polypeptides i) do not comprise an acidic amino acid [*e*.*g*., aspartic (D) or glutamic (E) acid] at the position corresponding to position 79 of SEQ ID NO: 1 or 2, and ii) do not comprise or consist of amino acids 29-109 of SEQ ID NO NO: 1 or 2. In certain embodiments, the ActRIIB-based ligand trap polypeptide comprises a polypeptide having an amino acid sequence that is at least 80%, 85%, 90%, 95%, 96%, 97%, 98%, 99%, or 100% identical to amino acid residues 25-131 of SEQ ID NO: 1 or 2. In preferred embodiments, ActRIIB-based ligand trap polypeptides do not comprise or consist of amino acids 25-131 of SEQ ID NO: 1 or 2. In certain embodiments, the ActRIIB-based trap polypeptide comprises a polypeptide having an amino acid sequence that is at least 80%, 85%, 90%, 95%, 96%, 97%, 98%, 99%, or 100% identical to SEQ ID NOs: 3, 4, 5, 6, 21, and 23, provided that: i) the ActRIIB-based trap polypeptide does not comprise an acidic amino acid [e.g., aspartic (D) or glutamic (E) acid] at the position corresponding to position 79 of SEQ ID NO: 1 or 2, and ii) the ActRIIB-based trap polypeptide does not comprise or consist of amino acids 29-109 of SEQ ID NO NO: 1 or 2.

The disclosure includes the results of an analysis of composite ActRIIB structures, shown in Figure 1, demonstrating that the ligand-binding pocket is defined, in part, by residues Y31, N33, N35, L38 through T41, E47, E50, Q53 through K55, L57, H58, Y60, S62, K74, W78 through N83, Y85, R87, A92, and E94 through F101. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Thus, a general formula for an ActRIIB-based ligand trap protein is one that comprises an amino acid sequence that is at least 80%, 85%, 90%, 95%, 96%, 97%, 98%, 99%, or 100% identical to amino acids 29-109 of SEQ ID NO: 1 or 2, optionally beginning at a position ranging from 20-24 (*e.g.*, 20, 21, 22, 23, or 24) or 22-25(*e.g.*, 22, 23, 24, or 25) and ending at a position ranging from 129-134 (*e.g.*, 129, 130, 131, 132, 133, or 134), and comprising no more than 1, 2, 5, 10 or 15 conservative amino acid changes in the ligand-binding pocket, and zero, one or more non-conservative alterations at positions 40, 53, 55, 74, 79 and/or 82 in the ligand-binding pocket. In preferred embodiments, ActRIIB-based trap polypeptides of the present disclosure do not comprise an acidic amino acid [*e*.*g*., aspartic (D) or glutamic (E) acid] at the position corresponding to position 79 of SEQ ID NO: 1 or 2. In other preferred embodiments, ActRIIB-based ligand trap polypeptides of the present disclosure do not comprise or consist of amino acids 29-109 of SEQ ID NO:1 or 2. Sites outside the binding pocket, at which variability may be particularly well tolerated, include the amino and carboxy termini of the extracellular domain (as noted above), and positions 42-46 and 65-73 (with respect to SEQ ID NO:1). An asparagine to alanine alteration at position 65 (N65A) actually improves ligand-binding in the A64 background, and is thus expected to have no detrimental effect on ligand-binding in the R64 background (see, *e.g.*, U.S. Patent No. 7,842,663). This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64 (see, *e.g.*, U.S. Patent No. 7,842,663).

ActRIIB is well-conserved across nearly all vertebrates, with large stretches of the extracellular domain conserved completely. Many of the ligands that bind to ActRIIB are also highly conserved. Accordingly, comparisons of ActRIIB sequences from various vertebrate organisms provide insights into residues that may be altered. Therefore, an active, human ActRIIB variant polypeptide useful in accordance with the presently disclosed methods may include one or more amino acids at corresponding positions from the sequence of another vertebrate ActRIIB, or may include a residue that is similar to that in the human or other vertebrate sequence. The following examples illustrate this approach to defining an active ActRIIB variant. L46 is a valine in *Xenopus* ActRIIB, and so this position may be altered, and optionally may be altered to another hydrophobic residue, such as V, I or F, or a non-polar residue such as A. E52 is a K in *Xenopus*, indicating that this site may be tolerant of a wide variety of changes, including polar residues, such as E, D, K, R, H, S, T, P, G, Y and probably A. T93 is a K in *Xenopus*, indicating that a wide structural variation is tolerated at this position, with polar residues favored, such as S, K, R, E, D, H, G, P, G and Y. F108 is a Y in *Xenopus*, and therefore Y or other hydrophobic group, such as I, V or L should be tolerated. E111 is K in *Xenopus*, indicating that charged residues will be tolerated at this position, including D, R, K and H, as well as Q and N. R112 is K in *Xenopus*, indicating that basic residues are tolerated at this position, including R and H. A at position 119 is relatively poorly conserved, and appears as P in rodents and V in *Xenopus*, thus essentially any amino acid should be tolerated at this position.

It has been previously demonstrated that the addition of a further N-linked glycosylation site (N-X-S/T) is well-tolerated relative to the ActRIIB(R64)-Fc form (see, *e.g.*, U.S. Patent No. 7,842,663). By introducing an asparagine at position 24 (A24N construct; with respect to SEQ ID NO:1), an NXT sequence is created that may confer a longer half-life. Other NX(T/S) sequences are found at 42-44 (NQS) and 65-67 (NSS), although the latter may not be efficiently glycosylated with the R at position 64. N-X-S/T sequences may be generally introduced at positions outside the ligand binding pocket defined in Figure 1. Particularly suitable sites for the introduction of non-endogenous N-X-S/T sequences include amino acids 20-29, 20-24, 22-25, 109-134, 120-134 or 129-134 (with respect to SEQ ID NO:1). N-X-S/T sequences may also be introduced into the linker between the ActRIIB sequence and an Fc domain or other fusion component. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E105N, R112N, G120N, E123N, P129N, A132N, R112S and R112T (with respect to SEQ ID NO:1). Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T (with respect to SEQ ID NO:1) are contemplated. Likewise, in an A24N variant, an S26T alteration may be used. Accordingly, an ActRIIB-based ligand trap polypeptide of the present disclosure may be an ActRIIB variant having one or more additional, non-endogenous N-linked glycosylation consensus sequences as described above.

The variations described may be combined in various ways. Additionally, the results of the mutagenesis program described herein indicate that there are amino acid positions in ActRIIB that are often beneficial to conserve. With respect to SEQ ID NO:1, these include position 64 (basic amino acid), position 80 (acidic or hydrophobic amino acid), position 78 (hydrophobic, and particularly tryptophan), position 37 (acidic, and particularly aspartic or glutamic acid), position 56 (basic amino acid), position 60 (hydrophobic amino acid, particularly phenylalanine or tyrosine). Thus, in each of the traps disclosed herein, the disclosure provides a framework of amino acids that may be conserved. Other positions that may be desirable to conserve are as follows: position 52 (acidic amino acid), position 55 (basic amino acid), position 81 (acidic), 98 (polar or charged, particularly E, D, R or K), all with respect to SEQ ID NO:1.

A general formula for an active ActRIIA polypeptide is one that comprises a polypeptide that is at least 80%, 85%, 90%, 95%, 96%, 97%, 98%, 99%, or 100% identical to amino acids 30-110 of SEQ ID NO:9. Accordingly, ActRIIA-based ligand traps of the present disclosure may comprise a polypeptide that is at least 80%, 85%, 90%, 95%, 96%, 97%, 98%, 99%, or 100% identical to amino acids 30-110 of SEQ ID NO:9. In preferred embodiments, ActRIIA-based ligand traps of the present disclosure do not comprise or consist of amino acids 30-110 of SEQ ID NO:9. Optionally, ActRIIA-based ligand trap polypeptides of the present disclosure comprise a polypeptide that is at least 80%, 85%, 90%, 95%, 96%, 97%, 98%, 99%, or 100% identical to amino acids amino acids 12-82 of SEQ ID NO:9 optionally beginning at a position ranging from 1-5 (*e.g.*, 1, 2, 3, 4, or 5) or 3-5 (*e.g.*, 3, 4, or 5) and ending at a position ranging from 110-116 (*e.g.*, 110, 111, 112, 113, 114, 115, or 116) or 110-115 (*e.g.*, 110, 111, 112, 113, 114, or 115), respectively, and comprising no more than 1, 2, 5, 10 or 15 conservative amino acid changes in the ligand binding pocket, and zero, one or more non-conservative alterations at positions 40, 53, 55, 74, 79 and/or 82 in the ligand-binding pocket (with respect to SEQ ID NO:9).

Functionally active fragments of ligand traps of the disclosure (*e*.*g*. GDF11 traps, activin B traps, or GDF11/activin B traps) can be obtained by screening polypeptides recombinantly produced from the corresponding fragment of the nucleic acid encoding an ligand trap polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of GDF11 and/or activin B but that do not substantially bind to and/or inhibit the activity of activin A.

Functional variants may be generated by modifying the structure of a ligand trap of the present disclosure (*e*.*g*. a GDF11 trap, activin B trap, or GDF11/activin B trap) for such purposes as enhancing therapeutic efficacy, or stability (*e*.*g*., shelf-life and resistance to proteolytic degradation *in vivo*). Such modified ligand trap polypeptides when selected to retain GDF11 and/or activin B binding, are considered functional equivalents of the naturally-occurring ActRII polypeptides. Modified ligand trap polypeptides can also be produced, for instance, by amino acid substitution, deletion, or addition. For instance, it is reasonable to expect that an isolated replacement of a leucine with an isoleucine or valine, an aspartate with a glutamate, a threonine with a serine, or a similar replacement of an amino acid with a structurally related amino acid (*e*.*g*., conservative mutations) will not have a major effect on the biological activity of the resulting molecule. Conservative replacements are those that take place within a family of amino acids that are related in their side chains. Whether a change in the amino acid sequence of an ligand trap polypeptide results in a functional homolog can be readily determined by assessing the ability of the variant ligand trap polypeptide to produce a response in cells in a fashion similar to the wild-type ActRII polypeptide, or to bind to one or more ligands, such as GDF11, activin A, activin B, activin C, activin E, GDF8, BMP6, BMP9, BMP10, GDF3, BMP3, BMP3B, Nodal, andGDF15, as compared to the unmodified ActRII polypeptide or a wild-type ActRII polypeptide.

In certain embodiments, the present disclosure contemplates specific mutations of the ligand trap polypeptides of the present disclosure (*e*.*g*. GDF11 traps, activin B traps, or GDF11/activin B traps) so as to alter the glycosylation of the polypeptide. Such mutations may be selected so as to introduce or eliminate one or more glycosylation sites, such as O-linked or N-linked glycosylation sites. Asparagine-linked glycosylation recognition sites generally comprise a tripeptide sequence, asparagine-X-threonine or asparagine-X-serine (where "X" is any amino acid) which is specifically recognized by appropriate cellular glycosylation enzymes. The alteration may also be made by the addition of, or substitution by, one or more serine or threonine residues to the sequence of the ligand trap polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on an ligand trap polypeptide is by chemical or enzymatic coupling of glycosides to the polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. Removal of one or more carbohydrate moieties present on an ActRII polypeptide may be accomplished chemically and/or enzymatically. Chemical deglycosylation may involve, for example, exposure of the ligand trap polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Enzymatic cleavage of carbohydrate moieties on ligand trap polypeptides can be achieved by the use of a variety of endo- and exo-glycosidases as described by Thotakura et al. [Meth. Enzymol. (1987) 138:350]. The sequence of an ligand trap polypeptide may be adjusted, as appropriate, depending on the type of expression system used, as mammalian, yeast, insect and plant cells may all introduce differing glycosylation patterns that can be affected by the amino acid sequence of the peptide. In general, ligand trap proteins for use in humans may be expressed in a mammalian cell line that provides proper glycosylation, such as HEK293 or CHO cell lines, although other mammalian expression cell lines are expected to be useful as well.

This disclosure further contemplates a method of generating mutants, particularly sets of combinatorial mutants of ligand trap polypeptide (*e.g.* a GDF11 trap, activin B trap, or GDF11/activin B trap), as well as truncation mutants; pools of combinatorial mutants are especially useful for identifying ligand trap sequences. The purpose of screening such combinatorial libraries may be to generate, for example, ligand trap polypeptides which bind to activin B, GDF11, and optionally other ligands but do not substantially bind to activin A. A variety of screening assays are provided below, and such assays may be used to evaluate variants. For example, a ligand trap may be screened for ability to bind to an ActRII ligand (*e.g.*, GDF11 and/or activin B), to prevent binding of an ActRII ligand to an ActRII polypeptide or to interfere with signaling caused by an ActRII ligand.

The activity of a ligand trap (*e.g.* a GDF11 trap, activin B trap, or GDF11/activin B trap) or its variants may also be tested in a cell-based or *in vivo* assay. For example, the effect of a ligand trap on the expression of genes involved in hematopoiesis may be assessed. This may, as needed, be performed in the presence of one or more recombinant ActRII ligand proteins (*e*.*g*., GDF11 and/or activin B), and cells may be transfected so as to produce a ligand trap polypeptide and/or variants thereof, and optionally, an ActRII ligand. Likewise, a ligand trap may be administered to a mouse or other animal, and one or more blood measurements, such as an RBC count, hemoglobin, or reticulocyte count may be assessed using art recognized methods.

Combinatorial-derived ligand traps (*e.g.* a GDF11 trap, activin B trap, or GDF11/activin B trap) can be generated which have a selective or generally increased potency relative to a reference ligand trap. Such variants, when expressed from recombinant DNA constructs, can be used in gene therapy protocols. Likewise, mutagenesis can give rise to variants which have intracellular half-lives dramatically different than the corresponding unmodified ligand trap. For example, the altered protein can be rendered either more stable or less stable to proteolytic degradation or other cellular processes which result in destruction of, or otherwise inactivation of an unmodified ligand trap. Such variants, and the genes which encode them, can be utilized to ligand trap polypeptide levels by modulating the half-life of the ligand trap. For instance, a short half-life can give rise to more transient biological effects and, when part of an inducible expression system, can allow tighter control of recombinant ligand trap polypeptide levels within the cell. In an Fc fusion protein, mutations may be made in the linker (if any) and/or the Fc portion to alter the half-life of the protein.

A combinatorial library may be produced by way of a degenerate library of genes encoding a library of polypeptides which each include at least a portion of potential ActRII polypeptide sequences. For instance, a mixture of synthetic oligonucleotides can be enzymatically ligated into gene sequences such that the degenerate set of potential ActRII polypeptide nucleotide sequences are expressible as individual polypeptides, or alternatively, as a set of larger fusion proteins (*e*.*g*., for phage display).

There are many ways by which the library of potential homologs can be generated from a degenerate oligonucleotide sequence. Chemical synthesis of a degenerate gene sequence can be carried out in an automatic DNA synthesizer, and the synthetic genes can then be ligated into an appropriate vector for expression. The synthesis of degenerate oligonucleotides is well known in the art [see, *e*.*g*., Narang, SA (1983) Tetrahedron 39:3; Itakura et al. (1981) Recombinant DNA, Proc. 3rd Cleveland Sympos. Macromolecules, ed. AG Walton, Amsterdam: Elsevier pp273-289; Itakura et al. (1984) Annu. Rev. Biochem. 53:323; Itakura et al. (1984) Science 198:1056; Ike et al. (1983) Nucleic Acid Res. 11:477]. Such techniques have been employed in the directed evolution of other proteins [see, *e.g.*, Scott et al., (1990) Science 249:386-390; Roberts et al. (1992) PNAS USA 89:2429-2433; Devlin et al. (1990) Science 249: 404-406; Cwirla et al., (1990) PNAS USA 87: 6378-6382; as well as U.S. Patent Nos: 5,223,409; 5,198,346; and 5,096,815)].

Alternatively, other forms of mutagenesis can be utilized to generate a combinatorial library. For example, ligand traps of the present disclosure can be generated and isolated from a library by screening using, for example, alanine scanning mutagenesis [see, *e.g.*, Ruf et al. (1994) Biochemistry 33:1565-1572; Wang et al. (1994) J. Biol. Chem. 269:3095-3099; Balint et al. (1993) Gene 137:109-118; Grodberg et al. (1993) Eur. J. Biochem. 218:597-601; Nagashima et al. (1993) J. Biol. Chem. 268:2888-2892; Lowman et al. (1991) Biochemistry 30:10832-10838; and Cunningham et al. (1989) Science 244:1081-1085], by linker scanning mutagenesis [see, *e.g.*, Gustin et al. (1993) Virology 193:653-660; and Brown et al. (1992) Mol. Cell Biol. 12:2644-2652; McKnight et al. (1982) Science 232:316)], by saturation mutagenesis [see, *e.g.,* Meyers et al., (1986) Science 232:613]; by PCR mutagenesis [see, *e.g.,* Leung et al. (1989) Method Cell Mol Biol 1:11-19]; or by random mutagenesis, including chemical mutagenesis [see, *e.g.,* Miller et al. (1992) A Short Course in Bacterial Genetics, CSHL Press, Cold Spring Harbor, NY; and Greener et al. (1994) Strategies in Mol Biol 7:32-34]. Linker scanning mutagenesis, particularly in a combinatorial setting, is an attractive method for identifying truncated (bioactive) forms of ActRII polypeptides.

A wide-range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ligand traps of the disclosure. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include GDF11, activin B, and/or activin binding assays and GDF11-, activin-B-, and/or activin-mediated cell signaling assays.

In certain embodiments, the ligand traps (*e.g.* a GDF11 trap, activin B trap, or GDF11/activin B trap) of the present disclosure may further comprise post-translational modifications in addition to any that are naturally present in the ActRII polypeptide (e.g., an ActRIIA or ActRIIB polypeptide). Such modifications include, but are not limited to, acetylation, carboxylation, glycosylation, phosphorylation, lipidation, and acylation. As a result, the modified ligand trap polypeptides may contain non-amino acid elements, such as polyethylene glycols, lipids, poly- or mono-saccharide, and phosphates. Effects of such non-amino acid elements on the functionality of a ligand trap polypeptide may be tested as described herein for other ligand trap polypeptide variants. When a ligand trap polypeptide is produced in cells by cleaving a nascent form of the ligand trap polypeptide, post-translational processing may also be important for correct folding and/or function of the protein. Different cells (*e.g.*, CHO, HeLa, MDCK, 293, WI38, NIH-3T3 or HEK293) have specific cellular machinery and characteristic mechanisms for such post-translational activities and may be chosen to ensure the correct modification and processing of the ligand trap polypeptides.

In certain aspects, ligand traps of the present disclosure (*e*.*g*. a GDF11 trap, activin B trap, or GDF11/activin B trap) include fusion proteins having at least a portion (domain) of an ActRII polypeptide (*e*.*g*., an ActRIIA or ActRIIB polypeptide) and one or more heterologous portions (domains). Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt- conjugated resins are used. Many of such matrices are available in "kit" form, such as the Pharmacia GST purification system and the QIAexpress^{™} system (Qiagen) useful with (HIS₆) fusion partners. As another example, a fusion domain may be selected so as to facilitate detection of the ligand trap polypeptides. Examples of such detection domains include the various fluorescent proteins (*e*.*g*., GFP) as well as "epitope tags," which are usually short peptide sequences for which a specific antibody is available. Well known epitope tags for which specific monoclonal antibodies are readily available include FLAG, influenza virus haemagglutinin (HA), and c-myc tags. In some cases, the fusion domains have a protease cleavage site, such as for Factor Xa or thrombin, which allows the relevant protease to partially digest the fusion proteins and thereby liberate the recombinant proteins therefrom. The liberated proteins can then be isolated from the fusion domain by subsequent chromatographic separation. In certain preferred embodiments, a ligand trap is fused with a domain that stabilizes the ligand trap polypeptide *in vivo* (a "stabilizer" domain). By "stabilizing" is meant anything that increases serum half-life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (*e*.*g*., dimerizing, tetramerizing) domains and functional domains (that confer an additional biological function, such as further stimulation of muscle growth).

In certain embodiments, the present disclosure provides ligand trap fusion proteins comprising a variant extracellular domain (*e*.*g*., a ligand-binding domain) of ActRII protein fused to the following Fc domain:

In other embodiments, the present disclosure provides a ligand trap fusion protein comprising a variant extracellular domain (*e*.*g*., a ligand-binding domain) of ActRIIB fused to an Fc domain:

An alternative form with an A64 substitution is as follows:

Optionally, the Fc domain has one or more mutations at residues such as Asp-265, lysine 322, and Asn-434. In certain cases, the mutant Fc domain having one or more of these mutations (e.g., Asp-265 mutation) has reduced ability of binding to the Fcγ receptor relative to a wild-type Fc domain. In other cases, the mutant Fc domain having one or more of these mutations (*e*.*g*., Asn-434 mutation) has increased ability of binding to the MHC class I-related Fc-receptor (FcRN) relative to a wildtype Fc domain.

It is understood that different elements of the fusion proteins may be arranged in any manner that is consistent with the desired functionality. For example, a ligand trap (*e.g.* a GDF11 trap, activin B trap, or GDF11/activin B trap) may be placed C-terminal to a heterologous domain, or alternatively, a heterologous domain may be placed C-terminal to a ligand trap domain. The ligand trap domain and the heterologous domain need not be adjacent in a fusion protein, and additional domains or amino acid sequences may be included C- or N-terminal to either domain or between the domains.

In certain embodiments, the ligand traps (*e.g.* a GDF11 traps, activin B traps, or GDF11/activin B traps) of the present disclosure contain one or more modifications that are capable of stabilizing the ligand trap polypeptides. For example, such modifications enhance the *in vitro* half-life of the ligand trap polypeptides, enhance circulatory half-life of the ligand trap polypeptides, and/or reducing proteolytic degradation of the ligand trap polypeptides. Such stabilizing modifications include, but are not limited to, fusion proteins (including, for example, fusion proteins comprising an ligand trap and a stabilizer domain), modifications of a glycosylation site (including, for example, addition of a glycosylation site to a ligand trap polypeptide), and modifications of carbohydrate moiety (including, for example, removal of carbohydrate moieties from a ligand trap polypeptide). As used herein, the term "stabilizer domain" not only refers to a fusion domain (*e*.*g*., an immunoglobulin Fc domain) as in the case of fusion proteins, but also includes nonproteinaceous modifications such as a carbohydrate moiety, or nonproteinaceous moiety, such as polyethylene glycol.

An example of an ActRIIA-Fc fusion protein comprising a TPA linker is provided below:

The TPA leader sequence is indicated by single underlining; the TGGG linker domain is indicated by double underlining; and the immunoglobulin Fc domain is indicated with **bold** font.

This polypeptide is encoded by the following nucleic acid sequence:

An example of a mature ActRIIA-Fc fusion protein as purified from a CHO cell line is provided below:

The TGGG linker domain is indicated by double underlining; and the immunoglobulin Fc domain is indicated with **bold** font.

An example of an ActRIIB-Fc fusion protein comprising a TPA linker is provided below:

The TPA leader sequence is indicated by single underlining; the TGGG linker domain is indicated by double underlining; and the immunoglobulin Fc domain is indicated with **bold** font.

This polypeptide is encoded by the following nucleic acid sequence:

An example of a mature ActRIIB-Fc fusion protein [referenced herein as ActRIIB(20-134)-Fc] as purified from a CHO cell line is provided below:

The immunoglobulin Fc domain is indicated by single underlining.

An alternative form with an L79D substitution [referenced herein as ActRIIB(L79D 20-134)-Fc] is as follows:

The immunoglobulin Fc domain is indicated with single underlining.

An alternative form comprising a double-truncated ActRIIB domain and an L79D substitution [referenced herein as ActRIIB(L79D 25-131)-Fc] is as follows:

The immunoglobulin Fc domain is indicated with single underlining.

In certain embodiments, a ligand trap fusion protein (*e.g.*, a GDF11 trap, an activin B trap, or a GDF11/activin B trap) of the present disclosure comprises an amino acid sequence that is at least 80%, 90%, 95%, 96%, 97%, 98%, 99%, or 100% identical to the amino acid sequence of any one of SEQ ID Nos: 18, 20, 21, 23, 48, and 49. In preferred embodiments, ligand trap fusion proteins of the present disclosure do not comprise or consist of the amino acid sequence of any one of SEQ ID Nos: 18, 20, 21, 23, 48 and 49. In other preferred embodiments, ActRIIB-based ligand trap fusion proteins comprising an amino acid sequence that is at least 80%, 90%, 95%, 96%, 97%, 98%, 99%, or 100% identical to the amino acid sequence of SEQ ID NO: 21 or 23 do not comprise an acidic amino acid at the position corresponding to position 79 of SEQ ID NO: 1 or 2.

In certain embodiments, the present disclosure makes available isolated and/or purified forms of the ActRII polypeptides, which are isolated from, or otherwise substantially free of, other proteins.

In certain embodiments, ligand trap polypeptides of the disclosure can be produced by a variety of art-known techniques. For example, ligand trap polypeptides can be synthesized using standard protein chemistry techniques such as those described in Bodansky, M. Principles of Peptide Synthesis, Springer Verlag, Berlin (1993) and Grant G. A. (ed.), Synthetic Peptides: A User's Guide, W. H. Freeman and Company, New York (1992). In addition, automated peptide synthesizers are commercially available (*see*, *e.g.*, Advanced ChemTech Model 396; Milligen/Biosearch 9600). Alternatively, the ligand trap polypeptides, fragments or variants thereof may be recombinantly produced using various expression systems (e.g., E. coli, Chinese Hamster Ovary (CHO) cells, COS cells, baculovirus) as is well known in the art. In a further embodiment, the modified or unmodified ligand trap polypeptides may be produced by digestion of recombinantly produced full-length ligand trap polypeptides by using, for example, a protease, *e*.*g*., trypsin, thermolysin, chymotrypsin, pepsin, or paired basic amino acid converting enzyme (PACE). Computer analysis (using a commercially available software, *e*.*g*., MacVector, Omega, PCGene, Molecular Simulation, Inc.) can be used to identify proteolytic cleavage sites. Alternatively, such ligand trap polypeptides may be produced from recombinantly produced full-length ligand trap polypeptides such as standard techniques known in the art, such as by chemical cleavage (*e*.*g*., cyanogen bromide, hydroxylamine).

In preferred embodiments, all proteins and polypeptides of the present disclosure (*e.g.*, GDF11/activin B traps, GDF11 traps, and activin B traps) to be used in accordance with the methods described herein are isolated proteins and polypeptides. As used herein, an isolated protein or polypeptide is one which has been separated from a component of its natural environment. In some embodiments, a protein or polypeptide is purified to greater than 95%, 96%, 97%, 98%, or 99% purity as determined by, for example, electrophoretic (*e*.*g*., SDS-PAGE, isoelectric focusing (IEF), capillary electrophoresis) or chromatographic (*e.g.*, ion exchange or reverse phase HPLC). Methods for assessment of antibody purity are well known in the art [see, *e.g.*, Flatman et al., (2007) J. Chromatogr. B 848:79-87].

Any of the ligand trap polypeptides disclosed herein (*e.g.*, a GDF11 trap, an activin B trap, or a GDF11/activin B trap) can be combined with one or more additional antagonist agents of the disclosure to achieve the desired effect. A ligand trap polypeptide disclosed herein (*e.g.*, a GDF11 trap polypeptide, an activin B trap polypeptide, or a GDF11/activin B trap polypeptide) can be combined with another ligand trap polypeptide disclosed herein, or an antibody directed to any of the targets of the disclosure (*e*.*g*., an anti-GDF11 antibody, an anti-activin A antibody, an anti-activin B antibody, an anti-activin C antibody, an anti-activin E antibody, an anti-GDF8 antibody, an anti-BMP6 antibody, an anti-ActRIIA antibody, an anti-ActRIIB antibody, an anti-GDF15 antibody, an anti-Nodal antibody, an anti-GDF3antibody, an anti-BMP3 antibody, an anti-BMP3B antibody, an anti-BMP9 antibody, or an anti-BMP 10 antibody), or a small-molecule antagonist directed to any of the targets of the disclosure (*e*.*g*., an activin B small-molecule antagonist, an activin B small-molecule antagonist, a GDF11 small-molecule antagonist, an activin C small-molecule antagonist, an activin E small-molecule antagonist, a GDF8 small-molecule antagonist, a BMP6 small-molecule antagonist, a GDF15 small-molecule antagonist, a Nodal small-molecule antagonist, a GDF3 small-molecule antagonist, a BMP3 small-molecule antagonist, a BMP3B small-molecule antagonist, BMP9 small-molecule antagonist, or a BMP10 small-molecule antagonist), or a polynucleotide antagonist of the disclosure (*e*.*g*., a polynucleotide antagonist of activin A, activin B, activin C, activin E, GDF11, GDF8, or BMP6), or a non-antibody binding polypeptide disclosed herein (*e.g.*, a GDF11 binding polypeptide, an activin B binding polypeptide, activin B binding polypeptide, an activin E binding polypeptide, an activin C binding polypeptide, a GDF8 binding polypeptide, a BMP6 binding polypeptide, a GDF15 binding polypeptide, a Nodal binding polypeptide, a BMP3 binding polypeptide, GDF3 binding polypeptide, a BMP3B binding polypeptide, a BMP9 binding polypeptide, or a BMP10 binding polypeptide). For example, a GDF11 trap polypeptide can be combined with an activin B antagonist of the disclosure (*e*.*g*., an activin B trap polypeptide, an anti-activin B antibody, a small-molecule antagonist of activin B, a polynucleotide antagonist of activin B, or a non-antibody polypeptide antagonist of activin B) to inhibit both GDF11 and activin B activity (*e.g.*, the ability to bind to and/or activate an ActRIIA and/or ActRIIB receptor). In an alternative embodiment, an activin B trap polypeptide can be combined with a GDF11 antagonist of the disclosure (*e*.*g*., a GDF trap polypeptide, an anti-GDF11 antibody, a small-molecule antagonist of GDF11, a polynucleotide antagonist of GDF11, or a non-antibody polypeptide antagonist of GDF11) to inhibit both a GDF11 and activin B activity.

### C. Nucleic Acids Encoding Trap Polypeptides and Recombinant Methods

In certain embodiments, the present disclosure provides isolated and/or recombinant nucleic acids encoding the ActRII polypeptides (*e*.*g*., soluble ActRIIA polypeptides and soluble ActRIIB polypeptides), including fragments, functional variants, and fusion proteins disclosed herein. For example, SEQ ID NO:12 encodes the naturally occurring human ActRIIA precursor polypeptide, while SEQ ID NO:13 encodes the processed extracellular domain of ActRIIA. For example, SEQ ID NO:7 encodes a naturally occurring human ActRIIB precursor polypeptide (the A64 variant described above), while SEQ ID NO:8 encodes the processed extracellular domain of ActRIIB (the A64 variant described above). The subject nucleic acids may be single-stranded or double stranded. Such nucleic acids may be DNA or RNA molecules. These nucleic acids may be used, for example, in methods for making ActRII-based ligand trap polypeptides of the present disclosure.

As used herein, isolated nucleic acid(s) refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

In certain embodiments, nucleic acids encoding ActRIIA-based ligand trap polypeptides of the present disclosure are understood to include nucleic acids that are variants of SEQ ID NO: 12 or 13. In certain aspects, nucleic acids encoding ActRIIB-based ligand trap polypeptides of the present disclosure are understood to include nucleic acids that are variants of SEQ ID NO: 7 or 8. Variant nucleotide sequences include sequences that differ by one or more nucleotide substitutions, additions or deletions, such as allelic variants. Nucleic acids of the disclosure encode ActRIIA- and ActRIIB-based ligand trap polypeptides that bind to and/or antagonize the activity of at least GDF11 and/or activin A. Optionally, nucleic acids of the disclosure encode ActRIIA- and ActRIIB-based ligand trap polypeptides that do not substantially bind to and/or inhibit activin A. In some embodiments, nucleic acids of the disclosure that encode ActRIIA- and ActRIIB-based ligand trap polypeptides that bind to and/or inhibit GDF11 and/or activin B further bind to and/or inhibit one or more of: activin A, activin C, activin E, GDF8, GDF15, Nodal, GDF3, BMP3, BMP3B, and BMP6.

In certain embodiments, ActRIIA- and ActRIIB-based ligand traps of the present disclosure are encoded by isolated or recombinant nucleic acid sequences that are at least 80%, 85%, 90%, 95%, 97%, 98%, 99%, or 100% identical to SEQ ID NOs: 8, 13, 19, and 22. In preferred embodiments, ActRIIA- and ActRIIB-based ligand traps of the present disclosure are not encoded by nucleic acid sequences that comprise or consist of any one of: SEQ ID NOs: 8, 13, 19, and 22. One of ordinary skill in the art will appreciate that nucleic acid sequences that are at least 80%, 85%, 90%, 95%, 97%, 98%, 99%, or 100% identical to the sequences complementary to SEQ ID NOs: 8, 13, 19, and 22, and variants thereof, are also within the scope of the present disclosure, provided that the sequences do not comprise or consist of sequences complementary to SEQ ID NOs 8, 13, 19, and 22. In further embodiments, the nucleic acid sequences of the disclosure can be isolated, recombinant, and/or fused with a heterologous nucleotide sequence, or in a DNA library.

In other embodiments, nucleic acids of the present disclosure also include nucleotide sequences that hybridize under highly stringent conditions to the nucleotide sequence designated in SEQ ID NOs: 8, 13, 19, and 22, complement sequence of SEQ ID NOs: 8, 13, 19, and 22, or fragments thereof, provided that they do not comprise or consist of the nucleotides of SEQ ID NOs: 8, 13, 19, and 22. As discussed above, one of ordinary skill in the art will understand readily that appropriate stringency conditions which promote DNA hybridization can be varied. For example, one could perform the hybridization at 6.0 × sodium chloride/sodium citrate (SSC) at about 45 °C, followed by a wash of 2.0 × SSC at 50 °C. For example, the salt concentration in the wash step can be selected from a low stringency of about 2.0 × SSC at 50 °C to a high stringency of about 0.2 × SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In one embodiment, the disclosure provides nucleic acids which hybridize under low stringency conditions of 6 × SSC at room temperature followed by a wash at 2 × SSC at room temperature.

Isolated nucleic acids which differ from the nucleic acids as set forth in SEQ ID NOs: 8, 13, 19, and 22 due to degeneracy in the genetic code are also within the scope of the disclosure. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms are within the scope of this disclosure.

In certain embodiments, the recombinant nucleic acids of the present disclosure may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the disclosure. The promoters may be either naturally occurring promoters, or hybrid promoters that combine elements of more than one promoter. An expression construct may be present in a cell on an episome, such as a plasmid, or the expression construct may be inserted in a chromosome. In some embodiments, the expression vector contains a selectable marker gene to allow the selection of transformed host cells. Selectable marker genes are well known in the art and will vary with the host cell used.

In certain aspects of the present disclosure, the subject nucleic acid is provided in an expression vector comprising a nucleotide sequence encoding an ActRII polypeptide and operably linked to at least one regulatory sequence. Regulatory sequences are art-recognized and are selected to direct expression of the ActRII polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding an ActRII polypeptide. Such useful expression control sequences, include, for example, the early and late promoters of SV40, *tet* promoter, adenovirus or cytomegalovirus immediate early promoter, RSV promoters, the lac system, the *trp* system, the TAC or TRC system, T7 promoter whose expression is directed by T7 RNA polymerase, the major operator and promoter regions of phage lambda , the control regions for fd coat protein, the promoter for 3-phosphoglycerate kinase or other glycolytic enzymes, the promoters of acid phosphatase, *e.g.*, Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the present disclosure can be produced by ligating the cloned gene, or a portion thereof, into a vector suitable for expression in either prokaryotic cells, eukaryotic cells (yeast, avian, insect or mammalian), or both. Expression vehicles for production of a recombinant ActRII polypeptide include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as *E. coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures [see, *e.g.*, Molecular Cloning A Laboratory Manual, 3rd Ed., ed. by Sambrook, Fritsch and Maniatis (Cold Spring Harbor Laboratory Press, 2001)]. In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the ß-gal containing pBlueBac III).

In a some embodiments, a vector will be designed for production of the subject ActRII polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wisc.). As will be apparent, the subject gene constructs can be used to cause expression of the subject ActRII polypeptides in cells propagated in culture, e.g., to produce proteins, including fusion proteins or variant proteins, for purification.

This disclosure also pertains to a host cell transfected with a recombinant gene including a coding sequence for one or more of the subject ActRII polypeptides. The host cell may be any prokaryotic or eukaryotic cell. For example, an ActRII polypeptide of the may be expressed in bacterial cells such as *E. coli*, insect cells (e.g., using a baculovirus expression system), yeast, or mammalian cells. Other suitable host cells are known to those skilled in the art.

Accordingly, the present disclosure further pertains to methods of producing the subject ActRII polypeptides. For example, a host cell transfected with an expression vector encoding an ActRIIA or ActRIIB polypeptide can be cultured under appropriate conditions to allow expression of the ActRII polypeptide to occur. The ActRII polypeptide may be secreted and isolated from a mixture of cells and medium containing the ActRII polypeptide. Alternatively, the ActRII polypeptide may be retained cytoplasmically or in a membrane fraction and the cells harvested, lysed and the protein isolated. A cell culture includes host cells, media and other byproducts. Suitable media for cell culture are well known in the art. The subject ActRII polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRII polypeptides and affinity purification with an agent that binds to a domain fused to the ActRII polypeptide (*e.g.*, a protein A column may be used to purify an ActRIIA-Fc or ActRIIB-Fc fusion). In some embodiments, the ActRII polypeptide is a fusion protein containing a domain which facilitates its purification. In some embodiments, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. An ActRIIB-hFc or ActRIIA-hFc protein may be purified to a purity of >90%, >95%, >96%, >98%, or >99% as determined by size exclusion chromatography and >90%, >95%, >96%, >98%, or >99% as determined by SDS PAGE. The target level of purity should be one that is sufficient to achieve desirable results in mammalian systems, particularly non-human primates, rodents (mice), and humans.

In another embodiment, a fusion gene coding for a purification leader sequence, such as a poly-(His)/enterokinase cleavage site sequence at the N-terminus of the desired portion of the recombinant ActRII polypeptide, can allow purification of the expressed fusion protein by affinity chromatography using a Ni²⁺ metal resin. The purification leader sequence can then be subsequently removed by treatment with enterokinase to provide the purified ActRII polypeptide. *See*, *e.g.*, Hochuli et al. (1987) J. Chromatography 411:177; and Janknecht et al. PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence [see, *e*.*g*., Current Protocols in Molecular Biology, eds. Ausubel et al., John Wiley & Sons: 1992].

### D. Other Binding Polypeptides

In another aspect, an antagonist agent, or combination of agents, of the present disclosure is a non-antibody binding polypeptide that binds to and/or inhibits the activity of at least GDF11 and/or activin B (*e.g.*, activation of ActRIIA or ActRIIB Smad2/3 signaling). Optionally, a non-antibody binding polypeptide, or combinations of non-antibody-binding polypeptides, of the disclosure does not bind to and/or inhibit the activity of activin A (*e.g.*, activin A-mediated activation of ActRIIA or ActRIIB Smad2/3 signaling). Optionally, a non-antibody binding polypeptide, or combinations of non-antibody-binding polypeptides, of the disclosure further binds to and/or inhibits the activity of GDF8 (*e*.*g*., GDF8-mediated activation of ActRIIA or ActRIIB Smad2/3 signaling). In some embodiments, a non-antibody binding polypeptide, or combinations of non-antibody-binding polypeptides, of the disclosure that binds to and/or inhibits the activity of GDF11 and/or activin B further binds to and/or inhibits activity of one or more of activin E, activin C, activin A, GDF8, BMP6, GDF15, Nodal, GDF3, BMP3, BMP3B, BMP9, and BMP10 (*e*.*g*., activation of ActRIIA or ActRIIB Smad2/3 and/or Smad 1/5/8 signaling). In certain embodiments, non-antibody-binding polypeptides that bind to BMP9 and/or BMP10 inhibit interaction between BMP9 and a type II receptor of the TGFβ superfamily (*e*.*g*., ActRIIA and/or ActRIIB) and/or BMP10 and a type II receptor of the TGFβ superfamily (*e*.*g*., ActRIIA and/or ActRIIB). Preferably, non-antibody-binding polypeptides that bind to BMP9 and/or BMP10 do not inhibit, or substantially inhibit, interaction between BMP9 and ALK1 and/or BMP10 and ALK1.

Binding polypeptides of the present disclosure may be chemically synthesized using known polypeptide synthesis methodology or may be prepared and purified using recombinant technology. Binding polypeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such binding polypeptides that are capable of binding, preferably specifically, to a target as described herein (*e.g.*, GDF11, activin B, activin E, activin C, GDF8, activin A, BMP6, GDF15, Nodal, GDF3, BMP3, BMP3B, BMP9, and BMP10). Binding polypeptides may be identified without undue experimentation using well known techniques. In this regard, it is noted that techniques for screening polypeptide libraries for binding polypeptides that are capable of specifically binding to a polypeptide target are well known in the art including, for example, U.S. Pat. Nos. 5,556,762; 5,750,373; 4,708,871; 4,833,092; 5,223,409; 5,403,484; 5,571,689; and 5,663,143; PCT Publication Nos. WO 84/03506 and WO84/03564; Geysen et al. (1984) Proc. Natl. Acad. Sci. U.S.A., 81:3998-4002; Geysen et al. (1985) Proc. Natl. Acad. Sci. U.S.A., 82:178-182; Geysen et al. (1986) in Synthetic Peptides as Antigens, 130-149; Geysen et al. (1987) J. Immunol. Meth, 102:259-274; Schoofs et al. (1988) J. Immunol., 140:611-616, Cwirla, S. E. et al. (1990) Proc. Natl. Acad. Sci. USA, 87:6378; Lowman, H. B. et al. (1991) Biochemistry, 30:10832; Clackson, T. et al. (1991) Nature, 352: 624; Marks, J. D. et al. (1991), J. Mol. Biol., 222:581; Kang, A. S. et al. (1991) Proc. Natl. Acad. Sci. USA, 88:8363, and Smith, G. P. (1991) Current Opin. Biotechnol., 2:668.

In this regard, bacteriophage (phage) display is one well known technique which allows one to screen large polypeptide libraries to identify member(s) of those libraries which are capable of specifically binding to a target polypeptide (*e*.*g*., GDF11, activin B, activin E, activin C, GDF8, BMP6, GDF15, Nodal, GDF3, BMP3, BMP3B, BMP9, and BMP10) . Phage display is a technique by which variant polypeptides are displayed as fusion proteins to the coat protein on the surface of bacteriophage particles [see, *e.g.,* Scott, J. K. and Smith, G. P. (1990) Science, 249: 386]. The utility of phage display lies in the fact that large libraries of selectively randomized protein variants (or randomly cloned cDNAs) can be rapidly and efficiently sorted for those sequences that bind to a target molecule with high affinity. Display of peptide [see, e.*g*., Cwirla, S. E. et al. (1990) Proc. Natl. Acad. Sci. USA, 87:6378] or protein [Lowman, H. B. et al. (1991) Biochemistry, 30:10832; Clackson, T. et al. (1991) Nature, 352: 624; Marks, J. D. et al. (1991), J. Mol. Biol., 222:581; Kang, A. S. et al. (1991) Proc. Natl. Acad. Sci. USA, 88:8363] libraries on phage have been used for screening millions of polypeptides or oligopeptides for ones with specific binding properties [see, *e.g.*, Smith, G. P. (1991) Current Opin. Biotechnol., 2:668]. Sorting phage libraries of random mutants requires a strategy for constructing and propagating a large number of variants, a procedure for affinity purification using the target receptor, and a means of evaluating the results of binding enrichments (see, *e.g.,* U.S. Pat. Nos. 5,223,409; 5,403,484; 5,571,689; and 5,663,143).

Although most phage display methods have used filamentous phage, lambdoid phage display systems (see, *e.g.*, WO 95/34683; and U.S. Pat. No. 5,627,024), T4 phage display systems [see, *e.g.*, Ren et al. (1998) Gene, 215: 439; Zhu et al. (1998) Cancer Research, 58(15): 3209-3214; Jiang et al. (1997) Infection & Immunity, 65(11): 4770-4777; Ren et al. (1997) Gene, 195(2):303-311; Ren (1996) Protein Sci., 5: 1833; Efimov et al. (1995) Virus Genes, 10: 173] and T7 phage display systems [see, *e.g.*, Smith and Scott (1993) Methods in Enzymology, 217: 228-257; and U.S. Pat. No. 5,766,905] are also known.

Additional improvements to enhance the ability of display systems to screen peptide libraries for binding to selected target molecules and to display functional proteins with the potential of screening these proteins for desired properties are known in the art. Combinatorial reaction devices for phage display reactions have been developed (see, *e.g.*, WO 98/14277) and phage display libraries have been used to analyze and control bimolecular interactions (see, *e.g.*, WO 98/20169; and WO 98/20159) and properties of constrained helical peptides (see, *e.g.*, WO 98/20036). International patent publication no. WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. International patent publication no. WO 97/46251 describes a method of biopanning a random phage display library with an affinity purified antibody and then isolating binding phage, followed by a micropanning process using microplate wells to isolate high affinity binding phage. The use of *Staphylococcus aureus* protein A as an affinity tag has also been reported [see, *e.g.*, Li et al. (1998) Mol. Biotech., 9:187.

WO 97/47314 describes the use of substrate subtraction libraries to distinguish enzyme specificities using a combinatorial library, which may be a phage display library. A method for selecting enzymes suitable for use in detergents using phage display is described in international patent publication no. WO 97/09446. Additional methods of selecting specific binding proteins are described in, for example, U.S. Pat. Nos. 5,498,538; 5,432,018; and international patent publication WO 98/15833.

Methods of generating peptide libraries and screening these libraries are also disclosed in, for example, U.S. Pat. Nos. 5,723,286; 5,432,018; 5,580,717; 5,427,908; 5,498,530; 5,770,434; 5,734,018; 5,698,426; 5,763,192; and 5,723,323.

Any of the non-antibody binding polypeptides disclosed herein (*e.g.*, a GDF11 binding polypeptide, an activin B binding polypeptide, an activin B binding polypeptide, an activin E binding polypeptide, an activin C binding polypeptide, a GDF8 binding polypeptide, a BMP6 binding polypeptide, a GDF15 binding polypeptide, a Nodal binding polypeptide, a GDF3 binding polypeptide, a BMP3binding polypeptide, a BMP3B binding polypeptide, a BMP9 binding polypeptide, or a BMP6 binding polypeptide) can be combined with one or more additional antagonist agents of the disclosure to achieve the desired effect. A non-antibody binding polypeptide disclosed herein (*e.g.*, a GDF11 binding polypeptide, an activin A binding polypeptide, an activin B binding polypeptide, an activin E binding polypeptide, an activin C binding polypeptide, a GDF8 binding polypeptide, a BMP6 binding polypeptide, a GDF15 binding polypeptide, a Nodal binding polypeptide, a GDF3 binding polypeptide, a BMP3binding polypeptide, a BMP3B binding polypeptide, a BMP9 binding polypeptide, or a BMP6 binding polypeptide) can be combined with another non-antibody binding polypeptide of the disclosure, or with an antibody directed to any of the targets of the disclosure (*e*.*g*., an anti-GDF11 antibody, an anti-activin B antibody, an anti-activin B antibody, an anti-activin C antibody, an anti-activin E antibody, an anti-GDF11 antibody, an anti-GDF8 antibody, an anti-BMP6 antibody, an anti-ActRIIA antibody, an anti-ActRIIB antibody, an anti-GDF15 antibody, an anti-Nodal antibody, an anti-GDF3 antibody, an anti-BMP3 antibody, an anti-BMP3B antibody, an anti-BMP9 antibody, or an anti-BMP 10 antibody) or a ligand trap polypeptide disclosed herein (*e.g.*, a GDF11 trap polypeptide, an activin B trap polypeptide, or a GDF11/activin B trap polypeptide), or a small molecule directed to any of the targets of the disclosure or a small-molecule antagonist directed to any of the targets of the disclosure (*e*.*g*., an activin B small-molecule antagonist, an activin B small-molecule antagonist, a GDF11 small-molecule antagonist, an activin C small-molecule antagonist, an activin E small-molecule antagonist, a GDF8 small-molecule antagonist, a BMP6 small-molecule antagonist, a GDF15 small-molecule antagonist, a Nodal small-molecule antagonist, a GDF3 small-molecule antagonist, a BMP3 small-molecule antagonist, a BMP3B small-molecule antagonist, a BMP9 small-molecule antagonist, or a BMP10 small-molecule antagonist), or a polynucleotide antagonist of the disclosure (*e*.*g*., a polynucleotide antagonist of activin B, a polynucleotide antagonist of activin B, activin C, activin E, GDF11, GDF8, or BMP6). For example, a GDF11-binding polypeptide can be combined with an activin B antagonist of the disclosure (*e*.*g*., an activin B trap polypeptide, an anti-activin B antibody, a small-molecule antagonist of activin B, a polynucleotide antagonist of activin B, or a non-antibody polypeptide antagonist of activin B) to inhibit both a GDF11 and an activin B activity (*e*.*g*., the ability to bind to and/or activate an ActRIIA and/or ActRIIB receptor). In an alternative embodiment, an activin-B-binding polypeptide can be combined with a GDF11 antagonist of the disclosure (*e*.*g*., a GDF-trap polypeptide, an anti-GDF11 antibody, a small-molecule antagonist of GDF11, a polynucleotide antagonist of GDF11, or a non-antibody polypeptide antagonist of GDF11) to inhibit both a GDF11 and an activin B activity.

### E. Small-Molecule Antagonists

In another aspect, an antagonist agent, or combination of agents, of the present disclosure is a small-molecule antagonist that inhibits the expression (*e*.*g*., transcription, translation, and/or cellular secretion) of at least GDF11 and/or activin B. Optionally, a small-molecule antagonist, or combinations of small-molecule antagonists, of the disclosure does not inhibit the expression of activin A. Optionally, a small-molecule antagonist, or combinations of small-molecule antagonists, of the disclosure further inhibits expression of GDF8. In some embodiments, a small-molecule antagonist, or combinations of small-molecule antagonists, of the disclosure that inhibits expression of GDF11 and/or activin B further inhibits expression of one or more of activin E, activin C, activin A, GDF8, GDF15, Nodal, GDF3, BMP3, and BMP3B.

In another aspect, an antagonist agent, or combination of agents, of the present disclosure is a small-molecule agent that binds to and inhibits the activity of at least GDF11 and/or activin B (*e*.*g*., activation of ActRIIA and/or ActRIIB Smad2/3 signaling). Optionally, a small-molecule antagonist, or combinations of small-molecule antagonists, of the disclosure does not bind to and/or inhibit activin A activity (*e*.*g*., activin A-mediated activation of ActRIIA and/or ActRIIB Smad2/3 signaling. Optionally, a small-molecule antagonist, or combinations of small-molecule antagonists, of the disclosure further binds to and inhibits the activity of GDF8 (*e*.*g*., GDF8-mediated activation of ActRIIA and/or ActRIIB Smad 2/3 signaling). In some embodiments, a small-molecule antagonist, or combinations of small-molecule antagonists, of the disclosure that binds to and inhibits an activity of GDF11 and/or activin B further binds to and inhibits an activity of one or more of GDF8, activin E, activin C, activin A, BMP6, GDF15, Nodal, GDF3, BMP3, BMP3B, BMP9, and BMP10 (*e.g.*, activation of ActRIIA and/or ActRIIB Smad2/3 and/or Smad 1/5/8 signaling). In certain embodiments, small molecules that bind to BMP9 and/or BMP10 inhibit interaction between BMP9 and a type II receptor of the TGFβ superfamily (*e*.*g*., ActRIIA and/or ActRIIB) and/or BMP10 and a type II receptor of the TGFβ superfamily (*e*.*g*., ActRIIA and/or ActRIIB). Preferably, small molecules that bind to BMP9 and/or BMP10 do not inhibit, or substantially inhibit, interaction between BMP9 and ALK1 and/or BMP10 and ALK1.

In a further aspect, an antagonist agent, or combination of agents, of the present disclosure is a small-molecule agent that indirectly antagonizes at least GDF11 and/or activin B activity. In some embodiments, an indirect small-molecule antagonist, or combination of indirect small-molecule antagonists, of the present disclosure is a small molecule that binds to an ActRII receptor (*e.g.*, an ActRIIA and/or an ActRIIB receptor) and inhibits at least GDF11 and/or activin B from binding to and/or activating an ActRII receptor (e.g., activation of ActRIIA and/or ActRIIB Smad2/3 signaling). Optionally, an indirect small-molecule antagonist, or combination of indirect small-molecule antagonists, of the present disclosure does not substantially inhibit activin A from binding to and/or activating an ActRII receptor (*e*.*g*., activin A-mediated ActRIIA and/or ActRIIB Smad 2/3 signaling). Optionally, an indirect small-molecule antagonist, or combination of indirect small-molecule antagonists, of the present disclosure binds to an ActRII receptor and further inhibits binding to and/or activation of the ActRII receptor by GDF8. In some embodiments, an indirect small-molecule antagonist, or combination of indirect small-molecule antagonists, of the present disclosure that bind to an ActRII receptor and inhibit at least GDF11 and/or activin B from binding to and/or activating an ActRII receptor further inhibit one or more of activin C, activin E, GDF8, BMP6, activin A, GDF15, Nodal, GDF3, BMP3, BMP3B, BMP9, and BMP10 from binding to and/or activating the ActRII receptor (*e*.*g*., activation of ActRIIA and/or ActRIIB Smad 2/3 and/or Smad 1/5/8 signaling).

Binding organic small-molecule antagonists of the present disclosure may be identified and chemically synthesized using known methodology (*see*, *e.g.*, PCT Publication Nos. WO 00/00823 and WO 00/39585). In general, small-molecule antagonists of the disclosure are usually less than about 2000 daltons in size, alternatively less than about 1500, 750, 500, 250 or 200 daltons in size, wherein such organic small molecules that are capable of binding, preferably specifically, to a polypeptide as described herein (activin B, activin C, activin E, GDF11, GDF8, and BMP6). Such small-molecule antagonists may be identified without undue experimentation using well-known techniques. In this regard, it is noted that techniques for screening organic small-molecule libraries for molecules that are capable of binding to a polypeptide target are well known in the art (see, *e*.*g*., international patent publication Nos. WO00/00823 and WO00/39585).

Binding organic small molecules of the present disclosure may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, and acid chlorides.

Any of the small-molecule antagonists disclosed herein (*e.g.*, a small-molecule antagonist of activin B, activin C, activin E, GDF11, GDF8, BMP6, GDF15, Nodal, GDF3, BMP3, BMP3B, BMP9, or BMP10) can be combined with one or more additional antagonist agents of the disclosure to achieve the desired effect. A small-molecule antagonist disclosed herein (*e.g.*, a small-molecule antagonist of activin A, activin B, activin C, activin E, GDF11, GDF8, BMP6, GDF15, Nodal, GDF3, BMP3, BMP3B, BMP9, or BMP10) can be combined with another small-molecule antagonist of the disclosure, or an antibody directed to any of the targets of the disclosure (*e*.*g*., an anti-GDF11 antibody, an anti-activin B antibody, an anti-activin B antibody, an anti-activin C antibody, an anti-activin E antibody, an anti-GDF8 antibody, an anti-BMP6 antibody, an anti-ActRIIA antibody, an anti-ActRIIB antibody, an anti-ActRIIB antibody, an anti-GDF15 antibody, an anti-Nodal antibody, an anti-GDF3 antibody, an anti-BMP3 antibody, an anti-BMP3B antibody, an anti-BMP9 antibody, or an anti-BMP 10 antibody), or a non-antibody binding polypeptide disclosed herein (*e.g.*, a GDF11-binding polypeptide, an activin-B-binding polypeptide, an activin-B-binding polypeptide, an activin-E-binding polypeptide, an activin-C-binding polypeptide, a GDF8-binding polypeptide, a BMP6-binding polypeptide, a GDF15-binding polypeptide, a Nodal-binding polypeptide, a GDF3-binding polypeptide, a BMP3-binding polypeptide, a BMP3B-binding polypeptide, a BMP9-binding polypeptide, or a BMP10-binding polypeptide), or a ligand trap polypeptide disclosed herein (*e.g.*, a GDF11-trap polypeptide, an activin-B trap polypeptide, or a GDF/activin-B trap polypeptide), or a polynucleotide antagonist of the disclosure (*e*.*g*., a polynucleotide antagonist of activin A, a polynucleotide antagonist of activin B, activin C, activin E, GDF11, GDF8, BMP6, GDF15, Nodal, GDF3, BMP3, or BMP3B). For example, a small-molecule antagonist of GDF11 can be combined with an activin-B antagonist of the disclosure (*e*.*g*., an activin-B trap polypeptide, an anti-activin-B antibody, a small-molecule antagonist of activin B, a polynucleotide antagonist of activin B, or a non-antibody polypeptide antagonist of activin B) to inhibit both a GDF11 and an activin B activity (*e*.*g*., the ability to bind to and/or activate an ActRIIA and/or ActRIIB receptor). In an alternative embodiment, a small-molecule antagonist of activin-B antibody can be combined with a GDF11 antagonist of the disclosure (*e*.*g*., a GDF-trap polypeptide, an anti-GDF11 antibody, a small-molecule antagonist of GDF11, a polynucleotide antagonist of GDF11, or a non-antibody polypeptide antagonist of GDF11) to inhibit both a GDF11 and an activin B activity.

### F. Antagonist Polynucleotides

In another aspect, an antagonist agent, or combination of agents, of the present disclosure is a polynucleotide antagonist that inhibits at least GDF11 and/or activin B. In some embodiments, an antagonist polynucleotide of the disclosure inhibits the expression (*e*.*g*., transcription, translation, and/or cellular secretion) of at least GDF11 and/or activin B. Optionally, a polynucleotide antagonist, or combinations of polynucleotide antagonists, of the disclosure does not inhibit activin A (*e*.*g*. inhibits expression and/or activity of activin A). Optionally, a polynucleotide antagonist, or combinations of polynucleotide antagonists, of the disclosure further inhibit GDF8 (*e*.*g*. inhibits expression and/or activity of GDF8). In some embodiments, a polynucleotide antagonist, or combinations of polynucleotide antagonists, of the disclosure that inhibits GDF11 and/or activin B (*e*.*g*. expression and/or activity of GDF11 and/or activin B) further inhibits (*e*.*g*., inhibits expression and/or activity) one or more of activin E, activin C, activin A, GDF8, BMP6, Nodal, GDF3, BMP3, and GDF3B.

The polynucleotide antagonists of the disclosure may be an antisense nucleic acid, an RNAi molecule [*e*.*g*., small interfering RNA (siRNA), small-hairpin RNA (shRNA), microRNA (miRNA)], an aptamer and/or a ribozyme. The nucleic acid and amino acid sequences of human GDF11, activin B, activin C, activin E, GDF8, activin A, BMP6, Nodal, GDF3, BMP3, and GDF3B are known in the art. In addition, many different methods of generating polynucleotide antagonists are well known in the art. Therefore polynucleotide antagonists for use in accordance with this disclosure may be routinely made by the skilled person in the art based on the knowledge in the art and teachings provided herein.

Antisense technology can be used to control gene expression through antisense DNA or RNA, or through triple-helix formation. Antisense techniques are discussed, for example, in Okano (1991) J. Neurochem. 56:560; Oligodeoxynucleotides as Antisense Inhibitors of Gene Expression, CRC Press, Boca Raton, Fla. (1988). Triple-helix formation is discussed in, for instance, Cooney et al. (1988) Science 241:456; and Dervan et al., (1991) Science 251:1300. The methods are based on binding of a polynucleotide to a complementary DNA or RNA. In some embodiments, the antisense nucleic acids comprise a single-stranded RNA or DNA sequence that is complementary to at least a portion of an RNA transcript of a gene disclosed herein (*e.g.*, GDF11, activin B, activin C, activin E, GDF8, activin A, BMP6, Nodal, GDF3, BMP3, and GDF3B). However, absolute complementarity, although preferred, is not required.

A sequence "complementary to at least a portion of an RNA," referred to herein, means a sequence having sufficient complementarity to be able to hybridize with the RNA, forming a stable duplex; in the case of double-stranded antisense nucleic acids of a gene disclosed herein (*e.g.*, GDF11, activin A, activin B, activin C, activin E, GDF8, activin A, BMP6, Nodal, GDF3, BMP3, and GDF3B), a single strand of the duplex DNA may thus be tested, or triplex formation may be assayed. The ability to hybridize will depend on both the degree of complementarity and the length of the antisense nucleic acid. Generally, the larger the hybridizing nucleic acid, the more base mismatches with an RNA it may contain and still form a stable duplex (or triplex as the case may be). One skilled in the art can ascertain a tolerable degree of mismatch by use of standard procedures to determine the melting point of the hybridized complex.

Polynucleotides that are complementary to the 5' end of the message, for example, the 5'-untranslated sequence up to and including the AUG initiation codon, should work most efficiently at inhibiting translation. However, sequences complementary to the 3'-untranslated sequences of mRNAs have been shown to be effective at inhibiting translation of mRNAs as well [see, *e*.*g*., Wagner, R., (1994) Nature 372:333-335]. Thus, oligonucleotides complementary to either the 5'- or 3'-non-translated, non-coding regions of a gene of the disclosure (*e*.*g*., GDF11, activin A, activin B, activin C, activin E, GDF8, BMP6, Nodal, GDF3, BMP3, and GDF3B), could be used in an antisense approach to inhibit translation of an endogenous mRNA (*e*.*g*., GDF11, activin A, activin B, activin C, activin E, GDF8, activin A, BMP6, Nodal, GDF3, BMP3, and GDF3B). Polynucleotides complementary to the 5'-untranslated region of the mRNA should include the complement of the AUG start codon. Antisense polynucleotides complementary to mRNA coding regions are less efficient inhibitors of translation but could be used in accordance with the methods of the present disclosure. Whether designed to hybridize to the 5'-, 3'- or coding region of an mRNA of the disclosure (*e*.*g*., GDF11, activin A, activin B, activin C, activin E, GDF8, activin A, BMP6, Nodal, GDF3, BMP3, and GDF3B), antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

In one embodiment, the antisense nucleic acid of the present disclosure (e.g., a GDF11, activin B, activin C, activin E, GDF8, activin A, BMP6, Nodal, GDF3, BMP3, and GDF3B and/or antisense nucleic acid) is produced intracellularly by transcription from an exogenous sequence. For example, a vector or a portion thereof, is transcribed, producing an antisense nucleic acid (RNA) of a gene of the disclosure. Such a vector would contain a sequence encoding the desired antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the sequence encoding desired genes of the instant disclosure, or fragments thereof, can be by any promoter known in the art to act in vertebrate, preferably human cells. Such promoters can be inducible or constitutive. Such promoters include, but are not limited to, the SV40 early promoter region [see , *e.g.*, Benoist and Chambon (1981) Nature 290:304-310], the promoter contained in the 3' long-terminal repeat of Rous sarcoma virus [see, *e.g.*, Yamamoto et al. (1980) Cell 22:787-797], the herpes thymidine promoter [see, *e.g.*, Wagner et al. (1981) Proc. Natl. Acad. Sci. U.S.A. 78:1441-1445], and the regulatory sequences of the metallothionein gene [see, *e*.*g*., Brinster, et al. (1982) Nature 296:39-42].

In some embodiments, the polynucleotide antagonists are interfering RNA (RNAi) molecules that target the expression of one or more of: GDF11, activin B, activin C, activin E, GDF8, activin A, BMP6, Nodal, GDF3, BMP3, and GDF3B. RNAi refers to the expression of an RNA which interferes with the expression of the targeted mRNA. Specifically, RNAi silences a targeted gene via interacting with the specific mRNA through a siRNA (small interfering RNA). The ds RNA complex is then targeted for degradation by the cell. An siRNA molecule is a double-stranded RNA duplex of 10 to 50 nucleotides in length, which interferes with the expression of a target gene which is sufficiently complementary (e.g. at least 80% identity to the gene). In some embodiments, the siRNA molecule comprises a nucleotide sequence that is at least 85, 90, 95, 96, 97, 98, 99, or 100% identical to the nucleotide sequence of the target gene.

Additional RNAi molecules include short-hairpin RNA (shRNA); also short-interfering hairpin and microRNA (miRNA). The shRNA molecule contains sense and antisense sequences from a target gene connected by a loop. The shRNA is transported from the nucleus into the cytoplasm, and it is degraded along with the mRNA. Pol III or U6 promoters can be used to express RNAs for RNAi. Paddison et al. [Genes & Dev. (2002) 16:948-958, 2002] have used small RNA molecules folded into hairpins as a means to effect RNAi. Accordingly, such short-hairpin RNA (shRNA) molecules are also advantageously used in the methods described herein. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the double-stranded RNA (dsRNA) products of the DICER RNase and, in any event, have the same capacity for inhibiting expression of a specific gene. The shRNA can be expressed from a lentiviral vector. An miRNA is a single-stranded RNA of about 10 to 70 nucleotides in length that are initially transcribed as pre-miRNA characterized by a "stem-loop" structure, which are subsequently processed into mature miRNA after further processing through the RISC.

Molecules that mediate RNAi, including without limitation siRNA, can be produced in vitro by chemical synthesis (Hohjoh, FEBS Lett 521:195-199, 2002), hydrolysis of dsRNA (Yang et al., Proc Natl Acad Sci USA 99:9942-9947, 2002), by *in vitro* transcription with T7 RNA polymerase (Donzeet et al., Nucleic Acids Res 30:e46, 2002; Yu et al., Proc Natl Acad Sci USA 99:6047-6052, 2002), and by hydrolysis of double-stranded RNA using a nuclease such as E. coli RNase III (Yang et al., Proc Natl Acad Sci USA 99:9942-9947, 2002).

According to another aspect, the disclosure provides polynucleotide antagonists including but not limited to, a decoy DNA, a double-stranded DNA, a single-stranded DNA, a complexed DNA, an encapsulated DNA, a viral DNA, a plasmid DNA, a naked RNA, an encapsulated RNA, a viral RNA, a double-stranded RNA, a molecule capable of generating RNA interference, or combinations thereof.

In some embodiments, the polynucleotide antagonists of the disclosure are aptamers. Aptamers are nucleic acid molecules, including double-stranded DNA and single-stranded RNA molecules, which bind to and form tertiary structures that specifically bind to a target molecule, such as a GDF11, activin B, activin C, activin E, GDF8, activin A, BMP6, Nodal, GDF3, BMP3, or GDF3B polypeptide. The generation and therapeutic use of aptamers are well established in the art (see, *e.g.*, U.S. Pat. No. 5,475,096). Additional information on aptamers can be found in U.S. Patent Application Publication No. 20060148748. Nucleic acid aptamers are selected using methods known in the art, for example via the Systematic Evolution of Ligands by Exponential Enrichment (SELEX) process. SELEX is a method for the in vitro evolution of nucleic acid molecules with highly specific binding to target molecules as described in, *e.g.*, U.S. Pat. Nos. 5,475,096; 5,580,737; 5,567,588; 5,707,796; 5,763,177; 6,011,577; and 6,699,843. Another screening method to identify aptamers is described in U.S. Pat. No. 5,270,163. The SELEX process is based on the capacity of nucleic acids for forming a variety of two- and three-dimensional structures, as well as the chemical versatility available within the nucleotide monomers to act as ligands (form specific binding pairs) with virtually any chemical compound, whether monomeric or polymeric, including other nucleic acid molecules and polypeptides. Molecules of any size or composition can serve as targets. The SELEX method involves selection from a mixture of candidate oligonucleotides and step-wise iterations of binding, partitioning and amplification, using the same general selection scheme, to achieve desired binding affinity and selectivity. Starting from a mixture of nucleic acids, which can comprise a segment of randomized sequence, the SELEX method includes steps of contacting the mixture with the target under conditions favorable for binding; partitioning unbound nucleic acids from those nucleic acids which have bound specifically to target molecules; dissociating the nucleic acid-target complexes; amplifying the nucleic acids dissociated from the nucleic acid-target complexes to yield a ligand enriched mixture of nucleic acids. The steps of binding, partitioning, dissociating and amplifying are repeated through as many cycles as desired to yield nucleic acid ligands which bind with high affinity and specificity to the target molecule.

Typically, such binding molecules are separately administered to the animal [see, *e.g.*, O'Connor (1991) J. Neurochem. 56:560], but such binding molecules can also be expressed in vivo from polynucleotides taken up by a host cell and expressed *in vivo* [see, *e*.*g*., Oligodeoxynucleotides as Antisense Inhibitors of Gene Expression, CRC Press, Boca Raton, Fla. (1988)].

Any of the polynucleotide antagonists disclosed herein (*e.g.*, a polynucleotide antagonist of activin A, activin B, activin C, activin E, GDF11, GDF8, BMP6, Nodal, GDF3, BMP3, or GDF3B) can be combined with one or more additional antagonist agents of the disclosure to achieve the desired effect. A polynucleotide antagonist disclosed herein (*e.g.*, a polynucleotide antagonist of activin A, activin B, activin C, activin E, GDF11, GDF8, BMP6, Nodal, GDF3, BMP3, or GDF3B) can be combined with another polynucleotide antagonist of the disclosure, or an antibody directed to any of the targets of the disclosure (*e*.*g*., an anti-GDF11 antibody, an anti-activin B antibody, an anti-activin B antibody, an anti-GDF8 antibody, an anti-activin C antibody, an anti-activin E antibody, an anti-BMP6 antibody, an anti-BMP6 antibody, an anti-GDF15 antibody, an anti-Nodal antibody, an anti-GDF3 antibody, an anti-BMP3 antibody, an anti-BMP3B antibody, an anti-BMP9 antibody, or an anti-BMP 10 antibody), or a non-antibody binding polypeptide disclosed herein (*e.g.*, a GDF11 binding polypeptide, an activin B binding polypeptide, an activin B binding polypeptide, an activin E binding polypeptide, an activin C binding polypeptide, a GDF8 binding polypeptide, a BMP6 binding polypeptide, a GDF15 binding polypeptide, a Nodal binding polypeptide, a GDF3 binding polypeptide, a BMP3 binding polypeptide, a BMP3B binding polypeptide, a BMP9 binding polypeptide, or a BMP10 binding polypeptide), or a ligand-trap polypeptide disclosed herein (*e.g.*, a GDF11-trap polypeptide, an activin-B trap polypeptide, or a GDF11/activin-B trap polypeptide), or a small-molecule antagonist directed to any of the targets of the disclosure (*e*.*g*., an activin-A small-molecule antagonist, an activin-B small-molecule antagonist, a GDF11 small-molecule antagonist, an activin-C small-molecule antagonist, an activin-E small-molecule antagonist, a GDF8 small-molecule antagonist, a BMP6 small-molecule antagonist, a BMP6 small-molecule antagonist, a GDF15 small-molecule antagonist, a Nodal small-molecule antagonist, a GDF3 small-molecule antagonist, a BMP3 small-molecule antagonist, a BMP3B small-molecule antagonist, a BMP9 small-molecule antagonist, or a BMP10 small-molecule antagonist). For example, an antisense antagonist of GDF11 can be combined with an activin-B antagonist of the disclosure (*e.g.*, an activin-B trap polypeptide, an anti-activin B antibody, a small-molecule antagonist of activin B, a polynucleotide antagonist of activin B, or a non-antibody polypeptide antagonist of activin B) to inhibit both a GDF11 and an activin B activity (*e.g.*, the ability to bind to and/or activate an ActRIIA and/or ActRIIB receptor). In an alternative embodiment, an antisense antagonist of activin-B antibody can be combined with a GDF11 antagonist of the disclosure (*e.g.*, a GDF-trap polypeptide, an anti-GDFl 1 antibody, a small-molecule antagonist of GDF11, a polynucleotide antagonist of GDF11, or a non-antibody polypeptide antagonist of GDF11) to inhibit both a GDF11 and an activin B activity.

### 3. Screening Assays

In certain aspects, the present disclosure relates to the use of disclosed ActRII polypeptides (*e.g*., soluble ActRIIA and ActRIIB polypeptides and variants thereof) to identify antagonists of one or more of GDF11, activin A, activin B, activin C, activin E, GDF8, activin A, BMP6, GDF15, Nodal, GDF3, BMP3, BMP3B, BMP9, and BMP10, particularly antagonist that inhibit one or more of these ligands from binding to and/or activating an ActRII receptor *(e.g.,* an ActRIIA and/or ActRIIB receptor), but that do not inhibit activin A from binding to and/or activating an ActRII receptor. Compounds identified through the screening assays disclosed herein can be tested to assess their ability to modulate red blood cell, hemoglobin and/or reticulocyte levels *in vivo* or *in vitro.* These compounds can be tested, for example, in animal models.

There are numerous approaches to screening for therapeutic agents for increasing red blood cell or hemoglobin levels by targeting ActRII signaling. In certain embodiments, high-throughput screening of compounds can be carried out to identify agents that perturb ActRII-mediated effects on a selected cell line. In certain embodiments, the assay is carried out to screen and identify compounds that specifically inhibit or reduce binding of an ActRII polypeptide to its binding partner, such as an ActRII ligand *(e.g.,* GDF11, activin A, activin B, activin C, activin E, GDF8, activin A , BMP6, GDF15, Nodal, GDF3, BMP3, BMP3B, BMP9, and BMP10). Alternatively, the assay can be used to identify compounds that do not substantially affect binding of an ActRII polypeptide to its binding partner, such as an ActRII ligand (*e.g.*, activin A). In a further embodiment, the compounds can be identified by their ability to interact with an ActRII polypeptide.

A variety of assay formats will suffice and, in light of the present disclosure, those not expressly described herein will nevertheless be comprehended by one of ordinary skill in the art. As described herein, the test compounds (agents) of the disclosure may be created by any combinatorial chemical method. Alternatively, the subject compounds may be naturally occurring biomolecules synthesized *in vivo* or *in vitro.* Compounds (agents) to be tested for their ability to act as modulators of tissue growth can be produced, for example, by bacteria, yeast, plants or other organisms *(e.g.,* natural products), produced chemically *(e.g.,* small molecules, including peptidomimetics), or produced recombinantly. Test compounds contemplated by the present disclosure include non-peptidyl organic molecules, peptides, polypeptides, peptidomimetics, sugars, hormones, and nucleic acid molecules. In a specific embodiment, the test agent is a small organic molecule having a molecular weight of less than about 2,000 Daltons.

The test compounds of the disclosure can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S transferase (GST), photoactivatible crosslinkers or any combinations thereof.

In many drug screening programs which test libraries of compounds and natural extracts, high-throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the *in vitro* system, the assay instead being focused primarily on the effect of the drug on the molecular target as may be manifest in an alteration of binding affinity between an ActRII polypeptide and its binding partner (*e.g*., GDF11, activin A, activin B, *etc*.).

Merely to illustrate, in an exemplary screening assay of the present disclosure, the compound of interest is contacted with an isolated and purified ActRIIB polypeptide which is ordinarily capable of binding to an ActRIIB ligand *(e.g.,* GDF11, activin A, activin B, activin C, activin E, GDF8, BMP6, activin A, BMP6, GDF15, Nodal, GDF3, BMP3, BMP3B, BMP9, and/or BMP10), as appropriate for the intention of the assay. To the mixture of the compound and ActRIIB polypeptide is then added to a composition containing an ActRIIB ligand. Detection and quantification of ActRIIB/ActRIIB ligand complexes provides a means for determining the compound's efficacy at inhibiting (or potentiating) complex formation between the ActRIIB polypeptide and its binding protein. The efficacy of the compound can be assessed by generating dose response curves from data obtained using various concentrations of the test compound. Moreover, a control assay can also be performed to provide a baseline for comparison. For example, in a control assay, isolated and purified ActRIIB ligand is added to a composition containing the ActRIIB polypeptide, and the formation of ActRIIB/ActRIIB ligand complex is quantitated in the absence of the test compound. It will be understood that, in general, the order in which the reactants may be admixed can be varied, and can be admixed simultaneously. Moreover, in place of purified proteins, cellular extracts and lysates may be used to render a suitable cell-free assay system.

Complex formation between the ActRII polypeptide and its binding protein may be detected by a variety of techniques. For instance, modulation of the formation of complexes can be quantitated using, for example, detectably labeled proteins such as radiolabeled (*e.g.,* ³²P, ³⁵S, ¹⁴C or ³H), fluorescently labeled *(e.g.,* FITC), or enzymatically labeled ActRII polypeptide or its binding protein, by immunoassay, or by chromatographic detection.

In certain embodiments, the present disclosure contemplates the use of fluorescence polarization assays and fluorescence resonance energy transfer (FRET) assays in measuring, either directly or indirectly, the degree of interaction between an ActRII polypeptide and its binding protein. Further, other modes of detection, such as those based on optical waveguides (see, *e.g.,* PCT Publication WO 96/26432 and U.S. Pat. No. 5,677,196), surface plasmon resonance (SPR), surface charge sensors, and surface force sensors, are compatible with many embodiments of the disclosure.

Moreover, the present disclosure contemplates the use of an interaction trap assay, also known as the "two hybrid assay," for identifying agents that disrupt or potentiate interaction between an ActRIIB polypeptide and its binding partner [see, *e.g.,* , U.S. Pat. No. 5,283,317; Zervos et al. (1993) Cell 72:223-232; Madura et al. (1993) J Biol Chem 268:12046-12054; Bartel et al. (1993) Biotechniques 14:920-924; and Iwabuchi et al. (1993) Oncogene 8:1693-1696)]. In certain embodiments, the present disclosure contemplates the use of reverse two hybrid systems to identify compounds (*e.g.*, small molecules or peptides) that dissociate interactions between an ActRII polypeptide and its binding protein [see, *e.g.,* Vidal and Legrain, (1999) Nucleic Acids Res 27:919-29; Vidal and Legrain (1999) Trends Biotechnol 17:374-81; and U.S. Pat. Nos. 5,525,490; 5,955,280; and 5,965,368].

In certain embodiments, the subject compounds are identified by their ability to interact with an ActRII polypeptide. The interaction between the compound and the ActRII polypeptide may be covalent or non-covalent. For example, such interaction can be identified at the protein level using *in vitro* biochemical methods, including photocrosslinking, radiolabeled ligand binding, and affinity chromatography [see, *e.g.,* Jakoby WB et al. (1974) Methods in Enzymology 46: 1]. In certain cases, the compounds may be screened in a mechanism based assay, such as an assay to detect compounds which bind to an ActRII polypeptide. This may include a solid phase or fluid phase binding event. Alternatively, the gene encoding an ActRII polypeptide can be transfected with a reporter system (*e.g.*, β-galactosidase, luciferase, or green fluorescent protein) into a cell and screened against the library preferably by a high-throughput screening or with individual members of the library. Other mechanism based binding assays may be used, for example, binding assays which detect changes in free energy. Binding assays can be performed with the target fixed to a well, bead or chip or captured by an immobilized antibody or resolved by capillary electrophoresis. The bound compounds may be detected usually using colorimetric or fluorescence or surface plasmon resonance.

### 4. Exemplary Therapeutic Uses

In certain aspects, antagonist agents, or combination of agents, of the disclosure that inhibit at least GDF11 and/or activin B can be used to increase red blood cell levels, treat or prevent an anemia, and/or treat or prevent ineffective erythropoiesis in a subject in need thereof. Optionally, antagonist agents, or combination of agents, to be used in accordance with the methods herein do not inhibit activin A. Optionally, antagonist agents, or combination of agents, to be used in accordance with the methods herein further inhibit GDF8. In certain aspects, antagonist agents, or combination of agents, to be used in accordance with the methods herein, in addition to inhibiting GDF11 and/or activin B, further inhibit one or more of GDF8, activin C, activin E, activin A, BMP6, GDF15, Nodal, GDF3, BMP3, BMP3B, BMP9, and BMP10.

The terms "subject," an "individual," or a "patient" are interchangeable throughout the specification and generally refer to mammals. Mammals include, but are not limited to, domesticated animals *(e.g.,* cows, sheep, cats, dogs, and horses), primates *(e.g.,* humans and non-human primates such as monkeys), rabbits, and rodents (*e.g.*, mice and rats). In certain aspects, an antagonist agent of the present disclosure may be used in combination with conventional therapeutic approaches for increasing red blood cell levels, particularly those used to treat anemias of multifactorial origin. Conventional therapeutic approaches for increasing red blood cell levels include, for example, red blood cell transfusion, administration of one or more EPO receptor activators, hematopoietic stem cell transplantation, immunosuppressive biologics and drugs (*e.g.*, corticosteroids). In certain embodiments, an antagonist agent of the present disclosure can be used to treat or prevent an anemia in a subject in need thereof. In certain embodiments, an antagonist agent of the present disclosure can be used to treat or prevent ineffective erythropoiesis and/or the disorders associated with ineffective erythropoiesis in a subject in need thereof. In certain aspects, an antagonist agent of the present disclosure can be used in combination with conventional therapeutic approaches for treating or preventing an anemia or ineffective erythropoiesis disorder, particularly those used to treat anemias of multifactorial origin.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

The term "treating" as used herein includes amelioration or elimination of the condition once it has been established.

In either case, prevention or treatment may be discerned in the diagnosis provided by a physician or other health care provider and the intended result of administration of the therapeutic agent.

In general, treatment or prevention of a disease or condition as described in the present disclosure is achieved by administering one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the present disclosure in an "effective amount". An effective amount of an agent refers to an amount effective, at dosages and for periods of time necessary, to achieve the desired therapeutic or prophylactic result. A "therapeutically effective amount" of an agent of the present disclosure may vary according to factors such as the disease state, age, sex, and weight of the individual, and the ability of the agent to elicit a desired response in the individual. A "prophylactically effective amount" refers to an amount effective, at dosages and for periods of time necessary, to achieve the desired prophylactic result.

In certain embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, may be used to increase red blood cell, hemoglobin, or reticulocyte levels in healthy individuals and selected patient populations. Examples of appropriate patient populations include those with undesirably low red blood cell or hemoglobin levels, such as patients having an anemia, and those that are at risk for developing undesirably low red blood cell or hemoglobin levels, such as those patients who are about to undergo major surgery or other procedures that may result in substantial blood loss. In one embodiment, a patient with adequate red blood cell levels is treated with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) to increase red blood cell levels, and then blood is drawn and stored for later use in transfusions.

One or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, may be used to increase red blood cell levels, hemoglobin levels, and/or hematocrit levels in a patient having an anemia. When observing hemoglobin and/or hematocrit levels in humans, a level of less than normal for the appropriate age and gender category may be indicative of anemia, although individual variations are taken into account. For example, a hemoglobin level from 10-12.5 g/dl, and typically about 11.0 g/dl is considered to be within the normal range in health adults, although, in terms of therapy, a lower target level may cause fewer cardiovascular side effects [see, *e.g.,* Jacobs et al. (2000) Nephrol Dial Transplant 15, 15-19]. Alternatively, hematocrit levels (percentage of the volume of a blood sample occupied by the cells) can be used as a measure for anemia. Hematocrit levels for healthy individuals range from about 41-51% for adult males and from 35-45% for adult females. In certain embodiments, a patient may be treated with a dosing regimen intended to restore the patient to a target level of red blood cells, hemoglobin, and/or hematocrit. As hemoglobin and hematocrit levels vary from person to person, optimally, the target hemoglobin and/or hematocrit level can be individualized for each patient.

Anemia is frequently observed in patients having a tissue injury, an infection, and/or a chronic disease, particularly cancer. In some subjects, anemia is distinguished by low erythropoietin levels and/or an inadequate response to erythropoietin in the bone marrow [see, *e.g*., Adamson (2008) Harrison's Principles of Internal Medicine, 17th ed.; McGraw Hill, New York, pp 628-634]. Potential causes of anemia include, for example, blood loss, nutritional deficits (e.g. reduced dietary intake of protein), medication reaction, various problems associated with the bone marrow, and many diseases. More particularly, anemia has been associated with a variety of disorders and conditions that include, for example, bone marrow transplantation; solid tumors (*e.g.*, breast cancer, lung cancer, and colon cancer); tumors of the lymphatic system (*e.g.*, chronic lymphocyte leukemia, non-Hodgkins lymphoma, and Hodgkins lymphoma); tumors of the hematopoietic system (*e.g*., leukemia, a myelodysplastic syndrome and multiple myeloma); radiation therapy; chemotherapy (*e.g*., platinum containing regimens); inflammatory and autoimmune diseases, including, but not limited to, rheumatoid arthritis, other inflammatory arthritides, systemic lupus erythematosis (SLE), acute or chronic skin diseases *(e.g.,* psoriasis), inflammatory bowel disease *(e.g.,* Crohn's disease and ulcerative colitis); acute or chronic renal disease or failure, including idiopathic or congenital conditions; acute or chronic liver disease; acute or chronic bleeding; situations where transfusion of red blood cells is not possible due to patient allo- or auto-antibodies and/or for religious reasons *(e.g.,* some Jehovah's Witnesses); infections *(e.g.,* malaria and osteomyelitis); hemoglobinopathies including, for example, sickle cell disease (anemia), thalassemias; drug use or abuse (*e.g*., alcohol misuse); pediatric patients with anemia from any cause to avoid transfusion; and elderly patients or patients with underlying cardiopulmonary disease with anemia who cannot receive transfusions due to concerns about circulatory overload [see, *e.g*., Adamson (2008) Harrison's Principles of Internal Medicine, 17th ed.; McGraw Hill, New York, pp 628-634]. In some embodiments, one or more GDF11 and/or activin B antagonist agents (optionally further antagonists of one or more of GDF8, activin A, activin C, activin E, and BMP6) of the disclosure could be used to treat or prevent anemia associated with one or more of the disorders or conditions disclosed herein.

Many factors can contribute to cancer-related anemia. Some are associated with the disease process itself and the generation of inflammatory cytokines such as interleukin-1, interferon-gamma, and tumor necrosis factor [Bron et al. (2001) Semin Oncol 28(Suppl 8):1-6]. Among its effects, inflammation induces the key iron-regulatory peptide hepcidin, thereby inhibiting iron export from macrophages and generally limiting iron availability for erythropoiesis [see, *e.g.,* Ganz (2007) J Am Soc Nephrol 18:394-400]. Blood loss through various routes can also contribute to cancer-related anemia. The prevalence of anemia due to cancer progression varies with cancer type, ranging from 5% in prostate cancer up to 90% in multiple myeloma. Cancer-related anemia has profound consequences for patients, including fatigue and reduced quality of life, reduced treatment efficacy, and increased mortality. In some embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, could be used to treat a cancer-related anemia.

A hypoproliferative anemia can result from primary dysfunction or failure of the bone marrow. Hypoproliferative anemias include: anemia of chronic disease, anemia of kidney disease, anemia associated with hypometabolic states, and anemia associated with cancer. In each of these types, endogenous erythropoietin levels are *inappropriately low* for the degree of anemia observed. Other hypoproliferative anemias include: early-stage iron-deficient anemia, and anemia caused by damage to the bone marrow. In these types, endogenous erythropoietin levels are *appropriately elevated* for the degree of anemia observed. Prominent examples would be myelosuppression caused by cancer and/or chemotherapeutic drugs or cancer radiation therapy. A broad review of clinical trials found that mild anemia can occur in 100% of patients after chemotherapy, while more severe anemia can occur in up to 80% of such patients [see, *e.g.,* Groopman et al. (1999) J Natl Cancer Inst 91:1616-1634]. Myelosuppressive drugs include, for example: 1) alkylating agents such as nitrogen mustards *(e.g.,* melphalan) and nitrosoureas *(e.g.,* streptozocin); 2) antimetabolites such as folic acid antagonists *(e.g.,* methotrexate), purine analogs *(e.g.,* thioguanine), and pyrimidine analogs (*e.g*., gemcitabine); 3) cytotoxic antibiotics such as anthracyclines *(e.g.,* doxorubicin); 4) kinase inhibitors *(e.g.,* gefitinib); 5) mitotic inhibitors such as taxanes *(e.g.,* paclitaxel) and vinca alkaloids *(e.g.,* vinorelbine); 6) monoclonal antibodies *(e.g.,* rituximab); and 7) topoisomerase inhibitors *(e.g.,* topotecan and etoposide). In addition, conditions resulting in a hypometabolic rate can produce a mild-to-moderate hypoproliferative anemia. Among such conditions are endocrine deficiency states. For example, anemia can occur in Addison's disease, hypothyroidism, hyperparathyroidism, or males who are castrated or treated with estrogen. In some embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, could be used to treat a hyperproliferative anemia.

Chronic kidney disease is sometimes associated with hypoproliferative anemia, and the degree of the anemia varies in severity with the level of renal impairment. Such anemia is primarily due to inadequate production of erythropoietin and reduced survival of red blood cells. Chronic kidney disease usually proceeds gradually over a period of years or decades to end-stage (Stage-5) disease, at which point dialysis or kidney transplantation is required for patient survival. Anemia often develops early in this process and worsens as disease progresses. The clinical consequences of anemia of kidney disease are well-documented and include development of left ventricular hypertrophy, impaired cognitive function, reduced quality of life, and altered immune function [see, *e.g.,* Levin et al. (1999) Am J Kidney Dis 27:347-354; Nissenson (1992) Am J Kidney Dis 20(Suppl 1):21-24; Revicki et al. (1995) Am J Kidney Dis 25:548-554; Gafter et al., (1994) Kidney Int 45:224-231]. In some embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10), optionally combined with an EPO receptor activator, could be used to treat anemia associated with acute or chronic renal disease or failure.

Anemia resulting from acute blood loss of sufficient volume, such as from trauma or postpartum hemorrhage, is known as acute post-hemorrhagic anemia. Acute blood loss initially causes hypovolemia without anemia since there is proportional depletion of RBCs along with other blood constituents. However, hypovolemia will rapidly trigger physiologic mechanisms that shift fluid from the extravascular to the vascular compartment, which results in hemodilution and anemia. If chronic, blood loss gradually depletes body iron stores and eventually leads to iron deficiency. In some embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) optionally combined with an EPO receptor activator, could be used to treat anemia resulting from acute blood loss.

Iron-deficiency anemia is the final stage in a graded progression of increasing iron deficiency which includes negative iron balance and iron-deficient erythropoiesis as intermediate stages. Iron deficiency can result from increased iron demand, decreased iron intake, or increased iron loss, as exemplified in conditions such as pregnancy, inadequate diet, intestinal malabsorption, acute or chronic inflammation, and acute or chronic blood loss. With mild-to-moderate anemia of this type, the bone marrow remains hypoproliferative, and RBC morphology is largely normal; however, even mild anemia can result in some microcytic hypochromic RBCs, and the transition to severe iron-deficient anemia is accompanied by hyperproliferation of the bone marrow and increasingly prevalent microcytic and hypochromic RBCs [see, *e.g.*, Adamson (2008) Harrison's Principles of Internal Medicine, 17th ed.; McGraw Hill, New York, pp 628-634]. Appropriate therapy for iron-deficiency anemia depends on its cause and severity, with oral iron preparations, parenteral iron formulations, and RBC transfusion as major conventional options. In some embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, could be used to treat a chronic iron-deficiency.

Myelodysplastic syndrome (MDS) is a diverse collection of hematological conditions characterized by ineffective production of myeloid blood cells and risk of transformation to acute myelogenous leukemia. In MDS patients, blood stem cells do not mature into healthy red blood cells, white blood cells, or platelets. MDS disorders include, for example, refractory anemia, refractory anemia with ringed sideroblasts, refractory anemia with excess blasts, refractory anemia with excess blasts in transformation, refractory cytopenia with multilineage dysplasia, and myelodysplastic syndrome associated with an isolated 5q chromosome abnormality. As these disorders manifest as irreversible defects in both quantity and quality of hematopoietic cells, most MDS patients are afflicted with chronic anemia. Therefore, MDS patients eventually require blood transfusions and/or treatment with growth factors (e.g., erythropoietin or G-CSF) to increase red blood cell levels. However, many MDS patients develop side-effects due to frequency of such therapies. For example, patients who receive frequent red blood cell transfusion can exhibit tissue and organ damage from the buildup of extra iron. Accordingly, one or more GDF11 and/or activin B antagonist agents (optionally further antagonists of one or more of GDF8, activin A, activin C, activin E, and BMP6) of the disclosure, may be used to treat patients having MDS. In certain embodiments, patients suffering from MDS may be treated using one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally in combination with an EPO receptor activator. In other embodiments, patient suffering from MDS may be treated using a combination of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure and one or more additional therapeutic agents for treating MDS including, for example, thalidomide, lenalidomide, azacitadine, decitabine, erythropoietins, deferoxamine, antithymocyte globulin, and filgrastrim (G-CSF).

Originally distinguished from aplastic anemia, hemorrhage, or peripheral hemolysis on the basis of ferrokinetic studies [see, *e.g.,* Ricketts et al. (1978) Clin Nucl Med 3:159-164], ineffective erythropoiesis describes a diverse group of anemias in which production of mature RBCs is less than would be expected given the number of erythroid precursors (erythroblasts) present in the bone marrow [Tanno et al. (2010) Adv Hematol 2010:358283]. In such anemias, tissue hypoxia persists despite elevated erythropoietin levels due to ineffective production of mature RBCs. A vicious cycle eventually develops in which elevated erythropoietin levels drive massive expansion of erythroblasts, potentially leading to splenomegaly (spleen enlargement) due to extramedullary erythropoiesis [see, *e.g.,* Aizawa et al. (2003) Am J Hematol 74:68-72], erythroblast-induced bone pathology [see, *e.g.,* Di Matteo et al. (2008) J Biol Regul Homeost Agents 22:211-216], and tissue iron overload, even in the absence of therapeutic RBC transfusions [see, *e.g.,* Pippard et al. (1979) Lancet 2:819-821]. Thus, by boosting erythropoietic effectiveness, a GDF11 and/or activin B antagonist of the present disclosure may break the aforementioned cycle and thus alleviate not only the underlying anemia but also the associated complications of elevated erythropoietin levels, splenomegaly, bone pathology, and tissue iron overload. In some embodiments, one or more antagonist agents of the present disclosure (*e.g*., an GDF11, activin A, activin B, activin C, activin E, GDF8, BMP6, GDF15, Nodal, GDF3, BMP3, BMP3B, BMP9, and/or BMP10 antagonist) can be used to treat or prevent ineffective erythropoiesis, including anemia and elevated EPO levels as well as complications such as splenomegaly, erythroblast-induced bone pathology, iron overload, and their attendant pathologies. With splenomegaly, such pathologies include thoracic or abdominal pain and reticuloendothelial hyperplasia. Extramedullary hematopoiesis can occur not only in the spleen but potentially in other tissues in the form of extramedullary hematopoietic pseudotumors [see, *e.g.,* Musallam et al. (2012) Cold Spring Harb Perspect Med 2:a013482]. With erythroblast-induced bone pathology, attendant pathologies include low bone mineral density, osteoporosis, and bone pain [see, *e.g.,* Haidar et al. (2011) Bone 48:425-432]. With iron overload, attendant pathologies include hepcidin suppression and hyperabsorption of dietary iron [see, *e.g.,* Musallam et al. (2012) Blood Rev 26(Suppl 1):S16-S19], multiple endocrinopathies and liver fibrosis/cirrhosis [see, *e.g.,* Galanello et al. (2010) Orphanet J Rare Dis 5:11], and iron-overload cardiomyopathy [Lekawanvijit et al., 2009, Can J Cardiol 25:213-218].

The most common causes of ineffective erythropoiesis are the thalassemia syndromes, hereditary hemoglobinopathies in which imbalances in the production of intact alpha- and beta-hemoglobin chains lead to increased apoptosis during erythroblast maturation [see, *e.g.,* Schrier (2002) Curr Opin Hematol 9:123-126]. Thalassemias are collectively among the most frequent genetic disorders worldwide, with changing epidemiologic patterns predicted to contribute to a growing public health problem in both the U.S. and globally [Vichinsky (2005) Ann NY Acad Sci 1054:18-24]. Thalassemia syndromes are named according to their severity. Thus, α-thalassemias include α-thalassemia minor (also known as α-thalassemia trait; two affected α-globin genes), hemoglobin H disease (three affected α-globin genes), and α-thalassemia major (also known as hydrops fetalis; four affected α-globin genes). β-Thalassemias include β-thalassemia minor (also known as β-thalassemia trait; one affected β-globin gene), β-thalassemia intermedia (two affected β-globin genes), hemoglobin E thalassemia (two affected β-globin genes), and β-thalassemia major (also known as Cooley's anemia; two affected β-globin genes resulting in a complete absence of β-globin protein). β-Thalassemia impacts multiple organs, is associated with considerable morbidity and mortality, and currently requires life-long care. Although life expectancy in patients with β-thalassemia has increased in recent years due to use of regular blood transfusions in combination with iron chelation, iron overload resulting both from transfusions and from excessive gastrointestinal absorption of iron can cause serious complications such as heart disease, thrombosis, hypogonadism, hypothyroidism, diabetes, osteoporosis, and osteopenia [see, *e.g.,* Rund et al. (2005) N Engl J Med 353:1135-1146]. In certain embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, can be used to treat or prevent a thalassemia syndrome.

In some embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, can be used for treating disorders of ineffective erythropoiesis besides thalassemia syndromes. Such disorders include siderblastic anemia (inherited or acquired); dyserythropoietic anemia (Types I and II); sickle cell anemia; hereditary spherocytosis; pyruvate kinase deficiency; megaloblastic anemias, potentially caused by conditions such as folate deficiency (due to congenital diseases, decreased intake, or increased requirements), cobalamin deficiency (due to congenital diseases, pernicious anemia, impaired absorption, pancreatic insufficiency, or decreased intake), certain drugs, or unexplained causes (congenital dyserythropoietic anemia, refractory megaloblastic anemia, or erythroleukemia); myelophthisic anemias including, for example, myelofibrosis (myeloid metaplasia) and myelophthisis; congenital erythropoietic porphyria; and lead poisoning.

In certain embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may be used in combination with supportive therapies for ineffective erythropoiesis. Such therapies include transfusion with either red blood cells or whole blood to treat anemia. In chronic or hereditary anemias, normal mechanisms for iron homeostasis are overwhelmed by repeated transfusions, eventually leading to toxic and potentially fatal accumulation of iron in vital tissues such as heart, liver, and endocrine glands. Thus, supportive therapies for patients chronically afflicted with ineffective erythropoiesis also include treatment with one or more iron-chelating molecules to promote iron excretion in the urine and/or stool and thereby prevent, or reverse, tissue iron overload [see, *e.g.,* Hershko (2006) Haematologica 91:1307-1312; Cao et al. (2011), Pediatr Rep 3(2):e17]. Effective iron-chelating agents should be able to selectively bind and neutralize ferric iron, the oxidized form of non-transferrin bound iron which likely accounts for most iron toxicity through catalytic production of hydroxyl radicals and oxidation products [see, *e.g.,* Esposito et al. (2003) Blood 102:2670-2677]. These agents are structurally diverse, but all possess oxygen or nitrogen donor atoms able to form neutralizing octahedral coordination complexes with individual iron atoms in stoichiometries of 1:1 (hexadentate agents), 2:1 (tridentate), or 3:1 (bidentate) [Kalinowski et al. (2005) Pharmacol Rev 57:547-583]. In general, effective iron-chelating agents also are relatively low molecular weight (*e.g*., less than 700 daltons), with solubility in both water and lipids to enable access to affected tissues. Specific examples of iron-chelating molecules include deferoxamine, a hexadentate agent of bacterial origin requiring daily parenteral administration, and the orally active synthetic agents deferiprone (bidentate) and deferasirox (tridentate). Combination therapy consisting of same-day administration of two iron-chelating agents shows promise in patients unresponsive to chelation monotherapy and also in overcoming issues of poor patient compliance with dereroxamine alone [Cao et al. (2011) Pediatr Rep 3(2):e17; Galanello et al. (2010) Ann NY Acad Sci 1202:79-86].

As used herein, "in combination with" or "conjoint administration" refers to any form of administration such that the second therapy is still effective in the body (e.g., the two compounds are simultaneously effective in the patient, which may include synergistic effects of the two compounds). Effectiveness may not correlate to measurable concentration of the agent in blood, serum, or plasma. For example, the different therapeutic compounds can be administered either in the same formulation or in separate formulations, either concomitantly or sequentially, and on different schedules. Thus, an individual who receives such treatment can benefit from a combined effect of different therapies. One or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure can be administered concurrently with, prior to, or subsequent to, one or more other additional agents or supportive therapies. In general, each therapeutic agent will be administered at a dose and/or on a time schedule determined for that particular agent. The particular combination to employ in a regimen will take into account compatibility of the antagonist of the present disclosure with the therapy and/or the desired therapeutic effect to be achieved.

In certain embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may be used in combination with hepcidin or a hepcidin agonist for ineffective erythropoiesis. A circulating polypeptide produced mainly in the liver, hepcidin is considered a master regulator of iron metabolism by virtue of its ability to induce the degradation of ferroportin, an iron-export protein localized on absorptive enterocytes, hepatocytes, and macrophages. Broadly speaking, hepcidin reduces availability of extracellular iron, so hepcidin agonists may be beneficial in the treatment of ineffective erythropoiesis [see, *e.g*., Nemeth (2010) Adv Hematol 2010:750643]. This view is supported by beneficial effects of increased hepcidin expression in a mouse model of β-thalassemia [Gardenghi et al. (2010) J Clin Invest 120:4466-4477].

One or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, optionally combined with an EPO receptor activator, would also be appropriate for treating anemias of disordered RBC maturation, which are characterized in part by undersized (microcytic), oversized (macrocytic), misshapen, or abnormally colored (hypochromic) RBCs.

In certain embodiments, the present disclosure provides methods of treating or preventing anemia in an individual in need thereof by administering to the individual a therapeutically effective amount of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure and a EPO receptor activator. In certain embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may be used in combination with EPO receptor activators to reduce the required dose of these activators in patients that are susceptible to adverse effects of EPO. These methods may be used for therapeutic and prophylactic treatments of a patient.

One or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may be used in combination with EPO receptor activators to achieve an increase in red blood cells, particularly at lower dose ranges of EPO receptor activators. This may be beneficial in reducing the known off-target effects and risks associated with high doses of EPO receptor activators. The primary adverse effects of EPO include, for example, an excessive increase in the hematocrit or hemoglobin levels and polycythemia. Elevated hematocrit levels can lead to hypertension (more particularly aggravation of hypertension) and vascular thrombosis. Other adverse effects of EPO which have been reported, some of which relate to hypertension, are headaches, influenza-like syndrome, obstruction of shunts, myocardial infarctions and cerebral convulsions due to thrombosis, hypertensive encephalopathy, and red cell blood cell aplasia. See, *e.g.,* Singibarti (1994) J. Clin Investig 72(suppl 6), S36-S43; Horl et al. (2000) Nephrol Dial Transplant 15(suppl 4), 51-56; Delanty et al. (1997) Neurology 49, 686-689; and Bunn (2002) N Engl J Med 346(7), 522-523).

Provided that antagonists of the present disclosure act by a different mechanism than EPO, these antagonists may be useful for increasing red blood cell and hemoglobin levels in patients that do not respond well to EPO. For example, an antagonist of the present disclosure may be beneficial for a patient in which administration of a normal-to-increased dose of EPO (>300 IU/kg/week) does not result in the increase of hemoglobin level up to the target level. Patients with an inadequate EPO response are found in all types of anemia, but higher numbers of non-responders have been observed particularly frequently in patients with cancers and patients with end-stage renal disease. An inadequate response to EPO can be either constitutive (observed upon the first treatment with EPO) or acquired (observed upon repeated treatment with EPO).

In certain embodiments, the present disclosure provides methods for managing a patient that has been treated with, or is a candidate to be treated with, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure by measuring one or more hematologic parameters in the patient. The hematologic parameters may be used to evaluate appropriate dosing for a patient who is a candidate to be treated with the antagonist of the present disclosure, to monitor the hematologic parameters during treatment, to evaluate whether to adjust the dosage during treatment with one or more antagonist of the disclosure, and/or to evaluate an appropriate maintenance dose of one or more antagonists of the disclosure. If one or more of the hematologic parameters are outside the normal level, dosing with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may be reduced, delayed or terminated.

Hematologic parameters that may be measured in accordance with the methods provided herein include, for example, red blood cell levels, blood pressure, iron stores, and other agents found in bodily fluids that correlate with increased red blood cell levels, using art-recognized methods. Such parameters may be determined using a blood sample from a patient. Increases in red blood cell levels, hemoglobin levels, and/or hematocrit levels may cause increases in blood pressure.

In one embodiment, if one or more hematologic parameters are outside the normal range or on the high side of normal in a patient who is a candidate to be treated with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, then onset of administration of the one or more antagonists of the disclosure may be delayed until the hematologic parameters have returned to a normal or acceptable level either naturally or via therapeutic intervention. For example, if a candidate patient is hypertensive or pre-hypertensive, then the patient may be treated with a blood pressure lowering agent in order to reduce the patient's blood pressure. Any blood pressure lowering agent appropriate for the individual patient's condition may be used including, for example, diuretics, adrenergic inhibitors (including alpha blockers and beta blockers), vasodilators, calcium channel blockers, angiotensin-converting enzyme (ACE) inhibitors, or angiotensin II receptor blockers. Blood pressure may alternatively be treated using a diet and exercise regimen. Similarly, if a candidate patient has iron stores that are lower than normal, or on the low side of normal, then the patient may be treated with an appropriate regimen of diet and/or iron supplements until the patient's iron stores have returned to a normal or acceptable level. For patients having higher than normal red blood cell levels and/or hemoglobin levels, then administration of the one or more antagonists of the disclosure may be delayed until the levels have returned to a normal or acceptable level.

In certain embodiments, if one or more hematologic parameters are outside the normal range or on the high side of normal in a patient who is a candidate to be treated with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure, then the onset of administration may not be delayed. However, the dosage amount or frequency of dosing of the one or more antagonists of the disclosure may be set at an amount that would reduce the risk of an unacceptable increase in the hematologic parameters arising upon administration of the one or more antagonists of the disclosure. Alternatively, a therapeutic regimen may be developed for the patient that combines one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure with a therapeutic agent that addresses the undesirable level of the hematologic parameter. For example, if the patient has elevated blood pressure, then a therapeutic regimen involving administration of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure and a blood pressure-lowering agent may be designed. For a patient having lower than desired iron stores, a therapeutic regimen of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) and iron supplementation may be developed.

In one embodiment, baseline parameter(s) for one or more hematologic parameters may be established for a patient who is a candidate to be treated with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure and an appropriate dosing regimen established for that patient based on the baseline value(s). Alternatively, established baseline parameters based on a patient's medical history could be used to inform an appropriate antagonist-dosing regimen for a patient. For example, if a healthy patient has an established baseline blood pressure reading that is above the defined normal range it may not be necessary to bring the patient's blood pressure into the range that is considered normal for the general population prior to treatment with the one or more antagonist of the disclosure. A patient's baseline values for one or more hematologic parameters prior to treatment with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may also be used as the relevant comparative values for monitoring any changes to the hematologic parameters during treatment with the one or more antagonists of the disclosure.

In certain embodiments, one or more hematologic parameters are measured in patients who are being treated with a one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure. The hematologic parameters may be used to monitor the patient during treatment and permit adjustment or termination of the dosing with the one or more antagonists of the disclosure or additional dosing with another therapeutic agent. For example, if administration of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure results in an increase in blood pressure, red blood cell level, or hemoglobin level, or a reduction in iron stores, then the dose of the one or more antagonists of the disclosure may be reduced in amount or frequency in order to decrease the effects of the one or more antagonist of the disclosure on the one or more hematologic parameters. If administration of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure results in a change in one or more hematologic parameters that is adverse to the patient, then the dosing of the one or more antagonist of the disclosure may be terminated either temporarily, until the hematologic parameter(s) return to an acceptable level, or permanently. Similarly, if one or more hematologic parameters are not brought within an acceptable range after reducing the dose or frequency of administration of the one or more antagonists of the disclosure, then the dosing may be terminated. As an alternative, or in addition to, reducing or terminating the dosing with the one or more antagonists of the disclosure, the patient may be dosed with an additional therapeutic agent that addresses the undesirable level in the hematologic parameter(s), such as, for example, a blood pressure-lowering agent or an iron supplement. For example, if a patient being treated with one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure has elevated blood pressure, then dosing with the one or more antagonists of the disclosure may continue at the same level and a blood pressure-lowering agent is added to the treatment regimen, dosing with the one or more antagonists of the disclosure may be reduced *(e.g.,* in amount and/or frequency) and a blood pressure-lowering agent is added to the treatment regimen, or dosing with the one or more antagonists of the disclosure may be terminated and the patient may be treated with a blood pressure-lowering agent.

### 5. Pharmaceutical Compositions

In certain embodiments, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure can be administered alone or as a component of a pharmaceutical formulation (therapeutic composition or pharmaceutical composition). A pharmaceutical formation refers to a preparation which is in such form as to permit the biological activity of an active ingredient contained therein to be effective and which contains no additional components which are unacceptably toxic to a subject to which the formulation would be administered. The subject compounds may be formulated for administration in any convenient way for use in human or veterinary medicine. For example, one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure may be formulated with a pharmaceutically acceptable carrier. A pharmaceutically acceptable carrier refers to an ingredient in a pharmaceutical formulation, other than an active ingredient, which is generally nontoxic to a subject. A pharmaceutically acceptable carrier includes, but is not limited to, a buffer, excipient, stabilizer, or preservative. In general, pharmaceutical formulations for use in the present disclosure are in a pyrogen-free, physiologically acceptable form when administered to a subject. Therapeutically useful agents other than the antagonist of the disclosure, which may optionally be included in the formulation as described above, may be administered in combination with the subject compounds in the methods of the present disclosure.

Typically, compounds will be administered parenterally [e.g., by intravenous (I.V.) injection, intraarterial injection, intraosseous injection, intramuscular injection, intrathecal injection, subcutaneous injection, or intradermal injection]. Pharmaceutical compositions suitable for parenteral administration may comprise one or more GDF11 and/or activin B antagonist agents (optionally further antagonists of one or more of GDF8, activin A, activin C, activin E, and BMP6) of the disclosure in combination with one or more pharmaceutically acceptable sterile isotonic aqueous or nonaqueous solutions, dispersions, suspensions or emulsions, or sterile powders which may be reconstituted into sterile injectable solutions or dispersions just prior to use. Injectable solutions or dispersions may contain antioxidants, buffers, bacteriostats, suspending agents, thickening agents, or solutes which render the formulation isotonic with the blood of the intended recipient. Examples of suitable aqueous and nonaqueous carriers which may be employed in the pharmaceutical formulations of the present disclosure include water, ethanol, polyols *(e.g.,* glycerol, propylene glycol, polyethylene glycol, *etc*.)*,* vegetable oils *(e.g.,* olive oil), injectable organic esters *(e.g.,* ethyl oleate), and suitable mixtures thereof. Proper fluidity can be maintained, for example, by the use of coating materials (*e.g*., lecithin), by the maintenance of the required particle size in the case of dispersions, and by the use of surfactants.

In certain embodiments, a therapeutic method of the present disclosure includes administering the formulation systemically, or locally, from an implant or device. Further, the composition may be encapsulated or injected in a form for delivery to a target tissue site *(e.g.,* bone marrow or muscle). In certain embodiments, compositions of the present disclosure may include a matrix capable of delivering one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure to a target tissue site *(e.g.,* bone marrow or muscle), providing a structure for the developing tissue and optimally capable of being resorbed into the body. For example, the matrix may provide slow release of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure. Such matrices may be formed of materials presently in use for other implanted medical applications.

The choice of matrix material may be based on one or more of: biocompatibility, biodegradability, mechanical properties, cosmetic appearance, and interface properties. The particular application of the subject compositions will define the appropriate formulation. Potential matrices for the compositions may be biodegradable and chemically defined calcium sulfate, tricalciumphosphate, hydroxyapatite, polylactic acid, and polyanhydrides. Other potential materials are biodegradable and biologically well-defined including, for example, bone or dermal collagen. Further matrices are comprised of pure proteins or extracellular matrix components. Other potential matrices are non-biodegradable and chemically defined including, for example, sintered hydroxyapatite, bioglass, aluminates, or other ceramics. Matrices may be comprised of combinations of any of the above mentioned types of material including, for example, polylactic acid and hydroxyapatite or collagen and tricalciumphosphate. The bioceramics may be altered in composition (*e.g*., calcium-aluminate-phosphate) and processing to alter one or more of pore size, particle size, particle shape, and biodegradability.

In certain embodiments, formulations (compositions) of present disclosure can be administered orally, for example, in the form of capsules, cachets, pills, tablets, lozenges (using a flavored basis such as sucrose and acacia or tragacanth), powders, granules, a solution or a suspension in an aqueous or non-aqueous liquid, an oil-in-water or water-in-oil liquid emulsion, or an elixir or syrup, or pastille (using an inert base, such as gelatin and glycerin, or sucrose and acacia), and/or a mouth wash, each containing a predetermined amount of a compound of the present disclosure and optionally one or more other active ingredients. A compound of the present disclosure and optionally one or more other active ingredients may also be administered as a bolus, electuary, or paste.

In solid dosage forms for oral administration (*e.g*., capsules, tablets, pills, dragees, powders, and granules), one or more compounds of the present disclosure may be mixed with one or more pharmaceutically acceptable carriers including, for example, sodium citrate, dicalcium phosphate, a filler or extender (*e.g*., a starch, lactose, sucrose, glucose, mannitol, and silicic acid), a binder (e.g. carboxymethylcellulose, an alginate, gelatin, polyvinyl pyrrolidone, sucrose, and acacia), a humectant *(e.g.,* glycerol), a disintegrating agent *(e.g.,* agar-agar, calcium carbonate, potato or tapioca starch, alginic acid, a silicate, and sodium carbonate), a solution retarding agent *(e.g.* paraffin), an absorption accelerator *(e.g.* a quaternary ammonium compound), a wetting agent *(e.g.,* cetyl alcohol and glycerol monostearate), an absorbent *(e.g.,* kaolin and bentonite clay), a lubricant *(e.g.,* a talc, calcium stearate, magnesium stearate, solid polyethylene glycols, sodium lauryl sulfate), a coloring agent, and mixtures thereof. In the case of capsules, tablets, and pills, the pharmaceutical formulation (composition) may also comprise a buffering agent. Solid compositions of a similar type may also be employed as fillers in soft and hard-filled gelatin capsules using one or more excipients including, e.g., lactose or a milk sugar as well as a high molecular-weight polyethylene glycol.

Liquid dosage forms for oral administration of the present formulations (compositions) may include pharmaceutically acceptable emulsions, microemulsions, solutions, suspensions, syrups, and elixirs. In addition to the active ingredient(s), the liquid dosage form may contain an inert diluent commonly used in the art including, for example, water or other solvent, a solubilizing agent and/or emulsifier [e.g., ethyl alcohol, isopropyl alcohol, ethyl carbonate, ethyl acetate, benzyl alcohol, benzyl benzoate, propylene glycol, or 1,3-butylene glycol, an oil (*e.g*., cottonseed, groundnut, corn, germ, olive, castor, and sesame oil), glycerol, tetrahydrofuryl alcohol, a polyethylene glycol, a fatty acid ester of sorbitan, and mixtures thereof]. Besides inert diluents, the oral formulation can also include an adjuvant including, for example, a wetting agent, an emulsifying and suspending agent, a sweetening agent, a flavoring agent, a coloring agent, a perfuming agent, a preservative agent, and combinations thereof.

Suspensions, in addition to the active compounds, may contain suspending agents including, for example, an ethoxylated isostearyl alcohol, polyoxyethylene sorbitol, a sorbitan ester, microcrystalline cellulose, aluminum metahydroxide, bentonite, agar-agar, tragacanth, and combinations thereof.

Prevention of the action and/or growth of microorganisms may be ensured by the inclusion of various antibacterial and antifungal agents including, for example, paraben, chlorobutanol, and phenol sorbic acid.

In certain embodiments, it may be desirable to include an isotonic agent including, for example, a sugar or sodium chloride into the compositions. In addition, prolonged absorption of an injectable pharmaceutical form may be brought about by the inclusion of an agent that delay absorption including, for example, aluminum monostearate and gelatin.

It is understood that the dosage regimen will be determined by the attending physician considering various factors which modify the action of the one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure. The various factors include, but are not limited to, the patient's red blood cell count, hemoglobin level, the desired target red blood cell count, the patient's age, the patient's sex, the patient's diet, the severity of any disease that may be contributing to a depressed red blood cell level, the time of administration, and other clinical factors. The addition of other known active agents to the final composition may also affect the dosage. Progress can be monitored by periodic assessment of one or more of red blood cell levels, hemoglobin levels, reticulocyte levels, and other indicators of the hematopoietic process.

In certain embodiments, the present disclosure also provides gene therapy for the *in vivo* production of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure. Such therapy would achieve its therapeutic effect by introduction of the antagonist sequences into cells or tissues having one or more of the disorders as listed above. Delivery of the antagonist sequences can be achieved, for example, by using a recombinant expression vector such as a chimeric virus or a colloidal dispersion system. Preferred therapeutic delivery of one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure is the use of targeted liposomes.

Various viral vectors which can be utilized for gene therapy as taught herein include adenovirus, herpes virus, vaccinia, or an RNA virus (*e.g*., a retrovirus). The retroviral vector may be a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous sarcoma virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure.

Alternatively, tissue culture cells can be directly transfected with plasmids encoding the retroviral structural genes (gag, pol, and env), by conventional calcium phosphate transfection. These cells are then transfected with the vector plasmid containing the genes of interest. The resulting cells release the retroviral vector into the culture medium.

Another targeted delivery system for one or more GDF11 and/or activin B antagonist agents (optionally further antagonists of one or more of GDF8, activin A, activin C, activin E, BMP6, GDF15, Nodal, GDF3, BMP3, BMP3B, BMP9, and BMP10) of the disclosure is a colloidal dispersion system. Colloidal dispersion systems include, for example, macromolecule complexes, nanocapsules, microspheres, beads, and lipid-based systems including oil-in-water emulsions, micelles, mixed micelles, and liposomes. In certain embodiments, the preferred colloidal system of this disclosure is a liposome. Liposomes are artificial membrane vesicles which are useful as delivery vehicles *in vitro* and *in vivo.* RNA, DNA, and intact virions can be encapsulated within the aqueous interior and be delivered to cells in a biologically active form [see, *e.g.,* Fraley, et al. (1981) Trends Biochem. Sci., 6:77]. Methods for efficient gene transfer using a liposome vehicle are known in the art [see, *e.g.,* Mannino, et al. (1988) Biotechniques, 6:682, 1988].

The composition of the liposome is usually a combination of phospholipids, which may include a steroid (e.g. cholesterol). The physical characteristics of liposomes depend on pH, ionic strength, and the presence of divalent cations. Other phospholipids or other lipids may also be used including, for example, a phosphatidyl compound (*e.g.*, phosphatidylglycerol, phosphatidylcholine, phosphatidylserine, phosphatidylethanolamine, a sphingolipid, a cerebroside, and a ganglioside), egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

### EXEMPLIFICATION

The invention now being generally described, it will be more readily understood by reference to the following examples, which are included merely for purposes of illustration of certain embodiments and embodiments of the present invention, and are not intended to limit the invention.

### Example 1: Characterization of Ligand Binding Specificity for ActRIIB(L79D 20-134)-Fc and ActRIIB(L79D 25-131)-Fc

It has been previously reported that a variant ActRIIB-Fc fusion protein comprising amino acids 20-134 of instant SEQ ID NO:1 with an acidic amino acid at position 79 with respect to SEQ ID NO:1 [referenced herein as the"ActRIIB(L79D 20-134)-Fc" fusion protein, construct, variant, *etc*.; see SEQ ID NO:23 of the present disclosure] is characterized by unique biological properties *in vitro* and *in vivo* (see, *e.g.,* U.S. Patent No. 8,058,229). In comparison to a corresponding sample of an unmodified fusion protein (an ActRIIB(20-134)-Fc fusion protein), the ActRIIB(L79D 20-134)-Fc variant is characterized, in part, by substantial loss of binding affinity for activin A, and therefore significantly diminished capacity to antagonize activin A activity, but retains near wild-type levels of binding and inhibition of GDF11. In *vivo,* the ActRIIB(L79D 20-134)-Fc variant was found to be significantly more potent in the capacity to increase red blood cell levels in comparison to the unmodified ActRIIB(20-134)-Fc fusion protein. These data therefore indicate the observed biological activity is not dependent on activin A inhibition.

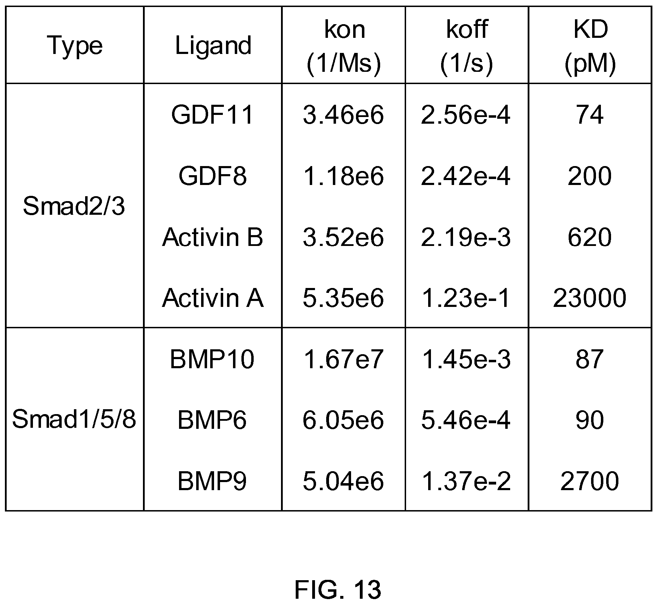

Similar results were previously reported for the double-truncated variant ActRIIB(L79D 25-131)-Fc (see, *e.g.,* SEQ ID NO:49 of the present disclosure as well as U.S. Patent No. 8,058,229), and the ligand binding profile for ActRIIB(L79D 25-131)-Fc was further characterized with respect to various ActRII ligands (*e.g*., GDF11, GDF8, activin A, activin B, BMP10, BMP6, and BMP9) by surface plasmon resonance. The results are depicted in Figure 13.

Upon consideration of this and other data, Applicants have determined that the ActRII ligands that should be inhibited to promote increased red blood cell levels are GDF11 and activin B. This data further suggests that it is also permissible to antagonize (inhibit) activin A, activin C, activin E, BMP6, and GDF8 in the context of a method for increasing red blood cell levels in a subject.

### Example 2: Bioassay for GDF-11, GDF-8, Activin B, Activin C, and Activin E-Mediated Signaling

An A-204 reporter gene assay is used to evaluate the effects of an anti-GDF11/activin B bispecific antibody on signaling by GDF11, activin B, activin A and/or GDF8. This assay has been previously described in the art (see, *e.g.,* U.S. Patent Application No. 2013/0243743). In brief, the A-204 reporter gene assay uses a human rhabdomyosarcoma cell line, which has been derived from muscle, and the reporter vector pGL3(CAGA)12 as described in Dennler et al. (1998) EMBO 17: 3091-3100. The CAGA12 motif is present in TGF-β responsive genes *(e.g.,* PAI-1 gene), so this vector is of general use for factors signaling through Smad2 and 3 *(e.g.,* GDF11, activin B, activin A, and GDF8).

At day 1, A-204 cells are transferred into one or more 48-well plates. At day 2, the A-204 cells are transfected with 10 µg pGL3(CAGA)12 or pGL3(CAGA) 12(10 µg) + pRLCMV (1 µg) and Fugene. At day 3, ligand factors (*e.g*., GDF11, activin B, activin A), which are diluted into medium + 0.1% BSA, are added to the cells along with the anti-GDF11/activin B bispecific antibody. Typically, the anti-GDF11/activin B bispecific antibody needs to be preincubated with factors for 1 hr before adding to cells. Approximately six hour later, the cells are rinsed with PBS and lysed.

The cell lysate is then subjected to a luciferase assay to determine the extent of Smad2/3 activation. The extent of inhibition of the anti-GDFl 1/activin B bispecific antibody is determined relative to appropriate controls.

### Example 3: Treatment with an Anti-GDF11/Anti-Activin B Bispecific Antibody

Nineteen-week-old male C57BL/6NTac mice are randomly assigned to one of two groups. Mice are dosed with vehicle (10 mM Tris-buffered saline, TBS) or an anti-GDF11/anti-activin B bispecific antibody by subcutaneous injection twice per week for three weeks. Blood is collected at baseline and after three weeks of dosing. The blood samples will be analyzed for cell distribution using a hematology analyzer (*e.g*., HM2, Abaxis, Inc.). In particular, mice will be monitored for changes in red blood cell parameters including, for example, red blood cell count (RBC), hemoglobin (HGB), and hematocrit (HCT).

### Example 4: Conjoint Administration of an Anti-GDF11 Antibody and an Anti-Activin B Antibody

Nineteen-week-old male C57BL/6NTac mice are randomly assigned to one of four groups: mice are dosed with vehicle (10 mM TBS), an anti-GDFl 1 antibody, an anti-activin B antibody, or both an anti-GDFl 1 and an anti-activin B antibody. Blood is collected at baseline and after three weeks of dosing. The blood samples will be analyzed for cell distribution using a hematology analyzer (*e.g.*, HM2, Abaxis, Inc.). In particular, mice will be monitored for changes in red blood cell parameters including, for example, red blood cell count (RBC), hemoglobin (HGB), and hematocrit (HCT).

### Example 5: Effects of Anti-Activin B, Anti-GDF8, and Anti-GDF8/GDF11 Antibodies on Erythropoiesis

Three antibodies, an anti-activin B, an anti-GDF8 antibody, and a bispecific anti-GDF8/GDF11 antibody, were evaluated for their ability to stimulate erythropoiesis (*e.g*., increase red blood cell, hemoglobin, and hematocrit levels) in mice. C57BL6 mice (8-10 weeks old) were divided into one of five treatment groups: i) treatment with vehicle (TBS, twice weekly; subcutaneously), ii) treatment with an anti-activin B antibody (10 mg/kg; twice weekly; subcutaneously), iii) treatment with an anti-GDF8 antibody (10 mg/kg, twice weekly; subcutaneously), iv) treatment with a bispecific anti-GDF8/GDF1 1 antibody (10 mg/kg, twice weekly; subcutaneously), and v) treatment with a combination of the anti-activin B antibody (10 mg/kg, twice weekly; subcutaneously) and the bispecific anti-GDF8/GDF1 1 antibody (10 mg/kg, twice weekly; subcutaneously). After two weeks of treatment, mice were euthanized and complete blood count parameters were measured.

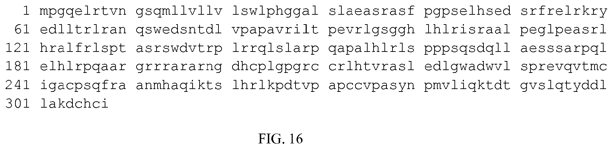

Compared to vehicle treated (control) subjects, there was a slight increase in red blood cell levels in mice treated with the anti-activin B antibody alone and the anti-GDF8 antibody alone. See Figure 16. A more substantial effect on red blood cell levels was observed in mice treated with the bispecific anti-GDF8/GDF1 1 antibody. See Figure 16. However, the greatest effect on erythropoiesis was observed in mice treated with a combination of the anti-activin B antibody and the bispecific anti-GDF8/GDF11 antibody, *e.g.,* the combination therapy significantly increased red blood cell levels (8.8%) compared to control subjects. See Figure 16. In addition, it was observed that combination treatment with the anti-activin B antibody and the bispecific anti-GDF8/GDF11 antibody resulted in increased levels of hemoglobin (9.6%) and hematocrit (9.1%) compared to control subjects.

Accordingly, among the ligands examined, there is a clear trend toward increased blood cell, hematocrit, and hemoglobin levels as additional ActRII ligand antagonists (inhibitors) are administered to the animals. Interestingly, inhibition of a single ActRII ligand did not have a significant effect on red blood cells levels. However, it appears that inhibition of at least two ActRII ligands can stimulate erythropoiesis and an even greater effect on red blood cell levels, as well as hemoglobin and hematocrit levels, is observed when three ActRII ligands are inhibited. Taken together, these data demonstrate that multiple ActRII ligands contribute erythropoiesis and further indicate that inhibiting just one of these ligands may not be sufficient to achieve significant increases in hematocrit, hemoglobin, and/or red blood cell levels. Therefore, from these experiments, it appears that an effective strategy for promoting erythropoiesis in a subject is to target multiple (*i.e.*, at least two) ActRII ligands.

## Claims

1. A composition for use in treating or preventing an anemia in a subject, wherein the composition comprises an effective amount of a combination of agents that inhibit activin B and GDF11; wherein the combination comprises one antibody or antigen binding fragment thereof that binds to GDF11, and, one antibody or antigen binding fragment thereof that binds to activin B.

2. The composition for use according to claim 1, wherein the combination of agents does not inhibit and/or bind to activin A.

3. The composition for use according to claim 1 or 2, wherein one or more of the agents further inhibits one or more of GDF8, activin C, activin E, GDF15, Nodal, BMP3, BMP3B, BMP9, and BMP10.

4. A composition for use in treating or preventing an anemia in a subject, wherein the composition comprises an effective amount of the composition of any one of claims 1-3.

5. A composition for use in treating or preventing an anemia in a subject as claimed in any one of claims 1-3, wherein the subject has myelodyspastic syndrome.

6. A composition for use in treating or preventing an anemia in a subject as claimed in any one of claims 1-3, wherein the subject has thalassemia.

7. A composition for use in treating or preventing an anemia in a subject as claimed in any one of claims 1-3, wherein the subject has sickle cell anemia.

8. The composition for use in treating or preventing an anemia in a subject as claimed in any one of claims 1-3, wherein the subject has beta-thalassemia.

## Patentansprüche

1. Zusammensetzung zur Verwendung bei der Behandlung oder Vorbeugung einer Anämie bei einem Subjekt, wobei die Zusammensetzung eine wirksame Menge einer Kombination von Mitteln umfasst, die Activin B und GDF11 hemmen; wobei die Kombination einen Antikörper oder ein Antigenbindungsfragment davon, der/das an GDF11 bindet, und einen Antikörper oder ein Antigenbindungsfragment davon, der/das an Activin B bindet, umfasst.

2. Zusammensetzung zur Verwendung nach Anspruch 1, wobei die Kombination von Mitteln Activin A nicht hemmt und/oder nicht daran bindet.

3. Zusammensetzung zur Verwendung nach den Ansprüchen 1 oder 2, wobei eines oder mehrere der Mittel ferner eines oder mehrere von GDF8, Activin C, Activin E, GDF15, Nodal, BMP3, BMP3B, BMP9 und BMP10 hemmt bzw. hemmen.

4. Zusammensetzung zur Verwendung bei der Behandlung oder Vorbeugung einer Anämie bei einem Subjekt, wobei die Zusammensetzung eine wirksame Menge der Zusammensetzung nach einem der Ansprüche 1-3 umfasst.

5. Zusammensetzung zur Verwendung bei der Behandlung oder Vorbeugung einer Anämie bei einem Subjekt nach einem der Ansprüche 1-3, wobei das Subjekt am myelodysplastischen Syndrom leidet.

6. Zusammensetzung zur Verwendung bei der Behandlung oder Vorbeugung einer Anämie bei einem Subjekt nach einem der Ansprüche 1-3, wobei das Subjekt an Thalassämie leidet.

7. Zusammensetzung zur Verwendung bei der Behandlung oder Vorbeugung einer Anämie bei einem Subjekt nach einem der Ansprüche 1-3, wobei das Subjekt an Sichelzellanämie leidet.

8. Zusammensetzung zur Verwendung bei der Behandlung oder Vorbeugung einer Anämie bei einem Subjekt nach einem der Ansprüche 1-3, wobei das Subjekt an beta-Thalassämie leidet.

## Revendications

1. Composition pour son utilisation dans le traitement ou la prévention d'une anémie chez un sujet, dans laquelle la composition comprend une quantité efficace d'une combinaison d'agents qui inhibent l'activine B et le GDF11 ; dans laquelle la combinaison comprend un anticorps ou un fragment de liaison d'antigène de celui-ci qui se lie au GDF11 et un anticorps ou fragment de liaison d'antigène de celui-ci qui se lie à l'activine B.

2. Composition pour son utilisation selon la revendication 1, dans laquelle la combinaison d'agents n'inhibe pas et/ou ne se lie pas à l'activine A.

3. Composition pour son utilisation selon les revendications 1 ou 2, dans laquelle un ou plusieurs des agents inhibent en outre un ou plusieurs parmi GDF8, activine C, activine E, GDF15, Nodal, BMP3, BMP3B, BMP9 et BMP10.

4. Composition pour son utilisation dans le traitement ou la prévention d'une anémie chez un sujet, dans laquelle la composition comprend une quantité efficace de la composition selon l'une quelconque des revendications 1 à 3.

5. Composition pour son utilisation dans le traitement ou la prévention d'une anémie chez un sujet selon l'une quelconque des revendications 1 à 3, dans laquelle le sujet a un syndrome myélodyspasique.

6. Composition pour son utilisation dans le traitement ou la prévention d'une anémie chez un sujet selon l'une quelconque des revendications 1 à 3, dans laquelle le sujet est atteint de thalassémie.

7. Composition pour son utilisation dans le traitement ou la prévention d'une anémie chez un sujet selon l'une quelconque des revendications 1 à 3, dans laquelle le sujet souffre d'anémie falciforme.

8. Composition pour son utilisation dans le traitement ou la prévention d'une anémie chez un sujet selon l'une quelconque des revendications 1 à 3, dans laquelle le sujet a une bêta-thalassémie.


### SEQUENCE LISTING

<110> ACCELERON PHARMA, INC.
<120> METHODS FOR INCREASING RED BLOOD CELL LEVELS AND TREATING INEFFECTIVE ERYTHROPOIESIS BY INHIBITING ACTIVIN B AND/OR GDF11
<130> P47879EP-PCT
<140> EP15765863.4
   <141> 2015-03-20
<150> US 62/021,923
   <151> 2014-07-08
<150> US 61/969,073
   <151> 2014-03-21
<160> 64
<170> PatentIn version 3.5
<210> 1
   <211> 512
   <212> PRT
   <213> Homo sapiens
<400> 1
   Met Thr Ala Pro Trp Val Ala Leu Ala Leu Leu Trp Gly Ser Leu Trp
   1               5                   10                  15
   Pro Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser Leu Ile Val Leu Leu Ala Phe Trp Met Tyr
   145                 150                 155                 160
   Arg His Arg Lys Pro Pro Tyr Gly His Val Asp Ile His Glu Asp Pro
                   165                 170                 175
   Gly Pro Pro Pro Pro Ser Pro Leu Val Gly Leu Lys Pro Leu Gln Leu
               180                 185                 190
   Leu Glu Ile Lys Ala Arg Gly Arg Phe Gly Cys Val Trp Lys Ala Gln
           195                 200                 205
   Leu Met Asn Asp Phe Val Ala Val Lys Ile Phe Pro Leu Gln Asp Lys
       210                 215                 220
   Gln Ser Trp Gln Ser Glu Arg Glu Ile Phe Ser Thr Pro Gly Met Lys
   225                 230                 235                 240
   His Glu Asn Leu Leu Gln Phe Ile Ala Ala Glu Lys Arg Gly Ser Asn
                   245                 250                 255
   Leu Glu Val Glu Leu Trp Leu Ile Thr Ala Phe His Asp Lys Gly Ser
               260                 265                 270
   Leu Thr Asp Tyr Leu Lys Gly Asn Ile Ile Thr Trp Asn Glu Leu Cys
           275                 280                 285
   His Val Ala Glu Thr Met Ser Arg Gly Leu Ser Tyr Leu His Glu Asp
       290                 295                 300
   Val Pro Trp Cys Arg Gly Glu Gly His Lys Pro Ser Ile Ala His Arg
   305                 310                 315                 320
   Asp Phe Lys Ser Lys Asn Val Leu Leu Lys Ser Asp Leu Thr Ala Val
                   325                 330                 335
   Leu Ala Asp Phe Gly Leu Ala Val Arg Phe Glu Pro Gly Lys Pro Pro
               340                 345                 350
   Gly Asp Thr His Gly Gln Val Gly Thr Arg Arg Tyr Met Ala Pro Glu
           355                 360                 365
   Val Leu Glu Gly Ala Ile Asn Phe Gln Arg Asp Ala Phe Leu Arg Ile
       370                 375                 380
   Asp Met Tyr Ala Met Gly Leu Val Leu Trp Glu Leu Val Ser Arg Cys
   385                 390                 395                 400
   Lys Ala Ala Asp Gly Pro Val Asp Glu Tyr Met Leu Pro Phe Glu Glu
                   405                 410                 415
   Glu Ile Gly Gln His Pro Ser Leu Glu Glu Leu Gln Glu Val Val Val
               420                 425                 430
   His Lys Lys Met Arg Pro Thr Ile Lys Asp His Trp Leu Lys His Pro
           435                 440                 445
   Gly Leu Ala Gln Leu Cys Val Thr Ile Glu Glu Cys Trp Asp His Asp
       450                 455                 460
   Ala Glu Ala Arg Leu Ser Ala Gly Cys Val Glu Glu Arg Val Ser Leu
   465                 470                 475                 480
   Ile Arg Arg Ser Val Asn Gly Thr Thr Ser Asp Cys Leu Val Ser Leu
                   485                 490                 495
   Val Thr Ser Val Thr Asn Val Asp Leu Pro Pro Lys Glu Ser Ser Ile
               500                 505                 510
<210> 2
   <211> 512
   <212> PRT
   <213> Homo sapiens
<400> 2
   Met Thr Ala Pro Trp Val Ala Leu Ala Leu Leu Trp Gly Ser Leu Trp
   1               5                   10                  15
   Pro Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Ala
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser Leu Ile Val Leu Leu Ala Phe Trp Met Tyr
   145                 150                 155                 160
   Arg His Arg Lys Pro Pro Tyr Gly His Val Asp Ile His Glu Asp Pro
                   165                 170                 175
   Gly Pro Pro Pro Pro Ser Pro Leu Val Gly Leu Lys Pro Leu Gln Leu
               180                 185                 190
   Leu Glu Ile Lys Ala Arg Gly Arg Phe Gly Cys Val Trp Lys Ala Gln
           195                 200                 205
   Leu Met Asn Asp Phe Val Ala Val Lys Ile Phe Pro Leu Gln Asp Lys
       210                 215                 220
   Gln Ser Trp Gln Ser Glu Arg Glu Ile Phe Ser Thr Pro Gly Met Lys
   225                 230                 235                 240
   His Glu Asn Leu Leu Gln Phe Ile Ala Ala Glu Lys Arg Gly Ser Asn
                   245                 250                 255
   Leu Glu Val Glu Leu Trp Leu Ile Thr Ala Phe His Asp Lys Gly Ser
               260                 265                 270
   Leu Thr Asp Tyr Leu Lys Gly Asn Ile Ile Thr Trp Asn Glu Leu Cys
           275                 280                 285
   His Val Ala Glu Thr Met Ser Arg Gly Leu Ser Tyr Leu His Glu Asp
       290                 295                 300
   Val Pro Trp Cys Arg Gly Glu Gly His Lys Pro Ser Ile Ala His Arg
   305                 310                 315                 320
   Asp Phe Lys Ser Lys Asn Val Leu Leu Lys Ser Asp Leu Thr Ala Val
                   325                 330                 335
   Leu Ala Asp Phe Gly Leu Ala Val Arg Phe Glu Pro Gly Lys Pro Pro
               340                 345                 350
   Gly Asp Thr His Gly Gln Val Gly Thr Arg Arg Tyr Met Ala Pro Glu
           355                 360                 365
   Val Leu Glu Gly Ala Ile Asn Phe Gln Arg Asp Ala Phe Leu Arg Ile
   370 375 380
   Asp Met Tyr Ala Met Gly Leu Val Leu Trp Glu Leu Val Ser Arg Cys
   385 390 395 400
   Lys Ala Ala Asp Gly Pro Val Asp Glu Tyr Met Leu Pro Phe Glu Glu
   405 410 415
   Glu Ile Gly Gln His Pro Ser Leu Glu Glu Leu Gln Glu Val Val Val
   420 425 430
   His Lys Lys Met Arg Pro Thr Ile Lys Asp His Trp Leu Lys His Pro
   435 440 445
   Gly Leu Ala Gln Leu Cys Val Thr Ile Glu Glu Cys Trp Asp His Asp
   450 455 460
   Ala Glu Ala Arg Leu Ser Ala Gly Cys Val Glu Glu Arg Val Ser Leu
   465 470 475 480
   Ile Arg Arg Ser Val Asn Gly Thr Thr Ser Asp Cys Leu Val Ser Leu
   485 490 495
   Val Thr Ser Val Thr Asn Val Asp Leu Pro Pro Lys Glu Ser Ser Ile
   500 505 510
<210> 3
   <211> 115
   <212> PRT
   <213> Homo sapiens
<400> 3
   Gly Arg Gly Glu Ala Glu Thr Arg Glu Cys Ile Tyr Tyr Asn Ala Asn
   1 5 10 15
   Trp Glu Leu Glu Arg Thr Asn Gln Ser Gly Leu Glu Arg Cys Glu Gly
   20 25 30
   Glu Gln Asp Lys Arg Leu His Cys Tyr Ala Ser Trp Arg Asn Ser Ser
   35 40 45
   Gly Thr Ile Glu Leu Val Lys Lys Gly Cys Trp Leu Asp Asp Phe Asn
   50 55 60
   Cys Tyr Asp Arg Gln Glu Cys Val Ala Thr Glu Glu Asn Pro Gln Val
   65 70 75 80
   Tyr Phe Cys Cys Cys Glu Gly Asn Phe Cys Asn Glu Arg Phe Thr His
                   85                  90                  95
   Leu Pro Glu Ala Gly Gly Pro Glu Val Thr Tyr Glu Pro Pro Pro Thr
               100                 105                 110
   Ala Pro Thr
           115
<210> 4
   <211> 115
   <212> PRT
   <213> Homo sapiens
<400> 4
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Ala Asn Ser Ser
           35                  40                  45
   Gly Thr Ile Glu Leu Val Lys Lys Gly Cys Trp Leu Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
                   85                  90                  95
   Leu Pro Glu Ala Gly Gly Pro Glu Val Thr Tyr Glu Pro Pro Pro Thr
               100                 105                 110
   Ala Pro Thr
           115
<210> 5
   <211> 100
   <212> PRT
   <213> Homo sapiens
<400> 5
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Arg Asn Ser Ser
           35                  40                  45
   Gly Thr Ile Glu Leu Val Lys Lys Gly Cys Trp Leu Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
                   85                  90                  95
   Leu Pro Glu Ala
               100
<210> 6
   <211> 100
   <212> PRT
   <213> Homo sapiens
<400> 6
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Ala Asn Ser Ser
           35                  40                  45
   Gly Thr Ile Glu Leu Val Lys Lys Gly Cys Trp Leu Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
                   85                  90                  95
   Leu Pro Glu Ala
               100
<210> 7
   <211> 1539
   <212> DNA
   <213> Homo sapiens
<400> 7
   atgacggcgc cctgggtggc cctcgccctc ctctggggat cgctgtggcc cggctctggg      60
   cgtggggagg ctgagacacg ggagtgcatc tactacaacg ccaactggga gctggagcgc     120
   accaaccaga gcggcctgga gcgctgcgaa ggcgagcagg acaagcggct gcactgctac     180
   gcctcctggg ccaacagctc tggcaccatc gagctcgtga agaagggctg ctggctagat     240
   gacttcaact gctacgatag gcaggagtgt gtggccactg aggagaaccc ccaggtgtac     300
   ttctgctgct gtgaaggcaa cttctgcaac gagcgcttca ctcatttgcc agaggctggg     360
   ggcccggaag tcacgtacga gccacccccg acagccccca ccctgctcac ggtgctggcc     420
   tactcactgc tgcccatcgg gggcctttcc ctcatcgtcc tgctggcctt ttggatgtac     480
   cggcatcgca agccccccta cggtcatgtg gacatccatg aggaccctgg gcctccacca     540
   ccatcccctc tggtgggcct gaagccactg cagctgctgg agatcaaggc tcgggggcgc     600
   tttggctgtg tctggaaggc ccagctcatg aatgactttg tagctgtcaa gatcttccca     660
   ctccaggaca agcagtcgtg gcagagtgaa cgggagatct tcagcacacc tggcatgaag     720
   cacgagaacc tgctacagtt cattgctgcc gagaagcgag gctccaacct cgaagtagag     780
   ctgtggctca tcacggcctt ccatgacaag ggctccctca cggattacct caaggggaac     840
   atcatcacat ggaacgaact gtgtcatgta gcagagacga tgtcacgagg cctctcatac     900
   ctgcatgagg atgtgccctg gtgccgtggc gagggccaca agccgtctat tgcccacagg     960
   gactttaaaa gtaagaatgt attgctgaag agcgacctca cagccgtgct ggctgacttt    1020
   ggcttggctg ttcgatttga gccagggaaa cctccagggg acacccacgg acaggtaggc    1080
   acgagacggt acatggctcc tgaggtgctc gagggagcca tcaacttcca gagagatgcc    1140
   ttcctgcgca ttgacatgta tgccatgggg ttggtgctgt gggagcttgt gtctcgctgc    1200
   aaggctgcag acggacccgt ggatgagtac atgctgccct ttgaggaaga gattggccag    1260
   cacccttcgt tggaggagct gcaggaggtg gtggtgcaca agaagatgag gcccaccatt   1320
   aaagatcact ggttgaaaca cccgggcctg gcccagcttt gtgtgaccat cgaggagtgc    1380
   tgggaccatg atgcagaggc tcgcttgtcc gcgggctgtg tggaggagcg ggtgtccctg    1440
   attcggaggt cggtcaacgg cactacctcg gactgtctcg tttccctggt gacctctgtc    1500
   accaatgtgg acctgccccc taaagagtca agcatctaa                           1539
<210> 8
   <211> 345
   <212> DNA
   <213> Homo sapiens
<400> 8
   gggcgtgggg aggctgagac acgggagtgc atctactaca acgccaactg ggagctggag        60
   cgcaccaacc agagcggcct ggagcgctgc gaaggcgagc aggacaagcg gctgcactgc       120
   tacgcctcct gggccaacag ctctggcacc atcgagctcg tgaagaaggg ctgctggcta       180
   gatgacttca actgctacga taggcaggag tgtgtggcca ctgaggagaa cccccaggtg       240
   tacttctgct gctgtgaagg caacttctgc aacgagcgct tcactcattt gccagaggct       300
   gggggcccgg aagtcacgta cgagccaccc ccgacagccc ccacc                       345
<210> 9
   <211> 513
   <212> PRT
   <213> Homo sapiens
<400> 9
   Met Gly Ala Ala Ala Lys Leu Ala Phe Ala Val Phe Leu Ile Ser Cys
   1               5                   10                  15
   Ser Ser Gly Ala Ile Leu Gly Arg Ser Glu Thr Gln Glu Cys Leu Phe
               20                  25                  30
   Phe Asn Ala Asn Trp Glu Lys Asp Arg Thr Asn Gln Thr Gly Val Glu
           35                  40                  45
   Pro Cys Tyr Gly Asp Lys Asp Lys Arg Arg His Cys Phe Ala Thr Trp
       50                  55                  60
   Lys Asn Ile Ser Gly Ser Ile Glu Ile Val Lys Gln Gly Cys Trp Leu
   65                  70                  75                  80
   Asp Asp Ile Asn Cys Tyr Asp Arg Thr Asp Cys Val Glu Lys Lys Asp
                   85                  90                  95
   Ser Pro Glu Val Tyr Phe Cys Cys Cys Glu Gly Asn Met Cys Asn Glu
               100                 105                 110
   Lys Phe Ser Tyr Phe Pro Glu Met Glu Val Thr Gln Pro Thr Ser Asn
           115                 120                 125
   Pro Val Thr Pro Lys Pro Pro Tyr Tyr Asn Ile Leu Leu Tyr Ser Leu
       130                 135                 140
   Val Pro Leu Met Leu Ile Ala Gly Ile Val Ile Cys Ala Phe Trp Val
   145                 150                 155                 160
   Tyr Arg His His Lys Met Ala Tyr Pro Pro Val Leu Val Pro Thr Gln
                   165                 170                 175
   Asp Pro Gly Pro Pro Pro Pro Ser Pro Leu Leu Gly Leu Lys Pro Leu
               180                 185                 190
   Gln Leu Leu Glu Val Lys Ala Arg Gly Arg Phe Gly Cys Val Trp Lys
   195 200 205
   Ala Gln Leu Leu Asn Glu Tyr Val Ala Val Lys Ile Phe Pro Ile Gln
   210 215 220
   Asp Lys Gln Ser Trp Gln Asn Glu Tyr Glu Val Tyr Ser Leu Pro Gly
   225 230 235 240
   Met Lys His Glu Asn Ile Leu Gln Phe Ile Gly Ala Glu Lys Arg Gly
   245 250 255
   Thr Ser Val Asp Val Asp Leu Trp Leu Ile Thr Ala Phe His Glu Lys
   260 265 270
   Gly Ser Leu Ser Asp Phe Leu Lys Ala Asn Val Val Ser Trp Asn Glu
   275 280 285
   Leu Cys His Ile Ala Glu Thr Met Ala Arg Gly Leu Ala Tyr Leu His
   290 295 300
   Glu Asp Ile Pro Gly Leu Lys Asp Gly His Lys Pro Ala Ile Ser His
   305 310 315 320
   Arg Asp Ile Lys Ser Lys Asn Val Leu Leu Lys Asn Asn Leu Thr Ala
   325 330 335
   Cys Ile Ala Asp Phe Gly Leu Ala Leu Lys Phe Glu Ala Gly Lys Ser
   340 345 350
   Ala Gly Asp Thr His Gly Gln Val Gly Thr Arg Arg Tyr Met Ala Pro
   355 360 365
   Glu Val Leu Glu Gly Ala Ile Asn Phe Gln Arg Asp Ala Phe Leu Arg
   370 375 380
   Ile Asp Met Tyr Ala Met Gly Leu Val Leu Trp Glu Leu Ala Ser Arg
   385 390 395 400
   Cys Thr Ala Ala Asp Gly Pro Val Asp Glu Tyr Met Leu Pro Phe Glu
   405 410 415
   Glu Glu Ile Gly Gln His Pro Ser Leu Glu Asp Met Gln Glu Val Val
   420 425 430
   Val His Lys Lys Lys Arg Pro Val Leu Arg Asp Tyr Trp Gln Lys His
   435 440 445
   Ala Gly Met Ala Met Leu Cys Glu Thr Ile Glu Glu Cys Trp Asp His
       450                 455                 460
   Asp Ala Glu Ala Arg Leu Ser Ala Gly Cys Val Gly Glu Arg Ile Thr
   465                 470                 475                 480
   Gln Met Gln Arg Leu Thr Asn Ile Ile Thr Thr Glu Asp Ile Val Thr
                   485                 490                 495
   Val Val Thr Met Val Thr Asn Val Asp Phe Pro Pro Lys Glu Ser Ser
               500                 505                 510
   Leu
<210> 10
   <211> 115
   <212> PRT
   <213> Homo sapiens
<400> 10
   Ile Leu Gly Arg Ser Glu Thr Gln Glu Cys Leu Phe Phe Asn Ala Asn
   1               5                   10                  15
   Trp Glu Lys Asp Arg Thr Asn Gln Thr Gly Val Glu Pro Cys Tyr Gly
               20                  25                  30
   Asp Lys Asp Lys Arg Arg His Cys Phe Ala Thr Trp Lys Asn Ile Ser
           35                  40                  45
   Gly Ser Ile Glu Ile Val Lys Gln Gly Cys Trp Leu Asp Asp Ile Asn
       50                  55                  60
   Cys Tyr Asp Arg Thr Asp Cys Val Glu Lys Lys Asp Ser Pro Glu Val
   65                  70                  75                  80
   Tyr Phe Cys Cys Cys Glu Gly Asn Met Cys Asn Glu Lys Phe Ser Tyr
                   85                  90                  95
   Phe Pro Glu Met Glu Val Thr Gln Pro Thr Ser Asn Pro Val Thr Pro
               100                 105                 110
   Lys Pro Pro
           115
<210> 11
   <211> 100
   <212> PRT
   <213> Homo sapiens
<400> 11
   Ile Leu Gly Arg Ser Glu Thr Gln Glu Cys Leu Phe Phe Asn Ala Asn
   1               5                   10                  15
   Trp Glu Lys Asp Arg Thr Asn Gln Thr Gly Val Glu Pro Cys Tyr Gly
               20                  25                  30
   Asp Lys Asp Lys Arg Arg His Cys Phe Ala Thr Trp Lys Asn Ile Ser
           35                  40                  45
   Gly Ser Ile Glu Ile Val Lys Gln Gly Cys Trp Leu Asp Asp Ile Asn
       50                  55                  60
   Cys Tyr Asp Arg Thr Asp Cys Val Glu Lys Lys Asp Ser Pro Glu Val
   65                  70                  75                  80
   Tyr Phe Cys Cys Cys Glu Gly Asn Met Cys Asn Glu Lys Phe Ser Tyr
                   85                  90                  95
   Phe Pro Glu Met
               100
<210> 12
   <211> 1542
   <212> DNA
   <213> Homo sapiens
<400> 12
   atgggagctg ctgcaaagtt ggcgtttgcc gtctttctta tctcctgttc ttcaggtgct 60
   atacttggta gatcagaaac tcaggagtgt cttttcttta atgctaattg ggaaaaagac 120
   agaaccaatc aaactggtgt tgaaccgtgt tatggtgaca aagataaacg gcggcattgt 180
   tttgctacct ggaagaatat ttctggttcc attgaaatag tgaaacaagg ttgttggctg 240
   gatgatatca actgctatga caggactgat tgtgtagaaa aaaaagacag ccctgaagta 300
   tatttttgtt gctgtgaggg caatatgtgt aatgaaaagt tttcttattt tccagagatg 360
   gaagtcacac agcccacttc aaatccagtt acacctaagc caccctatta caacatcctg 420
   ctctattcct tggtgccact tatgttaatt gcggggattg tcatttgtgc attttgggtg 480
   tacaggcatc acaagatggc ctaccctcct gtacttgttc caactcaaga cccaggacca 540
   cccccacctt ctccattact agggttgaaa ccactgcagt tattagaagt gaaagcaagg 600
   ggaagatttg gttgtgtctg gaaagcccag ttgcttaacg aatatgtggc tgtcaaaata 660
   tttccaatac aggacaaaca gtcatggcaa aatgaatacg aagtctacag tttgcctgga 720
   atgaagcatg agaacatatt acagttcatt ggtgcagaaa aacgaggcac cagtgttgat 780
   gtggatcttt ggctgatcac agcatttcat gaaaagggtt cactatcaga ctttcttaag 840
   gctaatgtgg tctcttggaa tgaactgtgt catattgcag aaaccatggc tagaggattg 900
   gcatatttac atgaggatat acctggccta aaagatggcc acaaacctgc catatctcac 960
   agggacatca aaagtaaaaa tgtgctgttg aaaaacaacc tgacagcttg cattgctgac 1020
   tttgggttgg ccttaaaatt tgaggctggc aagtctgcag gcgataccca tggacaggtt 1080
   ggtacccgga ggtacatggc tccagaggta ttagagggtg ctataaactt ccaaagggat 1140
   gcatttttga ggatagatat gtatgccatg ggattagtcc tatgggaact ggcttctcgc 1200
   tgtactgctg cagatggacc tgtagatgaa tacatgttgc catttgagga ggaaattggc 1260
   cagcatccat ctcttgaaga catgcaggaa gttgttgtgc ataaaaaaaa gaggcctgtt 1320
   ttaagagatt attggcagaa acatgctgga atggcaatgc tctgtgaaac cattgaagaa 1380
   tgttgggatc acgacgcaga agccaggtta tcagctggat gtgtaggtga aagaattacc 1440
   cagatgcaga gactaacaaa tattattacc acagaggaca ttgtaacagt ggtcacaatg 1500
   gtgacaaatg ttgactttcc tcccaaagaa tctagtctat ga 1542
<210> 13
   <211> 345
   <212> DNA
   <213> Homo sapiens
<400> 13
   atacttggta gatcagaaac tcaggagtgt cttttcttta atgctaattg ggaaaaagac        60
   agaaccaatc aaactggtgt tgaaccgtgt tatggtgaca aagataaacg gcggcattgt       120
   tttgctacct ggaagaatat ttctggttcc attgaaatag tgaaacaagg ttgttggctg       180
   gatgatatca actgctatga caggactgat tgtgtagaaa aaaaagacag ccctgaagta       240
   tatttttgtt gctgtgaggg caatatgtgt aatgaaaagt tttcttattt tccagagatg       300
   gaagtcacac agcccacttc aaatccagtt acacctaagc caccc                       345
<210> 14
   <211> 21
   <212> PRT
   <213> Apis sp.
<400> 14
   Met Lys Phe Leu Val Asn Val Ala Leu Val Phe Met Val Val Tyr Ile
   1               5                   10                  15
   Ser Tyr Ile Tyr Ala
               20
<210> 15
   <211> 22
   <212> PRT
   <213> Unknown
<220>
   <223> Description of Unknown: TPA signal sequence peptide
<400> 15
   Met Asp Ala Met Lys Arg Gly Leu Cys Cys Val Leu Leu Leu Cys Gly
   1               5                   10                  15
   Ala Val Phe Val Ser Pro
               20
<210> 16
   <211> 225
   <212> PRT
   <213> Artificial Sequence
<220>
   <223> Description of Artificial Sequence: Synthetic polypeptide
<220>
   <221> VARIANT
   <222> (43)..(43)
   <223> Ala
<220>
   <221> VARIANT
   <222> (100)..(100)
   <223> Ala
<220>
   <221> VARIANT
   <222> (212)..(212)
   <223> Ala
<400> 16
   Thr His Thr Cys Pro Pro Cys Pro Ala Pro Glu Leu Leu Gly Gly Pro
   1               5                   10                  15
   Ser Val Phe Leu Phe Pro Pro Lys Pro Lys Asp Thr Leu Met Ile Ser
               20                  25                  30
   Arg Thr Pro Glu Val Thr Cys Val Val Val Asp Val Ser His Glu Asp
           35                  40                  45
   Pro Glu Val Lys Phe Asn Trp Tyr Val Asp Gly Val Glu Val His Asn
       50                  55                  60
   Ala Lys Thr Lys Pro Arg Glu Glu Gln Tyr Asn Ser Thr Tyr Arg Val
   65                  70                  75                  80
   Val Ser Val Leu Thr Val Leu His Gln Asp Trp Leu Asn Gly Lys Glu
                   85                  90                  95
   Tyr Lys Cys Lys Val Ser Asn Lys Ala Leu Pro Val Pro Ile Glu Lys
   100 105 110
   Thr Ile Ser Lys Ala Lys Gly Gln Pro Arg Glu Pro Gln Val Tyr Thr
   115 120 125
   Leu Pro Pro Ser Arg Glu Glu Met Thr Lys Asn Gln Val Ser Leu Thr
   130 135 140
   Cys Leu Val Lys Gly Phe Tyr Pro Ser Asp Ile Ala Val Glu Trp Glu
   145 150 155 160
   Ser Asn Gly Gln Pro Glu Asn Asn Tyr Lys Thr Thr Pro Pro Val Leu
   165 170 175
   Asp Ser Asp Gly Pro Phe Phe Leu Tyr Ser Lys Leu Thr Val Asp Lys
   180 185 190
   Ser Arg Trp Gln Gln Gly Asn Val Phe Ser Cys Ser Val Met His Glu
   195 200 205
   Ala Leu His Asn His Tyr Thr Gln Lys Ser Leu Ser Leu Ser Pro Gly
   210 215 220
   Lys
   225
<210> 17
   <211> 344
   <212> PRT
   <213> Artificial Sequence
<220>
   <223> Description of Artificial Sequence: Synthetic polypeptide
<400> 17
   Ser Gly Arg Gly Glu Ala Glu Thr Arg Glu Cys Ile Tyr Tyr Asn Ala
   1               5                   10                  15
   Asn Trp Glu Leu Glu Arg Thr Asn Gln Ser Gly Leu Glu Arg Cys Glu
               20                  25                  30
   Gly Glu Gln Asp Lys Arg Leu His Cys Tyr Ala Ser Trp Ala Asn Ser
           35                  40                  45
   Ser Gly Thr Ile Glu Leu Val Lys Lys Gly Cys Trp Leu Asp Asp Phe
       50                  55                  60
   Asn Cys Tyr Asp Arg Gln Glu Cys Val Ala Thr Glu Glu Asn Pro Gln
   65                  70                  75                  80
   Val Tyr Phe Cys Cys Cys Glu Gly Asn Phe Cys Asn Glu Arg Phe Thr
   85 90 95
   His Leu Pro Glu Ala Gly Gly Pro Glu Val Thr Tyr Glu Pro Pro Pro
   100 105 110
   Thr Ala Pro Thr Gly Gly Gly Thr His Thr Cys Pro Pro Cys Pro Ala
   115 120 125
   Pro Glu Leu Leu Gly Gly Pro Ser Val Phe Leu Phe Pro Pro Lys Pro
   130 135 140
   Lys Asp Thr Leu Met Ile Ser Arg Thr Pro Glu Val Thr Cys Val Val
   145 150 155 160
   Val Asp Val Ser His Glu Asp Pro Glu Val Lys Phe Asn Trp Tyr Val
   165 170 175
   Asp Gly Val Glu Val His Asn Ala Lys Thr Lys Pro Arg Glu Glu Gln
   180 185 190
   Tyr Asn Ser Thr Tyr Arg Val Val Ser Val Leu Thr Val Leu His Gln
   195 200 205
   Asp Trp Leu Asn Gly Lys Glu Tyr Lys Cys Lys Val Ser Asn Lys Ala
   210 215 220
   Leu Pro Val Pro Ile Glu Lys Thr Ile Ser Lys Ala Lys Gly Gln Pro
   225 230 235 240
   Arg Glu Pro Gln Val Tyr Thr Leu Pro Pro Ser Arg Glu Glu Met Thr
   245 250 255
   Lys Asn Gln Val Ser Leu Thr Cys Leu Val Lys Gly Phe Tyr Pro Ser
   260 265 270
   Asp Ile Ala Val Glu Trp Glu Ser Asn Gly Gln Pro Glu Asn Asn Tyr
   275 280 285
   Lys Thr Thr Pro Pro Val Leu Asp Ser Asp Gly Ser Phe Phe Leu Tyr
   290 295 300
   Ser Lys Leu Thr Val Asp Lys Ser Arg Trp Gln Gln Gly Asn Val Phe
   305 310 315 320
   Ser Cys Ser Val Met His Glu Ala Leu His Asn His Tyr Thr Gln Lys
   325 330 335
   Ser Leu Ser Leu Ser Pro Gly Lys
   340
<210> 18
   <211> 369
   <212> PRT
   <213> Artificial Sequence
<220>
   <223> Description of Artificial Sequence: Synthetic polypeptide
<400> 18
   Met Asp Ala Met Lys Arg Gly Leu Cys Cys Val Leu Leu Leu Cys Gly
   1               5                   10                  15
   Ala Val Phe Val Ser Pro Gly Ala Ala Ile Leu Gly Arg Ser Glu Thr
               20                  25                  30
   Gln Glu Cys Leu Phe Phe Asn Ala Asn Trp Glu Lys Asp Arg Thr Asn
           35                  40                  45
   Gln Thr Gly Val Glu Pro Cys Tyr Gly Asp Lys Asp Lys Arg Arg His
       50                  55                  60
   Cys Phe Ala Thr Trp Lys Asn Ile Ser Gly Ser Ile Glu Ile Val Lys
   65                  70                  75                  80
   Gln Gly Cys Trp Leu Asp Asp Ile Asn Cys Tyr Asp Arg Thr Asp Cys
                   85                  90                  95
   Val Glu Lys Lys Asp Ser Pro Glu Val Tyr Phe Cys Cys Cys Glu Gly
               100                 105                 110
   Asn Met Cys Asn Glu Lys Phe Ser Tyr Phe Pro Glu Met Glu Val Thr
           115                 120                 125
   Gln Pro Thr Ser Asn Pro Val Thr Pro Lys Pro Pro Thr Gly Gly Gly
       130                 135                 140
   Thr His Thr Cys Pro Pro Cys Pro Ala Pro Glu Leu Leu Gly Gly Pro
   145                 150                 155                 160
   Ser Val Phe Leu Phe Pro Pro Lys Pro Lys Asp Thr Leu Met Ile Ser
                   165                 170                 175
   Arg Thr Pro Glu Val Thr Cys Val Val Val Asp Val Ser His Glu Asp
               180                 185                 190
   Pro Glu Val Lys Phe Asn Trp Tyr Val Asp Gly Val Glu Val His Asn
           195                 200                 205
   Ala Lys Thr Lys Pro Arg Glu Glu Gln Tyr Asn Ser Thr Tyr Arg Val
       210                 215                 220
   Val Ser Val Leu Thr Val Leu His Gln Asp Trp Leu Asn Gly Lys Glu
   225                 230                 235                 240
   Tyr Lys Cys Lys Val Ser Asn Lys Ala Leu Pro Val Pro Ile Glu Lys
                   245                 250                 255
   Thr Ile Ser Lys Ala Lys Gly Gln Pro Arg Glu Pro Gln Val Tyr Thr
               260                 265                 270
   Leu Pro Pro Ser Arg Glu Glu Met Thr Lys Asn Gln Val Ser Leu Thr
           275                 280                 285
   Cys Leu Val Lys Gly Phe Tyr Pro Ser Asp Ile Ala Val Glu Trp Glu
       290                 295                 300
   Ser Asn Gly Gln Pro Glu Asn Asn Tyr Lys Thr Thr Pro Pro Val Leu
   305                 310                 315                 320
   Asp Ser Asp Gly Ser Phe Phe Leu Tyr Ser Lys Leu Thr Val Asp Lys
                   325                 330                 335
   Ser Arg Trp Gln Gln Gly Asn Val Phe Ser Cys Ser Val Met His Glu
               340                 345                 350
   Ala Leu His Asn His Tyr Thr Gln Lys Ser Leu Ser Leu Ser Pro Gly
           355                 360                 365
   Lys
<210> 19
   <211> 1114
   <212> DNA
   <213> Artificial Sequence
<220>
   <223> Description of Artificial Sequence: Synthetic polynucleotide
<400> 19
   atggatgcaa tgaagagagg gctctgctgt gtgctgctgc tgtgtggagc agtcttcgtt      60
   tcgcccggcg ccgctatact tggtagatca gaaactcagg agtgtctttt tttaatgcta     120
   attgggaaaa agacagaacc aatcaaactg gtgttgaacc gtgttatggt gacaaagata     180
   aacggcggca ttgttttgct acctggaaga atatttctgg ttccattgaa tagtgaaaca     240
   aggttgttgg ctggatgata tcaactgcta tgacaggact gattgtgtag aaaaaaaaga     300
   cagccctgaa gtatatttct gttgctgtga gggcaatatg tgtaatgaaa agttttctta     360
   ttttccggag atggaagtca cacagcccac ttcaaatcca gttacaccta agccacccac     420
   cggtggtgga actcacacat gcccaccgtg cccagcacct gaactcctgg ggggaccgtc     480
   agtcttcctc ttccccccaa aacccaagga caccctcatg atctcccgga cccctgaggt     540
   cacatgcgtg gtggtggacg tgagccacga agaccctgag gtcaagttca actggtacgt     600
   ggacggcgtg gaggtgcata atgccaagac aaagccgcgg gaggagcagt acaacagcac     660
   gtaccgtgtg gtcagcgtcc tcaccgtcct gcaccaggac tggctgaatg gcaaggagta     720
   caagtgcaag gtctccaaca aagccctccc agtccccatc gagaaaacca tctccaaagc     780
   caaagggcag ccccgagaac cacaggtgta caccctgccc ccatcccggg aggagatgac     840
   caagaaccag gtcagcctga cctgcctggt caaaggcttc tatcccagcg acatcgccgt     900
   ggagtgggag agcaatgggc agccggagaa caactacaag accacgcctc ccgtgctgga     960
   ctccgacggc tccttcttcc tctatagcaa gctcaccgtg gacaagagca ggtggcagca    1020
   ggggaacgtc ttctcatgct ccgtgatgca tgaggctctg cacaaccact acacgcagaa    1080
   gagcctctcc ctgtctccgg gtaaatgaga attc                                1114
<210> 20
   <211> 344
   <212> PRT
   <213> Artificial Sequence
<220>
   <223> Description of Artificial Sequence: Synthetic polypeptide
<400> 20
   Ile Leu Gly Arg Ser Glu Thr Gln Glu Cys Leu Phe Phe Asn Ala Asn
   1               5                   10                  15
   Trp Glu Lys Asp Arg Thr Asn Gln Thr Gly Val Glu Pro Cys Tyr Gly
               20                  25                  30
   Asp Lys Asp Lys Arg Arg His Cys Phe Ala Thr Trp Lys Asn Ile Ser
           35                  40                  45
   Gly Ser Ile Glu Ile Val Lys Gln Gly Cys Trp Leu Asp Asp Ile Asn
       50                  55                  60
   Cys Tyr Asp Arg Thr Asp Cys Val Glu Lys Lys Asp Ser Pro Glu Val
   65 70 75 80
   Tyr Phe Cys Cys Cys Glu Gly Asn Met Cys Asn Glu Lys Phe Ser Tyr
   85 90 95
   Phe Pro Glu Met Glu Val Thr Gln Pro Thr Ser Asn Pro Val Thr Pro
   100 105 110
   Lys Pro Pro Thr Gly Gly Gly Thr His Thr Cys Pro Pro Cys Pro Ala
   115 120 125
   Pro Glu Leu Leu Gly Gly Pro Ser Val Phe Leu Phe Pro Pro Lys Pro
   130 135 140
   Lys Asp Thr Leu Met Ile Ser Arg Thr Pro Glu Val Thr Cys Val Val
   145 150 155 160
   Val Asp Val Ser His Glu Asp Pro Glu Val Lys Phe Asn Trp Tyr Val
   165 170 175
   Asp Gly Val Glu Val His Asn Ala Lys Thr Lys Pro Arg Glu Glu Gln
   180 185 190
   Tyr Asn Ser Thr Tyr Arg Val Val Ser Val Leu Thr Val Leu His Gln
   195 200 205
   Asp Trp Leu Asn Gly Lys Glu Tyr Lys Cys Lys Val Ser Asn Lys Ala
   210 215 220
   Leu Pro Val Pro Ile Glu Lys Thr Ile Ser Lys Ala Lys Gly Gln Pro
   225 230 235 240
   Arg Glu Pro Gln Val Tyr Thr Leu Pro Pro Ser Arg Glu Glu Met Thr
   245 250 255
   Lys Asn Gln Val Ser Leu Thr Cys Leu Val Lys Gly Phe Tyr Pro Ser
   260 265 270
   Asp Ile Ala Val Glu Trp Glu Ser Asn Gly Gln Pro Glu Asn Asn Tyr
   275 280 285
   Lys Thr Thr Pro Pro Val Leu Asp Ser Asp Gly Ser Phe Phe Leu Tyr
   290 295 300
   Ser Lys Leu Thr Val Asp Lys Ser Arg Trp Gln Gln Gly Asn Val Phe
   305 310 315 320
   Ser Cys Ser Val Met His Glu Ala Leu His Asn His Tyr Thr Gln Lys
   325 330 335
   Ser Leu Ser Leu Ser Pro Gly Lys
   340
<210> 21
   <211> 368
   <212> PRT
   <213> Artificial Sequence
<220>
   <223> Description of Artificial Sequence: Synthetic polypeptide
<400> 21
   Met Asp Ala Met Lys Arg Gly Leu Cys Cys Val Leu Leu Leu Cys Gly
   1               5                   10                  15
   Ala Val Phe Val Ser Pro Gly Ala Ser Gly Arg Gly Glu Ala Glu Thr
               20                  25                  30
   Arg Glu Cys Ile Tyr Tyr Asn Ala Asn Trp Glu Leu Glu Arg Thr Asn
           35                  40                  45
   Gln Ser Gly Leu Glu Arg Cys Glu Gly Glu Gln Asp Lys Arg Leu His
       50                  55                  60
   Cys Tyr Ala Ser Trp Arg Asn Ser Ser Gly Thr Ile Glu Leu Val Lys
   65                  70                  75                  80
   Lys Gly Cys Trp Leu Asp Asp Phe Asn Cys Tyr Asp Arg Gln Glu Cys
                   85                  90                  95
   Val Ala Thr Glu Glu Asn Pro Gln Val Tyr Phe Cys Cys Cys Glu Gly
               100                 105                 110
   Asn Phe Cys Asn Glu Arg Phe Thr His Leu Pro Glu Ala Gly Gly Pro
           115                 120                 125
   Glu Val Thr Tyr Glu Pro Pro Pro Thr Ala Pro Thr Gly Gly Gly Thr
       130                 135                 140
   His Thr Cys Pro Pro Cys Pro Ala Pro Glu Leu Leu Gly Gly Pro Ser
   145                 150                 155                 160
   Val Phe Leu Phe Pro Pro Lys Pro Lys Asp Thr Leu Met Ile Ser Arg
                   165                 170                 175
   Thr Pro Glu Val Thr Cys Val Val Val Asp Val Ser His Glu Asp Pro
               180                 185                 190
   Glu Val Lys Phe Asn Trp Tyr Val Asp Gly Val Glu Val His Asn Ala
           195                 200                 205
   Lys Thr Lys Pro Arg Glu Glu Gln Tyr Asn Ser Thr Tyr Arg Val Val
       210                 215                 220
   Ser Val Leu Thr Val Leu His Gln Asp Trp Leu Asn Gly Lys Glu Tyr
   225                 230                 235                 240
   Lys Cys Lys Val Ser Asn Lys Ala Leu Pro Val Pro Ile Glu Lys Thr
                   245                 250                 255
   Ile Ser Lys Ala Lys Gly Gln Pro Arg Glu Pro Gln Val Tyr Thr Leu
               260                 265                 270
   Pro Pro Ser Arg Glu Glu Met Thr Lys Asn Gln Val Ser Leu Thr Cys
           275                 280                 285
   Leu Val Lys Gly Phe Tyr Pro Ser Asp Ile Ala Val Glu Trp Glu Ser
       290                 295                 300
   Asn Gly Gln Pro Glu Asn Asn Tyr Lys Thr Thr Pro Pro Val Leu Asp
   305                 310                 315                 320
   Ser Asp Gly Ser Phe Phe Leu Tyr Ser Lys Leu Thr Val Asp Lys Ser
                   325                 330                 335
   Arg Trp Gln Gln Gly Asn Val Phe Ser Cys Ser Val Met His Glu Ala
               340                 345                 350
   Leu His Asn His Tyr Thr Gln Lys Ser Leu Ser Leu Ser Pro Gly Lys
           355                 360                 365
<210> 22
   <211> 1107
   <212> DNA
   <213> Artificial Sequence
<220>
   <223> Description of Artificial Sequence: Synthetic polynucleotide
<400> 22
   atggatgcaa tgaagagagg gctctgctgt gtgctgctgc tgtgtggagc agtcttcgtt 60
   tcgcccggcg cctctgggcg tggggaggct gagacacggg agtgcatcta ctacaacgcc 120
   aactgggagc tggagcgcac caaccagagc ggcctggagc gctgcgaagg cgagcaggac 180
   aagcggctgc actgctacgc ctcctggcgc aacagctctg gcaccatcga gctcgtgaag 240
   aagggctgct ggctcgatga cttcaactgc tacgataggc aggagtgtgt ggccactgag 300
   gagaaccccc aggtgtactt ctgctgctgt gaaggcaact tctgcaacga gcgcttcact 360
   catttgccag aggctggggg cccggaagtc acgtacgagc cacccccgac agcccccacc 420
   ggtggtggaa ctcacacatg cccaccgtgc ccagcacctg aactcctggg gggaccgtca 480
   gtcttcctct tccccccaaa acccaaggac accctcatga tctcccggac ccctgaggtc 540
   acatgcgtgg tggtggacgt gagccacgaa gaccctgagg tcaagttcaa ctggtacgtg 600
   gacggcgtgg aggtgcataa tgccaagaca aagccgcggg aggagcagta caacagcacg 660
   taccgtgtgg tcagcgtcct caccgtcctg caccaggact ggctgaatgg caaggagtac 720
   aagtgcaagg tctccaacaa agccctccca gtccccatcg agaaaaccat ctccaaagcc 780
   aaagggcagc cccgagaacc acaggtgtac accctgcccc catcccggga ggagatgacc 840
   aagaaccagg tcagcctgac ctgcctggtc aaaggcttct atcccagcga catcgccgtg 900
   gagtgggaga gcaatgggca gccggagaac aactacaaga ccacgcctcc cgtgctggac 960
   tccgacggct ccttcttcct ctatagcaag ctcaccgtgg acaagagcag gtggcagcag 1020
   gggaacgtct tctcatgctc cgtgatgcat gaggctctgc acaaccacta cacgcagaag 1080
   agcctctccc tgtctccggg taaatga 1107
<210> 23
   <211> 343
   <212> PRT
   <213> Artificial Sequence
<220>
   <223> Description of Artificial Sequence: Synthetic polypeptide
<400> 23
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Arg Asn Ser Ser
           35                  40                  45
   Gly Thr Ile Glu Leu Val Lys Lys Gly Cys Trp Asp Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
   85 90 95
   Leu Pro Glu Ala Gly Gly Pro Glu Val Thr Tyr Glu Pro Pro Pro Thr
   100 105 110
   Ala Pro Thr Gly Gly Gly Thr His Thr Cys Pro Pro Cys Pro Ala Pro
   115 120 125
   Glu Leu Leu Gly Gly Pro Ser Val Phe Leu Phe Pro Pro Lys Pro Lys
   130 135 140
   Asp Thr Leu Met Ile Ser Arg Thr Pro Glu Val Thr Cys Val Val Val
   145 150 155 160
   Asp Val Ser His Glu Asp Pro Glu Val Lys Phe Asn Trp Tyr Val Asp
   165 170 175
   Gly Val Glu Val His Asn Ala Lys Thr Lys Pro Arg Glu Glu Gln Tyr
   180 185 190
   Asn Ser Thr Tyr Arg Val Val Ser Val Leu Thr Val Leu His Gln Asp
   195 200 205
   Trp Leu Asn Gly Lys Glu Tyr Lys Cys Lys Val Ser Asn Lys Ala Leu
   210 215 220
   Pro Val Pro Ile Glu Lys Thr Ile Ser Lys Ala Lys Gly Gln Pro Arg
   225 230 235 240
   Glu Pro Gln Val Tyr Thr Leu Pro Pro Ser Arg Glu Glu Met Thr Lys
   245 250 255
   Asn Gln Val Ser Leu Thr Cys Leu Val Lys Gly Phe Tyr Pro Ser Asp
   260 265 270
   Ile Ala Val Glu Trp Glu Ser Asn Gly Gln Pro Glu Asn Asn Tyr Lys
   275 280 285
   Thr Thr Pro Pro Val Leu Asp Ser Asp Gly Ser Phe Phe Leu Tyr Ser
   290 295 300
   Lys Leu Thr Val Asp Lys Ser Arg Trp Gln Gln Gly Asn Val Phe Ser
   305 310 315 320
   Cys Ser Val Met His Glu Ala Leu His Asn His Tyr Thr Gln Lys Ser
   325 330 335
   Leu Ser Leu Ser Pro Gly Lys
   340
<210> 24
   <211> 4256
   <212> DNA
   <213> Homo sapiens
<400> 24
   tccccgcccc ccagtcctcc ctcccctccc ctccagcatg gtgctcgcgg cccgctgctg      60
   ctgggcttcc tgctcctcgc cctggagctg cggccccggg gggaggcggc cgagggcccc     120
   gcggcggcgg cggcggcggc ggcggcggcg gcagcggcgg gggtcggggg ggagcgctcc     180
   agccggccag ccccgtccgt ggcgcccgag ccggacggct gccccgtgtg cgtttggcgg     240
   cagcacagcc gcgagctgcg cctagagagc atcaagtcgc agatcttgag caaactgcgg     300
   ctcaaggagg cgcccaacat cagccgcgag gtggtgaagc agctgctgcc caaggcgccg     360
   ccgctgcagc agatcctgga cctacacgac ttccagggcg acgcgctgca gcccgaggac     420
   ttcctggagg aggacgagta ccacgccacc accgagaccg tcattagcat ggcccaggag     480
   acggacccag cagtacagac agatggcagc cctctctgct gccattttca cttcagcccc     540
   aaggtgatgt tcacaaaggt actgaaggcc cagctgtggg tgtacctacg gcctgtaccc     600
   cgcccagcca cagtctacct gcagatcttg cgactaaaac ccctaactgg ggaagggacc     660
   gcagggggag ggggcggagg ccggcgtcac atccgtatcc gctcactgaa gattgagctg     720
   cactcacgct caggccattg gcagagcatc gacttcaagc aagtgctaca cagctggttc     780
   cgccagccac agagcaactg gggcatcgag atcaacgcct ttgatcccag tggcacagac     840
   ctggctgtca cctccctggg gccgggagcc gaggggctgc atccattcat ggagcttcga     900
   gtcctagaga acacaaaacg ttcccggcgg aacctgggtc tggactgcga cgagcactca     960
   agcgagtccc gctgctgccg atatcccctc acagtggact ttgaggcttt cggctgggac    1020
   tggatcatcg cacctaagcg ctacaaggcc aactactgct ccggccagtg cgagtacatg    1080
   ttcatgcaaa aatatccgca tacccatttg gtgcagcagg ccaatccaag aggctctgct    1140
   gggccctgtt gtacccccac caagatgtcc ccaatcaaca tgctctactt caatgacaag    1200
   cagcagatta tctacggcaa gatccctggc atggtggtgg atcgctgtgg ctgctcttaa    1260
   ggtgggggat agaggatgcc tcccccacag accctacccc aagaccccta gccctgcccc    1320
   catcccccca agccctagag ctccctccac tcttcccgcg aacatcacac cgttccccga    1380
   ccaagccgtg tgcaatacaa cagagggagg caggtgggaa ttgagggtga ggggtttggg    1440
   ggaaagggga agcaggggca tagtcagggt ggggagtgtt tgaagtttgc agatgagaag    1500
   gtttgacaaa aagacagaga gatgtagaga cagtgataga gacagaggaa caaaaagagc    1560
   agcagtgaga aggcaaagag agaggcagaa gagacagacg aggcagagac aaaacactga 1620
   gaaagagact gaaatggagt aataaatgaa agccccacac caagcctcct ttcttccact 1680
   ggcaaggtga ggggcttggt atagtttggg gagatcccct gactattcag taggagaaga 1740
   aatcaaaaat ccattctttt ctccttctct ccctccaaca gtggccaggg gaaggggaag 1800
   tgagggcagg ggcaaaaaga tttgggaatt tttatttatt tatttattgt gacttttcat 1860
   ttttttggta tttggcttta ctggaatagg agggcccctg cccactgtgc cccgtttatc 1920
   ccttattccc caaaccctgc tctccccaac acctactcac ttaagcactt gtataaagcc 1980
   tccagggttg ggaatgggag taaagggcaa gagggcggac acatgaagtt tagtttctaa 2040
   cccatcatca ccctaactca accttttctg agccaaatgg cttgaattga agccagttgt 2100
   catggaaata gtaagaggtt agggtttaag agctggggat gcgggggtgg gagagagaac 2160
   cctcaacatc caggatctat ataatgagag ctactttaaa ccctcaggtc caccctcatg 2220
   atgctgagtt atttagccag agggtgcagc ctgcttatgc ccaaattccc tcagccaaga 2280
   gagagaccaa agagcctctg gaatggccct gctcccagcc tctatcttca ggtcaattag 2340
   agagagtata gagaccccag agtcccctgg gtctggaaag cgttaggaga ggtcaagaaa 2400
   ggagcagtaa ggaggctgaa ggttacaggg catttgaatc caaatcactg ctctgggcta 2460
   gggaatagag ccagcagacc aaggtgggaa ggattctgga agggggacat tttagtctcc 2520
   taaccccaaa gctcagggtg gaagagggga gaacaaggaa gcagagtgta taattatttt 2580
   ttccttttat ttttggaatc taacagtacc tggcagcagg gaggggaaag tacagtgggg 2640
   aaaagcatct gacaaggcca gttagaacag aggatgggaa ggatggagac tcccgggctt 2700
   ggaaggctag gaagcaggca gagactggtt gccatttcaa gtcactagct aggcccattc 2760
   attcctccca caaccctgac ccattctcct ctggactcac tgtgcctcag tttcttcccc 2820
   tcaatggaat gagaaatgac agcacccgcc acagccaaga gatgaattct gagcacttac 2880
   cacgggcact ttatggacat aaaatacctc tcgctgtggg acagataacc agggcaccag 2940
   agtagtggtg aagagatgtg aggcttaaga ggagtcacag gcttcagagt acaagttccc 3000
   ctctgcctcc cagctggaca gtgcctagaa gccaaggagt tgagaatctc ctgatccaca 3060
   ccctatcctt acttcaccac caggcctctt ggctccaggc aagagcttag aggatgtcag 3120
   gagaggtggg ggtaagaatc ttcagcaaaa ctgtcactct aagtagagcc agcagttacg 3180
   ggtctgataa aaacagtact gaactaaagt aaagcccaag ctggtgagca aactggatgg 3240
   ctcattcttc ccaagagcat gactctcccc cttggccagt tggtggaagg ggcaaaggta 3300
   tgtgaccacc cttgagaagg tgatgttggt gagctttaac atcttattcc tattcttata 3360
   gtgagaaagt gaaacaagat ctttcagtag aggaatgggc agggctgtag gctcttcagc 3420
   ttgccttcac ccatatagca gctatgctaa ccccaagcct ctctggccct gttcttcatc 3480
   cttccttctg ccccaatcct gaaggacaag acacacccgg ccatcaaacc actcacattt      3540
   ccttggtgga aggaaaggaa cagagaagtg aagaacagat acctccctcc aaggtcaaat      3600
   gcctcgtgat cttggcagag tagggattgg gcaataagca tcaggtatct tccctctaca      3660
   gattctagag agctggggca ttaaatatgg gggacactta gaatacagct ccttaaatac      3720
   caccaaataa agacctttgt gtgtgtgtgg tgggtggggg gggggcaggg gtctttctct      3780
   tatgaacata aatctgtgag ctgaagtctc attcccctgt tcctccctac ccccaaagag      3840
   gcacagagtg aagggacttg gggggcacag ctcagcaacc cagtgggagt tagcaccccc      3900
   tcccacctta tgatgtgtgt ggacctggcc agtgcccctc tgaacatatc attattagtg      3960
   taattatcat ttattttgtg tatttgtcac attgtgtgca tgacagcctt tgttaagggt      4020
   gtctgaggag tatggagctg acaggggcat tggaatgcca ggaaagaact tcttcaactg      4080
   agatcaaggc ttcctggagg gaaccactgc aaaaaggcca tcaggcagtt ttcaagttat      4140
   gtgacagagg gcaaagacgg ccatagggtg ctctgagttt tgggatggtc acatgacaca      4200
   atccagcact tgaacctgaa aaaaaaaata aaagcggtca aagagtttag aattca          4256
<210> 25
   <211> 406
   <212> PRT
   <213> Homo sapiens
<400> 25
   Met Val Leu Ala Ala Pro Leu Leu Leu Gly Phe Leu Leu Leu Ala Leu
   1               5                   10                  15
   Glu Leu Arg Pro Arg Gly Glu Ala Ala Glu Gly Pro Ala Ala Ala Ala
               20                  25                  30
   Ala Ala Ala Ala Ala Ala Ala Ala Ala Gly Val Gly Gly Glu Arg Ser
           35                  40                  45
   Ser Arg Ala Pro Ser Val Ala Pro Glu Pro Asp Gly Cys Pro Val Cys
       50                  55                  60
   Val Trp Arg Gln His Ser Arg Glu Leu Arg Leu Glu Ser Ile Lys Ser
   65                  70                  75                  80
   Gln Ile Leu Ser Lys Leu Arg Leu Lys Glu Ala Pro Asn Ile Ser Arg
                   85                  90                  95
   Glu Val Val Lys Gln Leu Leu Pro Lys Ala Pro Pro Leu Gln Gln Ile
               100                 105                 110
   Leu Asp Leu His Asp Phe Gln Gly Asp Ala Leu Gln Pro Glu Asp Phe
   115 120 125
   Leu Glu Glu Asp Glu Tyr His Ala Thr Thr Glu Thr Val Ile Ser Met
   130 135 140
   Ala Gln Glu Thr Asp Pro Ala Val Gln Thr Asp Gly Ser Pro Leu Cys
   145 150 155 160
   Cys His Phe His Phe Ser Pro Lys Val Met Phe Thr Lys Val Leu Lys
   165 170 175
   Ala Gln Leu Trp Val Tyr Leu Arg Pro Val Pro Arg Pro Ala Thr Val
   180 185 190
   Tyr Leu Gln Ile Leu Arg Leu Lys Pro Leu Thr Gly Glu Gly Thr Ala
   195 200 205
   Gly Gly Gly Gly Gly Gly Arg Arg His Ile Arg Ile Arg Ser Leu Lys
   210 215 220
   Ile Glu Leu His Ser Arg Ser Gly His Trp Gln Ser Ile Asp Phe Lys
   225 230 235 240
   Gln Val Leu His Ser Trp Phe Arg Gln Pro Gln Ser Asn Trp Gly Ile
   245 250 255
   Glu Ile Asn Ala Phe Asp Pro Ser Gly Thr Asp Leu Ala Val Thr Ser
   260 265 270
   Leu Gly Pro Gly Ala Glu Gly Leu His Pro Phe Met Glu Leu Arg Val
   275 280 285
   Leu Glu Asn Thr Lys Arg Ser Arg Arg Asn Leu Gly Leu Asp Cys Asp
   290 295 300
   Glu His Ser Ser Glu Ser Arg Cys Cys Arg Tyr Pro Leu Thr Val Asp
   305 310 315 320
   Phe Glu Ala Phe Gly Trp Asp Trp Ile Ile Ala Pro Lys Arg Tyr Lys
   325 330 335
   Ala Asn Tyr Cys Ser Gly Gln Cys Glu Tyr Met Phe Met Gln Lys Tyr
   340 345 350
   Pro His Thr His Leu Val Gln Gln Ala Asn Pro Arg Gly Ser Ala Gly
   355 360 365
   Pro Cys Cys Thr Pro Thr Lys Met Ser Pro Ile Asn Met Leu Tyr Phe
       370                 375                 380
   Asn Asp Lys Gln Gln Ile Ile Tyr Gly Lys Ile Pro Gly Met Val Val
   385                 390                 395                 400
   Asp Arg Cys Gly Cys Ser
                   405
<210> 26
   <211> 3217
   <212> DNA
   <213> Homo sapiens
<400> 26
   actcggctcg cctcgcggcg ggcgccctcg tcgccagcgg cgcaccatgg acgggctgcc      60
   cggtcgggcg ctgggggccg cctgccttct gctgctggcg gccggctggc tggggcctga     120
   ggcctggggc tcacccacgc ccccgccgac gcctgccgcg ccgccgccac ccccgccacc       180
   cggatccccg ggtggctcgc aggacacctg tacgtcgtgc ggcggcttcc ggcggccaga     240
   ggagctcggc cgagtggacg gcgacttcct ggaggcggtg aagcggcaca tcttgagccg     300
   cctgcagatg cggggccggc ccaacatcac gcacgccgtg cctaaggccg ccatggtcac     360
   ggccctgcgc aagctgcacg cgggcaaggt gcgcgaggac ggccgcgtgg agatcccgca     420
   cctcgacggc cacgccagcc cgggcgccga cggccaggag cgcgtttccg aaatcatcag     480
   cttcgccgag acagatggcc tcgcctcctc ccgggtccgc ctatacttct tcatctccaa     540
   cgaaggcaac cagaacctgt ttgtggtcca ggccagcctg tggctttacc tgaaactcct     600
   gccctacgtc ctggagaagg gcagccggcg gaaggtgcgg gtcaaagtgt acttccagga     660
   gcagggccac ggtgacaggt ggaacatggt ggagaagagg gtggacctca agcgcagcgg     720
   ctggcatacc ttcccactca cggaggccat ccaggccttg tttgagcggg gcgagcggcg     780
   actcaaccta gacgtgcagt gtgacagctg ccaggagctg gccgtggtgc cggtgttcgt     840
   ggacccaggc gaagagtcgc accggccctt tgtggtggtg caggctcggc tgggcgacag     900
   caggcaccgc attcgcaagc gaggcctgga gtgcgatggc cggaccaacc tctgttgcag     960
   gcaacagttc ttcattgact tccgcctcat cggctggaac gactggatca tagcacccac    1020
   cggctactac gggaactact gtgagggcag ctgcccagcc tacctggcag gggtccccgg    1080
   ctctgcctcc tccttccaca cggctgtggt gaaccagtac cgcatgcggg gtctgaaccc    1140
   cggcacggtg aactcctgct gcattcccac caagctgagc accatgtcca tgctgtactt    1200
   cgatgatgag tacaacatcg tcaagcggga cgtgcccaac atgattgtgg aggagtgcgg    1260
   ctgcgcctga cagtgcaagg caggggcacg gtggtggggc acggagggca gtcccgggtg    1320
   ggcttcttcc agcccccgcg ggaacggggg tacacggtgg gctgagtaca gtcattctgt    1380
   tgggctgtgg agatagtgcc agggtgcggc ctgagatatt tttctacagc ttcatagagc 1440
   aaccagtcaa aaccagagcg agaaccctca actgacatga aatactttaa aatgcacacg 1500
   tagccacgca cagccagacg catcctgcca cccacacagc agcctccagg ataccagcaa 1560
   atggatgcgg tgacaaatgg cagcttagct acaaatgcct gtcagtcgga gagaatgggg 1620
   tgagcagcca ccattcccac cagctggccc ggccactctg aattgcgcct tccgagcaca 1680
   cataaaagca caaagacaga gacgcagaga gagagagaga gccacggaga ggaaaagcag 1740
   atgcaggggt ggggagcgca gctcggcgga ggctgcgtgt gccccgtggc ttttaccagg 1800
   cctgctctgc ctggctcgat gtctgcttct tccccagcct gggatccttc gtgcttcaag 1860
   gcctggggag cctgtccttc catgcccttg tcgagggaaa gagacccaga aaggacacaa 1920
   cccgtcagag acctgggagc aggggcaatg accgtttgac tgtttgtggc ttgggcctct 1980
   gacatgactt atgtgtgtgt gtgtttttgg ggtggggagg gagggagaga agagggggct 2040
   aaatttgatg ctttaactga tctccaacag ttgacaggtc atccttgcca gttgtataac 2100
   tgaaaaagga cttttctacc aggtatgacc ttttaagtga aaatctgaat tgttctaaat 2160
   ggaaagaaaa aaagttgcaa tctgtgccct tcattgggga cattcctcta ggactggttt 2220
   ggggacgggt gggaatgacc cctaggcaag gggatgagac cgcaggagga aatggcgggg 2280
   aggaggcatt cttgaactgc tgaggatggg gggtgtcccc tcagcggagg ccaagggagg 2340
   ggagcagcct agttggtctt ggagagatgg ggaaggcttt cagctgattt gcagaagttg 2400
   cccatgtggg ccccagccat cagggctggc cgtggacgtg gcccctgccc actcacctgc 2460
   ccgcctgccc gcccgcccgc atagcacttg cagacctgcc tgaacgcaca tgacatagca 2520
   cttgccgatc tgcgtgtgtc cagaagtggc ccttggccga gcgccgaact cgctcgccct 2580
   ctagatgtcc aagtgccacg tgaactatgc aatttaaagg gttgacccac actagacgaa 2640
   actggactcg tacgactctt tttatatttt ttatacttga aatgaaatcc tttgcttctt 2700
   ttttaagcga atgattgctt ttaatgtttg cactgattta gttgcatgat tagtcagaaa 2760
   ctgccatttg aaaaaaagtt atttttatag cagcaaaaaa aaaaaaaaaa gaatacagtt 2820
   aaatgtatta tacataattt tggaaccaaa gaggccaaca gatcagtttt aattttatta 2880
   gacggtgagg ccatctgaga tgaggtggac gttctgagca gtcccttgag ggcctgccaa 2940
   cgtttcaggg tatgaatgga ttttgtttat tcggtttgat gtgtcttttc catccttaca 3000
   cacccagaag gtagagtaaa aatgactatg atagaatgca ggtgtgtatc cttaaatcct 3060
   catctttatg tttatttaat aaagctcccc ttagattctg tttcataata atttaaaacc 3120
   aaacaatttt cccatagact tgctgttaaa gtattgtacg tttgtgtaca gtttaagaaa 3180
   ataaaagatt gagtgccacg ggaaaaaaaa aaaaaaa 3217
<210> 27
   <211> 406
   <212> PRT
   <213> Homo sapiens
<400> 27
   Met Asp Gly Leu Pro Gly Arg Ala Leu Gly Ala Ala Cys Leu Leu Leu
   1               5                   10                  15
   Leu Ala Ala Gly Trp Leu Gly Pro Glu Ala Trp Gly Ser Pro Thr Pro
               20                  25                  30
   Pro Pro Thr Pro Ala Ala Pro Pro Pro Pro Pro Pro Pro Gly Ser Pro
           35                  40                  45
   Gly Gly Gln Asp Thr Cys Thr Ser Cys Gly Gly Phe Arg Arg Pro Glu
       50                  55                  60
   Glu Leu Gly Arg Val Asp Gly Asp Phe Leu Glu Ala Val Lys Arg His
   65                  70                  75                  80
   Ile Leu Ser Arg Leu Gln Met Arg Gly Arg Pro Asn Ile Thr His Ala
                   85                  90                  95
   Val Pro Lys Ala Ala Met Val Thr Ala Leu Arg Lys Leu His Ala Gly
               100                 105                 110
   Lys Val Arg Glu Asp Gly Arg Val Glu Ile Pro His Leu Asp Gly His
           115                 120                 125
   Ala Ser Pro Gly Ala Asp Gly Gln Glu Arg Val Ser Glu Ile Ile Ser
       130                 135                 140
   Phe Ala Glu Thr Asp Gly Leu Ala Ser Ser Arg Val Arg Leu Tyr Phe
   145                 150                 155                 160
   Phe Ile Ser Asn Glu Gly Asn Gln Asn Leu Phe Val Val Gln Ala Ser
                   165                 170                 175
   Leu Trp Leu Tyr Leu Lys Leu Leu Pro Tyr Val Leu Glu Lys Gly Ser
               180                 185                 190
   Arg Arg Lys Val Arg Val Lys Val Tyr Phe Gln Glu Gln Gly His Gly
           195                 200                 205
   Asp Arg Trp Asn Met Val Glu Lys Arg Val Asp Leu Lys Arg Ser Gly
       210                 215                 220
   Trp His Thr Phe Pro Leu Thr Glu Ala Ile Gln Ala Leu Phe Glu Arg
   225                 230                 235                 240
   Gly Glu Arg Arg Leu Asn Leu Asp Val Gln Cys Asp Ser Cys Gln Glu
                   245                 250                 255
   Leu Ala Val Val Pro Val Phe Val Asp Pro Gly Glu Glu Ser His Arg
               260                 265                 270
   Pro Phe Val Val Val Gln Ala Arg Leu Gly Asp Ser Arg His Arg Ile
           275                 280                 285
   Arg Lys Arg Gly Leu Glu Cys Asp Gly Arg Thr Asn Leu Cys Cys Arg
       290                 295                 300
   Gln Gln Phe Phe Ile Asp Phe Arg Leu Ile Gly Trp Asn Asp Trp Ile
   305                 310                 315                 320
   Ile Ala Pro Thr Gly Tyr Tyr Gly Asn Tyr Cys Glu Gly Ser Cys Pro
                   325                 330                 335
   Ala Tyr Leu Ala Gly Val Pro Gly Ser Ala Ser Ser Phe His Thr Ala
               340                 345                 350
   Val Val Asn Gln Tyr Arg Met Arg Gly Leu Asn Pro Gly Thr Val Asn
           355                 360                 365
   Ser Cys Cys Ile Pro Thr Lys Leu Ser Thr Met Ser Met Leu Tyr Phe
       370                 375                 380
   Asp Asp Glu Tyr Asn Ile Val Lys Arg Asp Val Pro Asn Met Ile Val
   385                 390                 395                 400
   Glu Glu Cys Gly Cys Ala
                   405
<210> 28
   <211> 2453
   <212> DNA
   <213> Homo sapiens
<400> 28
   cagacatgag ctgtgagggt caagcacagc tatccatcag atgatctact ttcagccttc        60
   ctgagtccca gacaatagaa gacaggtggc tgtacccttg gccaagggta ggtgtggcag       120
   tggtgtctgc tgtcactgtg ccctcattgg cccccagcaa tcagactcaa cagacggagc       180
   aactgccatc cgaggctcct gaaccagggc cattcaccag gagcatgcgg ctccctgatg       240
   tccagctctg gctggtgctg ctgtgggcac tggtgcgagc acaggggaca gggtctgtgt       300
   gtccctcctg tgggggctcc aaactggcac cccaagcaga acgagctctg gtgctggagc 360
   tagccaagca gcaaatcctg gatgggttgc acctgaccag tcgtcccaga ataactcatc 420
   ctccacccca ggcagcgctg accagagccc tccggagact acagccaggg agtgtggctc 480
   cagggaatgg ggaggaggtc atcagctttg ctactgtcac agactccact tcagcctaca 540
   gctccctgct cacttttcac ctgtccactc ctcggtccca ccacctgtac catgcccgcc 600
   tgtggctgca cgtgctcccc acccttcctg gcactctttg cttgaggatc ttccgatggg 660
   gaccaaggag gaggcgccaa gggtcccgca ctctcctggc tgagcaccac atcaccaacc 720
   tgggctggca taccttaact ctgccctcta gtggcttgag ggtgagaagg tctggtgtcc 780
   tgaaactgca actagactgc agacccctag aaggcaacag cacagttact ggacaaccga 840
   ggcggctctt ggacacagca ggacaccagc agcccttcct agagcttaag atccgagcca 900
   atgagcctgg agcaggccgg gccaggagga ggacccccac ctgtgagcct gcgaccccct 960
   tatgttgcag gcgagaccat tacgtagact tccaggaact gggatggcgg gactggatac 1020
   tgcagcccga ggggtaccag ctgaattact gcagtgggca gtgccctccc cacctggctg 1080
   gcagcccagg cattgctgcc tctttccatt ctgccgtctt cagcctcctc aaagccaaca 1140
   atccttggcc tgccagtacc tcctgttgtg tccctactgc ccgaaggccc ctctctctcc 1200
   tctacctgga tcataatggc aatgtggtca agacggatgt gccagatatg gtggtggagg 1260
   cctgtggctg cagctagcaa gaggacctgg ggctttggag tgaagagacc aagatgaagt 1320
   ttcccaggca cagggcatct gtgactggag gcatcagatt cctgatccac accccaaccc 1380
   aacaaccacc tggcaatatg actcacttga cccctatggg acccaaatgg gcactttctt 1440
   gtctgagact ctggcttatt ccaggttggc tgatgtgttg ggagatgggt aaagcgtttc 1500
   ttctaaaggg gtctacccag aaagcatgat ttcctgccct aagtcctgtg agaagatgtc 1560
   agggactagg gagggaggga gggaaggcag agaaaaatta cttagcctct cccaagatga 1620
   gaaagtcctc aagtgagggg aggaggaagc agatagatgg tccagcaggc ttgaagcagg 1680
   gtaagcaggc tggcccaggg taagggctgt tgaggtacct taagggaagg tcaagaggga 1740
   gatgggcaag gcgctgaggg aggatgctta ggggaccccc agaaacagga gtcaggaaaa 1800
   tgaggcacta agcctaagaa gttccctggt tttcccaggg ggacaggacc cactgggaga 1860
   caagcattta tactttcttt cttctttttt atttttttga gatcgagtct cgctctgtca 1920
   ccaggctgga gtgcagtgac acgatcttgg ctcactgcaa cctccgtctc ctgggttcaa 1980
   gtgattcttc tgcctcagcc tcccgagcag ctgggattac aggcgcccac taatttttgt 2040
   attcttagta gaaacgaggt ttcaacatgt tggccaggat ggtctcaatc tcttgacctc 2100
   ttgatccacc cgacttggcc tcccgaagtg atgagattat aggcgtgagc caccgcgcct 2160
   ggcttatact ttcttaataa aaaggagaaa gaaaatcaac aaatgtgagt cataaagaag 2220
   ggttagggtg atggtccaga gcaacagttc ttcaagtgta ctctgtaggc ttctgggagg 2280
   tcccttttca ggggtgtcca caaagtcaaa gctattttca taataatact aacatgttat 2340
   ttgccttttg aattctcatt atcttaaaat tgtattgtgg agttttccag aggccgtgtg 2400
   acatgtgatt acatcatctt tctgacatca ttgttaaaaa aaaaaaaaaa aaa 2453
<210> 29
   <211> 350
   <212> PRT
   <213> Homo sapiens
<400> 29
   Met Arg Leu Pro Asp Val Gln Leu Trp Leu Val Leu Leu Trp Ala Leu
   1               5                   10                  15
   Val Arg Ala Gln Gly Thr Gly Ser Val Cys Pro Ser Cys Gly Gly Ser
               20                  25                  30
   Lys Leu Ala Pro Gln Ala Glu Arg Ala Leu Val Leu Glu Leu Ala Lys
           35                  40                  45
   Gln Gln Ile Leu Asp Gly Leu His Leu Thr Ser Arg Pro Arg Ile Thr
       50                  55                  60
   His Pro Pro Pro Gln Ala Ala Leu Thr Arg Ala Leu Arg Arg Leu Gln
   65                  70                  75                  80
   Pro Gly Ser Val Ala Pro Gly Asn Gly Glu Glu Val Ile Ser Phe Ala
                   85                  90                  95
   Thr Val Thr Asp Ser Thr Ser Ala Tyr Ser Ser Leu Leu Thr Phe His
               100                 105                 110
   Leu Ser Thr Pro Arg Ser His His Leu Tyr His Ala Arg Leu Trp Leu
           115                 120                 125
   His Val Leu Pro Thr Leu Pro Gly Thr Leu Cys Leu Arg Ile Phe Arg
       130                 135                 140
   Trp Gly Pro Arg Arg Arg Arg Gln Gly Ser Arg Thr Leu Leu Ala Glu
   145                 150                 155                 160
   His His Ile Thr Asn Leu Gly Trp His Thr Leu Thr Leu Pro Ser Ser
                   165                 170                 175
   Gly Leu Arg Gly Glu Lys Ser Gly Val Leu Lys Leu Gln Leu Asp Cys
               180                 185                 190
   Arg Pro Leu Glu Gly Asn Ser Thr Val Thr Gly Gln Pro Arg Arg Leu
           195                 200                 205
   Leu Asp Thr Ala Gly His Gln Gln Pro Phe Leu Glu Leu Lys Ile Arg
       210                 215                 220
   Ala Asn Glu Pro Gly Ala Gly Arg Ala Arg Arg Arg Thr Pro Thr Cys
   225                 230                 235                 240
   Glu Pro Ala Thr Pro Leu Cys Cys Arg Arg Asp His Tyr Val Asp Phe
                   245                 250                 255
   Gln Glu Leu Gly Trp Arg Asp Trp Ile Leu Gln Pro Glu Gly Tyr Gln
               260                 265                 270
   Leu Asn Tyr Cys Ser Gly Gln Cys Pro Pro His Leu Ala Gly Ser Pro
           275                 280                 285
   Gly Ile Ala Ala Ser Phe His Ser Ala Val Phe Ser Leu Leu Lys Ala
       290                 295                 300
   Asn Asn Pro Trp Pro Ala Ser Thr Ser Cys Cys Val Pro Thr Ala Arg
   305                 310                 315                 320
   Arg Pro Leu Ser Leu Leu Tyr Leu Asp His Asn Gly Asn Val Val Lys
                   325                 330                 335
   Thr Asp Val Pro Asp Met Val Val Glu Ala Cys Gly Cys Ser
               340                 345                 350
<210> 30
   <211> 3314
   <212> DNA
   <213> Homo sapiens
<400> 30
   agattctgcc aagttctacc tgtaactggc ttcattttca agtcagacgt ttggctgctg 60
   ctctgtcccc tgcaacaagg agccatgcca gctggacaca cacttcttcc agggcctctg 120
   gcagccagga cagagttgag accacagctg ttgagaccct gagccctgag tctgtattgc 180
   tcaagaaggg ccttccccag caatgacctc ctcattgctt ctggcctttc tcctcctggc 240
   tccaaccaca gtggccactc ccagagctgg cggtcagtgt ccagcatgtg gggggcccac 300
   cttggaactg gagagccagc gggagctgct tcttgatctg gccaagagaa gcatcttgga 360
   caagctgcac ctcacccagc gcccaacact gaaccgccct gtgtccagag ctgctttgag 420
   gactgcactg cagcacctcc acggggtccc acagggggca cttctagagg acaacaggga 480
   acaggaatgt gaaatcatca gctttgctga gacaggcctc tccaccatca accagactcg 540
   tcttgatttt cacttctcct ctgatagaac tgctggtgac agggaggtcc agcaggccag 600
   tctcatgttc tttgtgcagc tcccttccaa taccacttgg accttgaaag tgagagtcct 660
   tgtgctgggt ccacataata ccaacctcac cttggctact cagtacctgc tggaggtgga 720
   tgccagtggc tggcatcaac tccccctagg gcctgaagct caagctgcct gcagccaggg 780
   gcacctgacc ctggagctgg tacttgaagg ccaggtagcc cagagctcag tcatcctggg 840
   tggagctgcc cataggcctt ttgtggcagc ccgggtgaga gttgggggca aacaccagat 900
   tcaccgacga ggcatcgact gccaaggagg gtccaggatg tgctgtcgac aagagttttt 960
   tgtggacttc cgtgagattg gctggcacga ctggatcatc cagcctgagg gctacgccat 1020
   gaacttctgc atagggcagt gcccactaca catagcaggc atgcctggta ttgctgcctc 1080
   ctttcacact gcagtgctca atcttctcaa ggccaacaca gctgcaggca ccactggagg 1140
   gggctcatgc tgtgtaccca cggcccggcg ccccctgtct ctgctctatt atgacaggga 1200
   cagcaacatt gtcaagactg acatacctga catggtagta gaggcctgtg ggtgcagtta 1260
   gtctatgtgt ggtatgggca gcccaaggtt gcatgggaaa acacgcccct acagaagtgc 1320
   acttccttga gaggagggaa tgacctcatt ctctgtccag aatgtggact ccctcttcct 1380
   gagcatctta tggaaattac cccacctttg acttgaagaa accttcatct aaagcaagtc 1440
   actgtgccat cttcctgacc actaccctct ttcctagggc atagtccatc ccgctagtcc 1500
   atcccgctag ccccactcca gggactcaga cccatctcca accatgagca atgccatctg 1560
   gttcccaggc aaagacaccc ttagctcacc tttaatagac cccataaccc actatgcctt 1620
   cctgtccttt ctactcaatg gtccccactc caagatgagt tgacacaacc ccttccccca 1680
   atttttgtgg atctccagag aggcccttct ttggattcac caaagtttag atcactgctg 1740
   cccaaaatag aggcttacct acccccctct ttgttgtgag cccctgtcct tcttagttgt 1800
   ccaggtgaac tactaaagct ctctttgcat accttcatcc attttttgtc cttctctgcc 1860
   tttctctatg cccttaaggg ctgacttgcc tgagctctat cacctgagct cccctgccct 1920
   ctggcttcct gctgaggtca gggcatttct tatccctgtt ccctctctgt ctaggtgtca 1980
   tggttctgtg taactgtggc tattctgtgt ccctacacta cctggctacc cccttccatg 2040
   gccccagctc tgcctacatt ctgatataac tgcttcaaca ctagggggtc ctaaaggctt 2100
   tctatcttgc tagtccctgg ggcctcaaca tctcatactg gttcccttaa ctctgcctat 2160
   acctctgtaa ataattcctt cactaagttc tcttgatgaa gcaaaaacag acagctgaaa 2220
   agtcctctat ctcctacaag ggccctaact ggcaccccag atgacacaga gcctgcctgc 2280
   ttatgctgta gtctgcctac tctgctgtct cttcacatgg tctcctcaga actgaactat 2340
   tgtatccatc tcacacttta tgcctcttct ttcttaggca ccccgtccct ccatccttcc 2400
   agaaccatct ttgaggtctc atggctaata aaaacctagg ctttacctgt tccctctgta 2460
   atccctccaa aagatgagac agatctatgc ttggtcatcc agtaaactga ccagctgtgg 2520
   gcacgcaagt gtgggaggca gaggcatgct cagagctggc tgccaggacc tctgacttgc 2580
   cttcctttca cccaccccca gtgctccacc caggagtcct gcctggaagc tggaatgggc 2640
   aagggctgct ggagtgggac agggagaaga ggaaggcctg gatgaggaga gggtggcatt 2700
   tgctctgaga ctgggtcctt tttagacctt tgcccgtcct cccccacatc tcctcccttt 2760
   ggctggacag tcctgaacca tgaggtcgat aatgtctgca gcccaaggcc gagtttgcgc 2820
   aaaacccatg tgttctttgg taaacgtgat gtctgtgttt gctcagttta tgaccccctc 2880
   ctatgagggt aagaggtccc tgaaatagga accctagagg agaaagtctg aaaaggactg 2940
   cctgggggac tgtaaatctg agcttgaggg cttcctgagc aacccatgga agttatccca 3000
   cctttgactt gaggagacct tcatctaagg agaatctaag gaggccttct ggtgtctccc 3060
   ccacacatcc ccgaccccca gatctaacct ccttcccaat tacagcttag tctccagggc 3120
   taggactggg gtaaagcaaa gtgagtcatt cacctggggg ggctaaattt taagggggtg 3180
   gtgaacaatt tattaatcaa gataggactt taatgcaata ttattttaaa tcaaaattaa 3240
   tgcaaaaaat ccatgatgaa caaaatagcc tacttttaaa taaaaacagg atcagcatta 3300
   aaaaaaaaaa aaaa 3314
<210> 31
   <211> 351
   <212> PRT
   <213> Homo sapiens
<400> 31
   Met Thr Ser Ser Leu Leu Leu Ala Phe Leu Leu Leu Ala Pro Thr Thr
   1               5                   10                  15
   Val Ala Thr Pro Arg Ala Gly Gly Gln Cys Pro Ala Cys Gly Gly Pro
               20                  25                  30
   Thr Leu Glu Leu Glu Ser Gln Arg Glu Leu Leu Leu Asp Leu Ala Lys
           35                  40                  45
   Arg Ser Leu Asp Lys Leu His Leu Thr Gln Arg Pro Thr Leu Asn Arg
       50                  55                  60
   Pro Val Ser Arg Ala Ala Leu Arg Thr Ala Leu Gln His Leu His Gly
   65                  70                  75                  80
   Val Pro Gln Gly Ala Leu Leu Glu Asp Asn Arg Glu Gln Glu Cys Glu
                   85                  90                  95
   Ile Ile Ser Phe Ala Glu Thr Gly Leu Ser Thr Ile Asn Gln Thr Arg
               100                 105                 110
   Leu Asp Phe His Phe Ser Ser Asp Arg Thr Ala Gly Asp Arg Glu Val
           115                 120                 125
   Gln Gln Ala Ser Leu Met Phe Phe Val Gln Leu Pro Ser Asn Thr Thr
       130                 135                 140
   Trp Thr Leu Lys Val Arg Val Leu Val Leu Gly Pro His Asn Thr Asn
   145                 150                 155                 160
   Leu Thr Leu Ala Thr Gln Tyr Leu Leu Glu Val Asp Ala Ser Gly Trp
                   165                 170                 175
   His Gln Leu Pro Leu Gly Pro Glu Ala Gln Ala Ala Cys Ser Gln Gly
               180                 185                 190
   His Leu Thr Leu Glu Leu Val Leu Glu Gly Gln Val Ala Gln Ser Ser
           195                 200                 205
   Val Ile Leu Gly Gly Ala Ala His Arg Pro Phe Val Ala Ala Arg Val
       210                 215                 220
   Arg Val Gly Gly Lys His Gln Ile His Arg Arg Gly Ile Asp Cys Gln
   225                 230                 235                 240
   Gly Gly Ser Arg Met Cys Cys Arg Gln Glu Phe Phe Val Asp Phe Arg
                   245                 250                 255
   Glu Ile Gly Trp His Asp Trp Ile Ile Gln Pro Glu Gly Tyr Ala Met
               260                 265                 270
   Asn Phe Cys Ile Gly Gln Cys Pro Leu His Ile Ala Gly Met Pro Gly
           275                 280                 285
   Ile Ala Ala Ser Phe His Thr Ala Val Leu Asn Leu Leu Lys Ala Asn
       290                 295                 300
   Thr Ala Ala Gly Thr Thr Gly Gly Gly Ser Cys Cys Val Pro Thr Ala
   305                 310                 315                 320
   Arg Arg Pro Leu Ser Leu Leu Tyr Tyr Asp Arg Asp Ser Asn Ile Val
                   325                 330                 335
   Lys Thr Asp Ile Pro Asp Met Val Val Glu Ala Cys Gly Cys Ser
   340 345 350
<210> 32
   <211> 2823
   <212> DNA
   <213> Homo sapiens
<400> 32
   agattcactg gtgtggcaag ttgtctctca gactgtacat gcattaaaat tttgcttggc 60
   attactcaaa agcaaaagaa aagtaaaagg aagaaacaag aacaagaaaa aagattatat 120
   tgattttaaa atcatgcaaa aactgcaact ctgtgtttat atttacctgt ttatgctgat 180
   tgttgctggt ccagtggatc taaatgagaa cagtgagcaa aaagaaaatg tggaaaaaga 240
   ggggctgtgt aatgcatgta cttggagaca aaacactaaa tcttcaagaa tagaagccat 300
   taagatacaa atcctcagta aacttcgtct ggaaacagct cctaacatca gcaaagatgt 360
   tataagacaa cttttaccca aagctcctcc actccgggaa ctgattgatc agtatgatgt 420
   ccagagggat gacagcagcg atggctcttt ggaagatgac gattatcacg ctacaacgga 480
   aacaatcatt accatgccta cagagtctga ttttctaatg caagtggatg gaaaacccaa 540
   atgttgcttc tttaaattta gctctaaaat acaatacaat aaagtagtaa aggcccaact 600
   atggatatat ttgagacccg tcgagactcc tacaacagtg tttgtgcaaa tcctgagact 660
   catcaaacct atgaaagacg gtacaaggta tactggaatc cgatctctga aacttgacat 720
   gaacccaggc actggtattt ggcagagcat tgatgtgaag acagtgttgc aaaattggct 780
   caaacaacct gaatccaact taggcattga aataaaagct ttagatgaga atggtcatga 840
   tcttgctgta accttcccag gaccaggaga agatgggctg aatccgtttt tagaggtcaa 900
   ggtaacagac acaccaaaaa gatccagaag ggattttggt cttgactgtg atgagcactc 960
   aacagaatca cgatgctgtc gttaccctct aactgtggat tttgaagctt ttggatggga 1020
   ttggattatc gctcctaaaa gatataaggc caattactgc tctggagagt gtgaatttgt 1080
   atttttacaa aaatatcctc atactcatct ggtacaccaa gcaaacccca gaggttcagc 1140
   aggcccttgc tgtactccca caaagatgtc tccaattaat atgctatatt ttaatggcaa 1200
   agaacaaata atatatggga aaattccagc gatggtagta gaccgctgtg ggtgctcatg 1260
   agatttatat taagcgttca taacttccta aaacatggaa ggttttcccc tcaacaattt 1320
   tgaagctgtg aaattaagta ccacaggcta taggcctaga gtatgctaca gtcacttaag 1380
   cataagctac agtatgtaaa ctaaaagggg gaatatatgc aatggttggc atttaaccat 1440
   ccaaacaaat catacaagaa agttttatga tttccagagt ttttgagcta gaaggagatc 1500
   aaattacatt tatgttccta tatattacaa catcggcgag gaaatgaaag cgattctcct 1560
   tgagttctga tgaattaaag gagtatgctt taaagtctat ttctttaaag ttttgtttaa 1620
   tatttacaga aaaatccaca tacagtattg gtaaaatgca ggattgttat ataccatcat 1680
   tcgaatcatc cttaaacact tgaatttata ttgtatggta gtatacttgg taagataaaa 1740
   ttccacaaaa atagggatgg tgcagcatat gcaatttcca ttcctattat aattgacaca 1800
   gtacattaac aatccatgcc aacggtgcta atacgatagg ctgaatgtct gaggctacca 1860
   ggtttatcac ataaaaaaca ttcagtaaaa tagtaagttt ctcttttctt caggtgcatt 1920
   ttcctacacc tccaaatgag gaatggattt tctttaatgt aagaagaatc atttttctag 1980
   aggttggctt tcaattctgt agcatacttg gagaaactgc attatcttaa aaggcagtca 2040
   aatggtgttt gtttttatca aaatgtcaaa ataacatact tggagaagta tgtaattttg 2100
   tctttggaaa attacaacac tgcctttgca acactgcagt ttttatggta aaataataga 2160
   aatgatcgac tctatcaata ttgtataaaa agactgaaac aatgcattta tataatatgt 2220
   atacaatatt gttttgtaaa taagtgtctc cttttttatt tactttggta tatttttaca 2280
   ctaaggacat ttcaaattaa gtactaaggc acaaagacat gtcatgcatc acagaaaagc 2340
   aactacttat atttcagagc aaattagcag attaaatagt ggtcttaaaa ctccatatgt 2400
   taatgattag atggttatat tacaatcatt ttatattttt ttacatgatt aacattcact 2460
   tatggattca tgatggctgt ataaagtgaa tttgaaattt caatggttta ctgtcattgt 2520
   gtttaaatct caacgttcca ttattttaat acttgcaaaa acattactaa gtataccaaa 2580
   ataattgact ctattatctg aaatgaagaa taaactgatg ctatctcaac aataactgtt 2640
   acttttattt tataatttga taatgaatat atttctgcat ttatttactt ctgttttgta 2700
   aattgggatt ttgttaatca aatttattgt actatgacta aatgaaatta tttcttacat 2760
   ctaatttgta gaaacagtat aagttatatt aaagtgtttt cacatttttt tgaaagacaa 2820
   aaa 2823
<210> 33
   <211> 375
   <212> PRT
   <213> Homo sapiens
<400> 33
   Met Gln Lys Leu Gln Leu Cys Val Tyr Ile Tyr Leu Phe Met Leu Ile
   1               5                   10                  15
   Val Ala Gly Pro Val Asp Leu Asn Glu Asn Ser Glu Gln Lys Glu Asn
               20                  25                  30
   Val Glu Lys Glu Gly Leu Cys Asn Ala Cys Thr Trp Arg Gln Asn Thr
           35                  40                  45
   Lys Ser Ser Arg Ile Glu Ala Ile Lys Ile Gln Ile Leu Ser Lys Leu
       50                  55                  60
   Arg Leu Glu Thr Ala Pro Asn Ile Ser Lys Asp Val Ile Arg Gln Leu
   65                  70                  75                  80
   Leu Pro Lys Ala Pro Pro Leu Arg Glu Leu Ile Asp Gln Tyr Asp Val
                   85                  90                  95
   Gln Arg Asp Asp Ser Ser Asp Gly Ser Leu Glu Asp Asp Asp Tyr His
               100                 105                 110
   Ala Thr Thr Glu Thr Ile Ile Thr Met Pro Thr Glu Ser Asp Phe Leu
           115                 120                 125
   Met Gln Val Asp Gly Lys Pro Lys Cys Cys Phe Phe Lys Phe Ser Ser
       130                 135                 140
   Lys Ile Gln Tyr Asn Lys Val Val Lys Ala Gln Leu Trp Ile Tyr Leu
   145                 150                 155                 160
   Arg Pro Val Glu Thr Pro Thr Thr Val Phe Val Gln Ile Leu Arg Leu
                   165                 170                 175
   Ile Lys Pro Met Lys Asp Gly Thr Arg Tyr Thr Gly Ile Arg Ser Leu
               180                 185                 190
   Lys Leu Asp Met Asn Pro Gly Thr Gly Ile Trp Gln Ser Ile Asp Val
           195                 200                 205
   Lys Thr Val Leu Gln Asn Trp Leu Lys Gln Pro Glu Ser Asn Leu Gly
       210                 215                 220
   Ile Glu Ile Lys Ala Leu Asp Glu Asn Gly His Asp Leu Ala Val Thr
   225                 230                 235                 240
   Phe Pro Gly Pro Gly Glu Asp Gly Leu Asn Pro Phe Leu Glu Val Lys
                   245                 250                 255
   Val Thr Asp Thr Pro Lys Arg Ser Arg Arg Asp Phe Gly Leu Asp Cys
               260                 265                 270
   Asp Glu His Ser Thr Glu Ser Arg Cys Cys Arg Tyr Pro Leu Thr Val
           275                 280                 285
   Asp Phe Glu Ala Phe Gly Trp Asp Trp Ile Ile Ala Pro Lys Arg Tyr
       290                 295                 300
   Lys Ala Asn Tyr Cys Ser Gly Glu Cys Glu Phe Val Phe Leu Gln Lys
   305                 310                 315                 320
   Tyr Pro His Thr His Leu Val His Gln Ala Asn Pro Arg Gly Ser Ala
                   325                 330                 335
   Gly Pro Cys Cys Thr Pro Thr Lys Met Ser Pro Ile Asn Met Leu Tyr
               340                 345                 350
   Phe Asn Gly Lys Glu Gln Ile Ile Tyr Gly Lys Ile Pro Ala Met Val
           355                 360                 365
   Val Asp Arg Cys Gly Cys Ser
       370                 375
<210> 34
   <211> 3105
   <212> DNA
   <213> Homo sapiens
<400> 34
   caactggggg cgccccggac gaccatgaga gataaggact gagggccagg aaggggaagc 60
   gagcccgccg agaggtggcg gggactgctc acgccaaggg ccacagcggc cgcgctccgg 120
   cctcgctccg ccgctccacg cctcgcggga tccgcggggg cagcccggcc gggcggggat 180
   gccggggctg gggcggaggg cgcagtggct gtgctggtgg tgggggctgc tgtgcagctg 240
   ctgcgggccc ccgccgctgc ggccgccctt gcccgctgcc gcggccgccg ccgccggggg 300
   gcagctgctg ggggacggcg ggagccccgg ccgcacggag cagccgccgc cgtcgccgca 360
   gtcctcctcg ggcttcctgt accggcggct caagacgcag gagaagcggg agatgcagaa 420
   ggagatcttg tcggtgctgg ggctcccgca ccggccccgg cccctgcacg gcctccaaca 480
   gccgcagccc ccggcgctcc ggcagcagga ggagcagcag cagcagcagc agctgcctcg 540
   cggagagccc cctcccgggc gactgaagtc cgcgcccctc ttcatgctgg atctgtacaa 600
   cgccctgtcc gccgacaacg acgaggacgg ggcgtcggag ggggagaggc agcagtcctg 660
   gccccacgaa gcagccagct cgtcccagcg tcggcagccg cccccgggcg ccgcgcaccc 720
   gctcaaccgc aagagccttc tggcccccgg atctggcagc ggcggcgcgt ccccactgac 780
   cagcgcgcag gacagcgcct tcctcaacga cgcggacatg gtcatgagct ttgtgaacct 840
   ggtggagtac gacaaggagt tctcccctcg tcagcgacac cacaaagagt tcaagttcaa 900
   cttatcccag attcctgagg gtgaggtggt gacggctgca gaattccgca tctacaagga 960
   ctgtgttatg gggagtttta aaaaccaaac ttttcttatc agcatttatc aagtcttaca 1020
   ggagcatcag cacagagact ctgacctgtt tttgttggac acccgtgtag tatgggcctc 1080
   agaagaaggc tggctggaat ttgacatcac ggccactagc aatctgtggg ttgtgactcc 1140
   acagcataac atggggcttc agctgagcgt ggtgacaagg gatggagtcc acgtccaccc 1200
   ccgagccgca ggcctggtgg gcagagacgg cccttacgac aagcagccct tcatggtggc 1260
   tttcttcaaa gtgagtgagg tgcacgtgcg caccaccagg tcagcctcca gccggcgccg 1320
   acaacagagt cgtaatcgct ctacccagtc ccaggacgtg gcgcgggtct ccagtgcttc 1380
   agattacaac agcagtgaat tgaaaacagc ctgcaggaag catgagctgt atgtgagttt 1440
   ccaagacctg ggatggcagg actggatcat tgcacccaag ggctatgctg ccaattactg 1500
   tgatggagaa tgctccttcc cactcaacgc acacatgaat gcaaccaacc acgcgattgt 1560
   gcagaccttg gttcacctta tgaaccccga gtatgtcccc aaaccgtgct gtgcgccaac 1620
   taagctaaat gccatctcgg ttctttactt tgatgacaac tccaatgtca ttctgaaaaa 1680
   atacaggaat atggttgtaa gagcttgtgg atgccactaa ctcgaaacca gatgctgggg 1740
   acacacattc tgccttggat tcctagatta catctgcctt aaaaaaacac ggaagcacag 1800
   ttggaggtgg gacgatgaga ctttgaaact atctcatgcc agtgccttat tacccaggaa 1860
   gattttaaag gacctcatta ataatttgct cacttggtaa atgacgtgag tagttgttgg 1920
   tctgtagcaa gctgagtttg gatgtctgta gcataaggtc tggtaactgc agaaacataa 1980
   ccgtgaagct cttcctaccc tcctccccca aaaacccacc aaaattagtt ttagctgtag 2040
   atcaagctat ttggggtgtt tgttagtaaa tagggaaaat aatctcaaag gagttaaatg 2100
   tattcttggc taaaggatca gctggttcag tactgtctat caaaggtaga ttttacagag 2160
   aacagaaatc ggggaagtgg ggggaacgcc tctgttcagt tcattcccag aagtccacag 2220
   gacgcacagc ccaggccaca gccagggctc cacggggcgc ccttgtctca gtcattgctg 2280
   ttgtatgttc gtgctggagt tttgttggtg tgaaaataca cttatttcag ccaaaacata 2340
   ccatttctac acctcaatcc tccatttgct gtactctttg ctagtaccaa aagtagactg 2400
   attacactga ggtgaggcta caaggggtgt gtaaccgtgt aacacgtgaa ggcaatgctc 2460
   acctcttctt taccagaacg gttctttgac cagcacatta acttctggac tgccggctct 2520
   agtacctttt cagtaaagtg gttctctgcc tttttactat acagcatacc acgccacagg 2580
   gttagaacca acgaagaaaa taaaatgagg gtgcccagct tataagaatg gtgttagggg 2640
   gatgagcatg ctgtttatga acggaaatca tgatttccct tgtagaaagt gaggctcaga 2700
   ttaaatttta gaatattttc taaatgtctt tttcacaatc atgtactggg aaggcaattt 2760
   catactaaac tgattaaata atacatttat aatctacaac tgtttgcact tacagctttt 2820
   tttgtaaata taaactataa tttattgtct attttatatc tgttttgctg taacattgaa 2880
   ggaaagacca gacttttaaa aaaaaagagt ttatttagaa agtatcatag tgtaaacaaa 2940
   caaattgtac cactttgatt ttcttggaat acaagactcg tgatgcaaag ctgaagttgt 3000
   gtgtacaaga ctcttgacag ttgtgcttct ctaggaggtt gggttttttt aaaaaaagaa 3060
   ttatctgtga accatacgtg attaataaag atttccttta aggca 3105
<210> 35
   <211> 513
   <212> PRT
   <213> Homo sapiens
<400> 35
<210> 36
   <211> 116
   <212> PRT
   <213> Homo sapiens
<400> 36
   Ile Leu Gly Arg Ser Glu Thr Gln Glu Cys Leu Phe Phe Asn Ala Asn
   1 5 10 15
   Trp Glu Lys Asp Arg Thr Asn Gln Thr Gly Val Glu Pro Cys Tyr Gly
   20 25 30
   Asp Lys Asp Lys Arg Arg His Cys Phe Ala Thr Trp Lys Asn Ile Ser
   35 40 45
   Gly Ser Ile Glu Ile Val Lys Gln Gly Cys Trp Leu Asp Asp Ile Asn
   50 55 60
   Cys Tyr Asp Arg Thr Asp Cys Val Glu Lys Lys Asp Ser Pro Glu Val
   65 70 75 80
   Tyr Phe Cys Cys Cys Glu Gly Asn Met Cys Asn Glu Lys Phe Ser Tyr
   85 90 95
   Phe Pro Glu Met Glu Val Thr Gln Pro Thr Ser Asn Pro Val Thr Pro
   100 105 110
   Lys Pro Pro Thr
   115
<210> 37
   <211> 150
   <212> PRT
   <213> Rattus sp.
<400> 37
   Met Thr Ala Pro Trp Ala Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Pro
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Pro Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 38
   <211> 150
   <212> PRT
   <213> Sus sp.
<400> 38
   Met Thr Ala Pro Trp Ala Ala Leu Ala Leu Leu Trp Gly Ser Leu Cys
   1               5                   10                  15
   Val Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 39
   <211> 150
   <212> PRT
   <213> Mus sp.
<400> 39
   Met Thr Ala Pro Trp Ala Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Pro Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
                       145                 150
<210> 40
   <211> 150
   <212> PRT
   <213> Homo sapiens
<400> 40
   Met Thr Ala Pro Trp Val Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 41
   <211> 150
   <212> PRT
   <213> Bos sp.
<400> 41
   Met Thr Ala Pro Trp Ala Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Arg Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Val Gly Gly Leu Ser
   145                 150
<210> 42
   <211> 150
   <212> PRT
   <213> Xenopus sp.
<400> 42
   Met Gly Ala Ser Val Ala Leu Thr Phe Leu Leu Leu Leu Ala Thr Phe
   1               5                   10                  15
   Arg Ala Gly Ser Gly His Asp Glu Val Glu Thr Arg Glu Cys Ile Tyr
               20                  25                  30
   Tyr Asn Ala Asn Trp Glu Leu Glu Lys Thr Asn Gln Ser Gly Val Glu
           35                  40                  45
   Arg Leu Val Glu Gly Lys Lys Asp Lys Arg Leu His Cys Tyr Ala Ser
       50                  55                  60
   Trp Arg Asn Asn Ser Gly Phe Ile Glu Leu Val Lys Lys Gly Cys Trp
   65                  70                  75                  80
   Leu Asp Asp Phe Asn Cys Tyr Asp Arg Gln Glu Cys Ile Ala Lys Glu
                   85                  90                  95
   Glu Asn Pro Gln Val Phe Phe Cys Cys Cys Glu Gly Asn Tyr Cys Asn
               100                 105                 110
   Lys Lys Phe Thr His Leu Pro Glu Val Glu Thr Phe Asp Pro Lys Pro
           115                 120                 125
   Gln Pro Ser Ala Ser Val Leu Asn Ile Leu Ile Tyr Ser Leu Leu Pro
       130                 135                 140
   Ile Val Gly Leu Ser Met
   145                 150
<210> 43
   <211> 150
   <212> PRT
   <213> Homo sapiens
<400> 43
   Met Gly Ala Ala Ala Lys Leu Ala Phe Ala Val Phe Leu Ile Ser Cys
   1               5                   10                  15
   Ser Ser Gly Ala Ile Leu Gly Arg Ser Glu Thr Gln Glu Cys Leu Phe
               20                  25                  30
   Phe Asn Ala Asn Trp Glu Lys Asp Arg Thr Asn Gln Thr Gly Val Glu
           35                  40                  45
   Pro Cys Tyr Gly Asp Lys Asp Lys Arg Arg His Cys Phe Ala Thr Trp
       50                  55                  60
   Lys Asn Ile Ser Gly Ser Ile Glu Ile Val Lys Gln Gly Cys Trp Leu
   65                  70                  75                  80
   Asp Asp Ile Asn Cys Tyr Asp Arg Thr Asp Cys Val Glu Lys Lys Asp
                   85                  90                  95
   Ser Pro Glu Val Tyr Phe Cys Cys Cys Glu Gly Asn Met Cys Asn Glu
               100                 105                 110
   Lys Phe Ser Tyr Phe Pro Glu Met Glu Val Thr Gln Pro Thr Ser Asn
           115                 120                 125
   Pro Val Thr Pro Lys Pro Pro Tyr Tyr Asn Ile Leu Leu Tyr Ser Leu
       130                 135                 140
   Val Pro Leu Met Leu Ile
   145                 150
<210> 44
   <211> 154
   <212> PRT
   <213> Artificial Sequence
<220>
   <223> Description of Artificial Sequence: Synthetic consensus polypeptide
<220>
   <221> VARIANT
   <222> (8)..(8)
   <223> Ala or not present
<220>
   <221> VARIANT
   <222> (121)..(121)
   <223> Ala, Val or Met
<400> 44
   Met Thr Ala Pro Trp Ala Ala Thr Leu Ala Leu Leu Trp Gly Ser Leu
   1               5                   10                  15
   Cys Ala Gly Ser Gly Arg Gly Glu Ala Glu Thr Arg Glu Cys Ile Tyr
               20                  25                  30
   Tyr Asn Ala Asn Trp Glu Leu Glu Arg Thr Asn Gln Ser Gly Leu Glu
           35                  40                  45
   Arg Leu Cys Glu Gly Glu Gln Asp Lys Arg Leu His Cys Tyr Ala Ser
       50                  55                  60
   Trp Arg Asn Ser Ser Gly Thr Ile Glu Leu Val Lys Lys Gly Cys Trp
   65                  70                  75                  80
   Leu Asp Asp Phe Asn Cys Tyr Asp Arg Gln Glu Cys Val Ala Thr Glu
                   85                  90                  95
   Glu Asn Pro Gln Val Tyr Phe Cys Cys Cys Glu Gly Asn Phe Cys Asn
               100                 105                 110
   Glu Arg Phe Thr His Leu Pro Glu Pro Gly Gly Pro Glu Val Thr Tyr
           115                 120                 125
   Glu Pro Lys Pro Pro Thr Ala Pro Thr Leu Leu Thr Val Leu Ala Tyr
       130                 135                 140
   Ser Leu Leu Pro Ile Gly Gly Leu Ser Met
   145                 150
<210> 45
   <211> 4
   <212> PRT
   <213> Artificial Sequence
<220>
   <223> Description of Artificial Sequence: Synthetic peptide
<400> 45
   Thr Gly Gly Gly
   1
<210> 46
   <211> 115
   <212> PRT
   <213> Homo sapiens
<400> 46
   Gly Arg Gly Glu Ala Glu Thr Arg Glu Cys Ile Tyr Tyr Asn Ala Asn
   1 5 10 15
   Trp Glu Leu Glu Arg Thr Asn Gln Ser Gly Leu Glu Arg Cys Glu Gly
   20 25 30
   Glu Gln Asp Lys Arg Leu His Cys Tyr Ala Ser Trp Arg Asn Ser Ser
   35 40 45
   Gly Thr Ile Glu Leu Val Lys Lys Gly Cys Trp Leu Asp Asp Phe Asn
   50 55 60
   Cys Tyr Asp Arg Gln Glu Cys Val Ala Thr Glu Glu Asn Pro Gln Val
   65 70 75 80
   Tyr Phe Cys Cys Cys Glu Gly Asn Phe Cys Asn Glu Arg Phe Thr His
   85 90 95
   Leu Pro Glu Ala Gly Gly Pro Glu Val Thr Tyr Glu Pro Pro Pro Thr
   100 105 110
   Ala Pro Thr
   115
<210> 47
   <211> 344
   <212> PRT
   <213> Artificial Sequence
<220>
   <223> Description of Artificial Sequence: Synthetic polypeptide
<400> 47
   Ser Gly Arg Gly Glu Ala Glu Thr Arg Glu Cys Ile Tyr Tyr Asn Ala
   1 5 10 15
   Asn Trp Glu Leu Glu Arg Thr Asn Gln Ser Gly Leu Glu Arg Cys Glu
   20 25 30
   Gly Glu Gln Asp Lys Arg Leu His Cys Tyr Ala Ser Trp Arg Asn Ser
           35                  40                  45
   Ser Gly Thr Ile Glu Leu Val Lys Lys Gly Cys Trp Leu Asp Asp Phe
       50                  55                  60
   Asn Cys Tyr Asp Arg Gln Glu Cys Val Ala Thr Glu Glu Asn Pro Gln
   65                  70                  75                  80
   Val Tyr Phe Cys Cys Cys Glu Gly Asn Phe Cys Asn Glu Arg Phe Thr
                   85                  90                  95
   His Leu Pro Glu Ala Gly Gly Pro Glu Val Thr Tyr Glu Pro Pro Pro
               100                 105                 110
   Thr Ala Pro Thr Gly Gly Gly Thr His Thr Cys Pro Pro Cys Pro Ala
           115                 120                 125
   Pro Glu Leu Leu Gly Gly Pro Ser Val Phe Leu Phe Pro Pro Lys Pro
       130                 135                 140
   Lys Asp Thr Leu Met Ile Ser Arg Thr Pro Glu Val Thr Cys Val Val
   145                 150                 155                 160
   Val Asp Val Ser His Glu Asp Pro Glu Val Lys Phe Asn Trp Tyr Val
                   165                 170                 175
   Asp Gly Val Glu Val His Asn Ala Lys Thr Lys Pro Arg Glu Glu Gln
               180                 185                 190
   Tyr Asn Ser Thr Tyr Arg Val Val Ser Val Leu Thr Val Leu His Gln
           195                 200                 205
   Asp Trp Leu Asn Gly Lys Glu Tyr Lys Cys Lys Val Ser Asn Lys Ala
       210                 215                 220
   Leu Pro Val Pro Ile Glu Lys Thr Ile Ser Lys Ala Lys Gly Gln Pro
   225                 230                 235                 240
   Arg Glu Pro Gln Val Tyr Thr Leu Pro Pro Ser Arg Glu Glu Met Thr
                   245                 250                 255
   Lys Asn Gln Val Ser Leu Thr Cys Leu Val Lys Gly Phe Tyr Pro Ser
               260                 265                 270
   Asp Ile Ala Val Glu Trp Glu Ser Asn Gly Gln Pro Glu Asn Asn Tyr
   275 280 285
   Lys Thr Thr Pro Pro Val Leu Asp Ser Asp Gly Ser Phe Phe Leu Tyr
   290 295 300
   Ser Lys Leu Thr Val Asp Lys Ser Arg Trp Gln Gln Gly Asn Val Phe
   305 310 315 320
   Ser Cys Ser Val Met His Glu Ala Leu His Asn His Tyr Thr Gln Lys
   325 330 335
   Ser Leu Ser Leu Ser Pro Gly Lys
   340
<210> 48
   <211> 343
   <212> PRT
   <213> Artificial Sequence
<220>
   <223> Description of Artificial Sequence: Synthetic polypeptide
<400> 48
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Arg Asn Ser Ser
           35                  40                  45
   Gly Thr Ile Glu Leu Val Lys Lys Gly Cys Trp Leu Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
                   85                  90                  95
   Leu Pro Glu Ala Gly Gly Pro Glu Val Thr Tyr Glu Pro Pro Pro Thr
               100                 105                 110
   Ala Pro Thr Gly Gly Gly Thr His Thr Cys Pro Pro Cys Pro Ala Pro
           115                 120                 125
   Glu Leu Leu Gly Gly Pro Ser Val Phe Leu Phe Pro Pro Lys Pro Lys
       130                 135                 140
   Asp Thr Leu Met Ile Ser Arg Thr Pro Glu Val Thr Cys Val Val Val
   145 150 155 160
   Asp Val Ser His Glu Asp Pro Glu Val Lys Phe Asn Trp Tyr Val Asp
   165 170 175
   Gly Val Glu Val His Asn Ala Lys Thr Lys Pro Arg Glu Glu Gln Tyr
   180 185 190
   Asn Ser Thr Tyr Arg Val Val Ser Val Leu Thr Val Leu His Gln Asp
   195 200 205
   Trp Leu Asn Gly Lys Glu Tyr Lys Cys Lys Val Ser Asn Lys Ala Leu
   210 215 220
   Pro Val Pro Ile Glu Lys Thr Ile Ser Lys Ala Lys Gly Gln Pro Arg
   225 230 235 240
   Glu Pro Gln Val Tyr Thr Leu Pro Pro Ser Arg Glu Glu Met Thr Lys
   245 250 255
   Asn Gln Val Ser Leu Thr Cys Leu Val Lys Gly Phe Tyr Pro Ser Asp
   260 265 270
   Ile Ala Val Glu Trp Glu Ser Asn Gly Gln Pro Glu Asn Asn Tyr Lys
   275 280 285
   Thr Thr Pro Pro Val Leu Asp Ser Asp Gly Ser Phe Phe Leu Tyr Ser
   290 295 300
   Lys Leu Thr Val Asp Lys Ser Arg Trp Gln Gln Gly Asn Val Phe Ser
   305 310 315 320
   Cys Ser Val Met His Glu Ala Leu His Asn His Tyr Thr Gln Lys Ser
   325 330 335
   Leu Ser Leu Ser Pro Gly Lys
   340
<210> 49
   <211> 335
   <212> PRT
   <213> Artificial Sequence
<220>
   <223> Description of Artificial Sequence: Synthetic polypeptide
<400> 49
   Glu Thr Arg Glu Cys Ile Tyr Tyr Asn Ala Asn Trp Glu Leu Glu Arg
   1               5                   10                  15
   Thr Asn Gln Ser Gly Leu Glu Arg Cys Glu Gly Glu Gln Asp Lys Arg
               20                  25                  30
   Leu His Cys Tyr Ala Ser Trp Arg Asn Ser Ser Gly Thr Ile Glu Leu
           35                  40                  45
   Val Lys Lys Gly Cys Trp Asp Asp Asp Phe Asn Cys Tyr Asp Arg Gln
       50                  55                  60
   Glu Cys Val Ala Thr Glu Glu Asn Pro Gln Val Tyr Phe Cys Cys Cys
   65                  70                  75                  80
   Glu Gly Asn Phe Cys Asn Glu Arg Phe Thr His Leu Pro Glu Ala Gly
                   85                  90                  95
   Gly Pro Glu Val Thr Tyr Glu Pro Pro Pro Thr Gly Gly Gly Thr His
               100                 105                 110
   Thr Cys Pro Pro Cys Pro Ala Pro Glu Leu Leu Gly Gly Pro Ser Val
           115                 120                 125
   Phe Leu Phe Pro Pro Lys Pro Lys Asp Thr Leu Met Ile Ser Arg Thr
       130                 135                 140
   Pro Glu Val Thr Cys Val Val Val Asp Val Ser His Glu Asp Pro Glu
   145                 150                 155                 160
   Val Lys Phe Asn Trp Tyr Val Asp Gly Val Glu Val His Asn Ala Lys
                   165                 170                 175
   Thr Lys Pro Arg Glu Glu Gln Tyr Asn Ser Thr Tyr Arg Val Val Ser
               180                 185                 190
   Val Leu Thr Val Leu His Gln Asp Trp Leu Asn Gly Lys Glu Tyr Lys
           195                 200                 205
   Cys Lys Val Ser Asn Lys Ala Leu Pro Ala Pro Ile Glu Lys Thr Ile
       210                 215                 220
   Ser Lys Ala Lys Gly Gln Pro Arg Glu Pro Gln Val Tyr Thr Leu Pro
   225                 230                 235                 240
   Pro Ser Arg Glu Glu Met Thr Lys Asn Gln Val Ser Leu Thr Cys Leu
                   245                 250                 255
   Val Lys Gly Phe Tyr Pro Ser Asp Ile Ala Val Glu Trp Glu Ser Asn
   260 265 270
   Gly Gln Pro Glu Asn Asn Tyr Lys Thr Thr Pro Pro Val Leu Asp Ser
   275 280 285
   Asp Gly Ser Phe Phe Leu Tyr Ser Lys Leu Thr Val Asp Lys Ser Arg
   290 295 300
   Trp Gln Gln Gly Asn Val Phe Ser Cys Ser Val Met His Glu Ala Leu
   305 310 315 320
   His Asn His Tyr Thr Gln Lys Ser Leu Ser Leu Ser Pro Gly Lys
   325 330 335
<210> 50
   <211> 308
   <212> PRT
   <213> Homo sapiens
<400> 50
   Met Pro Gly Gln Glu Leu Arg Thr Val Asn Gly Ser Gln Met Leu Leu
   1 5 10 15
   Val Leu Leu Val Leu Ser Trp Leu Pro His Gly Gly Ala Leu Ser Leu
   20 25 30
   Ala Glu Ala Ser Arg Ala Ser Phe Pro Gly Pro Ser Glu Leu His Ser
   35 40 45
   Glu Asp Ser Arg Phe Arg Glu Leu Arg Lys Arg Tyr Glu Asp Leu Leu
   50 55 60
   Thr Arg Leu Arg Ala Asn Gln Ser Trp Glu Asp Ser Asn Thr Asp Leu
   65 70 75 80
   Val Pro Ala Pro Ala Val Arg Ile Leu Thr Pro Glu Val Arg Leu Gly
   85 90 95
   Ser Gly Gly His Leu His Leu Arg Ile Ser Arg Ala Ala Leu Pro Glu
   100 105 110
   Gly Leu Pro Glu Ala Ser Arg Leu His Arg Ala Leu Phe Arg Leu Ser
   115 120 125
   Pro Thr Ala Ser Arg Ser Trp Asp Val Thr Arg Pro Leu Arg Arg Gln
   130 135 140
   Leu Ser Leu Ala Arg Pro Gln Ala Pro Ala Leu His Leu Arg Leu Ser
   145                 150                 155                 160
   Pro Pro Pro Ser Gln Ser Asp Gln Leu Leu Ala Glu Ser Ser Ser Ala
                   165                 170                 175
   Arg Pro Gln Leu Glu Leu His Leu Arg Pro Gln Ala Ala Arg Gly Arg
               180                 185                 190
   Arg Arg Ala Arg Ala Arg Asn Gly Asp His Cys Pro Leu Gly Pro Gly
           195                 200                 205
   Arg Cys Cys Arg Leu His Thr Val Arg Ala Ser Leu Glu Asp Leu Gly
       210                 215                 220
   Trp Ala Asp Trp Val Leu Ser Pro Arg Glu Val Gln Val Thr Met Cys
   225                 230                 235                 240
   Ile Gly Ala Cys Pro Ser Gln Phe Arg Ala Ala Asn Met His Ala Gln
                   245                 250                 255
   Ile Lys Thr Ser Leu His Arg Leu Lys Pro Asp Thr Val Pro Ala Pro
               260                 265                 270
   Cys Cys Val Pro Ala Ser Tyr Asn Pro Met Val Leu Ile Gln Lys Thr
           275                 280                 285
   Asp Thr Gly Val Ser Leu Gln Thr Tyr Asp Asp Leu Leu Ala Lys Asp
       290                 295                 300
   Cys His Cys Ile
   305
<210> 51
   <211> 1220
   <212> DNA
   <213> Homo sapiens
<400> 51
   agtcccagct cagagccgca acctgcacag ccatgcccgg gcaagaactc aggacggtga 60
   atggctctca gatgctcctg gtgttgctgg tgctctcgtg gctgccgcat gggggcgccc 120
   tgtctctggc cgaggcgagc cgcgcaagtt tcccgggacc ctcagagttg cactccgaag 180
   actccagatt ccgagagttg cggaaacgct acgaggacct gctaaccagg ctgcgggcca 240
   accagagctg ggaagattcg aacaccgacc tcgtcccggc ccctgcagtc cggatactca 300
   cgccagaagt gcggctggga tccggcggcc acctgcacct gcgtatctct cgggccgccc 360
   ttcccgaggg gctccccgag gcctcccgcc ttcaccgggc tctgttccgg ctgtccccga 420
   cggcgtcaag gtcgtgggac gtgacacgac cgctgcggcg tcagctcagc cttgcaagac 480
   cccaggcgcc cgcgctgcac ctgcgactgt cgccgccgcc gtcgcagtcg gaccaactgc 540
   tggcagaatc ttcgtccgca cggccccagc tggagttgca cttgcggccg caagccgcca 600
   gggggcgccg cagagcgcgt gcgcgcaacg gggaccactg tccgctcggg cccgggcgtt 660
   gctgccgtct gcacacggtc cgcgcgtcgc tggaagacct gggctgggcc gattgggtgc 720
   tgtcgccacg ggaggtgcaa gtgaccatgt gcatcggcgc gtgcccgagc cagttccggg 780
   cggcaaacat gcacgcgcag atcaagacga gcctgcaccg cctgaagccc gacacggtgc 840
   cagcgccctg ctgcgtgccc gccagctaca atcccatggt gctcattcaa aagaccgaca 900
   ccggggtgtc gctccagacc tatgatgact tgttagccaa agactgccac tgcatatgag 960
   cagtcctggt ccttccactg tgcacctgcg cggaggacgc gacctcagtt gtcctgccct 1020
   gtggaatggg ctcaaggttc ctgagacacc cgattcctgc ccaaacagct gtatttatat 1080
   aagtctgtta tttattatta atttattggg gtgaccttct tggggactcg ggggctggtc 1140
   tgatggaact gtgtatttat ttaaaactct ggtgataaaa ataaagctgt ctgaactgtt 1200
   aaaaaaaaaa aaaaaaaaaa 1220
<210> 52
   <211> 347
   <212> PRT
   <213> Homo sapiens
<400> 52
<210> 53
   <211> 2086
   <212> DNA
   <213> Homo sapiens
<400> 53
   ataagggctg gaggtgctgc tttcaggcct ggccagccca ccatgcacgc ccactgcctg 60
   cccttccttc tgcacgcctg gtgggcccta ctccaggcgg gtgctgcgac ggtggccact 120
   gcgctcctgc gtacgcgggg gcagccctcg tcgccatccc ctctggcgta catgctgagc 180
   ctctaccgcg acccgctgcc gagggcagac atcatccgca gcctacaggc agaagatgtg 240
   gcagtggatg ggcagaactg gacgtttgct tttgacttct ccttcctgag ccaacaagag 300
   gatctggcat gggctgagct ccggctgcag ctgtccagcc ctgtggacct ccccactgag 360
   ggctcacttg ccattgagat tttccaccag ccaaagcccg acacagagca ggcttcagac 420
   agctgcttag agcggtttca gatggaccta ttcactgtca ctttgtccca ggtcaccttt 480
   tccttgggca gcatggtttt ggaggtgacc aggcctctct ccaagtggct gaagcaccct 540
   ggggccctgg agaagcagat gtccagggta gctggagagt gctggccgcg gccccccaca 600
   ccgcctgcca ccaatgtgct ccttatgctc tactccaacc tctcgcagga gcagaggcag 660
   ctgggtgggt ccaccttgct gtgggaagcc gagagctcct ggcgggccca ggagggacag 720
   ctgtcctggg agtggggcaa gaggcaccgt cgacatcact tgccagacag aagtcaactg 780
   tgtcggaagg tcaagttcca ggtggacttc aacctgatcg gatggggctc ctggatcatc 840
   taccccaagc agtacaacgc ctatcgctgt gagggcgagt gtcctaatcc tgttggggag 900
   gagtttcatc cgaccaacca tgcatacatc cagagtctgc tgaaacgtta ccagccccac 960
   cgagtccctt ccacttgttg tgccccagtg aagaccaagc cgctgagcat gctgtatgtg 1020
   gataatggca gagtgctcct agatcaccat aaagacatga tcgtggaaga atgtgggtgc 1080
   ctctgatgac atcctggagg gagactggat ttgcctgcac tctggaaggc tgggaaactc 1140
   ctggaagaca tgataaccat ctaatccagt aaggagaaac agagaggggc aaagttgctc 1200
   tgcccaccag aactgaagag gaggggctgc ccactctgta aatgaagggc tcagtggagt 1260
   ctggccaagc acagaggctg ctgtcaggaa gagggaggaa gaagcctgtg cagggggctg 1320
   gctggatgtt ctctttactg aaaagacagt ggcaaggaaa agcacaagtg catgagttct 1380
   ttactggatt ttttaaaaac ctgtgaaccc cccgaaactg tatgtgaaag ttgagacata 1440
   tgtgcatgta ttttggaggt gggatgaagt cacctatagc tttcatgtat tctccaaagt 1500
   agtctgtgtg tgacctgtcc ccctccccaa agattaagga tcactgtata gattaaaaag 1560
   agtccgtcaa tctcattgcc tcaggctggg ttgggggagc cccacagctt tctggctggc 1620
   cagtggcaat ctactggcct tgtccagagg ctcactggag tggttctctg ctaatgagct 1680
   gtacaacaat aaagccattg tctagttctc ctgggccagc tggtgcctgt gaaggcagag 1740
   gcaggaactc atccaagagg accggccatg ttgggttaca gaagacatcc ctgcgtcagt 1800
   ctgcttcggc agacacagcc tgagtttgtt aaagttggtg acaatccacc tcagtctctc 1860
   aatgtgtgct attaatgagg cctctgagct tcctatccag cagtggtgaa ggccttgccc 1920
   tgggtggcaa gatacttgct ctatggtcac agctcagcca ctggaagctg tgcgacctca 1980
   ggtgagcaat tcactgtcca gtctccactt gtaaaaggaa cgctggtgaa tcctaatgca 2040
   ttcatattaa atgtctgttg tcaggctcag aagagccatg agcttt 2086
<210> 54
   <211> 364
   <212> PRT
   <213> Homo sapiens
<400> 54
<210> 55
   <211> 1224
   <212> DNA
   <213> Homo sapiens
<400> 55
   ggagctctcc ccggtctgac agccactcca gaggccatgc ttcgtttctt gccagatttg        60
   gctttcagct tcctgttaat tctggctttg ggccaggcag tccaatttca agaatatgtc       120
   tttctccaat ttctgggctt agataaggcg ccttcacccc agaagttcca acctgtgcct       180
   tatatcttga agaaaatttt ccaggatcgc gaggcagcag cgaccactgg ggtctcccga       240
   gacttatgct acgtaaagga gctgggcgtc cgcgggaatg tacttcgctt tctcccagac       300
   caaggtttct ttctttaccc aaagaaaatt tcccaagctt cctcctgcct gcagaagctc       360
   ctctacttta acctgtctgc catcaaagaa agggaacagt tgacattggc ccagctgggc       420
   ctggacttgg ggcccaattc ttactataac ctgggaccag agctggaact ggctctgttc       480
   ctggttcagg agcctcatgt gtggggccag accaccccta agccaggtaa aatgtttgtg       540
   ttgcggtcag tcccatggcc acaaggtgct gttcacttca acctgctgga tgtagctaag       600
   gattggaatg acaacccccg gaaaaatttc gggttattcc tggagatact ggtcaaagaa       660
   gatagagact caggggtgaa ttttcagcct gaagacacct gtgccagact aagatgctcc       720
   cttcatgctt ccctgctggt ggtgactctc aaccctgatc agtgccaccc ttctcggaaa       780
   aggagagcag ccatccctgt ccccaagctt tcttgtaaga acctctgcca ccgtcaccag       840
   ctattcatta acttccggga cctgggttgg cacaagtgga tcattgcccc caaggggttc       900
   atggcaaatt actgccatgg agagtgtccc ttctcactga ccatctctct caacagctcc       960
   aattatgctt tcatgcaagc cctgatgcat gccgttgacc cagagatccc ccaggctgtg      1020
   tgtatcccca ccaagctgtc tcccatttcc atgctctacc aggacaataa tgacaatgtc      1080
   attctacgac attatgaaga catggtagtc gatgaatgtg ggtgtgggta ggatgtcaga      1140
   aatgggaata gaaggagtgt tcttagggta aatcttttaa taaaactacc tatctggttt      1200
   atgaccactt agatcgaaat gtca                                              1224
<210> 56
   <211> 472
   <212> PRT
   <213> Homo sapiens
<400> 56
   Met Ala Gly Ala Ser Arg Leu Leu Phe Leu Trp Leu Gly Cys Phe Cys
   1 5 10 15
   Val Ser Leu Ala Gln Gly Glu Arg Pro Lys Pro Pro Phe Pro Glu Leu
   20 25 30
   Arg Lys Ala Val Pro Gly Asp Arg Thr Ala Gly Gly Gly Pro Asp Ser
   35 40 45
   Glu Leu Gln Pro Gln Asp Lys Val Ser Glu His Met Leu Arg Leu Tyr
   50 55 60
   Asp Arg Tyr Ser Thr Val Gln Ala Ala Arg Thr Pro Gly Ser Leu Glu
   65 70 75 80
   Gly Gly Ser Gln Pro Trp Arg Pro Arg Leu Leu Arg Glu Gly Asn Thr
   85 90 95
   Val Arg Ser Phe Arg Ala Ala Ala Ala Glu Thr Leu Glu Arg Lys Gly
               100                 105                 110
   Leu Tyr Ile Phe Asn Leu Thr Ser Leu Thr Lys Ser Glu Asn Ile Leu
           115                 120                 125
   Ser Ala Thr Leu Tyr Phe Cys Ile Gly Glu Leu Gly Asn Ile Ser Leu
       130                 135                 140
   Ser Cys Pro Val Ser Gly Gly Cys Ser His His Ala Gln Arg Lys His
   145                 150                 155                 160
   Ile Gln Ile Asp Leu Ser Ala Trp Thr Leu Lys Phe Ser Arg Asn Gln
                   165                 170                 175
   Ser Gln Leu Leu Gly His Leu Ser Val Asp Met Ala Lys Ser His Arg
               180                 185                 190
   Asp Ile Met Ser Trp Leu Ser Lys Asp Ile Thr Gln Leu Leu Arg Lys
           195                 200                 205
   Ala Lys Glu Asn Glu Glu Phe Leu Ile Gly Phe Asn Ile Thr Ser Lys
       210                 215                 220
   Gly Arg Gln Leu Pro Lys Arg Arg Leu Pro Phe Pro Glu Pro Tyr Ile
   225                 230                 235                 240
   Leu Val Tyr Ala Asn Asp Ala Ala Ile Ser Glu Pro Glu Ser Val Val
                   245                 250                 255
   Ser Ser Leu Gln Gly His Arg Asn Phe Pro Thr Gly Thr Val Pro Lys
               260                 265                 270
   Trp Asp Ser His Ile Arg Ala Ala Leu Ser Ile Glu Arg Arg Lys Lys
           275                 280                 285
   Arg Ser Thr Gly Val Leu Leu Pro Leu Gln Asn Asn Glu Leu Pro Gly
       290                 295                 300
   Ala Glu Tyr Gln Tyr Lys Lys Asp Glu Val Trp Glu Glu Arg Lys Pro
   305                 310                 315                 320
   Tyr Lys Thr Leu Gln Ala Gln Ala Pro Glu Lys Ser Lys Asn Lys Lys
                   325                 330                 335
   Lys Gln Arg Lys Gly Pro His Arg Lys Ser Gln Thr Leu Gln Phe Asp
               340                 345                 350
   Glu Gln Thr Leu Lys Lys Ala Arg Arg Lys Gln Trp Ile Glu Pro Arg
           355                 360                 365
   Asn Cys Ala Arg Arg Tyr Leu Lys Val Asp Phe Ala Asp Ile Gly Trp
       370                 375                 380
   Ser Glu Trp Ile Ile Ser Pro Lys Ser Phe Asp Ala Tyr Tyr Cys Ser
   385                 390                 395                 400
   Gly Ala Cys Gln Phe Pro Met Pro Lys Ser Leu Lys Pro Ser Asn His
                   405                 410                 415
   Ala Thr Ile Gln Ser Ile Val Arg Ala Val Gly Val Val Pro Gly Ile
               420                 425                 430
   Pro Glu Pro Cys Cys Val Pro Glu Lys Met Ser Ser Leu Ser Ile Leu
           435                 440                 445
   Phe Phe Asp Glu Asn Lys Asn Val Val Leu Lys Val Tyr Pro Asn Met
       450                 455                 460
   Thr Val Glu Ser Cys Ala Cys Arg
   465                 470
<210> 57
   <211> 5733
   <212> DNA
   <213> Homo sapiens
<400> 57
   agatcttgaa aacacccggg ccacacacgc cgcgacctac agctctttct cagcgttgga 60
   gtggagacgg cgcccgcagc gccctgcgcg ggtgaggtcc gcgcagctgc tggggaagag 120
   cccacctgtc aggctgcgct gggtcagcgc agcaagtggg gctggccgct atctcgctgc 180
   acccggccgc gtcccgggct ccgtgcgccc tcgccccagc tggtttggag ttcaaccctc 240
   ggctccgccg ccggctcctt gcgccttcgg agtgtcccgc agcgacgccg ggagccgacg 300
   cgccgcgcgg gtacctagcc atggctgggg cgagcaggct gctctttctg tggctgggct 360
   gcttctgcgt gagcctggcg cagggagaga gaccgaagcc acctttcccg gagctccgca 420
   aagctgtgcc aggtgaccgc acggcaggtg gtggcccgga ctccgagctg cagccgcaag 480
   acaaggtctc tgaacacatg ctgcggctct atgacaggta cagcacggtc caggcggccc 540
   ggacaccggg ctccctggag ggaggctcgc agccctggcg ccctcggctc ctgcgcgaag 600
   gcaacacggt tcgcagcttt cgggcggcag cagcagaaac tcttgaaaga aaaggactgt 660
   atatcttcaa tctgacatcg ctaaccaagt ctgaaaacat tttgtctgcc acactgtatt 720
   tctgtattgg agagctagga aacatcagcc tgagttgtcc agtgtctgga ggatgctccc 780
   atcatgctca gaggaaacac attcagattg atctttctgc atggaccctc aaattcagca 840
   gaaaccaaag tcaactcctt ggccatctgt cagtggatat ggccaaatct catcgagata 900
   ttatgtcctg gctgtctaaa gatatcactc aactcttgag gaaggccaaa gaaaatgaag 960
   agttcctcat aggatttaac attacgtcca agggacgcca gctgccaaag aggaggttac 1020
   cttttccaga gccttatatc ttggtatatg ccaatgatgc cgccatttct gagccagaaa 1080
   gtgtggtatc aagcttacag ggacaccgga attttcccac tggaactgtt cccaaatggg 1140
   atagccacat cagagctgcc ctttccattg agcggaggaa gaagcgctct actggggtct 1200
   tgctgcctct gcagaacaac gagcttcctg gggcagaata ccagtataaa aaggatgagg 1260
   tgtgggagga gagaaagcct tacaagaccc ttcaggctca ggcccctgaa aagagtaaga 1320
   ataaaaagaa acagagaaag gggcctcatc ggaagagcca gacgctccaa tttgatgagc 1380
   agaccctgaa aaaggcaagg agaaagcagt ggattgaacc tcggaattgc gccaggagat 1440
   acctcaaggt agactttgca gatattggct ggagtgaatg gattatctcc cccaagtcct 1500
   ttgatgccta ttattgctct ggagcatgcc agttccccat gccaaagtct ttgaagccat 1560
   caaatcatgc taccatccag agtatagtga gagctgtggg ggtcgttcct gggattcctg 1620
   agccttgctg tgtaccagaa aagatgtcct cactcagtat tttattcttt gatgaaaata 1680
   agaatgtagt gcttaaagta taccctaaca tgacagtaga gtcttgcgct tgcagataac 1740
   ctggcaaaga actcatttga atgcttaatt caatcattag tttattttta tggacttctt 1800
   cctgtttttt tttttttttt ttttgcactg ccaatgcatt ttgtttcaaa agattatttc 1860
   tatagtcaga ggggaatgag caaatagact gaagattgcc accaaggaaa agaactgtat 1920
   ttgtttctga atgtaactta aagcaagatt tttagtaaat atggacatct atttctcttt 1980
   ttgtaatcaa acacaacaac ttatcaaact gtttttagaa ctgttagaga acacactggt 2040
   ttatttttgt aatgttcttt gaaaacagaa tggagaagca gcaatagctt gtcatttatc 2100
   tcatttaatg actaatggga aatagagaac aatttcgcgt tttgaattag gcttattgcc 2160
   ttagaatcct gagaaagtgc taaataatca actctgatgt ttttcttaag ttcttgagac 2220
   tcttgtttat ccttgttttt cctccacaag tcattgtcta agtgtaatgg aaagtttatg 2280
   ctgagcgtta gtgtgtatgt atgtgcgtac atgcgccagg tgcctgtgcc ctctgtagga 2340
   tggtttgctt aatatggttt tataattcag tttacacagg attctttatt ttttttaatt 2400
   ttgtattttg gcaaacacca ttcagttata agaactttgc caaatatgat agaataattc 2460
   aagagcatat acagagagtt accacttgac ccagctattt aattgcaaat acagttgttt 2520
   tcatttcatt tcctaccaga aaaaggaatc agaaacctag tttttgaaaa cacaagtgta 2580
   attcctcttt tgtacttctt tttcacaaat gcttttattt attctaaatt gaatttaaaa 2640
   atccttccta aagccattaa ctctttaatt ctcctgatat gcctttactt cctatgaagt 2700
   tattggtaga tgttgaggcc caaaaactgg tagaatattg aagatcttct taaatgacca 2760
   atttaaccat aaccaaatat tgaatatcat tcttcagtca catctaagtc aggcactttt 2820
   tcacatagat cagggctttt ggctcagtca cgaaatctac aagttagcaa agcttacaaa 2880
   acattattcg tcaggtatgg gaatcaaata tagacacttg tttgtctttg tttccatttc 2940
   tatgtgtcac atacatatat gtgtcctctt ataactttag tcttcaaaat tatttcaata 3000
   tccttcttct cactatattt atttgtgtga tggaaatgct ttcaggccgt agatcattgt 3060
   tggtgttaat ctgtggttaa tcctcatttt agttccgtct tatctgatac ttagaaatat 3120
   ctcagccatt ttggaggctg tgcagtatca gaagacgtgg agtttgttct gtctctgcct 3180
   gtagctaatt atggtggttc agtcatttaa taaatatgtt ttgagcatct attttgtgaa 3240
   aggcactgtg ttacctgtgt gtctttagtg tcctcactgg taaaatgaag aggctggcca 3300
   tgagctggaa gggttaagtt tataattcca gctatttcac acccgtcttc cttgaaggaa 3360
   tgatagtgat agatataaaa acactgtaag tccctctttt aataaactaa atgaaagaac 3420
   atcctatact tcgctgtttg taaattagta tggcattcgc tttggtttaa gtggtatttt 3480
   attgcaaacc cattaaaaga ataactcatg aaaagaagct ctttgacacc ttggggtaca 3540
   caaatgttgg tgtgggtgtg tgttaattct gtgagtgaga cacaccagtt ctaaaaaaaa 3600
   tgagtgaagt tctggtgcct gagttaccat gctttcttct agttcttaca gtagcataaa 3660
   attaaagatt caaagtgaga tggaggataa aattactttt taatacatgt tctcaaacat 3720
   ttgaaaataa aagtatatga tagaaggggg ccagagtgtg gccaccatcc tgatcgtact 3780
   gtttttcaat aaagaaaact ttttcattgg tagatttggt gaaattctaa atttaggttt 3840
   ttttctagag ctgtatcaac caaaacttct ggcaattccc agtatcactt cttagccttc 3900
   ttatatccaa atgcctgttt attacctttc ttaatttgaa tcaatgccta gttattacag 3960
   attgcacccc acaatggcca aaaacccact acataataaa atttacaggt actaactagt 4020
   taagattata ttttaagtag caattgatat aaaattacaa cacaatgaaa gaacttgggt 4080
   aatctcttag caatggaaat aggttttaac cagcagtttt tctgggtgct ttgtaactat 4140
   cattttacta atgaattgag gatgtattat ggtttaaatt ggaagagttt tattcccaaa 4200
   gaataaagca agattatctt tcagtagtag agattgaagt aaatgtatta atattttaat 4260
   taatacagat ttactaagag tagttagaaa atttagtaag tgcctgtttt acaaattgtt 4320
   aggtactagt ttctgtataa ttcctacaca gaagctttag aaatctcctg atattaaatt 4380
   attaaattgg cattcatgaa aagagaagct acaattataa actccatttg ctaaatcatg 4440
   cataatactc tctctctctc ttcccccaca agtaatctct ctaccccatg cagtgtgcac 4500
   acacacacac acacagtcag ttactgaaaa aaataattct ttttcttttt ttttttaaat 4560
   ggagtttcac tcttgtcgcc caggctggag tgcagtggcg tgatctcggc ccactgcaac 4620
   ctccacctcc caggttcaag cagttctcct gcctcagcct cctgagtagc tgggattaca 4680
   ggtgtccgcc accatgcctg gctaattttt ttatttgata aaaagaattc tttttctcaa 4740
   taactgttct cttgaattca aattaaggga ctgccaaagt caattagaat attttaaaaa 4800
   tactttgttg taacctgtgt aaataatata caatttacag gatttgggat tgtagaactt 4860
   aaactggaag actggattcc tcagatctca ggactataac attccagata aatttttaca 4920
   ttccctttgc tgtatattaa ctgatgatca tttatatgtt aagatttttt accttaatat 4980
   ttctgaataa aactcttatt gcccatttaa tattttcata ggcaatcaaa tgtgagtaat 5040
   actgctaaga gtctgattta ttaaaaatat ttgtataatt cattcagttt agtttttcag 5100
   tttagtcttt ctgctttcac ttttctctgt gctaacaagt aactaatgtc tgggcattga 5160
   cttcttattg aatcaaagtt gggttaggca tagctatgca cacctgatgt gtaagattaa 5220
   agaagagatt aaataagaaa tcttgggtaa gttggacttt tctgtatagc tcttttttcc 5280
   tctgagttgt attttaatgt agtttataag tgataaaatg atccttgttt tctaaaagcc 5340
   agtccttccc ttcagctttc cacagtttct gtaaatgttt aatacttgta cagtcaatgg 5400
   caattttaaa tatatatata tatataatat atgtatatgg aaaaggttca aagatgcttt 5460
   taatttattt aatgactatt gccttcctat aataataatt ttcatcctta attatgataa 5520
   tacttttagc aagaaaaatt cctttttact acagttttta gatgcaaaat gcagtttggt 5580
   tctttagtca aatccactta gagggtatat tgcagtgaaa ctgtgaagga tacttcacta 5640
   ccaatgtata agctttgttg aatttgtatc attttctttc agtaatgaaa agctattcat 5700
   tatacagtat ggaaataaaa attgcttcat tga 5733
<210> 58
   <211> 478
   <212> PRT
   <213> Homo sapiens
<400> 58
   Met Ala His Val Pro Ala Arg Thr Ser Pro Gly Pro Gly Pro Gln Leu
   1               5                   10                  15
   Leu Leu Leu Leu Leu Pro Leu Phe Leu Leu Leu Leu Arg Asp Val Ala
               20                  25                  30
   Gly Ser His Arg Ala Pro Ala Trp Ser Ala Leu Pro Ala Ala Ala Asp
           35                  40                  45
   Gly Leu Gln Gly Asp Arg Asp Leu Gln Arg His Pro Gly Asp Ala Ala
       50                  55                  60
   Ala Thr Leu Gly Pro Ser Ala Gln Asp Met Val Ala Val His Met His
   65                  70                  75                  80
   Arg Leu Tyr Glu Lys Tyr Ser Arg Gln Gly Ala Arg Pro Gly Gly Gly
                   85                  90                  95
   Asn Thr Val Arg Ser Phe Arg Ala Arg Leu Glu Val Val Asp Gln Lys
               100                 105                 110
   Ala Val Tyr Phe Phe Asn Leu Thr Ser Met Gln Asp Ser Glu Met Ile
           115                 120                 125
   Leu Thr Ala Thr Phe His Phe Tyr Ser Glu Pro Pro Arg Trp Pro Arg
       130                 135                 140
   Ala Leu Glu Val Leu Cys Lys Pro Arg Ala Lys Asn Ala Ser Gly Arg
   145                 150                 155                 160
   Pro Leu Pro Leu Gly Pro Pro Thr Arg Gln His Leu Leu Phe Arg Ser
                   165                 170                 175
   Leu Ser Gln Asn Thr Ala Thr Gln Gly Leu Leu Arg Gly Ala Met Ala
               180                 185                 190
   Leu Ala Pro Pro Pro Arg Gly Leu Trp Gln Ala Lys Asp Ile Ser Pro
           195                 200                 205
   Ile Val Lys Ala Ala Arg Arg Asp Gly Glu Leu Leu Leu Ser Ala Gln
       210                 215                 220
   Leu Asp Ser Glu Glu Arg Asp Pro Gly Val Pro Arg Pro Ser Pro Tyr
   225                 230                 235                 240
   Ala Pro Tyr Ile Leu Val Tyr Ala Asn Asp Leu Ala Ile Ser Glu Pro
                   245                 250                 255
   Asn Ser Val Ala Val Thr Leu Gln Arg Tyr Asp Pro Phe Pro Ala Gly
               260                 265                 270
   Asp Pro Glu Pro Arg Ala Ala Pro Asn Asn Ser Ala Asp Pro Arg Val
           275                 280                 285
   Arg Arg Ala Ala Gln Ala Thr Gly Pro Leu Gln Asp Asn Glu Leu Pro
       290                 295                 300
   Gly Leu Asp Glu Arg Pro Pro Arg Ala His Ala Gln His Phe His Lys
   305                 310                 315                 320
   His Gln Leu Trp Pro Ser Pro Phe Arg Ala Leu Lys Pro Arg Pro Gly
                   325                 330                 335
   Arg Lys Asp Arg Arg Lys Lys Gly Gln Glu Val Phe Met Ala Ala Ser
               340                 345                 350
   Gln Val Leu Asp Phe Asp Glu Lys Thr Met Gln Lys Ala Arg Arg Lys
           355                 360                 365
   Gln Trp Asp Glu Pro Arg Val Cys Ser Arg Arg Tyr Leu Lys Val Asp
       370                 375                 380
   Phe Ala Asp Ile Gly Trp Asn Glu Trp Ile Ile Ser Pro Lys Ser Phe
   385                 390                 395                 400
   Asp Ala Tyr Tyr Cys Ala Gly Ala Cys Glu Phe Pro Met Pro Lys Ile
                   405                 410                 415
   Val Arg Pro Ser Asn His Ala Thr Ile Gln Ser Ile Val Arg Ala Val
               420                 425                 430
   Gly Ile Ile Pro Gly Ile Pro Glu Pro Cys Cys Val Pro Asp Lys Met
           435                 440                 445
   Asn Ser Leu Gly Val Leu Phe Leu Asp Glu Asn Arg Asn Val Val Leu
       450                 455                 460
   Lys Val Tyr Pro Asn Met Ser Val Asp Thr Cys Ala Cys Arg
   465                 470                 475
<210> 59
   <211> 2658
   <212> DNA
   <213> Homo sapiens
<400> 59
   gggccaggga cgaccctgtc agctgcagcc ccagaggtcc ggggcgcgca gccgggtccc      60
   ctcgagggcg cagccggccg ccccgccccg cccctcgaag cagccgggcc gggcgcgcag     120
   tgggctacaa actttcgcgg cgcgagtccg ccaaggcagc gcgccgactc gggctcggct     180
   cggctctgcg ctgctccgga cggctgtgac cgctggccgg gggctcgggc cgccggtacc     240
   cacggaccgc gcgcccgggt gcctgctccg ctaagcccct cgccccgcgc ggacctcggt     300
   atccagcgcc ctgctgcccg ggctctcccc gcgcgcccta ctgccgcgag gtcagtccgc     360
   agcctccggt gcgccagcgc tcgccttcct cctcctggac ttcggccctt tgccgccctc     420
   accacgccat ggctcatgtc cccgctcgga ccagcccggg acccgggccc cagctgctgc 480
   tgctgctgct gccgttgttt ctgctgttgc tccgggatgt ggccggcagc cacagggccc 540
   ccgcctggtc cgcactgccc gcggccgccg acggcctgca gggggacagg gatctccagc 600
   ggcaccctgg ggacgcggcc gccacgttgg gccccagcgc ccaggacatg gtcgctgtcc 660
   acatgcacag gctctatgag aagtacagcc ggcagggcgc gcggccggga gggggcaaca 720
   cggtccgcag cttcagggcc aggctggaag tggtcgacca gaaggccgtg tatttcttca 780
   acctgacttc catgcaagac tcggaaatga tccttacggc cactttccac ttctactcag 840
   agccgcctcg gtggcctcga gcgctcgagg tgctatgcaa gccgcgggcc aagaacgctt 900
   caggccgccc gctgcccctg ggcccgccca cacgccagca cctgctcttc cgcagcctct 960
   cgcagaacac ggccacacag gggctactcc gcggggccat ggccctggcg cccccaccgc 1020
   gcggcctgtg gcaggccaag gacatctccc ccatcgtcaa ggcggcccgc cgggatggcg 1080
   agctgctcct ctccgcccag ctggattctg aggagaggga cccgggggtg ccccggccca 1140
   gcccctatgc gccctacatc ctagtctatg ccaacgatct ggccatctcg gagcccaaca 1200
   gcgtggcagt gacgctgcag agatacgacc ccttccctgc cggagacccc gagccccgcg 1260
   cagcccccaa caactcagcg gacccccgcg tgcgccgagc cgcgcaggcc actgggcccc 1320
   tccaggacaa cgagctgccg gggctggatg agaggccgcc gcgcgcccac gcacagcact 1380
   tccacaagca ccagctgtgg cccagcccct tccgggcgct gaaaccccgg ccagggcgca 1440
   aagaccgcag gaagaagggc caggaggtgt tcatggccgc ctcgcaggtg ctggactttg 1500
   acgagaagac gatgcagaaa gcccggagga agcagtggga tgagccgagg gtgtgctccc 1560
   ggaggtacct gaaggtggac ttcgcagaca tcggctggaa tgaatggata atctcaccga 1620
   aatcttttga tgcctactac tgcgcgggag catgtgagtt ccccatgcct aagatcgttc 1680
   gtccatccaa ccatgccacc atccagagca ttgtcagggc tgtgggcatc atccctggca 1740
   tcccagagcc ctgctgtgtt cccgataaga tgaactccct tggggtcctc ttcctggatg 1800
   agaatcggaa tgtggttctg aaggtgtacc ccaacatgtc cgtggacacc tgtgcctgcc 1860
   ggtgagacca ctccagggtg gaaagaagcc acgcccagca gagctgcctt ctcggagcct 1920
   tctgcaacca ggacttgtgg tgcagctgca gacacagagc acagctcatg ggcaacatca 1980
   ctggggccca gagagagctg tccgccagtg catcattagg gggtctttca ttgctagtga 2040
   ctagcccctt aaatgccagc ctgagtacct gaaggaatct gggaattagc cctggcctga 2100
   aagtggccca tcattcatac ccactgttct gaaggcttga aaacaaaaca tatccacaac 2160
   attggcttga tgtgatcatc atctcataac tgagcaagaa gactatgcaa atcttagggc 2220
   gctcgctccc tgcacacgga aagaactctg tttaaatgct cagttcagaa cactttgggc 2280
   cacatagtga ttttggaaaa caggataatc gtggtgtaaa tgagtgtttc ctttcaaagt     2340
   ccactgcaga gcttttatcc atatggtatg cacatgtagc caatattggt ttctttttct     2400
   taatatatat attttatttt aaaacaacaa aaagggaggg cgttgacacc attccccaca     2460
   gagatagtca tgctgagtgt gggttgttta aacatgcata ttgaaataac acatatagta     2520
   acgtgggaat actaaaaaat aaccaagatt ttatattttt gtaaattata ctttctatac     2580
   tgtagattgt gtatgttatg tgtttttatg gaaagctaat aaattaaagg tacagtggta     2640
   tcttgaaaaa aaaaaaaa                                                   2658
<210> 60
   <211> 429
   <212> PRT
   <213> Homo sapiens
<400> 60
<210> 61
   <211> 1955
   <212> DNA
   <213> Homo sapiens
<400> 61
   aagcacaagt ggaggacaat ccagcccggc agcgggtgag agtgggtgct ggccaggacg 60
   gttccttcag agcaaacagc agggagatgc cggcccgctc cttcccagct cctccccgtg 120
   cccgctaaca cagcacggcc gcctgcagtc tcctctctgg gtgattgcgc gggcctaaga 180
   tgtgtcctgg ggcactgtgg gtggccctgc ccctgctgtc cctgctggct ggctccctac 240
   aggggaagcc actgcagagc tggggacgag ggtctgctgg gggaaacgcc cacagcccac 300
   tgggggtgcc tggaggtggg ctgcctgagc acaccttcaa cctgaagatg tttctggaga 360
   acgtgaaggt ggatttcctg cgcagcctta acctgagtgg ggtcccttcg caggacaaaa 420
   ccagggtgga gccgccgcag tacatgattg acctgtacaa caggtacacg tccgataagt 480
   cgactacgcc agcgtccaac attgtgcgga gcttcagcat ggaagatgcc atctccataa 540
   ctgccacaga ggacttcccc ttccagaagc acatcttgct cttcaacatc tccattccta 600
   ggcatgagca gatcaccaga gctgagctcc gactctatgt ctcctgtcaa aatcacgtgg 660
   acccctctca tgacctgaaa ggaagcgtgg tcatttatga tgttctggat ggaacagatg 720
   cctgggatag tgctacagag accaagacct tcctggtgtc ccaggacatt caggatgagg 780
   gctgggagac cttggaagtg tccagcgccg tgaagcgctg ggtccggtcc gactccacca 840
   agagcaaaaa taagctggaa gtgactgtgg agagccacag gaagggctgc gacacgctgg 900
   acatcagtgt ccccccaggt tccagaaacc tgcccttctt tgttgtcttc tccaatgacc 960
   acagcagtgg gaccaaggag accaggctgg agctgaggga gatgatcagc catgaacaag 1020
   agagcgtgct caagaagctg tccaaggacg gctccacaga ggcaggtgag agcagtcacg 1080
   aggaggacac ggatggccac gtggctgcgg ggtcgacttt agccaggcgg aaaaggagcg 1140
   ccggggctgg cagccactgt caaaagacct ccctgcgggt aaacttcgag gacatcggct 1200
   gggacagctg gatcattgca cccaaggagt atgaagccta cgagtgtaag ggcggctgct 1260
   tcttcccctt ggctgacgat gtgacgccga cgaaacacgc tatcgtgcag accctggtgc 1320
   atctcaagtt ccccacaaag gtgggcaagg cctgctgtgt gcccaccaaa ctgagcccca 1380
   tctccgtcct ctacaaggat gacatggggg tgcccaccct caagtaccat tacgagggca 1440
   tgagcgtggc agagtgtggg tgcaggtagt atctgcctgc ggggctgggg aggcaggcca 1500
   aaggggctcc acatgagagg tcctgcatgc ccctgggcac aacaaggact gattcaatct 1560
   gcatgccagc ctggaggagg aaagggagcc tgctctccct ccccacaccc cacccaaagc 1620
   atacaccgct gagctcaact gccagggaag gctaaggaaa tggggatttg agcacaacag 1680
   gaaagcctgg gagggttgtt gggatgcaag gaggtgatga aaaggagaca gggggaaaaa 1740
   taatccatag tcagcagaaa acaacagcag tgagccagag gagcacaggc gggcaggtca 1800
   ctgcagagac tgatggaagt tagagaggtg gaggaggcca gctcgctcca aaacccttgg 1860
   ggagtagagg gaaggagcag gccgcgtgtc acacccatca ttgtatgtta tttcccacaa 1920
   cccagttgga ggggcatggc ttccaattta gagac 1955
<210> 62
   <211> 424
   <212> PRT
   <213> Homo sapiens
<400> 62
   Met Gly Ser Leu Val Leu Thr Leu Cys Ala Leu Phe Cys Leu Ala Ala
   1               5                   10                  15
   Tyr Leu Val Ser Gly Ser Pro Ile Met Asn Leu Glu Gln Ser Pro Leu
               20                  25                  30
   Glu Glu Asp Met Ser Leu Phe Gly Asp Val Phe Ser Glu Gln Asp Gly
           35                  40                  45
   Val Asp Phe Asn Thr Leu Leu Gln Ser Met Lys Asp Glu Phe Leu Lys
       50                  55                  60
   Thr Leu Asn Leu Ser Asp Ile Pro Thr Gln Asp Ser Ala Lys Val Asp
   65                  70                  75                  80
   Pro Pro Glu Tyr Met Leu Glu Leu Tyr Asn Lys Phe Ala Thr Asp Arg
                   85                  90                  95
   Thr Ser Met Pro Ser Ala Asn Ile Ile Arg Ser Phe Lys Asn Glu Asp
               100                 105                 110
   Leu Phe Ser Gln Pro Val Ser Phe Asn Gly Leu Arg Lys Tyr Pro Leu
           115                 120                 125
   Leu Phe Asn Val Ser Ile Pro His His Glu Glu Val Ile Met Ala Glu
       130                 135                 140
   Leu Arg Leu Tyr Thr Leu Val Gln Arg Asp Arg Met Ile Tyr Asp Gly
   145                 150                 155                 160
   Val Asp Arg Lys Ile Thr Ile Phe Glu Val Leu Glu Ser Lys Gly Asp
                   165                 170                 175
   Asn Glu Gly Glu Arg Asn Met Leu Val Leu Val Ser Gly Glu Ile Tyr
               180                 185                 190
   Gly Thr Asn Ser Glu Trp Glu Thr Phe Asp Val Thr Asp Ala Ile Arg
           195                 200                 205
   Arg Trp Gln Lys Ser Gly Ser Ser Thr His Gln Leu Glu Val His Ile
       210                 215                 220
   Glu Ser Lys His Asp Glu Ala Glu Asp Ala Ser Ser Gly Arg Leu Glu
   225                 230                 235                 240
   Ile Asp Thr Ser Ala Gln Asn Lys His Asn Pro Leu Leu Ile Val Phe
                   245                 250                 255
   Ser Asp Asp Gln Ser Ser Asp Lys Glu Arg Lys Glu Glu Leu Asn Glu
               260                 265                 270
   Met Ile Ser His Glu Gln Leu Pro Glu Leu Asp Asn Leu Gly Leu Asp
           275                 280                 285
   Ser Phe Ser Ser Gly Pro Gly Glu Glu Ala Leu Leu Gln Met Arg Ser
       290                 295                 300
   Asn Ile Ile Tyr Asp Ser Thr Ala Arg Ile Arg Arg Asn Ala Lys Gly
   305                 310                 315                 320
   Asn Tyr Cys Lys Arg Thr Pro Leu Tyr Ile Asp Phe Lys Glu Ile Gly
                   325                 330                 335
   Trp Asp Ser Trp Ile Ile Ala Pro Pro Gly Tyr Glu Ala Tyr Glu Cys
               340                 345                 350
   Arg Gly Val Cys Asn Tyr Pro Leu Ala Glu His Leu Thr Pro Thr Lys
           355                 360                 365
   His Ala Ile Ile Gln Ala Leu Val His Leu Lys Asn Ser Gln Lys Ala
       370                 375                 380
   Ser Lys Ala Cys Cys Val Pro Thr Lys Leu Glu Pro Ile Ser Ile Leu
   385                 390                 395                 400
   Tyr Leu Asp Lys Gly Val Val Thr Tyr Lys Phe Lys Tyr Glu Gly Met
                   405                 410                 415
   Ala Val Ser Glu Cys Gly Cys Arg
               420
<210> 63
   <211> 1584
   <212> DNA
   <213> Homo sapiens
<400> 63
   ggggagagga agagtggtag ggggagggag agagagagga agagtttcca aacttgtctc      60
   cagtgacagg agacatttac gttccacaag ataaaactgc cacttagagc ccagggaagc     120
   taaaccttcc tggcttggcc taggagctcg agcggagtca tgggctctct ggtcctgaca     180
   ctgtgcgctc ttttctgcct ggcagcttac ttggtttctg gcagccccat catgaaccta     240
   gagcagtctc ctctggaaga agatatgtcc ctctttggtg atgttttctc agagcaagac     300
   ggtgtcgact ttaacacact gctccagagc atgaaggatg agtttcttaa gacactaaac     360
   ctctctgaca tccccacgca ggattcagcc aaggtggacc caccagagta catgttggaa     420
   ctctacaaca aatttgcaac agatcggacc tccatgccct ctgccaacat cattaggagt     480
   ttcaagaatg aagatctgtt ttcccagccg gtcagtttta atgggctccg aaaatacccc     540
   ctcctcttca atgtgtccat tcctcaccat gaagaggtca tcatggctga acttaggcta     600
   tacacactgg tgcaaaggga tcgtatgata tacgatggag tagaccggaa aattaccatt     660
   tttgaagtgc tggagagcaa aggggataat gagggagaaa gaaacatgct ggtcttggtg     720
   tctggggaga tatatggaac caacagtgag tgggagactt ttgatgtcac agatgccatc     780
   agacgttggc aaaagtcagg ctcatccacc caccagctgg aggtccacat tgagagcaaa     840
   cacgatgaag ctgaggatgc cagcagtgga cggctagaaa tagataccag tgcccagaat     900
   aagcataacc ctttgctcat cgtgttttct gatgaccaaa gcagtgacaa ggagaggaag     960
   gaggaactga atgaaatgat ttcccatgag caacttccag agctggacaa cttgggcctg    1020
   gatagctttt ccagtggacc tggggaagag gctttgttgc agatgagatc aaacatcatc    1080
   tatgactcca ctgcccgaat cagaaggaac gccaaaggaa actactgtaa gaggaccccg    1140
   ctctacatcg acttcaagga gattgggtgg gactcctgga tcatcgctcc gcctggatac    1200
   gaagcctatg aatgccgtgg tgtttgtaac taccccctgg cagagcatct cacacccaca    1260
   aagcatgcaa ttatccaggc cttggtccac ctcaagaatt cccagaaagc ttccaaagcc    1320
   tgctgtgtgc ccacaaagct agagcccatc tccatcctct atttagacaa aggcgtcgtc    1380
   acctacaagt ttaaatacga aggcatggcc gtctccgaat gtggctgtag atagaagaag    1440
   agtcctatgg cttatttaat aactgtaaat gtgtatattt ggtgttccta tttaatgaga    1500
   ttatttaata agggtgtaca gtaatagagg cttgctgcct tcaggaaatg gacaggtcag    1560
   tttgttgtag gaaatgcata tttt                                           1584
<210> 64
   <211> 6
   <212> PRT
   <213> Artificial Sequence
<220>
   <223> Description of Artificial Sequence: Synthetic 6xHis tag
<400> 64
   His His His His His His
   1               5